# EUROPEAN PATENT SPECIFICATION

(11) **EP 3 606 913 B1**
(45) Date of publication and mention of the grant of the patent: **27.04.2022**
(21) Application number: 18717271.3
(22) Date of filing: 29.03.2018
(51) Int. Cl.: C07D 271/06, A01N 43/836

(54) **MICROBIOCIDAL OXADIAZOLE DERIVATIVES**
MIKROBIOZIDE OXADIAZOLDERIVATE
DÉRIVÉS D'OXADIAZOLE MICROBIOCIDES

(30) Priority: 03.04.2017 IN 201711012036
(43) Date of publication of application: 12.02.2020
(73) Proprietor: Syngenta Participations AG, 4058 Basel (CH)
(72) Inventor: HOFFMAN, Thomas, James, 4332 Stein (CH); STIERLI, Daniel, 4332 Stein (CH); BEAUDEGNIES, Renaud, 4332 Stein (CH); PITTERNA, Thomas, 4332 Stein (CH); RAJAN, Ramya, Goa 403 110 (IN)
(74) Representative: SYNGENTA IP
(86) International application number: PCT/EP2018/058208
(87) International publication number: WO 2018/185013

(56) References cited:
- WO-A1-2015/185485
- WO-A1-2017/018803
- WO-A1-2017/178245
- WO-A1-2017/213252

## Description

The present invention relates to microbiocidal oxadiazole derivatives, e.g., as active ingredients, which have microbiocidal activity, in particular, fungicidal activity. The invention also relates to agrochemical compositions which comprise at least one of the oxadiazole derivatives, to processes of preparation of these compounds and to uses of the oxadiazole derivatives or compositions in agriculture or horticulture for controlling or preventing infestation of plants, harvested food crops, seeds or non-living materials by phytopathogenic microorganisms, preferably fungi.

Certain oxadiazole derivatives are known as insecticidal and acaricidal agents, e.g., from CN 1927860. WO 2013/064079, EP 0 276 432 and WO 2015/185485 describe the use of substituted oxadiazoles for combating phytopathogenic fungi.

According to the present invention, there is provided a compound of Formula (I): 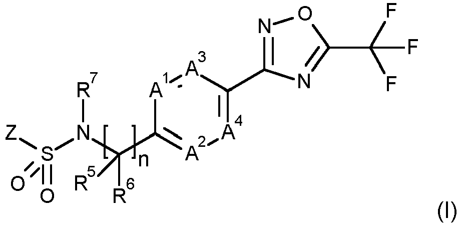 wherein
n is 0, 1, or 2;
A¹ represents N or CR¹, wherein R¹ represents hydrogen, halogen, or methyl;
A² represents N or CR², wherein R² represents hydrogen, halogen, or methyl;
A³ represents N or CR³, wherein R³ represents hydrogen or fluoro;
A⁴ represents N or CR⁴, wherein R⁴ represents hydrogen or fluoro; and
wherein at least 2 of A¹, A², A³ and A⁴ are C-H;
R⁵ and R⁶ independently represent hydrogen, methyl, cyano, difluoromethyl, or trifluoromethyl;
R⁷ represents hydroxy, C₁₋₄haloalkyl, C₁₋₄alkoxy, hydroxyC₂₋₄alkyl, C₁₋₂alkoxyC₂₋₄alkyl, C₁₋₂haloalkoxy, C₁₋₂haloalkoxyC₂₋₄alkyl, C₃₋₄alkenyloxy, C₃₋₄alkynyloxy or C₁₋₄alkylcarbonyloxy;
Z represents R⁸, wherein
R⁸ is C₁₋₆alkyl, C₂₋₆alkenyl, C₂₋₆alkynyl, cyanoC₁₋₄alkyl, C₁₋₄haloalkyl, C₁₋₄alkoxyC₂₋₄alkyl, C₁₋₂haloalkoxyC₂₋₄alkyl, C₁₋₄alkylcarbonylC₁₋₄alkyl, C₁₋₄alkoxycarbonylC₁₋₄alkyl, C₁₋₄alkylcarbonyloxyC₂₋₄alkyl, N-C₁₋₄alkylaminocarbonylC₁₋₄alkyl, N,N-diC₁₋₄alkylaminocarbonylC₁₋₄alkyl, N-C₁₋₄alkylaminoC₂₋₅alkyl, N,N-diC₁₋₄alkylaminoC₂₋₅alkyl, C₁₋₃alkylcarbonylaminoC₂₋₅alkyl, or C₁₋₄alkoxycarbonylaminoC₂₋₅alkyl; or
R⁸ is C₃₋₆cycloalkyl, C₃₋₆cycloalkylC₁₋₂alkyl, phenyl, phenylC₁₋₂alkyl, heteroaryl, heteroarylC₁₋₂alkyl, wherein the heteroaryl moiety is a 5- or 6-membered monocyclic aromatic ring which comprises 1, 2, 3 or 4 heteroatoms individually selected from N, O and S, heterocyclyl or heterocyclylC₁₋₂alkyl, wherein the heterocyclyl moiety is a 4- to 6-membered non-aromatic ring which comprises 1 or 2 heteroatoms individually selected from N, O and S;
wherein when R⁸ is C₃₋₆cycloalkyl, C₃₋₆cycloalkylC₁₋₂alkyl, phenyl, phenylC₁₋₂alkyl, heteroaryl, heteroarylC₁₋₂alkyl, heterocyclyl, or heterocyclylC₁₋₂alkyl, the C₃₋₆cycloalkyl, phenyl, heteroaryl, or heterocyclyl motif is optionally substituted by 1 or 2 substituents, which may be the same or different, selected from R⁹;
R⁹ represents cyano, fluoro, chloro, bromo, methyl, difluoromethyl, trifluoromethyl, methoxy, ethoxy, or difluoromethoxy; and wherein when R⁸ is substituted C₃₋₆cycloalkyl or heterocyclyl, these cycles may contain a carbonyl (C=O) or sulfonyl (S(O)₂) group; or
Z represents -N(R¹⁰)R¹¹, wherein
R¹⁰ represents hydrogen, C₁₋₆alkyl, C₁₋₆haloalkyl, C₁₋₄cyanoalkyl, hydroxyC₂₋₄alkyl, C₁₋₂alkoxyC₂₋₄alkyl, C₁₋₂haloalkoxyC₁₋₄alkyl, C₃₋₆alkenyl, C₃₋₆alkynyl, aminoC₂₋₄alkyl, N-C₁₋₄alkylaminoC₂₋₄alkyl, N,N-diC₁₋₄alkylaminoC₂₋₄alkyl, formyl, C₁₋₄alkylcarbonyl, C₁₋₄alkylcarbonylC₁₋₄alkyl, C₁₋₄alkoxycarbonylC₁₋₄alkyl, C₁₋₄alkylcarbonyloxyC₁₋₄alkyl, N-C₁₋₄alkylaminocarbonylC₁₋₄alkyl, N,N-diC₁₋₄alkylaminocarbonylC₁₋₄alkyl, C₁₋₄alkoxycarbonylaminoC₂₋₄alkyl, C₁₋₄alkylcarbonylaminoC₂₋₄alkyl, or C₁₋₄haloalkylcarbonylaminoC₂₋₄alkyl; or
R¹⁰ is C₃₋₆cycloalkyl, C₃₋₆cycloalkylC₁₋₂alkyl, phenyl, phenylC₁₋₂alkyl, heteroaryl, heteroarylC₁₋₂alkyl, wherein the heteroaryl moiety is a 5- or 6-membered monocyclic aromatic ring which comprises 1, 2, 3 or 4 heteroatoms individually selected from N, O and S, heterocyclyl or heterocyclylC₁₋₂alkyl, wherein the heterocyclyl moiety is a 4- to 6-membered non-aromatic ring which comprises 1 or 2 heteroatoms individually selected from N, O and S;
wherein when R¹⁰ is C₃₋₆cycloalkyl, C₃₋₆cycloalkylC₁₋₂alkyl, phenylC₁₋₂alkyl, phenyl, heteroaryl, heteroarylC₁₋₂alkyl, heterocyclyl, or heterocyclylC₁₋₂alkyl, the C₃₋₆cycloalkyl, phenyl, heteroaryl, or heterocyclyl motif is optionally substituted by 1 or 2 substituents, which may be the same or different, selected from R¹²;
R¹² represents cyano, fluoro, chloro, bromo, methyl, difluoromethyl, trifluoromethyl, methoxy, ethoxy, or difluoromethoxy; and wherein when R¹⁰ is substituted C₃₋₆cycloalkyl or heterocyclyl, these cycles may contain a carbonyl (C=O) or sulfonyl (S(O)₂) group;
R¹¹ represents hydrogen, hydroxyl, C₁₋₄alkyl, C₁₋₂haloalkyl, C₃₋₄alkenyl, C₃₋₄alkynyl, C₃₋₄cycloalkyl, C₃₋₄cycloalkylC₁₋₂alkyl, C₁₋₄alkoxy, C₃₋₄alkenyloxy, or C₃₋₄alkynyloxy; or
a salt or an N-oxide thereof.

Surprisingly, it has been found that the novel compounds of Formula (I) have, for practical purposes, a very advantageous level of biological activity for protecting plants against diseases that are caused by fungi.

According to a second aspect of the invention, there is provided an agrochemical composition comprising a fungicidally effective amount of a compound of Formula (I). Such an agricultural composition may further comprise at least one additional active ingredient and/or an agrochemically-acceptable diluent or carrier.

According to a third aspect of the invention, there is provided a method of controlling or preventing infestation of useful plants by phytopathogenic microorganisms, wherein a fungicidally effective amount of a compound of Formula (I), or a composition comprising this compound as active ingredient, is applied to the plants, to parts thereof or the locus thereof.

According to a fourth aspect of the invention, there is provided the use of a compound of Formula (I) as a fungicide, with the proviso that the use excludes methods for the treatment of the human or animal body by surgery or therapy.

As used herein, the term "halogen" or "halo" refers to fluorine (fluoro), chlorine (chloro), bromine (bromo) or iodine (iodo), preferably fluorine, chlorine or bromine.

As used herein, cyano means a -CN group.

As used herein, the term "hydroxyl" or "hydroxy" means an -OH group.

As used herein, amino means an -NH₂ group.

As used herein, acyl means a -C(O)CH₃ group.

As used herein, formyl means a -C(O)H group.

As used herein, the term "C₁₋₆alkyl" refers to a straight or branched hydrocarbon chain radical consisting solely of carbon and hydrogen atoms, containing no unsaturation, having from one to six carbon atoms, and which is attached to the rest of the molecule by a single bond. C₁₋₄alkyl, C₁₋₃alkyl and C₁₋₂alkyl are to be construed accordingly. Examples of C₁₋₆alkyl include, but are not limited to, methyl, ethyl, *n*-propyl, 1-methylethyl (isopropyl), *n*-butyl, and 1-dimethylethyl (*t*-butyl). A "C₁₋₂alkylene" group refers to the corresponding definition of C₁₋₂alkyl, except that such radical is attached to the rest of the molecule by two single bonds. Examples of C₁₋₂alkylene, are -CH₂- and -CH₂CH₂-.

As used herein, the term "C₂₋₆alkenyl" refers to a straight or branched hydrocarbon chain radical group consisting solely of carbon and hydrogen atoms, containing at least one double bond that can be of either the (*E*)- or (*Z*)-configuration, having from two to six carbon atoms, which is attached to the rest of the molecule by a single bond. C₃₋₆alkenyl, C₃₋₄alkenyl, and C₂₋₄alkenyl are to be construed accordingly. Examples of C₂₋₆alkenyl include, but are not limited to, ethenyl, prop-1-enyl and but-1-enyl.

As used herein, the term "C₂₋₆alkynyl" refers to a straight or branched hydrocarbon chain radical group consisting solely of carbon and hydrogen atoms, containing at least one triple bond, having from two to six carbon atoms, and which is attached to the rest of the molecule by a single bond. C₃₋₆alkynyl, C₃₋₄alkynyl, and C₂₋₄alkynyl, are to be construed accordingly. Examples of C₂₋₆alkynyl include, but are not limited to, ethynyl, prop-1-ynyl and but-1-ynyl.

As used herein, oxo means an =O group, e.g., as in a ketonyl (-C(O)-), sulfinyl (-S(O)-) or sulfonyl (-S(O)₂-) oxygen.

As used herein, the term "C₁₋₄alkoxy" refers to a radical of the formula -ORₓ where Rₓ is a C₁₋₄alkyl radical as generally defined above. The terms C₁₋₃alkoxy and C₁₋₂alkoxy are to be construed accordingly. Examples of C₁₋₄alkoxy include, but are not limited to, methoxy, ethoxy, propoxy, isopropoxy, and *t*-butoxy.

As used herein, the term "C₁₋₂haloalkoxy" refers to a C₁₋₂alkoxy group as defined above substituted by one or more of the same or different halogen atoms. Examples of C₁₋₂haloalkoxy include, but are not limited to, fluoromethoxy, fluoroethoxy, 1,1,1-trifluoromethoxy, and 2,2,2-trifluoroethoxy.

As used herein, the term "C₁₋₆haloalkyl" refers to a C₁₋₆alkyl radical as generally defined above substituted by one or more of the same or different halogen atoms. C₁₋₄haloalkyl, C₁₋₃haloalkyl and C₁₋₂haloalkyl are to be construed accordingly. Examples of C₁₋₃haloalkyl include, but are not limited to fluoromethyl, fluoroethyl, difluoromethyl, trifluoromethyl, 2,2,2-trifluoroethyl, and 3,3,3-trifluoropropyl.

As used herein, the term "C₁₋₂haloalkoxyC₁₋₄alkyl" refers to a C₁₋₄alkyl radical as generally defined above substituted by a C₁₋₂haloalkoxy group as defined above. C₁₋₂haloalkoxyC₁₋₄alkyl and C₁₋₂haloalkoxyC₁₋₂alkyl are to be construed accordingly.

As used herein, the term "hydroxyC₂₋₄alkyl" refers to a C₂₋₄alkyl radical as generally defined above substituted by one or more hydroxy groups. The term "hydroxyC₂₋₃alkyl" should be construed accordingly.

As used herein, the term "cyanoC₁₋₄alkyl" refers to refers to a C₁₋₄alkyl radical as generally defined above substituted by one or more cyano groups. CyanoC₁₋₂alkyl should be construed accordingly.

As used herein, the term "C₁₋₄alkylcarbonyl" refers to a radical of the formula -C(O)Rₓ where Rₓ is a C₁₋₄alkyl radical as generally defined above. C₁₋₂alkylcarbonyl should be construed accordingly.

As used herein, the term "C₃₋₄alkenoxy" refers to a radical of the formula -ORₓ where Rₓ is a C₃₋₄alkenyl radical as generally defined above.

As used herein, the term "C₃₋₄alkynoxy" refers to a radical of the formula -ORₓ where Rₓ is a C₃₋₄alkynyl radical as generally defined above.

As used herein, the term "C₁₋₄alkoxyC₂₋₄alkyl" refers to radical of the formula R_{y}-O-Rₓ- where R_{y} is a C₁₋₄alkyl radical as generally defined above, and Rₓ is a C₂₋₄alkylene radical as generally defined above. C₁₋₂alkoxyC₂₋₄alkyl and C₁₋₂alkoxyC₂₋₃alkyl should be construed accordingly.

As used herein, the term "C₁₋₄alkylcarbonyloxy" refers to a radical of the formula RₓC(O)O- where Rₓ is a C₁₋₄alkyl radical as generally defined above.

As used herein, the term "C₁₋₄alkylcarbonyloxyC₁₋₄alkyl" refers to a radical of the formula R_{y}C(O)ORₓ- where R_{y} is a C₁₋₄alkyl radical as generally defined above, and Rₓ is a C₁₋₄alkylene radical as generally defined above. C₁₋₄alkylcarbonyloxyC₂₋₄alkyl, C₁₋₃alkylcarbonyloxyC₂₋₄alkyl and C₁₋₂alkylcarbonyloxyC₁₋₂alkyl are to be construed accordingly.

As used herein, the term "C₁₋₄alkoxycarbonylC₁₋₄alkyl" refers to a radical of the formula R_{y}OC(O)Rx-, where R_{y} is a C₁₋₄alkyl radical as generally defined above, and Rₓ is a C₁₋₄alkylene radical as generally defined above. C₁₋₃alkoxycarbonylC₁₋₃alkyl and C₁₋₂alkoxycarbonylC₁₋₂alkyl are to be construed accordingly.

As used herein, the term "C₁₋₄alkylcarbonylC₁₋₄alkyl" refers to a radical of the formula R_{y}(O)Rₓ-where R_{y} is a C₁₋₄alkyl radical as generally defined above, and Rₓ is a C₁₋₄alkylene radical as generally defined above. C₁₋₃alkylcarbonylC₁₋₃alkyl and C₁₋₂alkylcarbonylC₁₋₂alkyl are to be construed accordingly.

As used herein, the term "aminoC₂₋₄alkyl" refers to a radical of the formula H₂NRₓ-, where Rₓ is a C₂₋₄alkylene radical as defined above.

As used herein, the term "N-C₁₋₄alkylaminoC₂₋₅alkyl" refers to a radical of the formula R_{y}NHRₓ-where R_{y} is a C₁₋₄alkyl radical as generally defined above, and Rₓ is a C₂₋₅alkylene radical as generally defined above.

As used herein, the term "N,N-diC₁₋₄alkylaminoC₂₋₅alkyl" refers to a radical of the formula (R_{y})R_{y}NHRₓ- where each R_{y} is independently a C₁₋₄alkyl radical as generally defined above, and Rₓ is a C₂₋₅alkylene radical as generally defined above.

As used herein, the term "N-C₁₋₄alkylaminocarbonylC₁₋₄alkyl" refers to a radical of the formula R_{y}NHC(O)Rₓ- where R_{y} is a C₁₋₄alkyl radical as generally defined above, and Rₓ is a C₁₋₄alkylene radical as generally defined above.

As used herein, the term "N,N-diC₁₋₄alkylaminocarbonylC₁₋₄alkyl" refers to a radical of the formula (R_{y})R_{y}NHC(O)Rₓ- where each R_{y} is independently a C₁₋₄alkyl radical as generally defined above, and Rₓ is a C₁₋₄alkylene radical as generally defined above.

As used herein, the term "C₁₋₄alkylcarbonylaminoC₁₋₄alkyl" refers to a radical of the formula R_{y}C(O)NHRₓ- where R_{y} is a C₁₋₄alkyl radical as generally defined above, and Rₓ is a C₁₋₄alkylene radical as generally defined above.

As used herein, the term "C₁₋₄haloalkylcarbonylaminoC₂₋₄alkyl" refers to a radical of the formula R_{y}C(O)NHRₓ- where R_{y} is a C₁₋₄alkyl radical as generally defined above substituted by one or more of the same or different halogen atoms, and Rₓ is a C₂₋₄alkylene radical as generally defined above.

As used herein, the term "C₁₋₄alkoxycarbonylaminoC₁₋₄alkyl" refers to a radical of the formula R_{y}O C(O)NHRₓ- where R_{y} is a C₁₋₄alkyl radical as generally defined above, and Rₓ is a C₁₋₄alkylene radical as generally defined above. C₁₋₄alkoxycarbonylaminoC₂₋₄alkyl is to be construed accordingly.

As used herein, the term "C₃₋₆cycloalkyl" may be mono- or bi-cyclic and contains 3 to 6 carbon atoms. C₃₋₅cycloalkyl and C₃₋₄cycloalkyl are to be construed accordingly. Examples of C₃₋₈cycloalkyl include, but are not limited to, cyclopropyl, 1-methylcyclopropyl, 2-methylcyclopropyl, 2,2-dichlorocyclopropyl, cyclobutyl, cyclopentyl, cyclohexyl, cyclopenten-1-yl, cyclopenten-3-yl, and cyclohexen-3-yl.

As used herein, the term "C₃₋₆cycloalkylC₁₋₂alkyl" refers to a C₃₋₆cycloalkyl ring as defined above attached to the rest of the molecule by a C₁₋₂alkylene radical as defined above. C₃₋₅cycloalkylC₁₋₂alkyl and C₃₋₄cycloalkylC₁₋₂alkyl are to be construed accordingly. Examples of C₃₋₆cycloalkylC₁₋₂alkyl include, but are not limited to cyclopropylmethyl, 2,2-chloropropylmethyl, cyclobutylethyl, and cyclopentylmethyl.

As used herein, the term "phenylC₁₋₂alkyl" refers to a phenyl ring attached to the rest of the molecule by a C₁₋₂alkylene radical as defined above. Examples of phenylC₁₋₂alkyl include, but are not limited to, benzyl.

As used herein, the term "heteroaryl" generally refers to a 5- or 6-membered monocyclic aromatic ring radical which comprises 1, 2, 3 or 4 heteroatoms individually selected from N, O and S. The heteroaryl radical is bonded to the rest of the molecule *via* a carbon atom or heteroatom. Examples of heteroaryl include but are not limited to, furanyl, pyrrolyl, thienyl, pyrazolyl, imidazolyl, thiazolyl, oxazolyl, isoxazolyl, triazolyl, tetrazolyl, pyrazinyl, pyridazinyl, pyrimidyl, pyridyl, and indolyl.

As used herein, the term "heteroarylC₁₋₂alkyl" generally refers to a 5- or 6-membered monocyclic aromatic ring radical which comprises 1, 2, 3 or 4 heteroatoms individually selected from N, O and S, which is attached to the rest of the molecule by a C₁₋₂alkylene radical as defined above. Examples of heteroarylC₁₋₂alkyl include but are not limited to, thienylmethyl, thienylethyl and pyridylmethyl.

As used herein, the term "heterocyclyl" or "heterocyclic" generally refers to a stable, saturated or partially saturated, 4- to 6-membered, non-aromatic monocyclic ring, which comprises 1 or 2 heteroatoms individually selected from N, O and S. The heterocyclyl radical may be bonded to the rest of the molecule *via* a carbon atom or heteroatom. Examples of heterocyclyl include, but are not limited to, azetidinyl, oxetanyl, pyrrolidyl, tetrahydrofuranyl, tetrahydrothienyl, tetrahydrothiopyranyl, piperidinyl, piperazinyl, tetrahydropyranyl, dioxolanyl, and morpholinyl.

As used herein, the term "heterocyclylC₁₋₂alkyl" generally refers to a stable, saturated or partially saturated, 4- to 6-membered, non-aromatic monocyclic ring, which comprises 1 or 2 heteroatoms individually selected from N, O and S, which is attached to the rest of the molecule by a C₁₋₂alkylene radical as defined above.

The presence of one or more possible asymmetric carbon atoms in a compound of Formula (I) means that the compounds may occur in chiral isomeric forms, i.e., enantiomeric or diastereomeric forms. Also, atropisomers may occur as a result of restricted rotation about a single bond. Formula (I) is intended to include all those possible isomeric forms and mixtures thereof. The present invention includes all those possible isomeric forms and mixtures thereof for a compound of Formula (I). Likewise, Formula (I) is intended to include all possible tautomers (including lactam-lactim tautomerism and keto-enol tautomerism) where present. The present invention includes all possible tautomeric forms for a compound of Formula (I).

In each case, the compounds of Formula (I) according to the invention are in free form, in oxidized form as an N-oxide, in covalently hydrated form, or in salt form, e.g., an agronomically usable or agrochemically acceptable salt form.

N-oxides are oxidized forms of tertiary amines or oxidized forms of nitrogen containing heteroaromatic compounds. They are described for instance in the book "Heterocyclic N-oxides" by A. Albini and S. Pietra, CRC Press, Boca Raton 1991.

The following list provides definitions, including preferred definitions, for substituents n, A¹, A², A³, A⁴, R¹, R², R³, R⁴, R⁵, R⁶, R⁷, Z, R⁸, R⁹, R¹⁰, R¹¹, and R¹², with reference to the compounds of Formula (I). For any one of these substituents, any of the definitions given below may be combined with any definition of any other substituent given below or elsewhere in this document.

n represents 0, 1 or 2. Preferably, n is 0 or 1. In some embodiments of the invention, n is 0. In other embodiments of the invention, n is 1.

A¹ represents N or CR¹, wherein R¹ represents hydrogen, halogen or methyl. Preferably, A¹ represents N or CR¹, wherein R¹ represents hydrogen, fluoro, chloro or methyl. More preferably, A¹ represents CR¹, wherein R¹ represents hydrogen, fluoro, chloro or methyl. More preferably still, A¹ represents CR¹, wherein R¹ represents hydrogen or fluoro.

A² represents N or CR², wherein R² represents hydrogen, halogen or methyl. Preferably, A² represents N or CR², wherein R² represents hydrogen, fluoro, chloro or methyl. More preferably, A² represents N or CR², wherein R² represents hydrogen or fluoro.

A³ represents N or CR³, wherein R³ represents hydrogen or fluoro. Preferably, A³ represents CR³, wherein R³ represents hydrogen or fluoro.

A⁴ represents N or CR⁴, wherein R⁴ represents hydrogen or fluoro. Preferably, A⁴ represents CR⁴, wherein R⁴ represents hydrogen or fluoro.

At least 2 of A¹, A², A³ and A⁴ are C-H.

Preferably, A¹ and A² each independently represent N, C-H or C-F, A³ and A⁴ each independently represent C-H or C-F, wherein at least 2 of A¹, A², A³ and A⁴ are C-H.

In one embodiment of the invention A¹ is C-F, and A², A³ and A⁴ are C-H. In another embodiment of the invention, A² is C-F, and A¹, A³ and A⁴ are C-H. In a further embodiment of the invention, A³ is C-F, and A¹, A² and A⁴ are C-H. In a further still embodiment of the invention A⁴ is C-F, and A¹, A² and A³ are C-H. In an additional embodiment of the invention, A² and A⁴ are each C-F, and A¹ and A³ are each C-H.

In one embodiment of the invention A² is N, and A¹, A³ and A⁴ are C-H. In a further embodiment of the invention A¹, A², A³ and A⁴ are all C-H.

R⁵ and R⁶ independently represent hydrogen, methyl, cyano, difluoromethyl, or trifluoromethyl. Preferably, R⁵ and R⁶ are both hydrogen or methyl, or R⁵ is hydrogen and R⁶ is methyl. More preferably, R⁵ and R⁶ are both hydrogen.

R⁷ represents hydroxy, C₁₋₄haloalkyl, C₁₋₄alkoxy, hydroxyC₂₋₄alkyl, C₁₋₂alkoxyC₂₋₄alkyl, C₁₋₂haloalkoxy, C₁₋₂haloalkoxyC₂₋₄alkyl, C₃₋₄alkenyloxy, C₃₋₄alkynyloxy or C₁₋₄alkylcarbonyloxy. Preferably, R⁷ represents hydroxy, C₁₋₄alkoxy, C₁₋₄haloalkyl, C₁₋₂haloalkoxy, C₃₋₄alkenyloxy or C₃₋₄alkynyloxy. More preferably, R⁷ represents methoxy.

Z represents R⁸ or -N(R¹⁰)R¹¹.

When Z represents R⁸, R⁸ represents C₁₋₆alkyl, C₂₋₆alkenyl, C₂₋₆alkynyl, cyanoC₁₋₄alkyl, C₁₋₄haloalkyl, C₁₋₄alkoxyC₂₋₄alkyl, C₁₋₂haloalkoxyC₂₋₄alkyl, C₁₋₄alkylcarbonylC₁₋₄alkyl, C₁₋₄alkoxycarbonylC₁₋₄alkyl, C₁₋₄alkylcarbonyloxyC₂₋₄alkyl, N-C₁₋₄alkylaminocarbonylC₁₋₄alkyl, N,N-diC₁₋₄alkylaminocarbonylC₁₋₄alkyl, N-C₁₋₄alkylaminoC₂₋₅alkyl, N,N-diC₁₋₄alkylaminoC₂₋₅alkyl, C₁₋₃alkylcarbonylaminoC₂₋₅alkyl, or C₁₋₄alkoxycarbonylaminoC₂₋₅alkyl; or
R⁸ is C₃₋₆cycloalkyl, C₃₋₆cycloalkylC₁₋₂alkyl, phenyl, phenylC₁₋₂alkyl, heteroaryl, heteroarylC₁₋₂alkyl, wherein the heteroaryl moiety is a 5- or 6-membered monocyclic aromatic ring which comprises 1, 2, 3 or 4 heteroatoms individually selected from N, O and S, heterocyclyl or heterocyclylC₁₋₂alkyl, wherein the heterocyclyl moiety is a 4- to 6-membered non-aromatic ring which comprises 1 or 2 heteroatoms individually selected from N, O and S;
wherein when R⁸ is C₃₋₆cycloalkyl, C₃₋₆cycloalkylC₁₋₂alkyl, phenyl, phenylC₁₋₂alkyl, heteroaryl, heteroarylC₁₋₂alkyl, heterocyclyl, or heterocyclylC₁₋₂alkyl, the C₃₋₆cycloalkyl, phenyl, heteroaryl, or heterocyclyl motif is optionally substituted by 1 or 2 substituents, which may be the same or different, selected from R⁹;
R⁹ represents cyano, fluoro, chloro, bromo, methyl, difluoromethyl, trifluoromethyl, methoxy, ethoxy, or difluoromethoxy; and wherein when R⁸ is substituted C₃₋₆cycloalkyl or heterocyclyl, these cycles may contain a carbonyl (C=O) or sulfonyl (S(O)₂) group;
Preferably, R⁸ is C₁₋₆alkyl, C₂₋₆alkenyl, C₂₋₆alkynyl, cyanoC₁₋₄alkyl, C₁₋₄haloalkyl, C₁₋₄alkoxyC₂₋₄alkyl, C₁₋₂haloalkoxyC₂₋₄alkyl, C₁₋₄alkylcarbonylC₁₋₄alkyl, C₁₋₄alkoxycarbonylC₁₋₄alkyl, or C₁₋₄alkylcarbonyloxyC₂₋₄alkyl; or
R⁸ is C₃₋₆cycloalkyl, C₃₋₆cycloalkylC₁₋₂alkyl, phenyl, phenylC₁₋₂alkyl, heteroaryl, heteroarylC₁₋₂alkyl, wherein the heteroaryl moiety is a 5- or 6-membered monocyclic aromatic ring which comprises 1, 2, or 3 heteroatoms individually selected from N, O and S, heterocyclyl or heterocyclylC₁₋₂alkyl, wherein the heterocyclyl moiety is a 4- to 6-membered non-aromatic ring which comprises 1 or 2 heteroatoms individually selected from N, O and S;
wherein when R⁸ is C₃₋₆cycloalkyl, C₃₋₆cycloalkylC₁₋₂alkyl, phenyl, phenylC₁₋₂alkyl, heteroaryl, heteroarylC₁₋₂alkyl, heterocyclyl, or heterocyclylC₁₋₂alkyl, the C₃₋₆cycloalkyl, phenyl, heteroaryl, or heterocyclyl motif is optionally substituted by 1 or 2 substituents, which may be the same or different, selected from R⁹;
R⁹ represents cyano, fluoro, chloro, bromo, methyl, difluoromethyl, trifluoromethyl, methoxy, ethoxy, or difluoromethoxy; and wherein when R⁸ is substituted C₃₋₆cycloalkyl or heterocyclyl, these cycles may contain a carbonyl (C=O) or sulfonyl (S(O)₂) group.

More preferably, R⁸ is C₁₋₄alkyl, C₂₋₄alkenyl, C₂₋₄alkynyl, cyanoC₁₋₂alkyl, C₁₋₃haloalkyl, C₁₋₄alkoxyC₂₋₄alkyl, C₁₋₂haloalkoxyC₂₋₄alkyl, C₁₋₃alkylcarbonylC₁₋₃alkyl, C₁₋₃alkoxycarbonylC₁₋₃alkyl, or C₁₋₃alkylcarbonyloxyC₂₋₄alkyl; or
R⁸ is C₃₋₅cycloalkyl, C₃₋₅cycloalkylC₁₋₂alkyl, phenyl, phenylC₁₋₂alkyl, heteroaryl, heteroarylC₁₋₂alkyl, wherein the heteroaryl moiety is a 5- or 6-membered monocyclic aromatic ring which comprises 1, 2, or 3 heteroatoms individually selected from N, O, and S, heterocyclyl or heterocyclylC₁₋₂alkyl, wherein the heterocyclyl moiety is a 4- to 6-membered non-aromatic ring which comprises 1 or 2 heteroatoms individually selected from N, O, and S;
wherein when R⁸ is C₃₋₅cycloalkyl, C₃₋₅cycloalkylC₁₋₂alkyl, phenyl, phenylC₁₋₂alkyl, heteroaryl, heteroarylC₁₋₂alkyl, heterocyclyl, or heterocyclylC₁₋₂alkyl, the C₃₋₅cycloalkyl, phenyl, heteroaryl, or heterocyclyl motif is optionally substituted by 1 or 2 substituents, which may be the same or different, selected from R⁹;
R⁹ represents cyano, fluoro, chloro, bromo, methyl, difluoromethyl, trifluoromethyl or methoxy; and wherein when R⁸ is substituted C₃₋₅cycloalkyl or heterocyclyl, R⁹ may also represent oxo (C=O).

More preferably, R⁸ is C₁₋₄alkyl, C₃₋₄alkenyl, C₁₋₃haloalkyl, C₁₋₂alkoxyC₂₋₃alkyl or C₁₋₂alkoxycarbonylC₁₋₂alkyl; or
R⁸ is C₃₋₅cycloalkyl, (C₃₋₅cycloalkyl)methyl, phenyl, benzyl, heteroaryl, (heteroaryl)methyl, wherein the heteroaryl moiety is a 5-membered monocyclic aromatic ring which comprises 1 or 2 heteroatoms individually selected from N, O, and S, heterocyclyl or (heterocyclyl)methyl, wherein the heterocyclyl moiety is a 5- or 6-membered non-aromatic ring which comprises 1 or 2 heteroatoms individually selected from N and O;
wherein when R⁸ is C₃₋₅cycloalkyl, (C₃₋₅cycloalkyl)methyl, phenyl, benzyl, heteroaryl, (heteroaryl)methyl, heterocyclyl, or (heterocyclyl)methyl, the C₃₋₅cycloalkyl, phenyl, heteroaryl, or heterocyclyl motif is optionally substituted by 1 or 2 substituents, which may be the same or different, selected from R⁹;
R⁹ represents cyano, fluoro, chloro, bromo, methyl or methoxy.

Even more preferably, R⁸ is methyl, ethyl, *n*-propyl, isopropyl, *n*-butyl, *t*-butyl, allyl, prop-1-enyl, isopropenyl, 1,1-difluoromethyl, 1,1,1-trifluoromethyl, 2,2-difluoroethyl, 2,2,2-trifluoroethyl, 3,3,3-trifluoropropyl, methoxymethyl, methoxyethyl, methoxycarbonylmethyl, methoxycarbonylethyl, ethoxycarbonylmethyl, ethoxycarbonylethyl, cyclopropyl, cyclobutyl, cyclopentyl, cyclopropylmethyl, cyclobutylmethyl, cyclopentylmethyl, 2,2-difluorocyclopropylmethyl, 2,2-dichlorocyclopropylmethyl, phenyl, benzyl, 2-fluorophenyl, 2,4-difluorophenyl, 2,4-dichlorophenyl, 4-chloro-2-fluorophenyl, 4-fluoro-2-chlorophenyl, pyrazol-3-yl, pyrazol-4-yl, 1-methylpyrazol-3-yl, 1-methylpyrazol-4-yl, thiophene-2-yl, thiophene-3-yl, 2-methylthiophene-5-yl, 2-methylthiophene-4-yl, tetrahydrofuran-2-yl, tetrahydrofuran-3-yl, tetrahydrofuran-2-yl methyl, tetrahydrofuran-3-yl methyl, morpholinyl, pyrrolidin-1-yl, pyrrolidin-2-yl, pyrrolidin-3-yl, piperidin-1-yl, piperidin-2-yl, piperidin-3-yl, or piperidin-4-yl.

More preferably still, R⁸ is methyl, ethyl, n-propyl, isopropyl, n-butyl, allyl, prop-1-enyl, 3,3,3-trifluoropropyl, methoxyethyl, methoxycarbonylmethyl, methoxycarbonylethyl, cyclopropyl, cyclopropylmethyl, 2,2-difluorocyclopropylmethyl, phenyl, benzyl, 2-fluorophenyl, 2,4-difluorophenyl, 4-chloro-2-fluorophenyl, 1-methylpyrazol-3-yl, 2-methylthiophene-5-yl, ortetrahydrofuran-3-yl methyl.

In a further set of preferable embodiments R⁸ is methyl, ethyl, methoxyethyl, cyclopropyl or cyclopropylmethyl.

When Z represents -N(R¹⁰)R¹¹, R¹⁰ represents hydrogen, C₁₋₆alkyl, C₁₋₆haloalkyl, cyanoC₁₋₄alkyl, hydroxyC₂₋₄alkyl, C₁₋₂alkoxyC₂₋₄alkyl, C₁₋₂haloalkoxyC₁₋₄alkyl, C₃₋₆alkenyl, C₃₋₆alkynyl, aminoC₂₋₄alkyl, N-C₁₋₄alkylaminoC₂₋₄alkyl, N,N-diC₁₋₄alkylaminoC₂₋₄alkyl, formyl, C₁₋₄alkylcarbonyl, C₁₋₄alkylcarbonylC₁₋₄alkyl, C₁₋₄alkoxycarbonylC₁₋₄alkyl, C₁₋₄alkylcarbonyloxyC₁₋₄alkyl, N-C₁₋₄alkylaminocarbonylC₁₋₄alkyl, N,N-diC₁₋₄alkylaminocarbonylC₁₋₄alkyl, C₁₋₄alkoxycarbonylaminoC₂₋₄alkyl, C₁₋₄alkylcarbonylaminoC₂₋₄alkyl, or C₁₋₄haloalkylcarbonylaminoC₂₋₄alkyl; or
R¹⁰ is C₃₋₆cycloalkyl, C₃₋₆cycloalkylC₁₋₂alkyl, phenyl, phenylC₁₋₂alkyl, heteroaryl, heteroarylC₁₋₂alkyl, wherein the heteroaryl moiety is a 5- or 6-membered monocyclic aromatic ring which comprises 1, 2, 3 or 4 heteroatoms individually selected from N, O and S, heterocyclyl or heterocyclylC₁₋₂alkyl, wherein the heterocyclyl moiety is a 4- to 6-membered non-aromatic ring which comprises 1 or 2 heteroatoms individually selected from N, O and S;
wherein when R¹⁰ is C₃₋₆cycloalkyl, C₃₋₆cycloalkylC₁₋₂alkyl, phenylC₁₋₂alkyl, phenyl, heteroaryl, heteroarylC₁₋₂alkyl, heterocyclyl, or heterocyclylC₁₋₂alkyl, the C₃₋₆cycloalkyl, phenyl, heteroaryl, or heterocyclyl motif is optionally substituted by 1 or 2 substituents, which may be the same or different, selected from R¹²;
R¹² represents cyano, fluoro, chloro, bromo, methyl, difluoromethyl, trifluoromethyl, methoxy, ethoxy, or difluoromethoxy; and wherein when R¹⁰ is substituted C₃₋₆cycloalkyl or heterocyclyl, these cycles may contain a carbonyl (C=O) or sulfonyl (S(O)₂) group.

Preferably, R¹⁰ represents hydrogen, C₁₋₆alkyl, C₁₋₆haloalkyl, cyanoC₁₋₄alkyl, hydroxyC₂₋₄alkyl, C₁₋₂alkoxyC₂₋₄alkyl, C₁₋₂haloalkoxyC₁₋₄alkyl, C₃₋₆alkenyl, C₃₋₆alkynyl, formyl, C₁₋₄alkylcarbonyl, C₁₋₄alkylcarbonylC₁₋₄alkyl, C₁₋₄alkoxycarbonylC₁₋₄alkyl, or C₁₋₄alkylcarbonyloxyC₁₋₄alkyl; or
R¹⁰ is C₃₋₆cycloalkyl, C₃₋₆cycloalkylC₁₋₂alkyl, phenyl, or phenylC₁₋₂alkyl;
wherein when R¹⁰ is C₃₋₆cycloalkyl, C₃₋₆cycloalkylC₁₋₂alkyl, phenyl, or phenylC₁₋₂alkyl, the C₃₋₆cycloalkyl or phenyl motif is optionally substituted by 1 or 2 substituents, which may be the same or different, selected from R¹²;
R¹² represents cyano, fluoro, chloro, bromo, methyl, difluoromethyl, trifluoromethyl, methoxy, ethoxy, or difluoromethoxy; and wherein when R¹⁰ is substituted C₃₋₆cycloalkyl or heterocyclyl, these cycles may contain a carbonyl (C=O) or sulfonyl (S(O)₂) group.

More preferably, R¹⁰ represents hydrogen, C₁₋₄alkyl, C₁₋₄haloalkyl, cyanoC₁₋₂alkyl, hydroxyC₂₋₄alkyl, C₁₋₂alkoxyC₂₋₄alkyl, C₁₋₂haloalkoxyC₁₋₂alkyl, C₃₋₄alkenyl, C₃₋₄alkynyl, formyl, C₁₋₂alkylcarbonyl, C₁₋₂alkylcarbonylC₁₋₂alkyl, C₁₋₂alkoxycarbonylC₁₋₂alkyl, or C₁₋₂alkylcarbonyloxyC₁₋₂alkyl; or
R¹⁰ is C₃₋₆cycloalkyl or C₃₋₆cycloalkylC₁₋₂alkyl;
wherein when R¹⁰ is C₃₋₆cycloalkyl or C₃₋₆cycloalkylC₁₋₂alkyl, C₃₋₆cycloalkyl motif is optionally substituted by 1 or 2 substituents, which may be the same or different, selected from R¹²;
R¹² represents cyano, fluoro, chloro, bromo, methyl, or methoxy; and wherein when R¹⁰ is substituted C₃₋₆cycloalkyl or heterocyclyl, these cycles may contain a carbonyl (C=O) group.

More preferably still, R¹⁰ represents hydrogen, methyl, ethyl, *n*-propyl, isopropyl, *n*-butyl, *t*-butyl, 1,1-difluoromethyl, cyanomethyl, hydroxymethyl, methoxymethyl, allyl, propargyl, formyl, methylcarbonyl, methoxycarbonylmethyl, cyclopropyl or cyclopropylmethyl.

Most preferably, R¹⁰ represents hydrogen or methyl.

R¹¹ represents hydrogen, hydroxy, C₁₋₄alkyl, C₁₋₂haloalkyl, C₃₋₄alkenyl, C₃₋₄alkynyl, C₃₋₄cycloalkyl, C₃₋₄cycloalkylC₁₋₂alkyl, C₁₋₄alkoxy, C₃₋₄alkenyloxy, or C₃₋₄alkynyloxy. Preferably, R¹¹ represents hydrogen, hydroxy, C₁₋₄alkyl, C₁₋₂haloalkyl, C₃₋₄alkenyl, C₃₋₄alkynyl, C₃₋₄cycloalkyl, C₃₋₄cycloalkylC₁₋₂alkyl, C₁₋₄alkoxy, C₃₋₄alkenyloxy, or C₃₋₄alkynyloxy. More preferably, R¹¹ represents hydrogen, hydroxy, C₁₋₄alkyl, C₁₋₂haloalkyl, C₃₋₄alkenyl, C₃₋₄alkynyl, C₃₋₄cycloalkyl, C₃₋₄cycloalkylC₁₋₂alkyl or C₁₋₄alkoxy. More preferably still, R¹¹ represents methyl, ethyl, n-propyl or propargyl. Most preferably, R¹¹ represents methyl, ethyl or n-propyl.

Preferably, the compound according to Formula (I) is selected from a compound 1.3, 1.5, 1.9, 1.18, 1.22, 1.40, 1.57, 1.58, 1.88 to 1.95, 1.101, 1.111 or 1.112 listed in Table T1 (below), or compound 4.2 listed in Table T4 (below).

Preferably, in a compound according to Formula (I) of the invention, n is 0 or 1;
A¹ represents C-H;
A² represents N or CR², wherein R² is hydrogen or fluoro;
A³ represents C-H;
A⁴ represents CR⁴, wherein R⁴ is hydrogen or fluoro;
R⁵ and R⁶ independently represent hydrogen or methyl;
R⁷ is methoxy;
Z represents R⁸;
R⁸ represents methyl, ethyl, n-propyl, isopropyl, n-butyl, allyl, prop-1-enyl, 3,3,3-trifluoropropyl, methoxyethyl, methoxycarbonylmethyl, methoxycarbonylethyl, cyclopropyl, cyclopropylmethyl, cyclopentylmethyl, 2,2-difluorocyclopropylmethyl, phenyl, benzyl, 2,4-difluorophenyl, 4-chloro-2-fluorophenyl, 1-methylpyrazol-3-yl, 2-methylthiophene-5-yl, tetrahydrofuran-3-yl methyl, pyrrolidinyl or morpholinyl.

Preferably, in a compound according to Formula (I) of the invention, n is 1;
A¹, A², A³, and A⁴ each independently represent C-H;
R⁵ and R⁶ independently represent hydrogen or methyl;
R⁷ is methoxy;
Z represents R⁸;
R⁸ represents methyl, ethyl, n-propyl, isopropyl, n-butyl, allyl, prop-1-enyl, 3,3,3-trifluoropropyl, methoxyethyl, methoxycarbonylmethyl, methoxycarbonylethyl, cyclopropyl, cyclopentylmethyl, 2,2-difluorocyclopropylmethyl, phenyl, benzyl, 1-methylpyrazol-3-yl, 2-methylthiophene-5-yl, tetrahydrofuran-3-yl methyl, pyrrolidinyl or morpholinyl.

More preferably, in a compound according to Formula (I) of the invention, n is 1;
A¹, A², A³, and A⁴ each independently represent C-H;
R⁵ and R⁶ both represent hydrogen;
R⁷ is methoxy;
Z represents R⁸;
R⁸ represents methyl, ethyl, methoxyethyl, cyclopropyl, cyclopropylmethyl, cyclopentylmethyl, or morpholinyl.

Preferably, in a compound according to Formula (I) of the invention, n is 1;
A¹, A², A³, and A⁴ each independently represent C-H;
R⁵ and R⁶ independently represent hydrogen or methyl;
R⁷ is methoxy;
Z represents -N(R¹⁰)R¹¹;
R¹⁰ represents hydrogen or methyl;
R¹¹ represents methyl, ethyl, n-propyl, or propargyl.

The following combinations represent particularly preferable embodiments:
A compound according to Formula (I) of the invention, wherein n is 1;
A¹, A², A³, and A⁴ are C-H;
R⁵ and R⁶ are hydrogen;
R⁷ represents methoxy;
Z represents R⁸, wherein R⁸ is methyl, ethyl, cyclopropyl, methoxyethyl, methoxycarbonylmethyl, methoxycarbonylethyl, 3,3,3-trifluoropropyl, 2,2-dichlorocyclopropylmethyl, prop-1-enyl, allyl, pyrrolidin-1-yl, 1-methylpyrazol-3-yl, or 2-methylthiophene-5-yl; or
Z represents N(R¹⁰)R¹¹, wherein R¹⁰ represents hydrogen or methyl, and R¹¹ represents methyl, ethyl, or n-propyl; or

A compound according to Formula (I) of the invention, wherein n is 2;
A¹, A², A³, and A⁴ are C-H;
R⁵ and R⁶ each independently represent hydrogen or methyl;
R⁷ represents methoxy;
Z represents R⁸, wherein R⁸ is methyl.

The compounds of the present invention may be enantiomers of the compound of Formula (I) as represented by a Formula (la) or a Formula (lb) when n is 1, wherein R⁵ and R⁶ are different (see below), or indeed when n is 2 and at only one of the two carbon positions bound to R⁵ and R⁶, R⁵ and R⁶ are different.

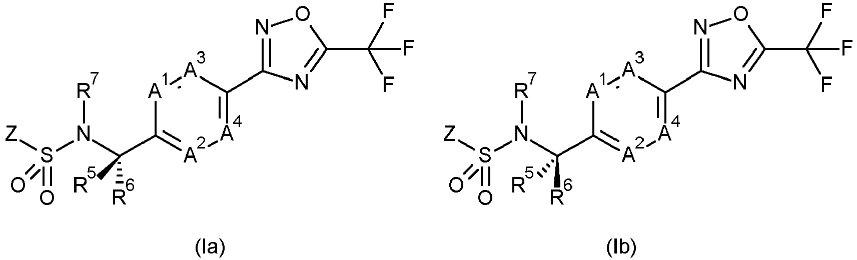

Likewise, the compounds of the present invention may be diastereomers of the compound of Formula (I) when n is 2, and wherein at each of the two carbon positions bound to R⁵ and R⁶, R⁵ and R⁶ are different.

It is understood that when in aqueous media, the compounds of Formula (I) according to the invention may be present in a reversible equilibrium with the corresponding covalently hydrated forms (i.e., the compounds of Formula (I-I) and Formula (I-II) as shown below, which may exist in tautomeric form as the compounds of formula (I-Ia) and formula (I-IIa)) at the CF₃-oxadiazole motif. This dynamic equilibrium may be important for the biological activity of the compounds of Formula (I). The designations of n, A¹, A², A³, A⁴, R¹, R², R³, R⁴, R⁵, R⁶, R⁷, Z, R⁸, R⁹, R¹⁰, R¹¹, and R¹², with reference to the compounds of Formula (I) of the present invention apply generally to the compounds of Formulae (I-I), (I-II), (I-Ia) and (I-IIa), as do the specific disclosures of combinations of n, A¹, A², A³, A⁴, R¹, R², R³, R⁴, R⁵, R⁶, R⁷, Z, R⁸, R⁹, R¹⁰, R¹¹, and R¹², as represented for compounds in Tables 1.2 and 2.2 below, or the compounds 1.3, 1.5, 1.9, 1.18, 1.22, 1.40, 1.57, 1.58, 1.88 to 1.95, 1.101, 1.111 or 1.112 described in Table T1 (below), or the compound 4.2 described in Table T4 (below).

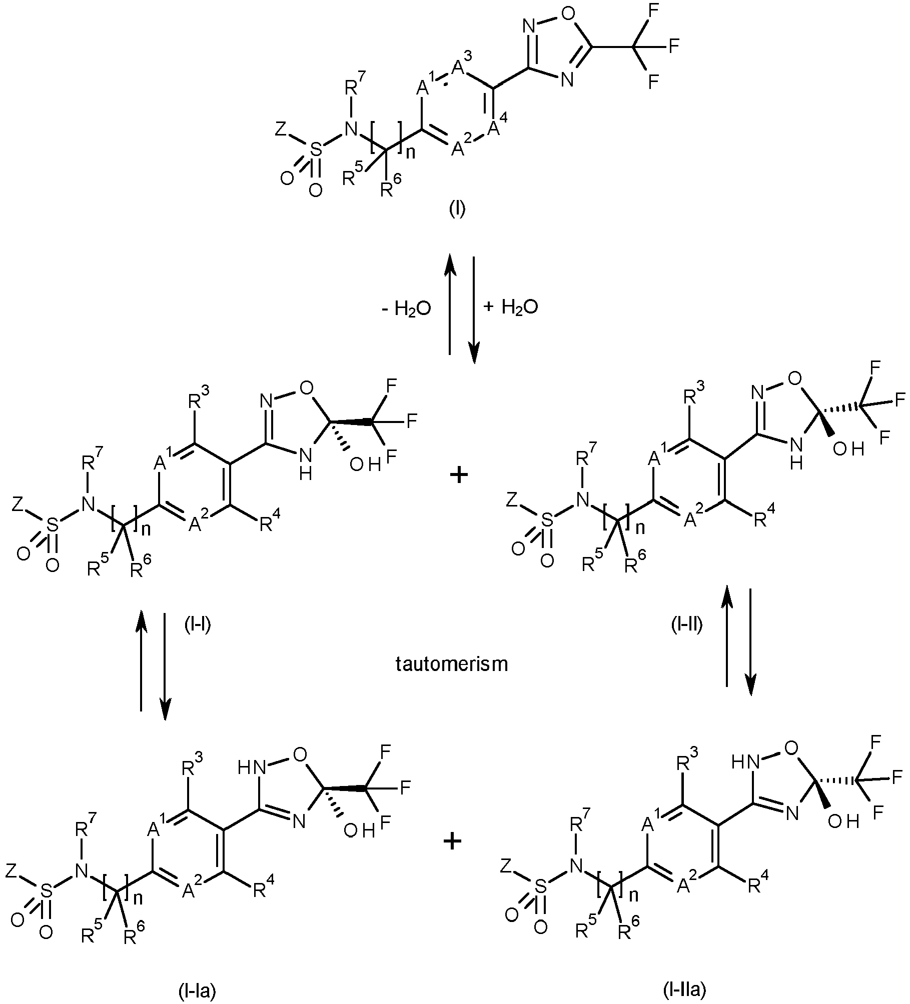

Compounds of the present invention can be made as shown in the following schemes 1 to 12, in which, unless otherwise stated, the definition of each variable is as defined above for a compound of Formula (I).

The compounds of Formula (I) can be obtained by a coupling transformation with compounds of Formula (II) and compounds of Formula (III), in a suitable solvent (e.g., dichloromethane) preferably at a temperature of between 0°C and 25°C, and optionally in the presence of a base such as triethylamine or pyridine, or under conditions described in the literature for an amide coupling. For examples, see WO 2012/068251 or Oshima, T. et al. Angew. Chem., Int. Ed. (2015), 54, 7193. Furthermore, compounds of Formula (III) are commercially available or can be prepared using known methods. For examples, see Lin, Wenwei et al. Eur. J.Med. Chem. (2016), 108, 505; WO 2002/053557; or WO 2008/115381. This reaction is shown in Scheme 1.

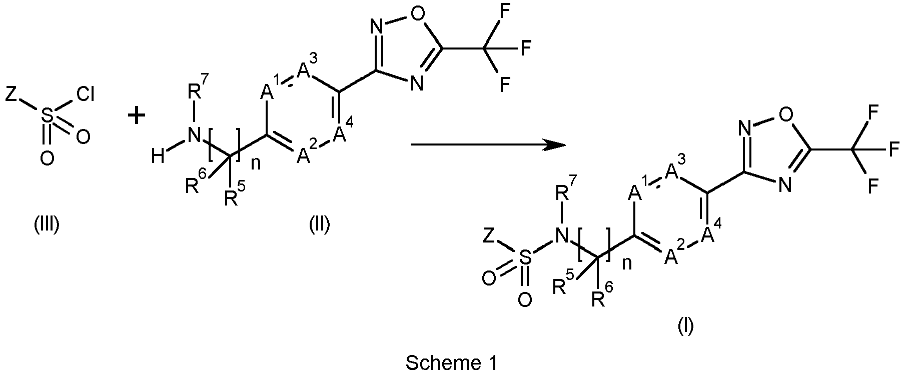

Alternatively, compounds of Formula (I) can be prepared from compounds of Formula (II) *via* treatment with SO₂Cl₂, in a suitable solvent (e.g. acetonitrile, or chloroform) at temperatures between 0°C and 40°C followed by the addition of suitable nucleophiles of Formula (IV), wherein Z-Nu is HN(R¹¹)R¹², in the presence of a base (e.g. trimethylamine or EtN(iPr)₂), optionally followed by heating at temperatures of 25°C and 75°C. For examples, see Lin, Wenwei et al. Eur. J.Med. Chem. (2016), 108, 505; WO 2002/053557; WO 2008/115381, or 2009002495. This reaction is shown in Scheme 2.

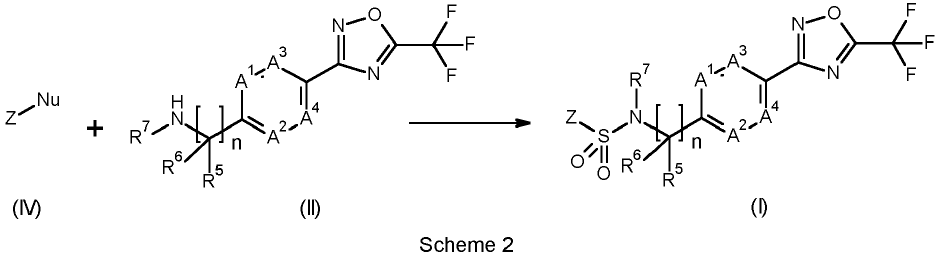

Additionally, compounds of Formula (I) can be prepared from compounds of Formula (V) by treatment with trifluoroacetic anhydride in the presence of a base (e.g. pyridine or 4-dimethylaminopyridine) in a suitable solvent, such as tetrahydrofuran or ethanol, at a temperature between 25°C and 75°C. For related examples, see WO 2003/028729 and WO 2010/045251. This reaction is shown in Scheme 3.

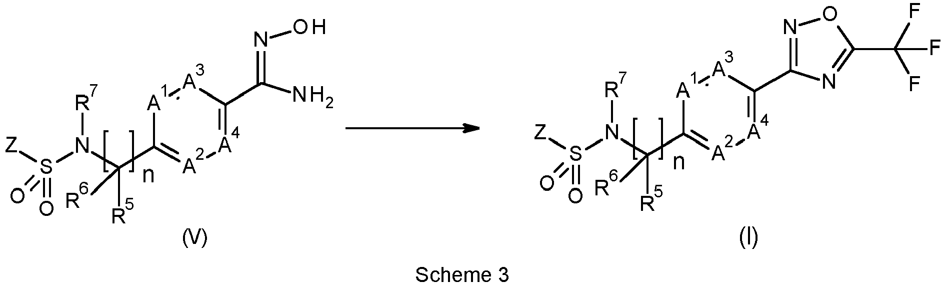

Compounds of Formula (V) can be prepared from compounds of Formula (VI) by treatment with a hydroxylamine hydrochloride salt in the presence of a base, such as triethylamine, in a suitable solvent, such as methanol, at a temperature between 0°C and 100°C. For related examples, see Kitamura, S. et al Chem. Pharm. Bull. (2001), 49, 268 and WO 2013/066838. This reaction is shown in Scheme 4.

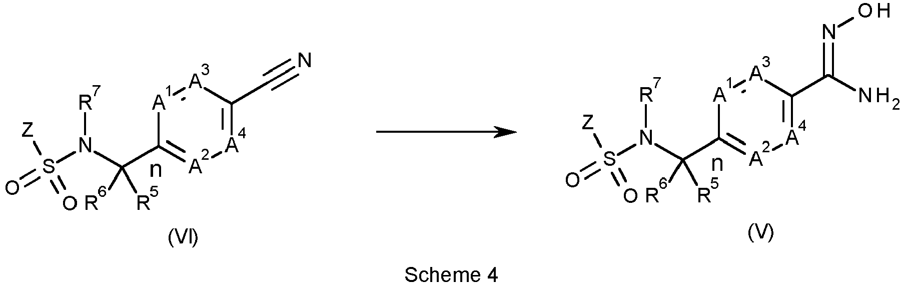

Compounds of Formula (VI) can be prepared from compounds of Formula (VII), wherein W is Br or I, *via* a metal-promoted reaction with a suitable cyanide reagent, such as Pd(0)/Zn(CN)₂ or CuCN, in a suitable solvent (e.g. dimethylformamide or N-methylpyrrolidone) at elevated temperature between 100°C and 120°C. For related examples, see US 2007/0155739 and WO 2009/022746. This reaction is shown in Scheme 5.

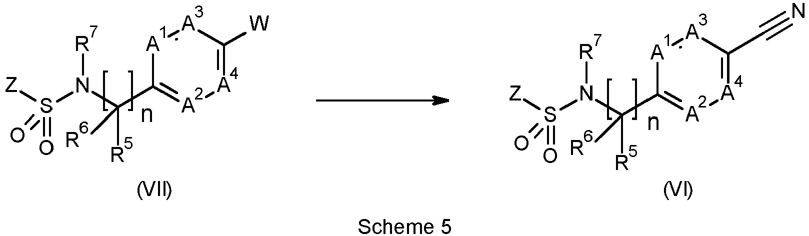

Compounds of Formula (VII), wherein the N-R⁷ bond contains a directly linked -CH₂ segment, can be prepared from compounds of Formula (VIII), wherein W is Br, I, or CN, *via* a base-promoted reaction (e.g. sodium hydride) with a suitable alkylating reagent (e.g. methyl iodide or propyl iodide), in a suitable solvent (e.g. dimethylformamide or N-methylpyrrolidone) at elevated temperature between 100°C and 120°C. This reaction is shown in Scheme 6.

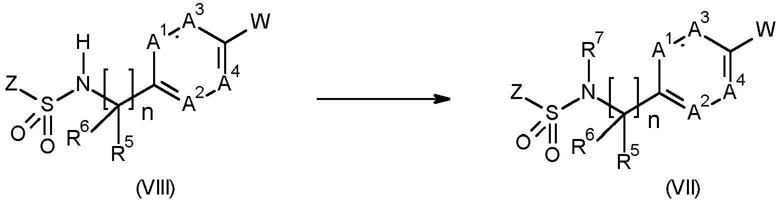

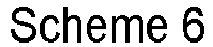

Alternatively, compounds of Formula (II), wherein n is preferably 1, can be prepared from compounds of Formula (X), wherein X is Cl or Br, by treatment with amines of Formula (IX), wherein Y is hydrogen or *tert*-butylcarboxylate, in a suitable solvent (e.g. tetrahydrofuran) at a temperature between 25°C and 60°C. Removal of the *tert*-butylcarboxylate groups with concomitant liberation of benzylamines of Formula (II) occurs upon treatment with HCl or trifluoroacetic acid in a suitable solvent (e.g. dioxane or MeOH). For related examples, see Miyawaki, K. et al. Heterocycles (2001), 54, 887, WO 2003/028729, and WO 2013/066839. This reaction is shown in Scheme 7.

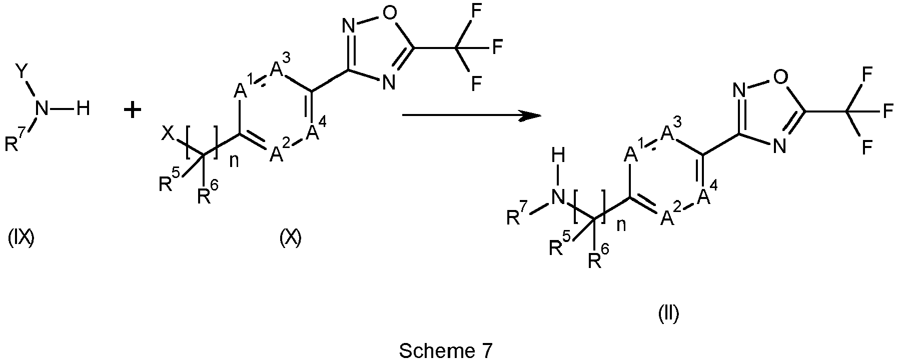

Compounds of Formula (X), wherein n is 1 and X is Cl or Br, can be prepared from compounds of Formula (XI), by treatment with a halogen source (e.g. N-bromosuccinimide (NBS) or N-chlorosuccinimide (NCS)) and a radical initiator (e.g. (PhCO₂)₂ or azobisisobutyronitrile (AIBN)) in a suitable solvent, such as tetrachloromethane, at temperatures between 55° and 100°C in the presence of ultraviolet light. For related examples, see Liu, S. et al. Synthesis (2001), 14, 2078 and Kompella, A. et al Org. Proc. Res. Dev. (2012), 16, 1794. This reaction is shown in Scheme 8.

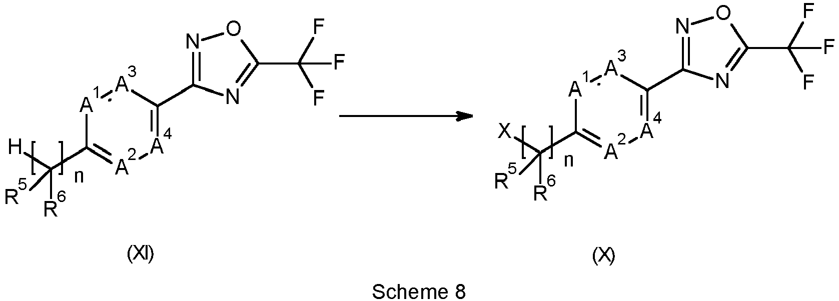

The compounds of Formula (VIII) can be obtained by a coupling transformation with compounds of Formula (XII) and compounds of Formula (III), in a suitable solvent (e.g. dichloromethane) preferably at a temperature of between 0°C and 25°C, and optionally in the presence of a base such as triethylamine or pyridine, or under conditions described in the literature for an amide coupling. For examples, see WO 2012/068251 or Oshima, T. et al. Angew. Chem., Int. Ed. (2015), 54, 7193. Furthermore, compounds of Formula (III) and compounds of Formula (XII) are commercially available or can be prepared using known methods. This reaction is shown in Scheme 9.

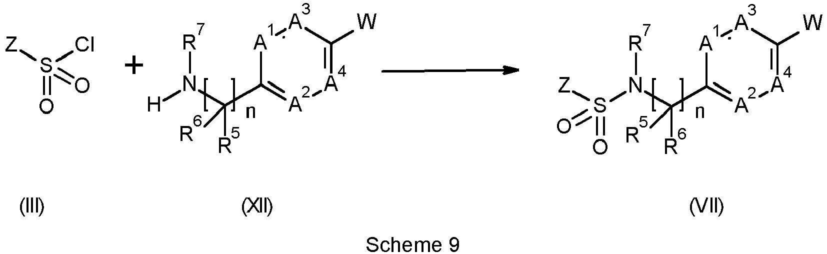

Alternatively, compounds of Formula (XII), wherein n is preferably 1, can be prepared from compounds of Formula (XIII), wherein X is Cl or Br and W is Br, I, or CN, by treatment with amines of Formula (IX), wherein Y is hydrogen or *tert*-butylcarboxylate, in a suitable solvent (e.g. tetrahydrofuran) at a temperature between 25°C and 60°C. Removal of the *tert*-butylcarboxylate groups with concomitant liberation of benzylamines of Formula (II) occurs upon treatment with HCl or trifluoroacetic acid in a suitable solvent (e.g. dioxane or MeOH). For related examples, see Miyawaki, K. et al. Heterocycles (2001), 54, 887, WO 2003/028729, and WO 2013/066839. This reaction is shown in Scheme 10.

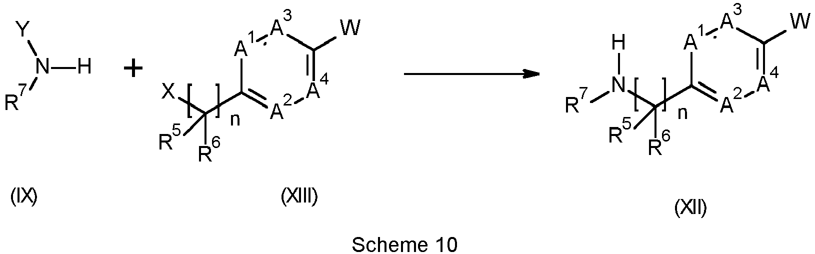

Compounds of Formula (XIII), wherein W is Br, I, or CN and X is Cl or Br and n is preferably 1, are either commercially available or can be prepared from compounds of Formula (XIX), by treatment with a halogen source, (e.g. N-bromosuccinimide (NBS) or N-chlorosuccinimide (NCS)) and a radical initiator, such as (PhCO₂)₂ or azobisisobutyronitrile (AIBN), in the presence of ultraviolet light, in a suitable solvent, such as tetrachloromethane, at temperatures between 55°C and 100°C. For related examples, see Liu, S. et al. Synthesis (2001), 14, 2078 and Kompella, A. et al Org. Proc. Res. Dev. (2012), 16, 1794. This reaction is shown in Scheme 11.

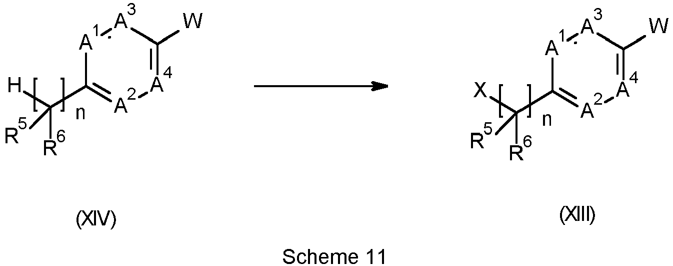

Alternatively, compounds of Formula (XIII), wherein X is Cl, Br, I, or -OSO₂Me and Z is Br, I, or CN and n is preferably 1, are either commercially available or can be prepared from compounds of Formula (XX), by treatment with a halogen source (e.g. CBr₄, CCl₄ or I₂) in the presence of triphenylphosphine, or with methanesulfonyl chloride (ClSO₂Me), in a suitable solvent, (e.g. dichloromethane) at a temperature between 0°C and 100°C. For related examples, see Liu, H. et al Bioorg. Med. Chem. (2008), 16, 10013, WO 2014/020350 and Kompella, A. et al Bioorg. Med. Chem. Lett. (2001), 1, 3161. Compounds of formula (XIV) are commercially available. This reaction is shown in Scheme 12.

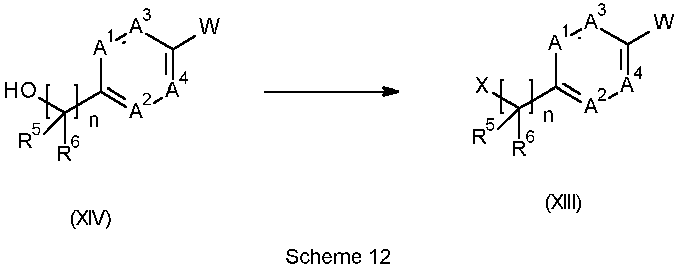

As already indicated, surprisingly, it has now been found that the compounds of Formula (I) of the present invention have, for practical purposes, a very advantageous level of biological activity for protecting plants against diseases that are caused by fungi.

The compounds of Formula (I) can be used in the agricultural sector and related fields of use, e.g., as active ingredients for controlling plant pests or on non-living materials for the control of spoilage microorganisms or organisms potentially harmful to man. The novel compounds are distinguished by excellent activity at low rates of application, by being well tolerated by plants and by being environmentally safe. They have very useful curative, preventive and systemic properties and can be used for protecting numerous cultivated plants. The compounds of Formula (I) can be used to inhibit or destroy the pests that occur on plants or parts of plants (fruit, blossoms, leaves, stems, tubers, roots) of different crops of useful plants, while at the same time protecting also those parts of the plants that grow later, e.g., from phytopathogenic microorganisms.

The present invention further relates to a method for controlling or preventing infestation of plants or plant propagation material and/or harvested food crops susceptible to microbial attack by treating plants or plant propagation material and/or harvested food crops wherein an effective amount a compound of Formula (I) is applied to the plants, to parts thereof or the locus thereof.

It is also possible to use compounds of Formula (I) as a fungicide. The term "fungicide" as used herein means a compound that controls, modifies, or prevents the growth of fungi. The term "fungicidally effective amount" where used means the quantity of such a compound or combination of such compounds that is capable of producing an effect on the growth of fungi. Controlling or modifying effects include all de*via*tion from natural development, such as killing, retardation and the like, and prevention includes barrier or other defensive formation in or on a plant to prevent fungal infection.

It may also be possible to use compounds of Formula (I) as dressing agents for the treatment of plant propagation material, e.g., seed, such as fruits, tubers or grains, or plant cuttings, for the protection against fungal infections as well as against phytopathogenic fungi occurring in the soil. The propagation material can be treated with a composition comprising a compound of Formula (I) before planting: seed, for example, can be dressed before being sown. The active compounds of Formula (I) can also be applied to grains (coating), either by impregnating the seeds in a liquid formulation or by coating them with a solid formulation. The composition can also be applied to the planting site when the propagation material is being planted, for example, to the seed furrow during sowing. The invention relates also to such methods of treating plant propagation material and to the plant propagation material so treated.

Furthermore, the compounds of Formula (I) can be used for controlling fungi in related areas, for example in the protection of technical materials, including wood and wood related technical products, in food storage, in hygiene management.

In addition, the invention could be used to protect non-living materials from fungal attack, e.g. lumber, wall boards and paint.

The compounds of Formula (I) are for example, effective against fungi and fungal vectors of disease as well as phytopathogenic bacteria and viruses. These fungi and fungal vectors of disease as well as phytopathogenic bacteria and viruses are for example:
Absidia corymbifera, Alternaria spp, Aphanomyces spp, Ascochyta spp, Aspergillus spp. including A. flavus, A. fumigatus, A. nidulans, A. niger, A. terrus, Aureobasidium spp. including A. pullulans, Blastomyces dermatitidis, Blumeria graminis, Bremia lactucae, Botryosphaeria spp. including B. dothidea, B. obtusa, Botrytis spp. inclusing B. cinerea, Candida spp. including C. albicans, C. glabrata, C. krusei, C. lusitaniae, C. parapsilosis, C. tropicalis, Cephaloascus fragrans, Ceratocystis spp, Cercospora spp. including C. arachidicola, Cercosporidium personatum, Cladosporium spp, Claviceps purpurea, Coccidioides immitis, Cochliobolus spp, Colletotrichum spp. including C. musae, Cryptococcus neoformans, Diaporthe spp, Didymella spp, Drechslera spp, Elsinoe spp,Epidermophyton spp, Erwinia amylovora, Erysiphe spp. including E. cichoracearum, Eutypa lata, Fusarium spp. including F. culmorum, F. graminearum, F. langsethiae, F. moniliforme, F. oxysporum, F. proliferatum, F. subglutinans, F. solani, Gaeumannomyces graminis, Gibberella fujikuroi, Gloeodes pomigena, Gloeosporium musarum, Glomerella cingulate, Guignardia bidwellii, Gymnosporangium juniperi-virginianae, Helminthosporium spp, Hemileia spp, Histoplasma spp. including H. capsulatum, Laetisaria fuciformis, Leptographium lindbergi, Leveillula taurica, Lophodermium seditiosum, Microdochium nivale, Microsporum spp, Monilinia spp, Mucor spp, Mycosphaerella spp. including M. graminicola, M. pomi, Oncobasidium theobromaeon, Ophiostoma piceae, Paracoccidioides spp, Penicillium spp. including P. digitatum, P. italicum, Petriellidium spp, Peronosclerospora spp. Including P. maydis, P. philippinensis and P. sorghi, Peronospora spp, Phaeosphaeria nodorum, Phakopsora pachyrhizi, Phellinus igniarus, Phialophora spp, Phoma spp, Phomopsis viticola, Phytophthora spp. including P. infestans, Plasmopara spp. including P. halstedii, P. viticola, Pleospora spp., Podosphaera spp. including P. leucotricha, Polymyxa graminis, Polymyxa betae, Pseudocercosporella herpotrichoides, Pseudomonas spp, Pseudoperonospora spp. including P. cubensis, P. humuli, Pseudopeziza tracheiphila, Puccinia Spp. including P. hordei, P. recondita, P. striiformis, P. triticina, Pyrenopeziza spp, Pyrenophora spp, Pyricularia spp. including P. oryzae, Pythium spp. including P. ultimum, Ramularia spp, Rhizoctonia spp, Rhizomucor pusillus, Rhizopus arrhizus, Rhynchosporium spp, Scedosporium spp. including S. apiospermum and S. prolificans, Schizothyrium pomi, Sclerotinia spp, Sclerotium spp, Septoria spp, including S. nodorum, S. tritici, Sphaerotheca macularis, Sphaerotheca fusca (Sphaerotheca fuliginea), Sporothorix spp, Stagonospora nodorum, Stemphylium spp,. Stereum hirsutum, Thanatephorus cucumeris, Thielaviopsis basicola, Tilletia spp, Trichoderma spp. including T. harzianum, T. pseudokoningii, T. viride, Trichophyton spp, Typhula spp, Uncinula necator, Urocystis spp, Ustilago spp, Venturia spp. including V. inaequalis, Verticillium spp, and Xanthomonas spp.

The compounds of Formula (I) may be used for example on turf, ornamentals, such as flowers, shrubs, broad-leaved trees or evergreens, for example conifers, as well as for tree injection, pest management and the like.

Within the scope of present invention, target crops and/or useful plants to be protected typically comprise perennial and annual crops, such as berry plants for example blackberries, blueberries, cranberries, raspberries and strawberries; cereals for example barley, maize (corn), millet, oats, rice, rye, sorghum triticale and wheat; fibre plants for example cotton, flax, hemp, jute and sisal; field crops for example sugar and fodder beet, coffee, hops, mustard, oilseed rape (canola), poppy, sugar cane, sunflower, tea and tobacco; fruit trees for example apple, apricot, avocado, banana, cherry, citrus, nectarine, peach, pear and plum; grasses for example Bermuda grass, bluegrass, bentgrass, centipede grass, fescue, ryegrass, St. Augustine grass and Zoysia grass; herbs such as basil, borage, chives, coriander, lavender, lovage, mint, oregano, parsley, rosemary, sage and thyme; legumes for example beans, lentils, peas and soya beans; nuts for example almond, cashew, ground nut, hazelnut, peanut, pecan, pistachio and walnut; palms for example oil palm; ornamentals for example flowers, shrubs and trees; other trees, for example cacao, coconut, olive and rubber; vegetables for example asparagus, aubergine, broccoli, cabbage, carrot, cucumber, garlic, lettuce, marrow, melon, okra, onion, pepper, potato, pumpkin, rhubarb, spinach and tomato; and vines for example grapes.

The term "useful plants" is to be understood as also including useful plants that have been rendered tolerant to herbicides like bromoxynil or classes of herbicides (such as, for example, HPPD inhibitors, ALS inhibitors, for example primisulfuron, prosulfuron and trifloxysulfuron, EPSPS (5-enol-pyrovyl-shikimate-3-phosphate-synthase) inhibitors, GS (glutamine synthetase) inhibitors or PPO (protoporphyrinogen-oxidase) inhibitors) as a result of conventional methods of breeding or genetic engineering. An example of a crop that has been rendered tolerant to imidazolinones, e.g. imazamox, by conventional methods of breeding (mutagenesis) is Clearfield^{®} summer rape (Canola). Examples of crops that have been rendered tolerant to herbicides or classes of herbicides by genetic engineering methods include glyphosate- and glufosinate-resistant maize varieties commercially available under the trade names RoundupReady^{®}, Herculex I^{®} and LibertyLink^{®}.

The term "useful plants" is to be understood as also including useful plants which have been so transformed by the use of recombinant DNA techniques that they are capable of synthesising one or more selectively acting toxins, such as are known, for example, from toxin-producing bacteria, especially those of the genus Bacillus.

Examples of such plants are: YieldGard^{®} (maize variety that expresses a CrylA(b) toxin); YieldGard Rootworm^{®} (maize variety that expresses a CryIIIB(b1) toxin); YieldGard Plus^{®} (maize variety that expresses a CryIA(b) and a CryIIIB(b1) toxin); Starlink^{®} (maize variety that expresses a Cry9(c) toxin); Herculex I^{®} (maize variety that expresses a CryIF(a2) toxin and the enzyme phosphinothricine N-acetyltransferase (PAT) to achieve tolerance to the herbicide glufosinate ammonium); NuCOTN 33B^{®} (cotton variety that expresses a CryIA(c) toxin); Bollgard I^{®} (cotton variety that expresses a CryIA(c) toxin); Bollgard II^{®} (cotton variety that expresses a CryIA(c) and a CryIIA(b) toxin); VIPCOT^{®} (cotton variety that expresses a VIP toxin); NewLeaf^{®} (potato variety that expresses a CryIIIA toxin); NatureGard^{®} Agrisure^{®} GT Advantage (GA21 glyphosate-tolerant trait), Agrisure^{®} CB Advantage (Bt11 corn borer (CB) trait), Agrisure^{®} RW (corn rootworm trait) and Protecta^{®}.

The term "crops" is to be understood as including also crop plants which have been so transformed by the use of recombinant DNA techniques that they are capable of synthesising one or more selectively acting toxins, such as are known, for example, from toxin-producing bacteria, especially those of the genus Bacillus.

Toxins that can be expressed by such transgenic plants include, for example, insecticidal proteins from Bacillus cereus or Bacillus popilliae; or insecticidal proteins from Bacillus thuringiensis, such as δ-endotoxins, e.g. Cry1Ab, Cry1Ac, Cry1F, Cry1Fa2, Cry2Ab, Cry3A, Cry3Bb1 or Cry9C, or vegetative insecticidal proteins (Vip), e.g. Vip1, Vip2, Vip3 or Vip3A; or insecticidal proteins of bacteria colonising nematodes, for example Photorhabdus spp. or Xenorhabdus spp., such as Photorhabdus luminescens, Xenorhabdus nematophilus; toxins produced by animals, such as scorpion toxins, arachnid toxins, wasp toxins and other insect-specific neurotoxins; toxins produced by fungi, such as Streptomycetes toxins, plant lectins, such as pea lectins, barley lectins or snowdrop lectins; agglutinins; proteinase inhibitors, such as trypsin inhibitors, serine protease inhibitors, patatin, cystatin, papain inhibitors; ribosome-inactivating proteins (RIP), such as ricin, maize-RIP, abrin, luffin, saporin or bryodin; steroid metabolism enzymes, such as 3-hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.

Further, in the context of the present invention there are to be understood by δ-endotoxins, for example Cry1Ab, Cry1Ac, Cry1F, Cry1Fa2, Cry2Ab, Cry3A, Cry3Bb1 or Cry9C, or vegetative insecticidal proteins (Vip), for example Vip1, Vip2, Vip3 or Vip3A, expressly also hybrid toxins, truncated toxins and modified toxins. Hybrid toxins are produced recombinantly by a new combination of different domains of those proteins (see, for example, WO 02/15701). Truncated toxins, for example a truncated Cry1Ab, are known. In the case of modified toxins, one or more amino acids of the naturally occurring toxin are replaced. In such amino acid replacements, preferably non-naturally present protease recognition sequences are inserted into the toxin, such as, for example, in the case of Cry3A055, a cathepsin-G-recognition sequence is inserted into a Cry3A toxin (see WO 03/018810).

Examples of such toxins or transgenic plants capable of synthesising such toxins are disclosed, for example, in EP-A-0 374 753, WO93/07278, WO95/34656, EP-A-0 427 529, EP-A-451 878 and WO 03/052073.

The processes for the preparation of such transgenic plants are generally known to the person skilled in the art and are described, for example, in the publications mentioned above. CryI-type deoxyribonucleic acids and their preparation are known, for example, from WO 95/34656, EP-A-0 367 474, EP-A-0 401 979 and WO 90/13651.

The toxin contained in the transgenic plants imparts to the plants tolerance to harmful insects. Such insects can occur in any taxonomic group of insects, but are especially commonly found in the beetles (Coleoptera), two-winged insects (Diptera) and butterflies (Lepidoptera).

Transgenic plants containing one or more genes that code for an insecticidal resistance and express one or more toxins are known and some of them are commercially available. Examples of such plants are: YieldGard^{®} (maize variety that expresses a Cry1Ab toxin); YieldGard Rootworm^{®} (maize variety that expresses a Cry3Bb1 toxin); YieldGard Plus^{®} (maize variety that expresses a Cry1Ab and a Cry3Bb1 toxin); Starlink^{®} (maize variety that expresses a Cry9C toxin); Herculex I^{®} (maize variety that expresses a Cry1Fa2 toxin and the enzyme phosphinothricine N-acetyltransferase (PAT) to achieve tolerance to the herbicide glufosinate ammonium); NuCOTN 33B^{®} (cotton variety that expresses a Cry1Ac toxin); Bollgard I^{®} (cotton variety that expresses a Cry1Ac toxin); Bollgard II^{®} (cotton variety that expresses a Cry1Ac and a Cry2Ab toxin); VipCot^{®} (cotton variety that expresses a Vip3A and a Cry1Ab toxin); NewLeaf^{®} (potato variety that expresses a Cry3A toxin); NatureGard^{®}, Agrisure^{®} GT Advantage (GA21 glyphosate-tolerant trait), Agrisure^{®} CB Advantage (Bt11 corn borer (CB) trait) and Protecta^{®}.

Further examples of such transgenic crops are:
1. **Bt11 Maize** from Syngenta Seeds SAS, Chemin de I'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a truncated Cry1Ab toxin. Bt11 maize also transgenically expresses the enzyme PAT to achieve tolerance to the herbicide glufosinate ammonium.
**2. Bt176 Maize** from Syngenta Seeds SAS, Chemin de I'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a Cry1Ab toxin. Bt176 maize also transgenically expresses the enzyme PAT to achieve tolerance to the herbicide glufosinate ammonium.
3. **MIR604 Maize** from Syngenta Seeds SAS, Chemin de I'Hobit 27, F-31 790 St. Sauveur, France, registration number C/FR/96/05/10. Maize which has been rendered insect-resistant by transgenic expression of a modified Cry3A toxin. This toxin is Cry3A055 modified by insertion of a cathepsin-G-protease recognition sequence. The preparation of such transgenic maize plants is described in WO 03/018810.
4. **MON 863 Maize** from Monsanto Europe S.A. 270-272 Avenue de Tervuren, B-1150 Brussels, Belgium, registration number C/DE/02/9. MON 863 expresses a Cry3Bb1 toxin and has resistance to certain Coleoptera insects.
5. **IPC 531 Cotton** from Monsanto Europe S.A. 270-272 Avenue de Tervuren, B-1150 Brussels, Belgium, registration number C/ES/96/02.
6. **1507 Maize** from Pioneer Overseas Corporation, Avenue Tedesco, 7 B-1160 Brussels, Belgium, registration number C/NL/00/10. Genetically modified maize for the expression of the protein Cry1F for achieving resistance to certain Lepidoptera insects and of the PAT protein for achieving tolerance to the herbicide glufosinate ammonium.
7. **NK603 × MON 810 Maize** from Monsanto Europe S.A. 270-272 Avenue de Tervuren, B-1150 Brussels, Belgium, registration number C/GB/02/M3/03. Consists of conventionally bred hybrid maize varieties by crossing the genetically modified varieties NK603 and MON 810. NK603 × MON 810 Maize transgenically expresses the protein CP4 EPSPS, obtained from *Agrobacterium sp.* strain CP4, which imparts tolerance to the herbicide Roundup^{®} (contains glyphosate), and also a Cry1Ab toxin obtained from *Bacillus thuringiensis subsp. kurstaki* which brings about tolerance to certain Lepidoptera, include the European corn borer.

The compounds of Formula (I) according to the present invention including a compound 1.3, 1.5, 1.9, 1.18, 1.22, 1.40, 1.57, 1.58, 1.88 to 1.95, 1.101, 1.111 or 1.112 listed in Table T1 (below), or compound 4.2 listed in Table T4 (below) may be used in controlling or preventing phytopathogenic diseases, especially phytopathogenic fungi (such as *Phakopsora pachyrhizi*) on soybean plants.

In particular, transgenic soybean plants expressing toxins, for example insecticidal proteins such as delta-endotoxins, e.g. Cry1Ac (Cry1Ac Bt protein). Accordingly, this may include transgenic soybean plants comprising event MON87701 (see U.S. Patent No. 8,049,071 and related applications and patents, as well as WO 2014/170327 A1 (eg, see paragraph [008] reference to Intacta RR2 PRO^{™} soybean)), event MON87751 (US. Patent Application Publication No. 2014/0373191) or event DAS-81419 (U.S. Patent No. 8632978 and related applications and patents).

Other transgenic soybean plants may comprise event SYHT0H2 - HPPD tolerance (U.S. Patent Application Publication No. 2014/0201860 and related applications and patents), event MON89788 - glyphosate tolerance (U.S. Pat. No. 7,632,985 and related applications and patents), event MON87708 - dicamba tolerance (U.S. Patent Application Publication No. US 2011/0067134 and related applications and patents), event DP-356043-5 - glyphosate and ALS tolerance (U.S. Patent Application Publication No. US 2010/0184079 and related applications and patents), event A2704-12 - glufosinate tolerance (U.S. Patent Application Publication No. US 2008/0320616 and related applications and patents), event DP-305423-1 - ALS tolerance (U.S. Patent Application Publication No. US 2008/0312082 and related applications and patents), event A5547-127 - glufosinate tolerance (U.S. Patent Application Publication No. US 2008/0196127 and related applications and patents), event DAS-40278-9 - tolerance to 2,4-dichlorophenoxyacetic acid and aryloxyphenoxypropionate (see WO 2011/022469, WO 2011/022470, WO 2011/022471, and related applications and patents), event 127 - ALS tolerance (WO 2010/080829 and related applications and patents), event GTS 40-3-2 - glyphosate tolerance, event DAS-68416-4-2,4-dichlorophenoxyacetic acid and glufosinate tolerance, event FG72 - glyphosate and isoxaflutole tolerance, event BPS-CV127-9 - ALS tolerance and GU262 - glufosinate tolerance or event SYHT04R - HPPD tolerance.

Under certain circumstances, compounds of Formula (I) according to the present invention when used in controlling or preventing phytopathogenic diseases, especially phytopathogenic fungi (such as *Phakopsora pachyrhizi*) on soy bean plants (in particular any of the transgenic soybean plants as described above), may display a synergistic interaction between the active ingredients.

The term "locus" as used herein means fields in or on which plants are growing, or where seeds of cultivated plants are sown, or where seed will be placed into the soil. It includes soil, seeds, and seedlings, as well as established vegetation.

The term "plants" refers to all physical parts of a plant, including seeds, seedlings, saplings, roots, tubers, stems, stalks, foliage, and fruits.

The term "plant propagation material" is understood to denote generative parts of the plant, such as seeds, which can be used for the multiplication of the latter, and vegetative material, such as cuttings or tubers, for example potatoes. There can be mentioned for example seeds (in the strict sense), roots, fruits, tubers, bulbs, rhizomes and parts of plants. Germinated plants and young plants which are to be transplanted after germination or after emergence from the soil, may also be mentioned. These young plants can be protected before transplantation by a total or partial treatment by immersion. Preferably "plant propagation material" is understood to denote seeds.

The compounds of Formula (I) may be used in unmodified form or, preferably, together with the adjuvants conventionally employed in the art of formulation. To this end they may be conveniently Formulated in known manner to emulsifiable concentrates, coatable pastes, directly sprayable or dilutable solutions or suspensions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances. The compositions may also contain further adjuvants such as stabilizers, antifoams, viscosity regulators, binders or tackifiers as well as fertilizers, micronutrient donors or other formulations for obtaining special effects.

Suitable carriers and adjuvants, e.g. for agricultural use, can be solid or liquid and are substances useful in formulation technology, e.g. natural or regenerated mineral substances, solvents, dispersants, wetting agents, tackifiers, thickeners, binders or fertilizers. Such carriers are for example described in WO 97/33890.

Suspension concentrates are aqueous formulations in which finely divided solid particles of the active compound are suspended. Such formulations include anti-settling agents and dispersing agents and may further include a wetting agent to enhance activity as well an anti-foam and a crystal growth inhibitor. In use, these concentrates are diluted in water and normally applied as a spray to the area to be treated. The amount of active ingredient may range from 0.5% to 95% of the concentrate.

Wettable powders are in the form of finely divided particles which disperse readily in water or other liquid carriers. The particles contain the active ingredient retained in a solid matrix. Typical solid matrices include fuller's earth, kaolin clays, silicas and other readily wet organic or inorganic solids. Wettable powders normally contain from 5% to 95% of the active ingredient plus a small amount of wetting, dispersing or emulsifying agent.

Emulsifiable concentrates are homogeneous liquid compositions dispersible in water or other liquid and may consist entirely of the active compound with a liquid or solid emulsifying agent, or may also contain a liquid carrier, such as xylene, heavy aromatic naphthas, isophorone and other nonvolatile organic solvents. In use, these concentrates are dispersed in water or other liquid and normally applied as a spray to the area to be treated. The amount of active ingredient may range from 0.5% to 95% of the concentrate.

Granular formulations include both extrudates and relatively coarse particles and are usually applied without dilution to the area in which treatment is required. Typical carriers for granular Formulations include sand, fuller's earth, attapulgite clay, bentonite clays, montmorillonite clay, vermiculite, perlite, calcium carbonate, brick, pumice, pyrophyllite, kaolin, dolomite, plaster, wood flour, ground corn cobs, ground peanut hulls, sugars, sodium chloride, sodium sulphate, sodium silicate, sodium borate, magnesia, mica, iron oxide, zinc oxide, titanium oxide, antimony oxide, cryolite, gypsum, diatomaceous earth, calcium sulphate and other organic or inorganic materials which absorb or which can be coated with the active compound. Granular Formulations normally contain 5% to 25% of active ingredients which may include surface-active agents such as heavy aromatic naphthas, kerosene and other petroleum fractions, or vegetable oils; and/or stickers such as dextrins, glue or synthetic resins.

Dusts are free-flowing admixtures of the active ingredient with finely divided solids such as talc, clays, flours and other organic and inorganic solids which act as dispersants and carriers.

Microcapsules are typically droplets or granules of the active ingredient enclosed in an inert porous shell which allows escape of the enclosed material to the surroundings at controlled rates. Encapsulated droplets are typically 1 to 50 microns in diameter. The enclosed liquid typically constitutes 50 to 95% of the weight of the capsule and may include solvent in addition to the active compound. Encapsulated granules are generally porous granules with porous membranes sealing the granule pore openings, retaining the active species in liquid form inside the granule pores. Granules typically range from 1 millimetre to 1 centimetre and preferably 1 to 2 millimetres in diameter. Granules are formed by extrusion, agglomeration or prilling, or are naturally occurring. Examples of such materials are vermiculite, sintered clay, kaolin, attapulgite clay, sawdust and granular carbon. Shell or membrane materials include natural and synthetic rubbers, cellulosic materials, styrene-butadiene copolymers, polyacrylonitriles, polyacrylates, polyesters, polyamides, polyureas, polyurethanes and starch xanthates.

Other useful formulations for agrochemical applications include simple solutions of the active ingredient in a solvent in which it is completely soluble at the desired concentration, such as acetone, alkylated naphthalenes, xylene and other organic solvents. Pressurised sprayers, wherein the active ingredient is dispersed in finely-divided form as a result of vaporisation of a low boiling dispersant solvent carrier, may also be used.

Suitable agricultural adjuvants and carriers that are useful in formulating the compositions of the invention in the formulation types described above are well known to those skilled in the art.

Liquid carriers that can be employed include, for example, water, toluene, xylene, petroleum naphtha, crop oil, acetone, methyl ethyl ketone, cyclohexanone, acetic anhydride, acetonitrile, acetophenone, amyl acetate, 2-butanone, chlorobenzene, cyclohexane, cyclohexanol, alkyl acetates, diacetonalcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, N,N-dimethyl formamide, dimethyl sulfoxide, 1,4-dioxane, dipropylene glycol, dipropylene glycol methyl ether, dipropylene glycol dibenzoate, diproxitol, alkyl pyrrolidinone, ethyl acetate, 2-ethyl hexanol, ethylene carbonate, 1,1,1-trichloroethane, 2-heptanone, alpha pinene, d-limonene, ethylene glycol, ethylene glycol butyl ether, ethylene glycol methyl ether, gamma-butyrolactone, glycerol, glycerol diacetate, glycerol monoacetate, glycerol triacetate, hexadecane, hexylene glycol, isoamyl acetate, isobornyl acetate, isooctane, isophorone, isopropyl benzene, isopropyl myristate, lactic acid, laurylamine, mesityl oxide, methoxy-propanol, methyl isoamyl ketone, methyl isobutyl ketone, methyl laurate, methyl octanoate, methyl oleate, methylene chloride, m-xylene, n-hexane, n-octylamine, octadecanoic acid, octyl amine acetate, oleic acid, oleylamine, o-xylene, phenol, polyethylene glycol (PEG400), propionic acid, propylene glycol, propylene glycol monomethyl ether, p-xylene, toluene, triethyl phosphate, triethylene glycol, xylene sulfonic acid, paraffin, mineral oil, trichloroethylene, perchloroethylene, ethyl acetate, amyl acetate, butyl acetate, methanol, ethanol, isopropanol, and higher molecular weight alcohols such as amyl alcohol, tetrahydrofurfuryl alcohol, hexanol, octanol, etc., ethylene glycol, propylene glycol, glycerine and N-methyl-2-pyrrolidinone. Water is generally the carrier of choice for the dilution of concentrates.

Suitable solid carriers include, for example, talc, titanium dioxide, pyrophyllite clay, silica, attapulgite clay, kieselguhr, chalk, diatomaxeous earth, lime, calcium carbonate, bentonite clay, fuller's earth, cotton seed hulls, wheat flour, soybean flour, pumice, wood flour, walnut shell flour and lignin.

A broad range of surface-active agents are advantageously employed in both said liquid and solid compositions, especially those designed to be diluted with carrier before application. These agents, when used, normally comprise from 0.1% to 15% by weight of the formulation. They can be anionic, cationic, non-ionic or polymeric in character and can be employed as emulsifying agents, wetting agents, suspending agents or for other purposes. Typical surface active agents include salts of alkyl sulfates, such as diethanolammonium lauryl sulphate; alkylarylsulfonate salts, such as calcium dodecylbenzenesulfonate; alkylphenol-alkylene oxide addition products, such as nonylphenol-C.sub. 18 ethoxylate; alcohol-alkylene oxide addition products, such as tridecyl alcohol-C.sub. 16 ethoxylate; soaps, such as sodium stearate; alkylnaphthalenesulfonate salts, such as sodium dibutylnaphthalenesulfonate; dialkyl esters of sulfosuccinate salts, such as sodium di(2-ethylhexyl) sulfosuccinate; sorbitol esters, such as sorbitol oleate; quaternary amines, such as lauryl trimethylammonium chloride; polyethylene glycol esters of fatty acids, such as polyethylene glycol stearate; block copolymers of ethylene oxide and propylene oxide; and salts of mono and dialkyl phosphate esters.

Other adjuvants commonly utilized in agricultural compositions include crystallisation inhibitors, viscosity modifiers, suspending agents, spray droplet modifiers, pigments, antioxidants, foaming agents, anti-foaming agents, light-blocking agents, compatibilizing agents, antifoam agents, sequestering agents, neutralising agents and buffers, corrosion inhibitors, dyes, odorants, spreading agents, penetration aids, micronutrients, emollients, lubricants and sticking agents.

In addition, further, other biocidally active ingredients or compositions may be combined with the compositions of the invention and used in the methods of the invention and applied simultaneously or sequentially with the compositions of the invention. When applied simultaneously, these further active ingredients may be formulated together with the compositions of the invention or mixed in, for example, the spray tank. These further biocidally active ingredients may be fungicides, herbicides, insecticides, bactericides, acaricides, nematicides and/or plant growth regulators.

Pesticidal agents are referred to herein using their common name are known, for example, from "The Pesticide Manual", 15th Ed., British Crop Protection Council 2009.

In addition, the compositions of the invention may also be applied with one or more systemically acquired resistance inducers ("SAR" inducer). SAR inducers are known and described in, for example, United States Patent No. US 6,919,298 and include, for example, salicylates and the commercial SAR inducer acibenzolar-S-methyl.

The compounds of Formula (I) are normally used in the form of agrochemical compositions and can be applied to the crop area or plant to be treated, simultaneously or in succession with further compounds. These further compounds can be e.g. fertilizers or micronutrient donors or other preparations, which influence the growth of plants. They can also be selective herbicides or non-selective herbicides as well as insecticides, fungicides, bactericides, nematicides, molluscicides or mixtures of several of these preparations, if desired together with further carriers, surfactants or application promoting adjuvants customarily employed in the art of formulation.

The compounds of Formula (I) may be used in the form of (fungicidal) compositions for controlling or protecting against phytopathogenic microorganisms, comprising as active ingredient at least one compound of Formula (I) or of at least one preferred individual compound as defined herein, in free form or in agrochemically usable salt form, and at least one of the above-mentioned adjuvants.

The invention therefore provides a composition, preferably a fungicidal composition, comprising at least one compound Formula (I) an agriculturally acceptable carrier and optionally an adjuvant. An agricultural acceptable carrier is for example a carrier that is suitable for agricultural use. Agricultural carriers are well known in the art. Preferably said composition may comprise at least one or more pesticidally-active compounds, for example an additional fungicidal active ingredient in addition to the compound of Formula (I).

The compound of Formula (I) may be the sole active ingredient of a composition or it may be admixed with one or more additional active ingredients such as a pesticide, fungicide, synergist, herbicide or plant growth regulator where appropriate. An additional active ingredient may, in some cases, result in unexpected synergistic activities.

Examples of suitable additional active ingredients include the following: acycloamino acid fungicides, aliphatic nitrogen fungicides, amide fungicides, anilide fungicides, antibiotic fungicides, aromatic fungicides, arsenical fungicides, aryl phenyl ketone fungicides, benzamide fungicides, benzanilide fungicides, benzimidazole fungicides, benzothiazole fungicides, botanical fungicides, bridged diphenyl fungicides, carbamate fungicides, carbanilate fungicides, conazole fungicides, copper fungicides, dicarboximide fungicides, dinitrophenol fungicides, dithiocarbamate fungicides, dithiolane fungicides, furamide fungicides, furanilide fungicides, hydrazide fungicides, imidazole fungicides, mercury fungicides, morpholine fungicides, organophosphorous fungicides, organotin fungicides, oxathiin fungicides, oxazole fungicides, phenylsulfamide fungicides, polysulfide fungicides, pyrazole fungicides, pyridine fungicides, pyrimidine fungicides, pyrrole fungicides, quaternary ammonium fungicides, quinoline fungicides, quinone fungicides, quinoxaline fungicides, strobilurin fungicides, sulfonanilide fungicides, thiadiazole fungicides, thiazole fungicides, thiazolidine fungicides, thiocarbamate fungicides, thiophene fungicides, triazine fungicides, triazole fungicides, triazolopyrimidine fungicides, urea fungicides, valinamide fungicides, and zinc fungicides.

Examples of suitable additional active ingredients also include the following: 3-difluoromethyl-1-methyl-1H-pyrazole-4-carboxylic acid (9-dichloromethylene-1,2,3,4-tetrahydro-1,4-methano-naphthalen-5-yl)-amide , 3-difluoromethyl-1-methyl-1H-pyrazole-4-carboxylic acid methoxy-[1-methyl-2-(2,4,6-trichlorophenyl)-ethyl]-amide , 1-methyl-3-difluoromethyl-1H-pyrazole-4-carboxylic acid (2-dichloromethylene-3-ethyl-1-methyl-indan-4-yl)-amide (1072957-71-1), 1-methyl-3-difluoromethyl-1H-pyrazole-4-carboxylic acid (4'-methylsulfanyl-biphenyl-2-yl)-amide, 1-methyl-3-difluoromethyl-4H-pyrazole-4-carboxylic acid [2-(2,4-dichloro-phenyl)-2-methoxy-1-methyl-ethyl]-amide, (5-Chloro-2,4-dimethyl-pyridin-3-yl)-(2,3,4-trimethoxy-6-methyl-phenyl)-methanone, (5-Bromo-4-chloro-2-methoxy-pyridin-3-yl)-(2,3,4-trimethoxy-6-methyl-phenyl)-methanone, 2-{2-[(E)-3-(2,6-Dichloro-phenyl)-1-methyl-prop-2-en-(E)-ylideneaminooxymethyl]-phenyl}-2-[(Z)-methoxyimino]-N-methyl-acetamide, 3-[5-(4-Chloro-phenyl)-2,3-dimethyl-isoxazolidin-3-yl]-pyridine, (E)-N-methyl-2- [2- (2, 5-dimethylphenoxymethyl) phenyl]-2-methoxy-iminoacetamide, 4-bromo-2-cyano-N, N-dimethyl-6-trifluoromethylbenzimidazole-1-sulphonamide, a- [N-(3-chloro-2, 6-xylyl)-2-methoxyacetamido]-y-butyrolactone, 4-chloro-2-cyano-N,N - dimethyl-5-p-tolylimidazole-1-sulfonamide, N-allyl-4, 5,-dimethyl-2-trimethylsilylthiophene-3-carboxamide, N- (I-cyano-1, 2-dimethylpropyl)-2- (2, 4-dichlorophenoxy) propionamide, N- (2-methoxy-5-pyridyl)-cyclopropane carboxamide, (.+-.)-cis-1-(4-chlorophenyl)-2-(1H-1,2,4-triazol-1-yl)-cycloheptanol, 2-(1-*tert*-butyl)-1-(2-chlorophenyl)-3-(1,2,4-triazol-1-yl)-propan-2-ol, 2',6'-dibromo-2-methyl-4-trifluoromethoxy-4'-trifluoromethyl-1,3-thiazole- 5-carboxanilide, 1-imidazolyl-1-(4'-chlorophenoxy)-3,3-dimethylbutan-2-one, methyl (E)-2-[2-[6-(2-cyanophenoxy)pyrimidin-4-yloxy]phenyl]3-methoxyacrylate, methyl (E)-2-[2-[6-(2-thioamidophenoxy)pyrimidin-4-yloxy]phenyl]-3-methoxyacrylate, methyl (E)-2-[2-[6-(2-fluorophenoxy)pyrimidin-4-yloxy]phenyl]-3-methoxyacrylate, methyl (E)-2-[2-[6-(2,6-difluorophenoxy)pyrimidin-4-yloxy]phenyl]-3-methoxyacryla te, methyl (E)-2-[2-[3-(pyrimidin-2-yloxy)phenoxy]phenyl]-3-methoxyacrylate, methyl (E)-2-[2-[3-(5-methylpyrimidin-2-yloxy)-phenoxy]phenyl]-3-methoxyacrylate, methyl (E)-2-[2-[3-(phenyl-sulphonyloxy)phenoxy]phenyl-3-methoxyacrylate, methyl (E)-2-[2-[3-(4-nitrophenoxy)phenoxy]phenyl]-3-methoxyacrylate, methyl (E)-2-[2-phenoxyphenyl]-3-methoxyacrylate, methyl (E)-2-[2-(3,5-dimethyl-benzoyl)pyrrol-1-yl]-3-methoxyacrylate, methyl (E)-2-[2-(3-methoxyphenoxy)phenyl]-3-methoxyacrylate, methyl (E)-2[2-(2-phenylethen-1-yl)-phenyl]-3-methoxyacrylate, methyl (E)-2-[2-(3,5-dichlorophenoxy)pyridin-3-yl]-3-methoxyacrylate, methyl (E)-2-(2-(3-(1,1,2,2-tetrafluoroethoxy)phenoxy)phenyl)-3-methoxyacrylate, methyl (E)-2-(2-[3-(alpha-hydroxybenzyl)phenoxy]phenyl)-3-methoxyacrylate, methyl (E)-2-(2-(4-phenoxypyridin-2-yloxy)phenyl)-3-methoxyacrylate, methyl (E)-2-[2-(3-n-propyloxy-phenoxy)phenyl]3-methoxyacrylate, methyl (E)-2-[2-(3-isopropyloxyphenoxy)phenyl]-3-methoxyacrylate, methyl (E)-2-[2-[3-(2-fluorophenoxy)phenoxy]phenyl]-3-methoxyacrylate, methyl (E)-2-[2-(3-ethoxyphenoxy)phenyl]-3-methoxyacrylate, methyl (E)-2-[2-(4-*tert*-butyl-pyridin-2-yloxy)phenyl]-3-methoxyacrylate, methyl (E)-2-[2-[3-(3-cyanophenoxy)phenoxy]phenyl]-3-methoxyacrylate, methyl (E)-2-[2-[(3-methyl-pyridin-2-yloxymethyl)phenyl]-3-methoxyacrylate, methyl (E)-2-[2-[6-(2-methyl-phenoxy)pyrimidin-4-yloxy]phenyl]-3-methoxyacrylate, methyl (E)-2-[2-(5-bromo-pyridin-2-yloxymethyl)phenyl]-3-methoxyacrylate, methyl (E)-2-[2-(3-(3-iodopyridin-2-yloxy)phenoxy)phenyl]-3-methoxyacrylate, methyl (E)-2-[2-[6-(2-chloropyridin-3-yloxy)pyrimidin-4-yloxy]phenyl]-3-methoxyac rylate, methyl (E),(E)-2-[2-(5,6-dimethylpyrazin-2-ylmethyloximinomethyl)phenyl]-3-methox yacrylate, methyl (E)-2-{2-[6-(6-methylpyridin-2-yloxy)pyrimidin-4-yloxy]phenyl}-3-methoxy-a crylate, methyl (E),(E)-2-{ 2-(3-methoxyphenyl)methyloximinomethyl]-phenyl}-3-methoxyacrylate, methyl (E)-2-{2-(6-(2-azidophenoxy)-pyrimidin-4-yloxy]phenyl}-3-methoxyacrylate, methyl (E),(E)-2-{2-[6-phenylpyrimidin-4-yl)-methyloximinomethyl]phenyl}-3-methox yacrylate, methyl (E),(E)-2-{2-[(4-chlorophenyl)-methyloximinomethyl]-phenyl}-3-methoxyacryl ate, methyl (E)-2-{2-[6-(2-n-propylphenoxy)-1,3,5-triazin-4-yloxy]phenyl}-3-methoxyacr ylate, methyl (E),(E)-2-{2-[(3-nitrophenyl)methyloximinomethyl]phenyl}-3-methoxyacrylate, 3-chloro-7-(2-aza-2,7,7-trimethyl-oct-3-en-5-ine), 2,6-dichloro-N-(4-trifluoromethylbenzyl)-benzamide, 3-iodo-2-propinyl alcohol, 4-chlorophenyl-3-iodopropargyl formal, 3-bromo-2,3-diiodo-2-propenyl ethylcarbamate, 2,3,3-triiodoallyl alcohol, 3-bromo-2,3-diiodo-2-propenyl alcohol, 3-iodo-2-propinyl n-butylcarbamate, 3-iodo-2-propinyl n-hexylcarbamate, 3-iodo-2-propinyl cyclohexyl-carbamate, 3-iodo-2-propinyl phenylcarbamate; phenol derivatives, such as tribromophenol, tetrachlorophenol, 3-methyl-4-chlorophenol, 3,5-dimethyl-4-chlorophenol, phenoxyethanol, dichlorophene, o-phenylphenol, m-phenylphenol, p-phenylphenol, 2-benzyl-4-chlorophenol, 5-hydroxy-2(5H)-furanone; 4,5-dichlorodithiazolinone, 4,5-benzodithiazolinone, 4,5-trimethylenedithiazolinone, 4,5-dichloro-(3H)-1,2-dithiol-3-one, 3,5-dimethyl-tetrahydro-1,3,5-thiadiazine-2-thione, N-(2-p-chlorobenzoylethyl)-hexaminium chloride, acibenzolar, acypetacs, alanycarb, albendazole, aldimorph, allicin, allyl alcohol, ametoctradin, amisulbrom, amobam, ampropylfos, anilazine, asomate, aureofungin, azaconazole, azafendin, azithiram, azoxystrobin, barium polysulfide, benalaxyl, benalaxyl-M, benodanil, benomyl, benquinox, bentaluron, benthiavalicarb, benthiazole, benzalkonium chloride, benzamacril, benzamorf, benzohydroxamic acid, benzovindiflupyr, berberine, bethoxazin, biloxazol, binapacryl, biphenyl, bitertanol, bithionol, bixafen, blasticidin-S, boscalid, bromothalonil, bromuconazole, bupirimate, buthiobate, butylamine calcium polysulfide, captafol, captan, carbamorph, carbendazim, carbendazim chlorhydrate, carboxin, carpropamid, carvone, CGA41396, CGA41397, chinomethionate, chitosan, chlobenthiazone, chloraniformethan, chloranil, chlorfenazole, chloroneb, chloropicrin, chlorothalonil, chlorozolinate, chlozolinate, climbazole, clotrimazole, clozylacon, copper containing compounds such as copper acetate, copper carbonate, copper hydroxide, copper naphthenate, copper oleate, copper oxychloride, copper oxyquinolate, copper silicate, copper sulphate, copper tallate, copper zinc chromate and Bordeaux mixture, cresol, cufraneb, cuprobam, cuprous oxide, cyazofamid, cyclafuramid, cycloheximide, cyflufenamid, cymoxanil, cypendazole, cyproconazole, cyprodinil, dazomet, debacarb, decafentin, dehydroacetic acid, di-2-pyridyl disulphide 1, 1'-dioxide, dichlofluanid, diclomezine, dichlone, dicloran, dichlorophen, dichlozoline, diclobutrazol, diclocymet, diethofencarb, difenoconazole, difenzoquat, diflumetorim, O, O-di-iso-propyl-S-benzyl thiophosphate, dimefluazole, dimetachlone, dimetconazole, dimethomorph, dimethirimol, diniconazole, diniconazole-M, dinobuton, dinocap, dinocton, dinopenton, dinosulfon, dinoterbon, diphenylamine, dipyrithione, disulfiram, ditalimfos, dithianon, dithioether, dodecyl dimethyl ammonium chloride, dodemorph, dodicin, dodine, doguadine, drazoxolon, edifenphos, enestroburin, epoxiconazole, etaconazole, etem, ethaboxam, ethirimol, ethoxyquin, ethilicin, ethyl (Z)-N-benzyl-N ([methyl (methyl-thioethylideneamino- oxycarbonyl) amino] thio)-β-alaninate, etridiazole, famoxadone, fenamidone, fenaminosulf, fenapanil, fenarimol, fenbuconazole, fenfuram, fenhexamid, fenitropan, fenoxanil, fenpiclonil, fenpicoxamid, fenpropidin, fenpropimorph, fenpyrazamine, fentin acetate, fentin hydroxide, ferbam, ferimzone, fluazinam, fludioxonil, flumetover, flumorph, flupicolide, fluopyram, fluoroimide, fluotrimazole, fluoxastrobin, fluquinconazole, flusilazole, flusulfamide, flutanil, flutolanil, flutriafol, fluxapyroxad, folpet, formaldehyde, fosetyl, fuberidazole, furalaxyl, furametpyr, furcarbanil, furconazole, furfural, furmecyclox, furophanate, glyodin, griseofulvin, guazatine, halacrinate, hexa chlorobenzene, hexachlorobutadiene, hexachlorophene, hexaconazole, hexylthiofos, hydrargaphen, hydroxyisoxazole, hymexazole, imazalil, imazalil sulphate, imibenconazole, iminoctadine, iminoctadine triacetate, inezin, iodocarb, ipconazole, ipfentrifluconazole, iprobenfos, iprodione, iprovalicarb, isopropanyl butyl carbamate, isoprothiolane, isopyrazam, isotianil, isovaledione, izopamfos, kasugamycin, kresoxim-methyl, LY186054, LY211795, LY248908, mancozeb, mandipropamid, maneb, mebenil, mecarbinzid, mefenoxam, mefentrifluconazole, mepanipyrim, mepronil, mercuric chloride, mercurous chloride, meptyldinocap, metalaxyl, metalaxyl-M, metam, metazoxolon, metconazole, methasulfocarb, methfuroxam, methyl bromide, methyl iodide, methyl isothiocyanate, metiram, metiram-zinc, metominostrobin, metrafenone, metsulfovax, milneb, moroxydine, myclobutanil, myclozolin, nabam, natamycin, neoasozin, nickel dimethyldithiocarbamate, nitrostyrene, nitrothal-isopropyl, nuarimol, octhilinone, ofurace, organomercury compounds, orysastrobin, osthol, oxadixyl, oxasulfuron, oxine-copper, oxolinic acid, oxpoconazole, oxycarboxin, parinol, pefurazoate, penconazole, pencycuron, penflufen, pentachlorophenol, penthiopyrad, phenamacril, phenazin oxide, phosdiphen, phosetyl-Al, phosphorus acids, phthalide, picoxystrobin, piperalin, polycarbamate, polyoxin D, polyoxrim, polyram, probenazole, prochloraz, procymidone, propamidine, propamocarb, propiconazole, propineb, propionic acid, proquinazid, prothiocarb, prothioconazole, pydiflumetofen, pyracarbolid, pyraclostrobin, pyrametrostrobin, pyraoxystrobin, pyrazophos, pyribencarb, pyridinitril, pyrifenox, pyrimethanil, pyriofenone, pyroquilon, pyroxychlor, pyroxyfur, pyrrolnitrin, quaternary ammonium compounds, quinacetol, quinazamid, quinconazole, quinomethionate, quinoxyfen, quintozene, rabenzazole, santonin, sedaxane, silthiofam, simeconazole, sipconazole, sodium pentachlorophenate, spiroxamine, streptomycin, sulphur, sultropen, tebuconazole, tebfloquin, tecloftalam, tecnazene, tecoram, tetraconazole, thiabendazole, thiadifluor, thicyofen, thifluzamide, 2-(thiocyanomethylthio) benzothiazole, thiophanate-methyl, thioquinox, thiram, tiadinil, timibenconazole, tioxymid, tolclofos-methyl, tolylfluanid, triadimefon, triadimenol, triamiphos, triarimol, triazbutil, triazoxide, tricyclazole, tridemorph, trifloxystrobin, triflumazole, triforine, triflumizole, triticonazole, uniconazole, urbacide, validamycin, valifenalate, vapam, vinclozolin, zarilamid, zineb, ziram, and zoxamide.

The compounds of the invention may also be used in combination with anthelmintic agents. Such anthelmintic agents include, compounds selected from the macrocyclic lactone class of compounds such as ivermectin, avermectin, abamectin, emamectin, eprinomectin, doramectin, selamectin, moxidectin, nemadectin and milbemycin derivatives as described in EP- 357460, EP-444964 and EP-594291. Additional anthelmintic agents include semisynthetic and biosynthetic avermectin/milbemycin derivatives such as those described in US-5015630, WO-9415944 and WO-9522552. Additional anthelmintic agents include the benzimidazoles such as albendazole, cambendazole, fenbendazole, flubendazole, mebendazole, oxfendazole, oxibendazole, parbendazole, and other members of the class. Additional anthelmintic agents include imidazothiazoles and tetrahydropyrimidines such as tetramisole, levamisole, pyrantel pamoate, oxantel or morantel. Additional anthelmintic agents include flukicides, such as triclabendazole and clorsulon and the cestocides, such as praziquantel and epsiprantel.

The compounds of the invention may be used in combination with derivatives and analogues of the paraherquamide/marcfortine class of anthelmintic agents, as well as the antiparasitic oxazolines such as those disclosed in US-5478855, US- 4639771 and DE-19520936.

The compounds of the invention may be used in combination with derivatives and analogues of the general class of dioxomorpholine antiparasitic agents as described in WO 96/15121 and also with anthelmintic active cyclic depsipeptides such as those described in WO 96/11945, WO 93/19053, WO 93/25543, EP 0 626 375, EP 0 382 173, WO 94/19334, EP 0 382 173, and EP 0 503 538.

The compounds of the invention may be used in combination with other ectoparasiticides; for example, fipronil; pyrethroids; organophosphates; insect growth regulators such as lufenuron; ecdysone agonists such as tebufenozide and the like; neonicotinoids such as imidacloprid and the like.

The compounds of the invention may be used in combination with terpene alkaloids, for example those described in International Patent Application Publication Numbers WO 95/19363 or WO 04/72086, particularly the compounds disclosed therein.

Other examples of such biologically active compounds that the compounds of the invention may be used in combination with include but are not restricted to the following:
Organophosphates: acephate, azamethiphos, azinphos-ethyl, azinphos- methyl, bromophos, bromophos-ethyl, cadusafos, chlorethoxyphos, chlorpyrifos, chlorfenvinphos, chlormephos, demeton, demeton-S-methyl, demeton-S-methyl sulphone, dialifos, diazinon, dichlorvos, dicrotophos, dimethoate, disulfoton, ethion, ethoprophos, etrimfos, famphur, fenamiphos, fenitrothion, fensulfothion, fenthion, flupyrazofos, fonofos, formothion, fosthiazate, heptenophos, isazophos, isothioate, isoxathion, malathion, methacriphos, methamidophos, methidathion, methyl- parathion, mevinphos, monocrotophos, naled, omethoate, oxydemeton-methyl, paraoxon, parathion, parathion-methyl, phenthoate, phosalone, phosfolan, phosphocarb, phosmet, phosphamidon, phorate, phoxim, pirimiphos, pirimiphos- methyl, profenofos, propaphos, proetamphos, prothiofos, pyraclofos, pyridapenthion, quinalphos, sulprophos, temephos, terbufos, tebupirimfos, tetrachlorvinphos, thimeton, triazophos, trichlorfon, vamidothion.

Carbamates: alanycarb, aldicarb, 2-sec-butylphenyl methylcarbamate, benfuracarb, carbaryl, carbofuran, carbosulfan, cloethocarb, ethiofencarb, fenoxycarb, fenthiocarb, furathiocarb, HCN-801, isoprocarb, indoxacarb, methiocarb, methomyl, 5-methyl-m-cumenylbutyryl(methyl)carbamate, oxamyl, pirimicarb, propoxur, thiodicarb, thiofanox, triazamate, UC-51717.

Pyrethroids: acrinathin, allethrin, alphametrin, 5-benzyl-3-furylmethyl (E)-(1 R)-cis-2,2-dimethyl-3-(2-oxothiolan-3-ylidenemethyl)cyclopropanecarboxylate, bifenthrin, beta-cyfluthrin, cyfluthrin, a-cypermethrin, beta-cypermethrin, bioallethrin, bioallethrin((S)-cyclopentylisomer), bioresmethrin, bifenthrin, NCI-85193, cycloprothrin, cyhalothrin, cythithrin, cyphenothrin, deltamethrin, empenthrin, esfenvalerate, ethofenprox, fenfluthrin, fenpropathrin, fenvalerate, flucythrinate, flumethrin, fluvalinate (D isomer), imiprothrin, cyhalothrin, lambda-cyhalothrin, permethrin, phenothrin, prallethrin, pyrethrins (natural products), resmethrin, tetramethrin, transfluthrin, theta-cypermethrin, silafluofen, t-fluvalinate, tefluthrin, tralomethrin, Zeta-cypermethrin.

Arthropod growth regulators: a) chitin synthesis inhibitors: benzoylureas: chlorfluazuron, diflubenzuron, fluazuron, flucycloxuron, flufenoxuron, hexaflumuron, lufenuron, novaluron, teflubenzuron, triflumuron, buprofezin, diofenolan, hexythiazox, etoxazole, chlorfentazine; b) ecdysone antagonists: halofenozide, methoxyfenozide, tebufenozide; c) juvenoids: pyriproxyfen, methoprene (including S-methoprene), fenoxycarb; d) lipid biosynthesis inhibitors: spirodiclofen.

Other antiparasitics: acequinocyl, amitraz, AKD-1022, ANS-118, azadirachtin, Bacillus thuringiensis, bensultap, bifenazate, binapacryl, bromopropylate, BTG-504, BTG-505, camphechlor, cartap, chlorobenzilate, chlordimeform, chlorfenapyr, chromafenozide, clothianidine, cyromazine, diacloden, diafenthiuron, DBI-3204, dinactin, dihydroxymethyldihydroxypyrrolidine, dinobuton, dinocap, endosulfan, ethiprole, ethofenprox, fenazaquin, flumite, MTI- 800, fenpyroximate, fluacrypyrim, flubenzimine, flubrocythrinate, flufenzine, flufenprox, fluproxyfen, halofenprox, hydramethylnon, IKI-220, kanemite, NC-196, neem guard, nidinorterfuran, nitenpyram, SD-35651, WL-108477, pirydaryl, propargite, protrifenbute, pymethrozine, pyridaben, pyrimidifen, NC-1111, R-195,RH-0345, RH-2485, RYI-210, S-1283, S-1833, SI-8601, silafluofen, silomadine, spinosad, tebufenpyrad, tetradifon, tetranactin, thiacloprid, thiocyclam, thiamethoxam, tolfenpyrad, triazamate, triethoxyspinosyn, trinactin, verbutin, vertalec, YI-5301.

Biological agents: Bacillus thuringiensis ssp aizawai, kurstaki, Bacillus thuringiensis delta endotoxin, baculovirus, entomopathogenic bacteria, virus and fungi.

Bactericides: chlortetracycline, oxytetracycline, streptomycin.

Other biological agents: enrofloxacin, febantel, penethamate, moloxicam, cefalexin, kanamycin, pimobendan, clenbuterol, omeprazole, tiamulin, benazepril, pyriprole, cefquinome, florfenicol, buserelin, cefovecin, tulathromycin, ceftiour, carprofen, metaflumizone, praziquarantel, triclabendazole.

The following mixtures of the compounds of Formula (I) with active ingredients are preferred. The abbreviation "TX" means one compound selected from the group consisting of the compounds as represented in Tables 1.2 and 2.2 (below), or a compound 1.3, 1.5, 1.9, 1.18, 1.22, 1.40, 1.57, 1.58, 1.88 to 1.95, 1.101, 1.111 or 1.112 listed in Table T1 (below), or compound 4.2 listed in Table T4 (below).
an adjuvant selected from the group of substances consisting of petroleum oils (alternative name) (628) + TX,
an acaricide selected from the group of substances consisting of 1,1-bis(4-chlorophenyl)-2-ethoxyethanol (IUPAC name) (910) + TX, 2,4-dichlorophenyl benzenesulfonate (IUPAC/Chemical Abstracts name) (1059) + TX, 2-fluoro-*N*-methyl-*N*-1-naphthylacetamide (IUPAC name) (1295) + TX, 4-chlorophenyl phenyl sulfone (IUPAC name) (981) + TX, abamectin (1) + TX, acequinocyl (3) + TX, acetoprole [CCN] + TX, acrinathrin (9) + TX, aldicarb (16) + TX, aldoxycarb (863) + TX, alpha-cypermethrin (202) + TX, amidithion (870) + TX, amidoflumet [CCN] + TX, amidothioate (872) + TX, amiton (875) + TX, amiton hydrogen oxalate (875) + TX, amitraz (24) + TX, aramite (881) + TX, arsenous oxide (882) + TX, AVI 382 (compound code) + TX, AZ 60541 (compound code) + TX, azinphos-ethyl (44) + TX, azinphos-methyl (45) + TX, azobenzene (IUPAC name) (888) + TX, azocyclotin (46) + TX, azothoate (889) + TX, benomyl (62) + TX, benoxafos (alternative name) [CCN] + TX, benzoximate (71) + TX, benzyl benzoate (IUPAC name) [CCN] + TX, bifenazate (74) + TX, bifenthrin (76) + TX, binapacryl (907) + TX, brofenvalerate (alternative name) + TX, bromocyclen (918) + TX, bromophos (920) + TX, bromophos-ethyl (921) + TX, bromopropylate (94) + TX, buprofezin (99) + TX, butocarboxim (103) + TX, butoxycarboxim (104) + TX, butylpyridaben (alternative name) + TX, calcium polysulfide (IUPAC name) (111) + TX, camphechlor (941) + TX, carbanolate (943) + TX, carbaryl (115) + TX, carbofuran (118) + TX, carbophenothion (947) + TX, CGA 50'439 (development code) (125) + TX, chinomethionat (126) + TX, chlorbenside (959) + TX, chlordimeform (964) + TX, chlordimeform hydrochloride (964) + TX, chlorfenapyr (130) + TX, chlorfenethol (968) + TX, chlorfenson (970) + TX, chlorfensulfide (971) + TX, chlorfenvinphos (131) + TX, chlorobenzilate (975) + TX, chloromebuform (977) + TX, chloromethiuron (978) + TX, chloropropylate (983) + TX, chlorpyrifos (145) + TX, chlorpyrifos-methyl (146) + TX, chlorthiophos (994) + TX, cinerin I (696) + TX, cinerin II (696) + TX, cinerins (696) + TX, clofentezine (158) + TX, closantel (alternative name) [CCN] + TX, coumaphos (174) + TX, crotamiton (alternative name) [CCN] + TX, crotoxyphos (1010) + TX, cufraneb (1013) + TX, cyanthoate (1020) + TX, cyflumetofen (CAS Reg. No.: 400882-07-7) + TX, cyhalothrin (196) + TX, cyhexatin (199) + TX, cypermethrin (201) + TX, DCPM (1032) + TX, DDT (219) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulfon (1039) + TX, diafenthiuron (226) + TX, dialifos (1042) + TX, diazinon (227) + TX, dichlofluanid (230) + TX, dichlorvos (236) + TX, dicliphos (alternative name) + TX, dicofol (242) + TX, dicrotophos (243) + TX, dienochlor (1071) + TX, dimefox (1081) + TX, dimethoate (262) + TX, dinactin (alternative name) (653) + TX, dinex (1089) + TX, dinex-diclexine (1089) + TX, dinobuton (269) + TX, dinocap (270) + TX, dinocap-4 [CCN] + TX, dinocap-6 [CCN] + TX, dinocton (1090) + TX, dinopenton (1092) + TX, dinosulfon (1097) + TX, dinoterbon (1098) + TX, dioxathion (1102) + TX, diphenyl sulfone (IUPAC name) (1103) + TX, disulfiram (alternative name) [CCN] + TX, disulfoton (278) + TX, DNOC (282) + TX, dofenapyn (1113) + TX, doramectin (alternative name) [CCN] + TX, endosulfan (294) + TX, endothion (1121) + TX, EPN (297) + TX, eprinomectin (alternative name) [CCN] + TX, ethion (309) + TX, ethoate-methyl (1134) + TX, etoxazole (320) + TX, etrimfos (1142) + TX, fenazaflor (1147) + TX, fenazaquin (328) + TX, fenbutatin oxide (330) + TX, fenothiocarb (337) + TX, fenpropathrin (342) + TX, fenpyrad (alternative name) + TX, fenpyroximate (345) + TX, fenson (1157) + TX, fentrifanil (1161) + TX, fenvalerate (349) + TX, fipronil (354) + TX, fluacrypyrim (360) + TX, fluazuron (1166) + TX, flubenzimine (1167) + TX, flucycloxuron (366) + TX, flucythrinate (367) + TX, fluenetil (1169) + TX, flufenoxuron (370) + TX, flumethrin (372) + TX, fluorbenside (1174) + TX, fluvalinate (1184) + TX, FMC 1137 (development code) (1185) + TX, formetanate (405) + TX, formetanate hydrochloride (405) + TX, formothion (1192) + TX, formparanate (1193) + TX, gamma-HCH (430) + TX, glyodin (1205) + TX, halfenprox (424) + TX, heptenophos (432) + TX, hexadecyl cyclopropanecarboxylate (IUPAC/Chemical Abstracts name) (1216) + TX, hexythiazox (441) + TX, iodomethane (IUPAC name) (542) + TX, isocarbophos (alternative name) (473) + TX, isopropyl *O*-(methoxyaminothiophosphoyl)salicylate (IUPAC name) (473) + TX, ivermectin (alternative name) [CCN] + TX, jasmolin I (696) + TX, jasmolin II (696) + TX, jodfenphos (1248) + TX, lindane (430) + TX, lufenuron (490) + TX, malathion (492) + TX, malonoben (1254) + TX, mecarbam (502) + TX, mephosfolan (1261) + TX, mesulfen (alternative name) [CCN] + TX, methacrifos (1266) + TX, methamidophos (527) + TX, methidathion (529) + TX, methiocarb (530) + TX, methomyl (531) + TX, methyl bromide (537) + TX, metolcarb (550) + TX, mevinphos (556) + TX, mexacarbate (1290) + TX, milbemectin (557) + TX, milbemycin oxime (alternative name) [CCN] + TX, mipafox (1293) + TX, monocrotophos (561) + TX, morphothion (1300) + TX, moxidectin (alternative name) [CCN] + TX, naled (567) + TX, NC-184 (compound code) + TX, NC-512 (compound code) + TX, nifluridide (1309) + TX, nikkomycins (alternative name) [CCN] + TX, nitrilacarb (1313) + TX, nitrilacarb 1:1 zinc chloride complex (1313) + TX, NNI-0101 (compound code) + TX, NNI-0250 (compound code) + TX, omethoate (594) + TX, oxamyl (602) + TX, oxydeprofos (1324) + TX, oxydisulfoton (1325) + TX, pp'-DDT (219) + TX, parathion (615) + TX, permethrin (626) + TX, petroleum oils (alternative name) (628) + TX, phenkapton (1330) + TX, phenthoate (631) + TX, phorate (636) + TX, phosalone (637) + TX, phosfolan (1338) + TX, phosmet (638) + TX, phosphamidon (639) + TX, phoxim (642) + TX, pirimiphos-methyl (652) + TX, polychloroterpenes (traditional name) (1347) + TX, polynactins (alternative name) (653) + TX, proclonol (1350) + TX, profenofos (662) + TX, promacyl (1354) + TX, propargite (671) + TX, propetamphos (673) + TX, propoxur (678) + TX, prothidathion (1360) + TX, prothoate (1362) + TX, pyrethrin I (696) + TX, pyrethrin II (696) + TX, pyrethrins (696) + TX, pyridaben (699) + TX, pyridaphenthion (701) + TX, pyrimidifen (706) + TX, pyrimitate (1370) + TX, quinalphos (711) + TX, quintiofos (1381) + TX, R-1492 (development code) (1382) + TX, RA-17 (development code) (1383) + TX, rotenone (722) + TX, schradan (1389) + TX, sebufos (alternative name) + TX, selamectin (alternative name) [CCN] + TX, SI-0009 (compound code) + TX, sophamide (1402) + TX, spirodiclofen (738) + TX, spiromesifen (739) + TX, SSI-121 (development code) (1404) + TX, sulfiram (alternative name) [CCN] + TX, sulfluramid (750) + TX, sulfotep (753) + TX, sulfur (754) + TX, SZI-121 (development code) (757) + TX, tau-fluvalinate (398) + TX, tebufenpyrad (763) + TX, TEPP (1417) + TX, terbam (alternative name) + TX, tetrachlorvinphos (777) + TX, tetradifon (786) + TX, tetranactin (alternative name) (653) + TX, tetrasul (1425) + TX, thiafenox (alternative name) + TX, thiocarboxime (1431) + TX, thiofanox (800) + TX, thiometon (801) + TX, thioquinox (1436) + TX, thuringiensin (alternative name) [CCN] + TX, triamiphos (1441) + TX, triarathene (1443) + TX, triazophos (820) + TX, triazuron (alternative name) + TX, trichlorfon (824) + TX, trifenofos (1455) + TX, trinactin (alternative name) (653) + TX, vamidothion (847) + TX, vaniliprole [CCN] and YI-5302 (compound code) + TX,
an algicide selected from the group of substances consisting of bethoxazin [CCN] + TX, copper dioctanoate (IUPAC name) (170) + TX, copper sulfate (172) + TX, cybutryne [CCN] + TX, dichlone (1052) + TX, dichlorophen (232) + TX, endothal (295) + TX, fentin (347) + TX, hydrated lime [CCN] + TX, nabam (566) + TX, quinoclamine (714) + TX, quinonamid (1379) + TX, simazine (730) + TX, triphenyltin acetate (IUPAC name) (347) and triphenyltin hydroxide (IUPAC name) (347) + TX,
an anthelmintic selected from the group of substances consisting of abamectin (1) + TX, crufomate (1011) + TX, doramectin (alternative name) [CCN] + TX, emamectin (291) + TX, emamectin benzoate (291) + TX, eprinomectin (alternative name) [CCN] + TX, ivermectin (alternative name) [CCN] + TX, milbemycin oxime (alternative name) [CCN] + TX, moxidectin (alternative name) [CCN] + TX, piperazine [CCN] + TX, selamectin (alternative name) [CCN] + TX, spinosad (737) and thiophanate (1435) + TX,
an avicide selected from the group of substances consisting of chloralose (127) + TX, endrin (1122) + TX, fenthion (346) + TX, pyridin-4-amine (IUPAC name) (23) and strychnine (745) + TX,
a bactericide selected from the group of substances consisting of 1-hydroxy-1*H*-pyridine-2-thione (IUPAC name) (1222) + TX, 4-(quinoxalin-2-ylamino)benzenesulfonamide (IUPAC name) (748) + TX, 8-hydroxyquinoline sulfate (446) + TX, bronopol (97) + TX, copper dioctanoate (IUPAC name) (170) + TX, copper hydroxide (IUPAC name) (169) + TX, cresol [CCN] + TX, dichlorophen (232) + TX, dipyrithione (1105) + TX, dodicin (1112) + TX, fenaminosulf (1144) + TX, formaldehyde (404) + TX, hydrargaphen (alternative name) [CCN] + TX, kasugamycin (483) + TX, kasugamycin hydrochloride hydrate (483) + TX, nickel bis(dimethyldithiocarbamate) (IUPAC name) (1308) + TX, nitrapyrin (580) + TX, octhilinone (590) + TX, oxolinic acid (606) + TX, oxytetracycline (611) + TX, potassium hydroxyquinoline sulfate (446) + TX, probenazole (658) + TX, streptomycin (744) + TX, streptomycin sesquisulfate (744) + TX, tecloftalam (766) + TX, and thiomersal (alternative name) [CCN] + TX,
a biological agent selected from the group of substances consisting of *Adoxophyes orana* GV (alternative name) (12) + TX, *Agrobacterium radiobacter* (alternative name) (13) + TX, *Amblyseius* spp. (alternative name) (19) + TX, *Anagrapha falcifera* NPV (alternative name) (28) + TX, *Anagrus atomus* (alternative name) (29) + TX, *Aphelinus abdominalis* (alternative name) (33) + TX, *Aphidius colemani* (alternative name) (34) + TX, *Aphidoletes aphidimyza* (alternative name) (35) + TX, *Autographa californica* NPV (alternative name) (38) + TX, *Bacillus firmus* (alternative name) (48) + TX, *Bacillus sphaericus* Neide (scientific name) (49) + TX, *Bacillus thuringiensis* Berliner (scientific name) (51) + TX, *Bacillus thuringiensis* subsp. *aizawai* (scientific name) (51) + TX, *Bacillus thuringiensis* subsp. *israelensis* (scientific name) (51) + TX, *Bacillus thuringiensis* subsp. *japonensis* (scientific name) (51) + TX, *Bacillus thuringiensis* subsp. *kurstaki* (scientific name) (51) + TX, *Bacillus thuringiensis* subsp. *tenebrionis* (scientific name) (51) + TX, *Beauveria bassiana* (alternative name) (53) + TX, *Beauveria brongniartii* (alternative name) (54) + TX, *Chrysoperla carnea* (alternative name) (151) + TX, *Cryptolaemus montrouzieri* (alternative name) (178) + TX, *Cydia pomonella* GV (alternative name) (191) + TX, *Dacnusa sibirica* (alternative name) (212) + TX, *Diglyphus isaea* (alternative name) (254) + TX, *Encarsia formosa* (scientific name) (293) + TX, *Eretmocerus eremicus* (alternative name) (300) + TX, *Helicoverpa zea* NPV (alternative name) (431) + TX, *Heterorhabditis bacteriophora* and H. *megidis* (alternative name) (433) + TX, *Hippodamia convergens* (alternative name) (442) + TX, *Leptomastix dactylopii* (alternative name) (488) + TX, *Macrolophus caliginosus* (alternative name) (491) + TX, *Mamestra brassicae* NPV (alternative name) (494) + TX, *Metaphycus helvolus* (alternative name) (522) + TX, *Metarhizium anisopliae* var. *acridum* (scientific name) (523) + TX, *Metarhizium anisopliae* var. *anisopliae* (scientific name) (523) + TX, *Neodiprion sertifer* NPV and *N. lecontei* NPV (alternative name) (575) + TX, *Orius* spp. (alternative name) (596) + TX, *Paecilomyces fumosoroseus* (alternative name) (613) + TX, *Phytoseiulus persimilis* (alternative name) (644) + TX, *Spodoptera exigua* multicapsid nuclear polyhedrosis virus (scientific name) (741) + TX, *Steinernema bibionis* (alternative name) (742) + TX, *Steinernema carpocapsae* (alternative name) (742) + TX, *Steinernema feltiae* (alternative name) (742) + TX, *Steinernema glaseri* (alternative name) (742) + TX, *Steinernema riobrave* (alternative name) (742) + TX, *Steinernema riobravis* (alternative name) (742) + TX, *Steinernema scapterisci* (alternative name) (742) + TX, *Steinernema* spp. (alternative name) (742) + TX, *Trichogramma* spp. (alternative name) (826) + TX, *Typhlodromus occidentalis* (alternative name) (844) and *Verticillium lecanii* (alternative name) (848) + TX, *bacillus subtilis* var. amyloliquefaciens Strain FZB24 (available from Novozymes Biologicals Inc., 5400 Corporate Circle, Salem, VA 24153, U.S.A. and known under the trade name Taegro^{®}) + TX,
a soil sterilant selected from the group of substances consisting of iodomethane (IUPAC name) (542) and methyl bromide (537) + TX,
a chemosterilant selected from the group of substances consisting of apholate [CCN] + TX, bisazir (alternative name) [CCN] + TX, busulfan (alternative name) [CCN] + TX, diflubenzuron (250) + TX, dimatif (alternative name) [CCN] + TX, hemel [CCN] + TX, hempa [CCN] + TX, metepa [CCN] + TX, methiotepa [CCN] + TX, methyl apholate [CCN] + TX, morzid [CCN] + TX, penfluron (alternative name) [CCN] + TX, tepa [CCN] + TX, thiohempa (alternative name) [CCN] + TX, thiotepa (alternative name) [CCN] + TX, tretamine (alternative name) [CCN] and uredepa (alternative name) [CCN] + TX,
an insect pheromone selected from the group of substances consisting of (*E*)-dec-5-en-1-yl acetate with (*E*)-dec-5-en-1-ol (IUPAC name) (222) + TX, (*E*)-tridec-4-en-1-yl acetate (IUPAC name) (829) + TX, (*E*)-6-methylhept-2-en-4-ol (IUPAC name) (541) + TX, (E,Z)-tetradeca-4,10-dien-1-yl acetate (IUPAC name) (779) + TX, (Z)-dodec-7-en-1-yl acetate (IUPAC name) (285) + TX, (Z)-hexadec-11-enal (IUPAC name) (436) + TX, (Z)-hexadec-11-en-1-yl acetate (IUPAC name) (437) + TX, (*Z*)-hexadec-13-en-11-yn-1-yl acetate (IUPAC name) (438) + TX, (*Z*)-icos-13-en-10-one (IUPAC name) (448) + TX, (Z)-tetradec-7-en-1-al (IUPAC name) (782) + TX, (*Z*)-tetradec-9-en-1-ol (IUPAC name) (783) + TX, (Z)-tetradec-9-en-1-yl acetate (IUPAC name) (784) + TX, (7*E*,9*Z*)-dodeca-7,9-dien-1-yl acetate (IUPAC name) (283) + TX, (9*Z*,11*E*)-tetradeca-9,11-dien-1-yl acetate (IUPAC name) (780) + TX, (9*Z*,12*E*)-tetradeca-9,12-dien-1-yl acetate (IUPAC name) (781) + TX, 14-methyloctadec-1-ene (IUPAC name) (545) + TX, 4-methylnonan-5-ol with 4-methylnonan-5-one (IUPAC name) (544) + TX, alpha-multistriatin (alternative name) [CCN] + TX, brevicomin (alternative name) [CCN] + TX, codlelure (alternative name) [CCN] + TX, codlemone (alternative name) (167) + TX, cuelure (alternative name) (179) + TX, disparlure (277) + TX, dodec-8-en-1-yl acetate (IUPAC name) (286) + TX, dodec-9-en-1-yl acetate (IUPAC name) (287) + TX, dodeca-8 + TX, 10-dien-1-yl acetate (IUPAC name) (284) + TX, dominicalure (alternative name) [CCN] + TX, ethyl 4-methyloctanoate (IUPAC name) (317) + TX, eugenol (alternative name) [CCN] + TX, frontalin (alternative name) [CCN] + TX, gossyplure (alternative name) (420) + TX, grandlure (421) + TX, grandlure I (alternative name) (421) + TX, grandlure II (alternative name) (421) + TX, grandlure III (alternative name) (421) + TX, grandlure IV (alternative name) (421) + TX, hexalure [CCN] + TX, ipsdienol (alternative name) [CCN] + TX, ipsenol (alternative name) [CCN] + TX, japonilure (alternative name) (481) + TX, lineatin (alternative name) [CCN] + TX, litlure (alternative name) [CCN] + TX, looplure (alternative name) [CCN] + TX, medlure [CCN] + TX, megatomoic acid (alternative name) [CCN] + TX, methyl eugenol (alternative name) (540) + TX, muscalure (563) + TX, octadeca-2,13-dien-1-yl acetate (IUPAC name) (588) + TX, octadeca-3,13-dien-1-yl acetate (IUPAC name) (589) + TX, orfralure (alternative name) [CCN] + TX, oryctalure (alternative name) (317) + TX, ostramone (alternative name) [CCN] + TX, siglure [CCN] + TX, sordidin (alternative name) (736) + TX, sulcatol (alternative name) [CCN] + TX, tetradec-11-en-1-yl acetate (IUPAC name) (785) + TX, trimedlure (839) + TX, trimedlure A (alternative name) (839) + TX, trimedlure B₁ (alternative name) (839) + TX, trimedlure B₂ (alternative name) (839) + TX, trimedlure C (alternative name) (839) and trunc-call (alternative name) [CCN] + TX,
an insect repellent selected from the group of substances consisting of 2-(octylthio)ethanol (IUPAC name) (591) + TX, butopyronoxyl (933) + TX, butoxy(polypropylene glycol) (936) + TX, dibutyl adipate (IUPAC name) (1046) + TX, dibutyl phthalate (1047) + TX, dibutyl succinate (IUPAC name) (1048) + TX, diethyltoluamide [CCN] + TX, dimethyl carbate [CCN] + TX, dimethyl phthalate [CCN] + TX, ethyl hexanediol (1137) + TX, hexamide [CCN] + TX, methoquin-butyl (1276) + TX, methylneodecanamide [CCN] + TX, oxamate [CCN] and picaridin [CCN] + TX,
an insecticide selected from the group of substances consisting of 1-dichloro-1-nitroethane (IUPAC/ChemicalAbstracts name) (1058) + TX, 1,1-dichloro-2,2-bis(4-ethylphenyl)ethane (IUPAC name) (1056), + TX, 1,2-dichloropropane (IUPAC/ChemicalAbstracts name) (1062) + TX, 1,2-dichloropropane with 1,3-dichloropropene (IUPAC name) (1063) + TX, 1-bromo-2-chloroethane (IUPAC/ChemicalAbstracts name) (916) + TX, 2,2,2-trichloro-1-(3,4-dichlorophenyl)ethyl acetate (IUPAC name) (1451) + TX, 2,2-dichlorovinyl 2-ethylsulfinylethyl methyl phosphate (IUPAC name) (1066) + TX, 2-(1,3-dithiolan-2-yl)phenyl dimethylcarbamate (IUPAC/ Chemical Abstracts name) (1109) + TX, 2-(2-butoxyethoxy)ethyl thiocyanate (IUPAC/ChemicalAbstracts name) (935) + TX, 2-(4,5-dimethyl-1,3-dioxolan-2-yl)phenyl methylcarbamate (IUPAC/ Chemical Abstracts name) (1084) + TX, 2-(4-chloro-3,5-xylyloxy)ethanol (IUPAC name) (986) + TX, 2-chlorovinyl diethyl phosphate (IUPAC name) (984) + TX, 2-imidazolidone (IUPAC name) (1225) + TX, 2-isovalerylindan-1,3-dione (IUPAC name) (1246) + TX, 2-methyl(prop-2-ynyl)aminophenyl methylcarbamate (IUPAC name) (1284) + TX, 2-thiocyanatoethyl laurate (IUPAC name) (1433) + TX, 3-bromo-1-chloroprop-1-ene (IUPAC name) (917) + TX, 3-methyl-1-phenylpyrazol-5-yl dimethylcarbamate (IUPAC name) (1283) + TX, 4-methyl(prop-2-ynyl)amino-3,5-xylyl methylcarbamate (IUPAC name) (1285) + TX, 5,5-dimethyl-3-oxocyclohex-1-enyl dimethylcarbamate (IUPAC name) (1085) + TX, abamectin (1) + TX, acephate (2) + TX, acetamiprid (4) + TX, acethion (alternative name) [CCN] + TX, acetoprole [CCN] + TX, acrinathrin (9) + TX, acrylonitrile (IUPAC name) (861) + TX, alanycarb (15) + TX, aldicarb (16) + TX, aldoxycarb (863) + TX, aldrin (864) + TX, allethrin (17) + TX, allosamidin (alternative name) [CCN] + TX, allyxycarb (866) + TX, alpha-cypermethrin (202) + TX, alpha-ecdysone (alternative name) [CCN] + TX, aluminium phosphide (640) + TX, amidithion (870) + TX, amidothioate (872) + TX, aminocarb (873) + TX, amiton (875) + TX, amiton hydrogen oxalate (875) + TX, amitraz (24) + TX, anabasine (877) + TX, athidathion (883) + TX, AVI 382 (compound code) + TX, AZ 60541 (compound code) + TX, azadirachtin (alternative name) (41) + TX, azamethiphos (42) + TX, azinphos-ethyl (44) + TX, azinphos-methyl (45) + TX, azothoate (889) + TX, *Bacillus thuringiensis* delta endotoxins (alternative name) (52) + TX, barium hexafluorosilicate (alternative name) [CCN] + TX, barium polysulfide (IUPAC/ChemicalAbstracts name) (892) + TX, barthrin [CCN] + TX, Bayer 22/190 (development code) (893) + TX, Bayer 22408 (development code) (894) + TX, bendiocarb (58) + TX, benfuracarb (60) + TX, bensultap (66) + TX, beta-cyfluthrin (194) + TX, beta-cypermethrin (203) + TX, bifenthrin (76) + TX, bioallethrin (78) + TX, bioallethrin S-cyclopentenyl isomer (alternative name) (79) + TX, bioethanomethrin [CCN] + TX, biopermethrin (908) + TX, bioresmethrin (80) + TX, bis(2-chloroethyl) ether (IUPAC name) (909) + TX, bistrifluron (83) + TX, borax (86) + TX, brofenvalerate (alternative name) + TX, bromfenvinfos (914) + TX, bromocyclen (918) + TX, bromo-DDT (alternative name) [CCN] + TX, bromophos (920) + TX, bromophos-ethyl (921) + TX, bufencarb (924) + TX, buprofezin (99) + TX, butacarb (926) + TX, butathiofos (927) + TX, butocarboxim (103) + TX, butonate (932) + TX, butoxycarboxim (104) + TX, butylpyridaben (alternative name) + TX, cadusafos (109) + TX, calcium arsenate [CCN] + TX, calcium cyanide (444) + TX, calcium polysulfide (IUPAC name) (111) + TX, camphechlor (941) + TX, carbanolate (943) + TX, carbaryl (115) + TX, carbofuran (118) + TX, carbon disulfide (IUPAC/Chemical Abstracts name) (945) + TX, carbon tetrachloride (IUPAC name) (946) + TX, carbophenothion (947) + TX, carbosulfan (119) + TX, cartap (123) + TX, cartap hydrochloride (123) + TX, cevadine (alternative name) (725) + TX, chlorbicyclen (960) + TX, chlordane (128) + TX, chlordecone (963) + TX, chlordimeform (964) + TX, chlordimeform hydrochloride (964) + TX, chlorethoxyfos (129) + TX, chlorfenapyr (130) + TX, chlorfenvinphos (131) + TX, chlorfluazuron (132) + TX, chlormephos (136) + TX, chloroform [CCN] + TX, chloropicrin (141) + TX, chlorphoxim (989) + TX, chlorprazophos (990) + TX, chlorpyrifos (145) + TX, chlorpyrifos-methyl (146) + TX, chlorthiophos (994) + TX, chromafenozide (150) + TX, cinerin I (696) + TX, cinerin II (696) + TX, cinerins (696) + TX, cis-resmethrin (alternative name) + TX, cismethrin (80) + TX, clocythrin (alternative name) + TX, cloethocarb (999) + TX, closantel (alternative name) [CCN] + TX, clothianidin (165) + TX, copper acetoarsenite [CCN] + TX, copper arsenate [CCN] + TX, copper oleate [CCN] + TX, coumaphos (174) + TX, coumithoate (1006) + TX, crotamiton (alternative name) [CCN] + TX, crotoxyphos (1010) + TX, crufomate (1011) + TX, cryolite (alternative name) (177) + TX, CS 708 (development code) (1012) + TX, cyanofenphos (1019) + TX, cyanophos (184) + TX, cyanthoate (1020) + TX, cyclethrin [CCN] + TX, cycloprothrin (188) + TX, cyfluthrin (193) + TX, cyhalothrin (196) + TX, cypermethrin (201) + TX, cyphenothrin (206) + TX, cyromazine (209) + TX, cythioate (alternative name) [CCN] + TX, *d*-limonene (alternative name) [CCN] + TX, *d*-tetramethrin (alternative name) (788) + TX, DAEP (1031) + TX, dazomet (216) + TX, DDT (219) + TX, decarbofuran (1034) + TX, deltamethrin (223) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulphon (1039) + TX, diafenthiuron (226) + TX, dialifos (1042) + TX, diamidafos (1044) + TX, diazinon (227) + TX, dicapthon (1050) + TX, dichlofenthion (1051) + TX, dichlorvos (236) + TX, dicliphos (alternative name) + TX, dicresyl (alternative name) [CCN] + TX, dicrotophos (243) + TX, dicyclanil (244) + TX, dieldrin (1070) + TX, diethyl 5-methylpyrazol-3-yl phosphate (IUPAC name) (1076) + TX, diflubenzuron (250) + TX, dilor (alternative name) [CCN] + TX, dimefluthrin [CCN] + TX, dimefox (1081) + TX, dimetan (1085) + TX, dimethoate (262) + TX, dimethrin (1083) + TX, dimethylvinphos (265) + TX, dimetilan (1086) + TX, dinex (1089) + TX, dinex-diclexine (1089) + TX, dinoprop (1093) + TX, dinosam (1094) + TX, dinoseb (1095) + TX, dinotefuran (271) + TX, diofenolan (1099) + TX, dioxabenzofos (1100) + TX, dioxacarb (1101) + TX, dioxathion (1102) + TX, disulfoton (278) + TX, dithicrofos (1108) + TX, DNOC (282) + TX, doramectin (alternative name) [CCN] + TX, DSP (1115) + TX, ecdysterone (alternative name) [CCN] + TX, EI 1642 (development code) (1118) + TX, emamectin (291) + TX, emamectin benzoate (291) + TX, EMPC (1120) + TX, empenthrin (292) + TX, endosulfan (294) + TX, endothion (1121) + TX, endrin (1122) + TX, EPBP (1123) + TX, EPN (297) + TX, epofenonane (1124) + TX, eprinomectin (alternative name) [CCN] + TX, esfenvalerate (302) + TX, etaphos (alternative name) [CCN] + TX, ethiofencarb (308) + TX, ethion (309) + TX, ethiprole (310) + TX, ethoate-methyl (1134) + TX, ethoprophos (312) + TX, ethyl formate (IUPAC name) [CCN] + TX, ethyl-DDD (alternative name) (1056) + TX, ethylene dibromide (316) + TX, ethylene dichloride (chemical name) (1136) + TX, ethylene oxide [CCN] + TX, etofenprox (319) + TX, etrimfos (1142) + TX, EXD (1143) + TX, famphur (323) + TX, fenamiphos (326) + TX, fenazaflor (1147) + TX, fenchlorphos (1148) + TX, fenethacarb (1149) + TX, fenfluthrin (1150) + TX, fenitrothion (335) + TX, fenobucarb (336) + TX, fenoxacrim (1153) + TX, fenoxycarb (340) + TX, fenpirithrin (1155) + TX, fenpropathrin (342) + TX, fenpyrad (alternative name) + TX, fensulfothion (1158) + TX, fenthion (346) + TX, fenthion-ethyl [CCN] + TX, fenvalerate (349) + TX, fipronil (354) + TX, flonicamid (358) + TX, flubendiamide (CAS. Reg. No.: 272451-65-7) + TX, flucofuron (1168) + TX, flucycloxuron (366) + TX, flucythrinate (367) + TX, fluenetil (1169) + TX, flufenerim [CCN] + TX, flufenoxuron (370) + TX, flufenprox (1171) + TX, flumethrin (372) + TX, fluvalinate (1184) + TX, FMC 1137 (development code) (1185) + TX, fonofos (1191) + TX, formetanate (405) + TX, formetanate hydrochloride (405) + TX, formothion (1192) + TX, formparanate (1193) + TX, fosmethilan (1194) + TX, fospirate (1195) + TX, fosthiazate (408) + TX, fosthietan (1196) + TX, furathiocarb (412) + TX, furethrin (1200) + TX, gamma-cyhalothrin (197) + TX, gamma-HCH (430) + TX, guazatine (422) + TX, guazatine acetates (422) + TX, GY-81 (development code) (423) + TX, halfenprox (424) + TX, halofenozide (425) + TX, HCH (430) + TX, HEOD (1070) + TX, heptachlor (1211) + TX, heptenophos (432) + TX, heterophos [CCN] + TX, hexaflumuron (439) + TX, HHDN (864) + TX, hydramethylnon (443) + TX, hydrogen cyanide (444) + TX, hydroprene (445) + TX, hyquincarb (1223) + TX, imidacloprid (458) + TX, imiprothrin (460) + TX, indoxacarb (465) + TX, iodomethane (IUPAC name) (542) + TX, IPSP (1229) + TX, isazofos (1231) + TX, isobenzan (1232) + TX, isocarbophos (alternative name) (473) + TX, isodrin (1235) + TX, isofenphos (1236) + TX, isolane (1237) + TX, isoprocarb (472) + TX, isopropyl *O*-(methoxyaminothiophosphoryl)salicylate (IUPAC name) (473) + TX, isoprothiolane (474) + TX, isothioate (1244) + TX, isoxathion (480) + TX, ivermectin (alternative name) [CCN] + TX, jasmolin I (696) + TX, jasmolin II (696) + TX, jodfenphos (1248) + TX, juvenile hormone I (alternative name) [CCN] + TX, juvenile hormone II (alternative name) [CCN] + TX, juvenile hormone III (alternative name) [CCN] + TX, kelevan (1249) + TX, kinoprene (484) + TX, lambda-cyhalothrin (198) + TX, lead arsenate [CCN] + TX, lepimectin (CCN) + TX, leptophos (1250) + TX, lindane (430) + TX, lirimfos (1251) + TX, lufenuron (490) + TX, lythidathion (1253) + TX, m-cumenyl methylcarbamate (IUPAC name) (1014) + TX, magnesium phosphide (IUPAC name) (640) + TX, malathion (492) + TX, malonoben (1254) + TX, mazidox (1255) + TX, mecarbam (502) + TX, mecarphon (1258) + TX, menazon (1260) + TX, mephosfolan (1261) + TX, mercurous chloride (513) + TX, mesulfenfos (1263) + TX, metaflumizone (CCN) + TX, metam (519) + TX, metam-potassium (alternative name) (519) + TX, metam-sodium (519) + TX, methacrifos (1266) + TX, methamidophos (527) + TX, methanesulfonyl fluoride (IUPAC/Chemical Abstracts name) (1268) + TX, methidathion (529) + TX, methiocarb (530) + TX, methocrotophos (1273) + TX, methomyl (531) + TX, methoprene (532) + TX, methoquin-butyl (1276) + TX, methothrin (alternative name) (533) + TX, methoxychlor (534) + TX, methoxyfenozide (535) + TX, methyl bromide (537) + TX, methyl isothiocyanate (543) + TX, methylchloroform (alternative name) [CCN] + TX, methylene chloride [CCN] + TX, metofluthrin [CCN] + TX, metolcarb (550) + TX, metoxadiazone (1288) + TX, mevinphos (556) + TX, mexacarbate (1290) + TX, milbemectin (557) + TX, milbemycin oxime (alternative name) [CCN] + TX, mipafox (1293) + TX, mirex (1294) + TX, monocrotophos (561) + TX, morphothion (1300) + TX, moxidectin (alternative name) [CCN] + TX, naftalofos (alternative name) [CCN] + TX, naled (567) + TX, naphthalene (IUPAC/ChemicalAbstracts name) (1303) + TX, NC-170 (development code) (1306) + TX, NC-184 (compound code) + TX, nicotine (578) + TX, nicotine sulfate (578) + TX, nifluridide (1309) + TX, nitenpyram (579) + TX, nithiazine (1311) + TX, nitrilacarb (1313) + TX, nitrilacarb 1:1 zinc chloride complex (1313) + TX, NNI-0101 (compound code) + TX, NNI-0250 (compound code) + TX, nornicotine (traditional name) (1319) + TX, novaluron (585) + TX, noviflumuron (586) + TX, *O*-5-dichloro-4-iodophenyl O-ethyl ethylphosphonothioate (IUPAC name) (1057) + TX, *O*,*O*-diethyl *O*-4-methyl-2-oxo-2*H*-chromen-7-yl phosphorothioate (IUPAC name) (1074) + TX, *O*,*O*-diethyl *O*-6-methyl-2-propylpyrimidin-4-yl phosphorothioate (IUPAC name) (1075) + TX, *O*,*O*,*O*',*O*'-tetrapropyl dithiopyrophosphate (IUPAC name) (1424) + TX, oleic acid (IUPAC name) (593) + TX, omethoate (594) + TX, oxamyl (602) + TX, oxydemeton-methyl (609) + TX, oxydeprofos (1324) + TX, oxydisulfoton (1325) + TX, pp'-DDT (219) + TX, para-dichlorobenzene [CCN] + TX, parathion (615) + TX, parathion-methyl (616) + TX, penfluron (alternative name) [CCN] + TX, pentachlorophenol (623) + TX, pentachlorophenyl laurate (IUPAC name) (623) + TX, permethrin (626) + TX, petroleum oils (alternative name) (628) + TX, PH 60-38 (development code) (1328) + TX, phenkapton (1330) + TX, phenothrin (630) + TX, phenthoate (631) + TX, phorate (636) + TX, phosalone (637) + TX, phosfolan (1338) + TX, phosmet (638) + TX, phosnichlor (1339) + TX, phosphamidon (639) + TX, phosphine (IUPAC name) (640) + TX, phoxim (642) + TX, phoxim-methyl (1340) + TX, pirimetaphos (1344) + TX, pirimicarb (651) + TX, pirimiphos-ethyl (1345) + TX, pirimiphos-methyl (652) + TX, polychlorodicyclopentadiene isomers (IUPAC name) (1346) + TX, polychloroterpenes (traditional name) (1347) + TX, potassium arsenite [CCN] + TX, potassium thiocyanate [CCN] + TX, prallethrin (655) + TX, precocene I (alternative name) [CCN] + TX, precocene II (alternative name) [CCN] + TX, precocene III (alternative name) [CCN] + TX, primidophos (1349) + TX, profenofos (662) + TX, profluthrin [CCN] + TX, promacyl (1354) + TX, promecarb (1355) + TX, propaphos (1356) + TX, propetamphos (673) + TX, propoxur (678) + TX, prothidathion (1360) + TX, prothiofos (686) + TX, prothoate (1362) + TX, protrifenbute [CCN] + TX, pymetrozine (688) + TX, pyraclofos (689) + TX, pyrazophos (693) + TX, pyresmethrin (1367) + TX, pyrethrin I (696) + TX, pyrethrin II (696) + TX, pyrethrins (696) + TX, pyridaben (699) + TX, pyridalyl (700) + TX, pyridaphenthion (701) + TX, pyrimidifen (706) + TX, pyrimitate (1370) + TX, pyriproxyfen (708) + TX, quassia (alternative name) [CCN] + TX, quinalphos (711) + TX, quinalphos-methyl (1376) + TX, quinothion (1380) + TX, quintiofos (1381) + TX, R-1492 (development code) (1382) + TX, rafoxanide (alternative name) [CCN] + TX, resmethrin (719) + TX, rotenone (722) + TX, RU 15525 (development code) (723) + TX, RU 25475 (development code) (1386) + TX, ryania (alternative name) (1387) + TX, ryanodine (traditional name) (1387) + TX, sabadilla (alternative name) (725) + TX, schradan (1389) + TX, sebufos (alternative name) + TX, selamectin (alternative name) [CCN] + TX, SI-0009 (compound code) + TX, SI-0205 (compound code) + TX, SI-0404 (compound code) + TX, SI-0405 (compound code) + TX, silafluofen (728) + TX, SN 72129 (development code) (1397) + TX, sodium arsenite [CCN] + TX, sodium cyanide (444) + TX, sodium fluoride (IUPAC/ChemicalAbstracts name) (1399) + TX, sodium hexafluorosilicate (1400) + TX, sodium pentachlorophenoxide (623) + TX, sodium selenate (IUPAC name) (1401) + TX, sodium thiocyanate [CCN] + TX, sophamide (1402) + TX, spinosad (737) + TX, spiromesifen (739) + TX, spirotetrmat (CCN) + TX, sulcofuron (746) + TX, sulcofuron-sodium (746) + TX, sulfluramid (750) + TX, sulfotep (753) + TX, sulfuryl fluoride (756) + TX, sulprofos (1408) + TX, tar oils (alternative name) (758) + TX, tau-fluvalinate (398) + TX, tazimcarb (1412) + TX, TDE (1414) + TX, tebufenozide (762) + TX, tebufenpyrad (763) + TX, tebupirimfos (764) + TX, teflubenzuron (768) + TX, tefluthrin (769) + TX, temephos (770) + TX, TEPP (1417) + TX, terallethrin (1418) + TX, terbam (alternative name) + TX, terbufos (773) + TX, tetrachloroethane [CCN] + TX, tetrachlorvinphos (777) + TX, tetramethrin (787) + TX, theta-cypermethrin (204) + TX, thiacloprid (791) + TX, thiafenox (alternative name) + TX, thiamethoxam (792) + TX, thicrofos (1428) + TX, thiocarboxime (1431) + TX, thiocyclam (798) + TX, thiocyclam hydrogen oxalate (798) + TX, thiodicarb (799) + TX, thiofanox (800) + TX, thiometon (801) + TX, thionazin (1434) + TX, thiosultap (803) + TX, thiosultap-sodium (803) + TX, thuringiensin (alternative name) [CCN] + TX, tolfenpyrad (809) + TX, tralomethrin (812) + TX, transfluthrin (813) + TX, transpermethrin (1440) + TX, triamiphos (1441) + TX, triazamate (818) + TX, triazophos (820) + TX, triazuron (alternative name) + TX, trichlorfon (824) + TX, trichlormetaphos-3 (alternative name) [CCN] + TX, trichloronat (1452) + TX, trifenofos (1455) + TX, triflumuron (835) + TX, trimethacarb (840) + TX, triprene (1459) + TX, vamidothion (847) + TX, vaniliprole [CCN] + TX, veratridine (alternative name) (725) + TX, veratrine (alternative name) (725) + TX, XMC (853) + TX, xylylcarb (854) + TX, Yl-5302 (compound code) + TX, zeta-cypermethrin (205) + TX, zetamethrin (alternative name) + TX, zinc phosphide (640) + TX, zolaprofos (1469) and ZXI 8901 (development code) (858) + TX, cyantraniliprole [736994-63-19 + TX, chlorantraniliprole [500008-45-7] + TX, cyenopyrafen [560121-52-0] + TX, cyflumetofen [400882-07-7] + TX, pyrifluquinazon [337458-27-2] + TX, spinetoram [187166-40-1 + 187166-15-0] + TX, spirotetramat [203313-25-1] + TX, sulfoxaflor [946578-00-3] + TX, flufiprole [704886-18-0] + TX, meperfluthrin [915288-13-0] + TX, tetramethylfluthrin [84937-88-2] + TX, triflumezopyrim (disclosed in WO 2012/092115) + TX,
a molluscicide selected from the group of substances consisting of bis(tributyltin) oxide (IUPAC name) (913) + TX, bromoacetamide [CCN] + TX, calcium arsenate [CCN] + TX, cloethocarb (999) + TX, copper acetoarsenite [CCN] + TX, copper sulfate (172) + TX, fentin (347) + TX, ferric phosphate (IUPAC name) (352) + TX, metaldehyde (518) + TX, methiocarb (530) + TX, niclosamide (576) + TX, niclosamide-olamine (576) + TX, pentachlorophenol (623) + TX, sodium pentachlorophenoxide (623) + TX, tazimcarb (1412) + TX, thiodicarb (799) + TX, tributyltin oxide (913) + TX, trifenmorph (1454) + TX, trimethacarb (840) + TX, triphenyltin acetate (IUPAC name) (347) and triphenyltin hydroxide (IUPAC name) (347) + TX, pyriprole [394730-71-3] + TX,
a nematicide selected from the group of substances consisting of AKD-3088 (compound code) + TX, 1,2-dibromo-3-chloropropane (IUPAC/ChemicalAbstracts name) (1045) + TX, 1,2-dichloropropane (IUPAC/ Chemical Abstracts name) (1062) + TX, 1,2-dichloropropane with 1,3-dichloropropene (IUPAC name) (1063) + TX, 1,3-dichloropropene (233) + TX, 3,4-dichlorotetrahydrothiophene 1,1-dioxide (IUPAC/ChemicalAbstracts name) (1065) + TX, 3-(4-chlorophenyl)-5-methylrhodanine (IUPAC name) (980) + TX, 5-methyl-6-thioxo-1,3,5-thiadiazinan-3-ylacetic acid (IUPAC name) (1286) + TX, 6-isopentenylaminopurine (alternative name) (210) + TX, abamectin (1) + TX, acetoprole [CCN] + TX, alanycarb (15) + TX, aldicarb (16) + TX, aldoxycarb (863) + TX, AZ 60541 (compound code) + TX, benclothiaz [CCN] + TX, benomyl (62) + TX, butylpyridaben (alternative name) + TX, cadusafos (109) + TX, carbofuran (118) + TX, carbon disulfide (945) + TX, carbosulfan (119) + TX, chloropicrin (141) + TX, chlorpyrifos (145) + TX, cloethocarb (999) + TX, cytokinins (alternative name) (210) + TX, dazomet (216) + TX, DBCP (1045) + TX, DCIP (218) + TX, diamidafos (1044) + TX, dichlofenthion (1051) + TX, dicliphos (alternative name) + TX, dimethoate (262) + TX, doramectin (alternative name) [CCN] + TX, emamectin (291) + TX, emamectin benzoate (291) + TX, eprinomectin (alternative name) [CCN] + TX, ethoprophos (312) + TX, ethylene dibromide (316) + TX, fenamiphos (326) + TX, fenpyrad (alternative name) + TX, fensulfothion (1158) + TX, fosthiazate (408) + TX, fosthietan (1196) + TX, furfural (alternative name) [CCN] + TX, GY-81 (development code) (423) + TX, heterophos [CCN] + TX, iodomethane (IUPAC name) (542) + TX, isamidofos (1230) + TX, isazofos (1231) + TX, ivermectin (alternative name) [CCN] + TX, kinetin (alternative name) (210) + TX, mecarphon (1258) + TX, metam (519) + TX, metam-potassium (alternative name) (519) + TX, metam-sodium (519) + TX, methyl bromide (537) + TX, methyl isothiocyanate (543) + TX, milbemycin oxime (alternative name) [CCN] + TX, moxidectin (alternative name) [CCN] + TX, *Myrothecium verrucaria* composition (alternative name) (565) + TX, NC-184 (compound code) + TX, oxamyl (602) + TX, phorate (636) + TX, phosphamidon (639) + TX, phosphocarb [CCN] + TX, sebufos (alternative name) + TX, selamectin (alternative name) [CCN] + TX, spinosad (737) + TX, terbam (alternative name) + TX, terbufos (773) + TX, tetrachlorothiophene (IUPAC/ Chemical Abstracts name) (1422) + TX, thiafenox (alternative name) + TX, thionazin (1434) + TX, triazophos (820) + TX, triazuron (alternative name) + TX, xylenols [CCN] + TX, YI-5302 (compound code) and zeatin (alternative name) (210) + TX, fluensulfone [318290-98-1] + TX,
a nitrification inhibitor selected from the group of substances consisting of potassium ethylxanthate [CCN] and nitrapyrin (580) + TX,
a plant activator selected from the group of substances consisting of acibenzolar (6) + TX, acibenzolar-S-methyl (6) + TX, probenazole (658) and *Reynoutria sachalinensis* extract (alternative name) (720) + TX,
a rodenticide selected from the group of substances consisting of 2-isovalerylindan-1,3-dione (IUPAC name) (1246) + TX, 4-(quinoxalin-2-ylamino)benzenesulfonamide (IUPAC name) (748) + TX, alpha-chlorohydrin [CCN] + TX, aluminium phosphide (640) + TX, antu (880) + TX, arsenous oxide (882) + TX, barium carbonate (891) + TX, bisthiosemi (912) + TX, brodifacoum (89) + TX, bromadiolone (91) + TX, bromethalin (92) + TX, calcium cyanide (444) + TX, chloralose (127) + TX, chlorophacinone (140) + TX, cholecalciferol (alternative name) (850) + TX, coumachlor (1004) + TX, coumafuryl (1005) + TX, coumatetralyl (175) + TX, crimidine (1009) + TX, difenacoum (246) + TX, difethialone (249) + TX, diphacinone (273) + TX, ergocalciferol (301) + TX, flocoumafen (357) + TX, fluoroacetamide (379) + TX, flupropadine (1183) + TX, flupropadine hydrochloride (1183) + TX, gamma-HCH (430) + TX, HCH (430) + TX, hydrogen cyanide (444) + TX, iodomethane (IUPAC name) (542) + TX, lindane (430) + TX, magnesium phosphide (IUPAC name) (640) + TX, methyl bromide (537) + TX, norbormide (1318) + TX, phosacetim (1336) + TX, phosphine (IUPAC name) (640) + TX, phosphorus [CCN] + TX, pindone (1341) + TX, potassium arsenite [CCN] + TX, pyrinuron (1371) + TX, scilliroside (1390) + TX, sodium arsenite [CCN] + TX, sodium cyanide (444) + TX, sodium fluoroacetate (735) + TX, strychnine (745) + TX, thallium sulfate [CCN] + TX, warfarin (851) and zinc phosphide (640) + TX,
a synergist selected from the group of substances consisting of 2-(2-butoxyethoxy)ethyl piperonylate (IUPAC name) (934) + TX, 5-(1,3-benzodioxol-5-yl)-3-hexylcyclohex-2-enone (IUPAC name) (903) + TX, farnesol with nerolidol (alternative name) (324) + TX, MB-599 (development code) (498) + TX, MGK 264 (development code) (296) + TX, piperonyl butoxide (649) + TX, piprotal (1343) + TX, propyl isomer (1358) + TX, S421 (development code) (724) + TX, sesamex (1393) + TX, sesasmolin (1394) and sulfoxide (1406) + TX,
an animal repellent selected from the group of substances consisting of anthraquinone (32) + TX, chloralose (127) + TX, copper naphthenate [CCN] + TX, copper oxychloride (171) + TX, diazinon (227) + TX, dicyclopentadiene (chemical name) (1069) + TX, guazatine (422) + TX, guazatine acetates (422) + TX, methiocarb (530) + TX, pyridin-4-amine (IUPAC name) (23) + TX, thiram (804) + TX, trimethacarb (840) + TX, zinc naphthenate [CCN] and ziram (856) + TX,
a virucide selected from the group of substances consisting of imanin (alternative name) [CCN] and ribavirin (alternative name) [CCN] + TX,
a wound protectant selected from the group of substances consisting of mercuric oxide (512) + TX, octhilinone (590) and thiophanate-methyl (802) + TX,
and biologically active compounds selected from the group consisting of ametoctradin [865318-97-4] + TX, amisulbrom [348635-87-0] + TX, azaconazole [60207-31-0] + TX, benzovindiflupyr [1072957-71-1] + TX, bitertanol [70585-36-3] + TX, bixafen [581809-46-3] + TX, bromuconazole [116255-48-2] + TX, coumoxystrobin [850881-70-8] + TX, cyproconazole [94361-06-5] + TX, difenoconazole [119446-68-3] + TX, diniconazole [83657-24-3] + TX, enoxastrobin [238410-11-2] + TX, epoxiconazole [106325-08-0] + TX, fenbuconazole [114369-43-6] + TX, fenpyrazamine [473798-59-3] + TX, fluquinconazole [136426-54-5] + TX, flusilazole [85509-19-9] + TX, flutriafol [76674-21-0] + TX, fluxapyroxad [907204-31-3] + TX, fluopyram [658066-35-4] + TX, fenaminstrobin [366815-39-6] + TX, isofetamid [875915-78-9] + TX, hexaconazole [79983-71-4] + TX, imazalil [35554-44-0] + TX, imiben-conazole [86598-92-7] + TX, ipconazole [125225-28-7] + TX, ipfentrifluconazole [1417782-08-1] + TX, isotianil [224049-04-1] + TX, mandestrobin [173662-97-0] (can be prepared according to the procedures described in WO 2010/093059) + TX, mefentrifluconazole [1417782-03-6] + TX, metconazole [125116-23-6] + TX, myclobutanil [88671-89-0] + TX, paclobutrazol [76738-62-0] + TX, pefurazoate [101903-30-4] + TX, penflufen [494793-67-8] + TX, penconazole [66246-88-6] + TX, prothioconazole [178928-70-6] + TX, pyrifenox [88283-41-4] + TX, prochloraz [67747-09-5] + TX, propiconazole [60207-90-1] + TX, simeconazole [149508-90-7] + TX, tebuconazole [107534-96-3] + TX, tetraconazole [112281-77-3] + TX, triadimefon [43121-43-3] + TX, triadimenol [55219-65-3] + TX, triflumizole [99387-89-0] + TX, triticonazole [131983-72-7] + TX, ancymidol [12771-68-5] + TX, fenarimol [60168-88-9] + TX, nuarimol [63284-71-9] + TX, bupirimate [41483-43-6] + TX, dimethirimol [5221-53-4] + TX, ethirimol [23947-60-6] + TX, dodemorph [1593-77-7] + TX, fenpropidin [67306-00-7] + TX, fenpropimorph [67564-91-4] + TX, spiroxamine [118134-30-8] + TX, tridemorph [81412-43-3] + TX, cyprodinil [121552-61-2] + TX, mepanipyrim [110235-47-7] + TX, pyrimethanil [53112-28-0] + TX, fenpiclonil [74738-17-3] + TX, fludioxonil [131341-86-1] + TX, fluindapyr [1383809-87-7] + TX, benalaxyl [71626-11-4] + TX, furalaxyl [57646-30-7] + TX, metalaxyl [57837-19-1] + TX, R-metalaxyl [70630-17-0] + TX, ofurace [58810-48-3] + TX, oxadixyl [77732-09-3] + TX, benomyl [17804-35-2] + TX, carbendazim [10605-21-7] + TX, debacarb [62732-91-6] + TX, fuberidazole [3878-19-1] + TX, thiabendazole [148-79-8] + TX, chlozolinate [84332-86-5] + TX, dichlozoline [24201-58-9] + TX, iprodione [36734-19-7] + TX, myclozoline [54864-61-8] + TX, procymidone [32809-16-8] + TX, vinclozoline [50471-44-8] + TX, boscalid [188425-85-6] + TX, carboxin [5234-68-4] + TX, fenfuram [24691-80-3] + TX, flutolanil [66332-96-5] + TX, flutianil [958647-10-4] + TX, mepronil [55814-41-0] + TX, oxycarboxin [5259-88-1] + TX, penthiopyrad [183675-82-3] + TX, thifluzamide [130000-40-7] + TX, guazatine [108173-90-6] + TX, dodine [2439-10-3] [112-65-2] (free base) + TX, iminoctadine [13516-27-3] + TX, azoxystrobin [131860-33-8] + TX, dimoxystrobin [149961-52-4] + TX, enestroburin {Proc. BCPC, Int. Congr., Glasgow, 2003, 1, 93} + TX, fluoxastrobin [361377-29-9] + TX, kresoxim-methyl [143390-89-0] + TX, metominostrobin [133408-50-1] + TX, trifloxystrobin [141517-21-7] + TX, orysastrobin [248593-16-0] + TX, picoxystrobin [117428-22-5] + TX, pyraclostrobin [175013-18-0] + TX, pyraoxystrobin [862588-11-2] + TX, ferbam [14484-64-1] + TX, mancozeb [8018-01-7] + TX, maneb [12427-38-2] + TX, metiram [9006-42-2] + TX, propineb [12071-83-9] + TX, thiram [137-26-8] + TX, zineb [12122-67-7] + TX, ziram [137-30-4] + TX, captafol [2425-06-1] + TX, captan [133-06-2] + TX, dichlofluanid [1085-98-9] + TX, fluoroimide [41205-21-4] + TX, folpet [133-07-3 ] + TX, tolylfluanid [731-27-1] + TX, bordeaux mixture [8011-63-0] + TX, copperhydroxid [20427-59-2] + TX, copperoxychlorid [1332-40-7] + TX, coppersulfat [7758-98-7] + TX, copperoxid [1317-39-1] + TX, mancopper [53988-93-5] + TX, oxine-copper [10380-28-6] + TX, dinocap [131-72-6] + TX, nitrothal-isopropyl [10552-74-6] + TX, edifenphos [17109-49-8] + TX, iprobenphos [26087-47-8] + TX, isoprothiolane [50512-35-1] + TX, phosdiphen [36519-00-3] + TX, pyrazophos [13457-18-6] + TX, tolclofos-methyl [57018-04-9] + TX, acibenzolar-S-methyl [135158-54-2] + TX, anilazine [101-05-3] + TX, benthiavalicarb [413615-35-7] + TX, blasticidin-S [2079-00-7] + TX, chinomethionat [2439-01-2] + TX, chloroneb [2675-77-6] + TX, chlorothalonil [1897-45-6] + TX, cyflufenamid [180409-60-3] + TX, cymoxanil [57966-95-7] + TX, dichlone [117-80-6] + TX, diclocymet [139920-32-4] + TX, diclomezine [62865-36-5] + TX, dicloran [99-30-9] + TX, diethofencarb [87130-20-9] + TX, dimethomorph [110488-70-5] + TX, SYP-LI90 (Flumorph) [211867-47-9] + TX, dithianon [3347-22-6] + TX, ethaboxam [162650-77-3] + TX, etridiazole [2593-15-9] + TX, famoxadone [131807-57-3] + TX, fenamidone [161326-34-7] + TX, fenoxanil [115852-48-7] + TX, fentin [668-34-8] + TX, ferimzone [89269-64-7] + TX, fluazinam [79622-59-6] + TX, fluopicolide [239110-15-7] + TX, flusulfamide [106917-52-6] + TX, fenhexamid [126833-17-8] + TX, fosetyl-aluminium [39148-24-8] + TX, hymexazol [10004-44-1] + TX, iprovalicarb [140923-17-7] + TX, IKF-916 (Cyazofamid) [120116-88-3] + TX, kasugamycin [6980-18-3] + TX, methasulfocarb [66952-49-6] + TX, metrafenone [220899-03-6] + TX, pencycuron [66063-05-6] + TX, phthalide [27355-22-2] + TX, picarbutrazox [500207-04-5] + TX, polyoxins [11113-80-7] + TX, probenazole [27605-76-1] + TX, propamocarb [25606-41-1] + TX, proquinazid [189278-12-4] + TX, pydiflumetofen [1228284-64-7] + TX, pyrametostrobin [915410-70-7] + TX, pyroquilon [57369-32-1] + TX, pyriofenone [688046-61-9] + TX, pyribencarb [799247-52-2] + TX, pyrisoxazole [847749-37-5] + TX, quinoxyfen [124495-18-7] + TX, quintozene [82-68-8] + TX, sulfur [7704-34-9] + TX, Timorex Gold^{™} (plant extract containing tea tree oil from the Stockton Group) + TX, tebufloquin [376645-78-2] + TX, tiadinil [223580-51-6] + TX, triazoxide [72459-58-6] + TX, tolprocarb [911499-62-2] + TX, triclopyricarb [902760-40-1] + TX, tricyclazole [41814-78-2] + TX, triforine [26644-46-2] + TX, validamycin [37248-47-8] + TX, valifenalate [283159-90-0] + TX, zoxamide (RH7281) [156052-68-5] + TX, mandipropamid [374726-62-2] + TX, isopyrazam [881685-58-1] + TX, phenamacril + TX, sedaxane [874967-67-6] + TX, trinexapac-ethyl [95266-40-3] + TX, 3-difluoromethyl-1-methyl-1H-pyrazole-4-carboxylic acid (9-dichloromethylene-1,2,3,4-tetrahydro-1,4-methano-naphthalen-5-yl)-amide (dislosed in WO 2007/048556) + TX, 3-difluoromethyl-1-methyl-1H-pyrazole-4-carboxylic acid (3',4',5'-trifluoro-biphenyl-2-yl)-amide (disclosed in WO 2006/087343) + TX, [(3S,4R,4aR,6S,6aS,12R,12aS,12bS)-3-[(cyclopropylcarbonyl)oxy]- 1,3,4,4a,5,6,6a,12,12a,12b-decahydro-6,12-dihydroxy-4,6a,12b-trimethyl-11-oxo-9-(3-pyridinyl)-2*H*,11*H*naphtho[2,1-b]pyrano[3,4-e]pyran-4-yl]methyl-cyclopropanecarboxylate [915972-17-7] + TX and 1,3,5-trimethyl-N-(2-methyl-1-oxopropyl)-N-[3-(2-methylpropyl)-4-[2,2,2-trifluoro-1-methoxy-1-(trifluoromethyl)ethyl]phenyl]-1H-pyrazole-4-carboxamide [926914-55-8] + TX,
or a biologically active compound selected from the group consisting of N-[(5-chloro-2-isopropyl-phenyl)methyl]-N-cyclopropyl-3-(difluoromethyl)-5-fluoro-1-methyl-pyrazole-4-carboxamide (can be prepared according to the procedures described in WO 2010/130767) + TX, 2,6-Dimethyl-1H,5H-[1,4]dithiino[2,3-c:5,6-c']dipyrrole-1,3,5,7(2H,6H)-tetrone (can be prepared according to the procedures described in WO 2011/138281) + TX, 6-ethyl-5,7-dioxo-pyrrolo[4,5][1,4]dithiino[1,2-c]isothiazole-3-carbonitrile + TX, 4-(2-bromo-4-fluoro-phenyl)-N-(2-chloro-6-fluoro-phenyl)-2,5-dimethyl-pyrazol-3-amine (can be prepared according to the procedures described in WO 2012/031061) + TX, 3-(difluoromethyl)-N-(7-fluoro-1,1,3-trimethyl-indan-4-yl)-1-methyl-pyrazole-4-carboxamide (can be prepared according to the procedures described in WO 2012/084812) + TX, CAS 850881-30-0 + TX, 3-(3,4-dichloro-1,2-thiazol-5-ylmethoxy)-1,2-benzothiazole 1,1-dioxide (can be prepared according to the procedures described in WO 2007/129454) + TX, 2-[2-[(2,5-dimethylphenoxy)methyl]phenyl]-2-methoxy-N-methyl-acetamide + TX, 3-(4,4-difluoro-3,4-dihydro-3,3-dimethylisoquinolin-1-yl)quinolone (can be prepared according to the procedures described in WO 2005/070917) + TX, 2-[2-fluoro-6-[(8-fluoro-2-methyl-3-quinolyl)oxy]phenyl]propan-2-ol (can be prepared according to the procedures described in WO 2011/081174) + TX, 2-[2-[(7,8-difluoro-2-methyl-3-quinolyl)oxy]-6-fluoro-phenyl]propan-2-ol (can be prepared according to the procedures described in WO 2011/081174) + TX, oxathiapiprolin + TX [1003318-67-9], tert-butyl N-[6-[[[(1-methyltetrazol-5-yl)-phenyl-methylene]amino]oxymethyl]-2-pyridyl]carbamate + TX, N-[2-(3,4-difluorophenyl)phenyl]-3-(trifluoromethyl)pyrazine-2-carboxamide (can be prepared according to the procedures described in WO 2007/ 072999) + TX, 3-(difluoromethyl)-1-methyl-N-[(3R)-1,1,3-trimethylindan-4-yl]pyrazole-4-carboxamide (can be prepared according to the procedures described in WO 2014/013842) + TX, 2,2,2-trifluoroethyl N-[2-methyl-1-[[(4-methylbenzoyl)amino]methyl]propyl]carbamate + TX, (2RS)-2-[4-(4-chlorophenoxy)-α,α,α-trifluoro-o-tolyl]-1-(1H-1,2,4-triazol-1-yl)propan-2-ol + TX, (2RS)-2-[4-(4-chlorophenoxy)-α,α,α-trifluoro-o-tolyl]-3-methyl-1-(1H-1,2,4-triazol-1-yl)butan-2-ol + TX, 2-(difluoromethyl)-N-[(3R)-3-ethyl-1,1-dimethyl-indan-4-yl]pyridine-3-carboxamide + TX, 2-(difluoromethyl)-N-[3-ethyl-1,1-dimethyl-indan-4-yl]pyridine-3-carboxamide + TX, N'-(2,5-dimethyl-4-phenoxy-phenyl)-N-ethyl-N-methyl-formamidine + TX, N'-[4-(4,5-dichlorothiazol-2-yl)oxy-2,5-dimethyl-phenyl]-N-ethyl-N-methyl-formamidine (can be prepared according to the procedures described in WO 2007/031513) + TX, [2-[3-[2-[1-[2-[3,5-bis(difluoromethyl)pyrazol-1-yl]acetyl]-4-piperidyl]thiazol-4-yl]-4,5-dihydroisoxazol-5-yl]-3-chloro-phenyl] methanesulfonate (can be prepared according to the procedures described in WO 2012/025557) + TX, but-3-ynyl N-[6-[[(Z)-[(1-methyltetrazol-5-yl)-phenyl-methylene]amino]oxymethyl]-2-pyridyl]carbamate (can be prepared according to the procedures described in WO 2010/000841) + TX, 2-[[3-(2-chlorophenyl)-2-(2,4-difluorophenyl)oxiran-2-yl]methyl]-4H-1,2,4-triazole-3-thione (can be prepared according to the procedures described in WO 2010/146031) + TX, methyl N-[[5-[4-(2,4-dimethylphenyl)triazol-2-yl]-2-methyl-phenyl]methyl]carbamate + TX, 3-chloro-6-methyl-5-phenyl-4-(2,4,6-trifluorophenyl)pyridazine (can be prepared according to the procedures described in WO 2005/121104) + TX, 2-[2-chloro-4-(4-chlorophenoxy)phenyl]-1-(1,2,4-triazol-1-yl)propan-2-ol (can be prepared according to the procedures described in WO 2013/024082) + TX, 3-chloro-4-(2,6-difluorophenyl)-6-methyl-5-phenyl-pyridazine (can be prepared according to the procedures described in WO 2012/020774) + TX, 4-(2,6-difluorophenyl)-6-methyl-5-phenyl-pyridazine-3-carbonitrile (can be prepared according to the procedures described in WO 2012/020774) + TX, (R)-3-(difluoromethyl)-1-methyl-N-[1,1,3-trimethylindan-4-yl]pyrazole-4-carboxamide (can be prepared according to the procedures described in WO 2011/162397 ) + TX, 3-(difluoromethyl)-N-(7-fluoro-1,1,3-trimethyl-indan-4-yl)-1-methyl-pyrazole-4-carboxamide (can be prepared according to the procedures described in WO 2012/084812) + TX, 1-[2-[[1-(4-chlorophenyl)pyrazol-3-yl]oxymethyl]-3-methyl-phenyl]-4-methyl-tetrazol-5-one (can be prepared according to the procedures described in WO 2013/162072) + TX, 1-methyl-4-[3-methyl-2-[[2-methyl-4-(3,4,5-trimethylpyrazol-1-yl)phenoxy]methyl]phenyl]tetrazol-5-one (can be prepared according to the procedures described in WO 2014/051165) + TX, (Z,2E)-5-[1-(4-chlorophenyl)pyrazol-3-yl]oxy-2-methoxyimino-N,3-dimethyl-pent-3-enamide + TX, (4-phenoxyphenyl)methyl 2-amino-6-methyl-pyridine-3-carboxylate + TX, N-(5-chloro-2-isopropylbenzyl)-N-cyclopropyl-3-(difluoromethyl)-5-fluoro-1-methylpyrazole-4-carboxamide [1255734-28-1] (can be prepared according to the procedures described in WO 2010/130767) + TX, 3-(difluoromethyl)-N-[(R)-2,3-dihydro-1,1,3-trimethyl-1H-inden-4-yl]-1-methylpyrazole-4-carboxamide [1352994-67-2] + TX, N'-(2,5-dimethyl-4-phenoxy-phenyl)-N-ethyl-N-methyl-formamidine + TX, N'-[4-(4,5-dichloro-thiazol-2-yloxy)-2,5-dimethyl-phenyl]-N-ethyl-N-methyl-formamidine + TX, N'-(2,5-dimethyl-4-phenoxy-phenyl)-N-ethyl-N-methyl-formamidine + TX, N'-[4-(4,5-dichloro-thiazol-2-yloxy)-2,5-dimethyl-phenyl]-N-ethyl-N-methyl-formamidine + TX, 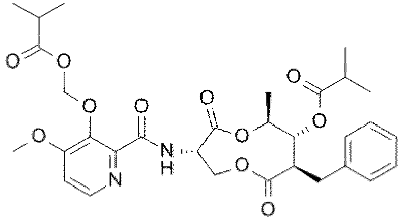 (fenpicoxamid [517875-34-2] (as described in WO 2003/035617)) + TX, (1S)-2,2-bis(4-fluorophenyl)-1-methylethyl N-{[3-(acetyloxy)-4-methoxy-2-pyridyl]carbonyl}-L-alaninate [1961312-55-9] (as described in WO 2016/122802) + TX, 2-(difluoromethyl)-N-(1,1,3-trimethylindan-4-yl)pyridine-3-carboxamide + TX, 2-(difluoromethyl)-N-(3-ethyl-1,1-dimethyl-indan-4-yl)pyridine-3-carboxamide + TX, 2-(difluoromethyl)-N-(1,1-dimethyl-3-propyl-indan-4-yl)pyridine-3-carboxamide + TX, 2-(difluoromethyl)-N-(3-isobutyl-1,1-dimethyl-indan-4-yl)pyridine-3-carboxamide + TX, 2-(difluoromethyl)-N-[(3R)-1,1,3-trimethylindan-4-yl]pyridine-3-carboxamide + TX, 2-(difluoromethyl)-N-[(3R)-3-ethyl-1,1-dimethyl-indan-4-yl]pyridine-3-carboxamide + TX, and 2-(difluoromethyl)-N-[(3R)-1,1-dimethyl-3-propyl-indan-4-yl]pyridine-3-carboxamide + TX, wherein each of these carboxamide compounds can be prepared according to the procedures described in WO 2014/095675 and/or WO 2016/139189.

The references in brackets behind the active ingredients, e.g. *[*3878-19-1] refer to the Chemical Abstracts Registry number. The above described mixing partners are known. Where the active ingredients are included in "The Pesticide Manual" [The Pesticide Manual - A World Compendium; Thirteenth Edition; Editor: C. D. S. TomLin; The British Crop Protection Council], they are described therein under the entry number given in round brackets hereinabove for the particular compound; for example, the compound "abamectin" is described under entry number (1). Where "[CCN]" is added hereinabove to the particular compound, the compound in question is included in the "Compendium of Pesticide Common Names", which is accessible on the internet [A. Wood; Compendium of Pesticide Common Names, Copyright © 1995-2004]; for example, the compound "acetoprole" is described under the internet address http://www.alanwood.net/pesticides/acetoprole.html.

Most of the active ingredients described above are referred to hereinabove by a so-called "common name", the relevant "ISO common name" or another "common name" being used in individual cases. If the designation is not a "common name", the nature of the designation used instead is given in round brackets for the particular compound; in that case, the IUPAC name, the IUPAC/Chemical Abstracts name, a "chemical name", a "traditional name", a "compound name" or a "develoment code" is used or, if neither one of those designations nor a "common name" is used, an "alternative name" is employed. "CAS Reg. No" means the Chemical Abstracts Registry Number.

The active ingredient mixture of the compounds of Formula (I) selected from one compound as represented in Tables 1.2 and 2.2 (below), or a compound 1.3, 1.5, 1.9, 1.18, 1.22, 1.40, 1.57, 1.58, 1.88 to 1.95, 1.101, 1.111 or 1.112 listed in Table T1 (below), or compound 4.2 listed in Table T4 (below), is preferably in a mixing ratio of from 100:1 to 1:6000, especially from 50:1 to 1:50, more especially in a ratio of from 20:1 to 1:20, even more especially from 10:1 to 1:10, very especially from 5:1 and 1:5, special preference being given to a ratio of from 2:1 to 1:2, and a ratio of from 4:1 to 2:1 being likewise preferred, above all in a ratio of 1:1, or 5:1, or 5:2, or 5:3, or 5:4, or 4:1, or 4:2, or 4:3, or 3:1, or 3:2, or 2:1, or 1:5, or 2:5, or 3:5, or 4:5, or 1:4, or 2:4, or 3:4, or 1:3, or 2:3, or 1:2, or 1:600, or 1:300, or 1:150, or 1:35, or 2:35, or 4:35, or 1:75, or 2:75, or 4:75, or 1:6000, or 1:3000, or 1:1500, or 1:350, or 2:350, or 4:350, or 1:750, or 2:750, or 4:750. Those mixing ratios are by weight.

The mixtures as described above can be used in a method for controlling pests, which comprises applying a composition comprising a mixture as described above to the pests or their environment, with the exception of a method for treatment of the human or animal body by surgery or therapy and diagnostic methods practised on the human or animal body.

The mixtures comprising a compound as represented in Tables 1.2 and 2.2 (below), or a compound 1.3, 1.5, 1.9, 1.18, 1.22, 1.40, 1.57, 1.58, 1.88 to 1.95, 1.101, 1.111 or 1.112 listed in Table

T1 (below), or compound 4.2 listed in Table T4 (below), and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying a compound as represented in in Tables 1.2 and 2.2 (below), or a compound 1.3, 1.5, 1.9, 1.18, 1.22, 1.40, 1.57, 1.58, 1.88 to 1.95, 1.101, 1.111 or 1.112 listed in Table T1 (below)), or compound 4.2 listed in Table T4 (below), and the active ingredient(s) as described above, is not essential for working the present invention.

The compositions according to the invention can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers, fertilizers or other active ingredients for achieving specific effects, for example bactericides, fungicides, nematocides, plant activators, molluscicides or herbicides.

The compositions according to the invention are prepared in a manner known per se, in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds (I) for the preparation of these compositions are also a subject of the invention.

Another aspect of the invention is related to the use of a compound of Formula (I) or of a preferred individual compound as defined herein, of a composition comprising at least one compound of Formula (I) or at least one preferred individual compound as above-defined, or of a fungicidal or insecticidal mixture comprising at least one compound of Formula (I) or at least one preferred individual compound as above-defined, in admixture with other fungicides or insecticides as described above, for controlling or preventing infestation of plants, e.g. useful plants such as crop plants, propagation material thereof, e.g. seeds, harvested crops, e.g. harvested food crops, or non-living materials by insects or by phytopathogenic microorganisms, preferably fungal organisms.

A further aspect of the invention is related to a method of controlling or preventing an infestation of plants, e.g., useful plants such as crop plants, propagation material thereof, e.g. seeds, harvested crops, e.g., harvested food crops, or of non-living materials by insects or by phytopathogenic or spoilage microorganisms or organisms potentially harmful to man, especially fungal organisms, which comprises the application of a compound of Formula (I) or of a preferred individual compound as above-defined as active ingredient to the plants, to parts of the plants or to the locus thereof, to the propagation material thereof, or to any part of the non-living materials.

Controlling or preventing means reducing infestation by phytopathogenic or spoilage microorganisms or organisms potentially harmful to man, especially fungal organisms, to such a level that an improvement is demonstrated.

A preferred method of controlling or preventing an infestation of crop plants by phytopathogenic microorganisms, especially fungal organisms, or insects which comprises the application of a compound of Formula (I), or an agrochemical composition which contains at least one of said compounds, is foliar application. The frequency of application and the rate of application will depend on the risk of infestation by the corresponding pathogen or insect. However, the compounds of Formula (I) can also penetrate the plant through the roots *via* the soil (systemic action) by drenching the locus of the plant with a liquid Formulation, or by applying the compounds in solid form to the soil, e.g. in granular form (soil application). In crops of water rice such granulates can be applied to the flooded rice field. The compounds of Formula (I) may also be applied to seeds (coating) by impregnating the seeds or tubers either with a liquid formulation of the fungicide or coating them with a solid formulation.

A formulation, e.g. a composition containing the compound of Formula (I), and, if desired, a solid or liquid adjuvant or monomers for encapsulating the compound of Formula (I), may be prepared in a known manner, typically by intimately mixing and/or grinding the compound with extenders, for example solvents, solid carriers and, optionally, surface active compounds (surfactants).

Advantageous rates of application are normally from 5g to 2kg of active ingredient (a.i.) per hectare (ha), preferably from 10g to 1kg a.i./ha, most preferably from 20g to 600g a.i./ha. When used as seed drenching agent, convenient dosages are from 10mg to 1g of active substance per kg of seeds.

When the combinations of the present invention are used for treating seed, rates of 0.001 to 50 g of a compound of Formula (I) per kg of seed, preferably from 0.01 to 10g per kg of seed are generally sufficient.

Suitably, a composition comprising a compound of Formula (I) according to the present invention is applied either preventative, meaning prior to disease development or curative, meaning after disease development.

The compositions of the invention may be employed in any conventional form, for example in the form of a twin pack, a powder for dry seed treatment (DS), an emulsion for seed treatment (ES), a flowable concentrate for seed treatment (FS), a solution for seed treatment (LS), a water dispersible powder for seed treatment (WS), a capsule suspension for seed treatment (CF), a gel for seed treatment (GF), an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a wettable powder (WP) or any technically feasible formulation in combination with agriculturally acceptable adjuvants.

Such compositions may be produced in conventional manner, e.g. by mixing the active ingredients with appropriate formulation inerts (diluents, solvents, fillers and optionally other formulating ingredients such as surfactants, biocides, anti-freeze, stickers, thickeners and compounds that provide adjuvancy effects). Also conventional slow release formulations may be employed where long lasting efficacy is intended. Particularly, formulations to be applied in spraying forms, such as water dispersible concentrates (e.g. EC, SC, DC, OD, SE, EW, EO and the like), wettable powders and granules, may contain surfactants such as wetting and dispersing agents and other compounds that provide adjuvancy effects, e.g. the condensation product of formaldehyde with naphthalene sulphonate, an alkylarylsulphonate, a lignin sulphonate, a fatty alkyl sulphate, and ethoxylated alkylphenol and an ethoxylated fatty alcohol.

A seed dressing formulation is applied in a manner known per se to the seeds employing the combination of the invention and a diluent in suitable seed dressing formulation form, e.g. as an aqueous suspension or in a dry powder form having good adherence to the seeds. Such seed dressing formulations are known in the art. Seed dressing formulations may contain the single active ingredients or the combination of active ingredients in encapsulated form, e.g. as slow release capsules or microcapsules.

In general, the formulations include from 0.01 to 90% by weight of active agent, from 0 to 20% agriculturally acceptable surfactant and 10 to 99.99% solid or liquid formulation inerts and adjuvant(s), the active agent consisting of at least the compound of Formula (I) optionally together with other active agents, particularly microbiocides or conservatives or the like. Concentrated forms of compositions generally contain in between about 2 and 80%, preferably between about 5 and 70% by weight of active agent. Application forms of formulation may for example contain from 0.01 to 20% by weight, preferably from 0.01 to 5% by weight of active agent. Whereas commercial products will preferably be formulated as concentrates, the end user will normally employ diluted formulations.

Whereas it is preferred to formulate commercial products as concentrates, the end user will normally use dilute formulations.

### Table 1.1: This table discloses 26 specific compounds of the formula (T-1):

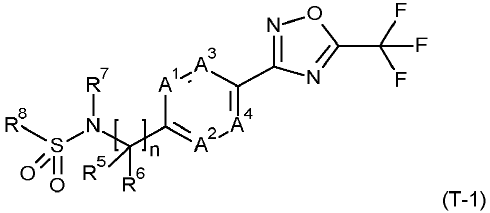

wherein n is 1, A¹ is C-R¹, A² is C-R², A³ is C-R³, A⁴ is C-R⁴ and R¹, R², R³, R⁴, R⁵, and R⁶ are hydrogen, R⁷ is methyl, and R⁸ is as defined below in Table 1.

Each of Tables 1.2 to 1.7 (which follow Table 1.1) make available 26 individual compounds of the Formula (T-1) in which n, A¹, A², A³, A⁴, R¹, R², R³, R⁴, R⁵, R⁶, and R⁷ are as specifically defined in Tables 1.2 to 1.7, which refer to Table 1 wherein R⁸ is specifically defined.

**Table 1**

| Compound no. | R⁸ | Compound no. | R⁸ |
|---|---|---|---|
| 1.001 | methyl | 1.014 | cyclopentylmethyl |
| 1.002 | ethyl | 1.015 | tetrahydrofuran-3-ylmethyl |
| 1.003 | propyl | 1.016 | phenyl |
| 1.004 | isopropyl | 1.017 | benzyl |
| 1.005 | n-butyl | 1.018 | 4-methoxy-phenyl |
| 1.006 | 3,3,3-trifluoropropyl | 1.019 | 2-fluorophenyl |
| 1.007 | 2-methoxyethyl | 1.020 | 2,4-difluorophenyl |
| 1.008 | methyl 1-ethanoate | 1.021 | 2,4-dichlorophenylmethyl |
| 1.009 | methyl 1-propanoate | 1.022 | 4-chloro-2-fluoro-phenylmethyl |
| 1.010 | prop-1-enyl | 1.023 | 5-methyl-thiophen-2-yl |
| 1.011 | cyclopropyl | 1.024 | 1-methyl-pyrazol-3-yl |
| 1.012 | cyclopropylmethyl | 1.025 | pyrrolidin-1-yl |
| 1.013 | 2,2-dichlorocyclopropylmethyl | 1.026 | morpholin-4-yl |

Table 1.2: This table discloses 26 specific compounds of Formula (T-1) wherein n is 1, A¹ is C-R¹, A² is C-R², A³ is C-R³, A⁴ is C-R⁴ and R¹, R², R³, R⁴, R⁵, and R⁶ are hydrogen, and R⁷ is methoxy, and R⁸ is as defined above in Table 1.

Reference Table 1.3: This table discloses 26 specific compounds of Formula (T-1) wherein n is 1, A¹ is C-R¹, A² is C-R², A³ is C-R³, A⁴ is C-R⁴ and R¹, R², R³, R⁴, R⁵, and R⁶ are hydrogen, and R⁷ is ethyl, and R⁸ is as defined above in Table 1.

Reference Table 1.4: This table discloses 26 specific compounds of Formula (T-1) wherein n is 1, A¹ is C-R¹, A² is C-R², A³ is C-R³, A⁴ is C-R⁴ and R¹, R², R³, R⁴, R⁵, and R⁶ are hydrogen, and R⁷ is propyl, and R⁸ is as defined above in Table 1.

Reference Table 1.5: This table discloses 26 specific compounds of Formula (T-1) wherein n is 1, A¹ is C-R¹, A² is C-R², A³ is C-R³, A⁴ is C-R⁴ and R¹, R², R³, R⁴, R⁵, and R⁶ are hydrogen, and R⁷ is isopropyl, and R⁸ is as defined above in Table 1.

Reference Table 1.6: This table discloses 26 specific compounds of Formula (T-1) wherein n is 1, A¹ is C-R¹, A² is C-R², A³ is C-R³, A⁴ is C-R⁴ and R¹, R², R³, R⁴, R⁵, and R⁶ are hydrogen, and R⁷ is cyclopropyl, and R⁸ is as defined above in Table 1.

Reference Table 1.7: This table discloses 26 specific compounds of Formula (T-1) wherein n is 1, A¹ is C-R¹, A² is C-R², A³ is C-R³, A⁴ is C-R⁴ and R¹, R², R³, R⁴, R⁵, R⁶ and R⁷ are hydrogen, and R⁸ is as defined above in Table 1.

### Table 2.1: This table discloses 6 specific compounds of the Formula (T-2):

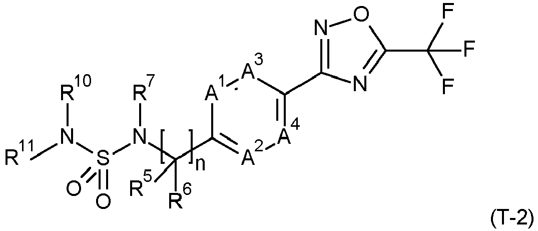

wherein n is 1, A¹ is C-R¹, A² is C-R², A³ is C-R³, A⁴ is C-R⁴ and R¹, R², R³, R⁴, R⁵, and R⁶ are hydrogen, R⁷ is methyl, and R⁸ is as defined below in Table 2.

Each of Tables 2.2 to 2.7 (which follow Table 2.1) make available 6 individual compounds of the formula (T-2) in which n, A¹, A², A³, A⁴, R¹, R², R³, R⁴, R⁵, R⁶, and R⁷ are as specifically defined in Tables 2.2 to 2.7, which refer to Table 2 wherein -N(R¹⁰)R¹¹ is specifically defined.

**Table 2**

| Compound no. | -N(R¹⁰)R¹¹ | Compound no. | -N(R¹⁰)R¹¹ |
|---|---|---|---|
| 2.001 | N-methylamino | 2.004 | N-cyclopropylamino |
| 2.002 | N-ethylamino | 2.005 | N-prop-2-ynylamino |
| 2.003 | N-propylamino | 2.006 | N,N-dimethylamino |

Table 2.2: This table discloses 6 specific compounds of Formula (T-2) wherein n is 1, A¹ is C-R¹, A² is C-R², A³ is C-R³, A⁴ is C-R⁴ and R¹, R², R³, R⁴, R⁵, and R⁶ are hydrogen, and R⁷ is methoxy, and-N(R¹⁰)R¹¹ is as defined above in Table 2.

Reference Table 2.3: This table discloses 6 specific compounds of Formula (T-2) wherein n is 1, A¹ is C-R¹, A² is C-R², A³ is C-R³, A⁴ is C-R⁴ and R¹, R², R³, R⁴, R⁵, and R⁶ are hydrogen, and R⁷ is ethyl, and -N(R¹⁰)R¹¹ is as defined above in Table 2.

Reference Table 2.4: This table discloses 6 specific compounds of Formula (T-2) wherein n is 1, A¹ is C-R¹, A² is C-R², A³ is C-R³, A⁴ is C-R⁴ and R¹, R², R³, R⁴, R⁵, and R⁶ are hydrogen, and R⁷ is propyl, and -N(R¹⁰)R¹¹ is as defined above in Table 2.

Reference Table 2.5: This table discloses 6 specific compounds of Formula (T-2) wherein n is 1, A¹ is C-R¹, A² is C-R², A³ is C-R³, A⁴ is C-R⁴ and R¹, R², R³, R⁴, R⁵, and R⁶ are hydrogen, and R⁷ is isopropyl, and -N(R¹⁰)R¹¹ is as defined above in Table 2.

Reference Table 2.6: This table discloses 6 specific compounds of Formula (T-2) wherein n is 1, A¹ is C-R¹, A² is C-R², A³ is C-R³, A⁴ is C-R⁴ and R¹, R², R³, R⁴, R⁵, and R⁶ are hydrogen, and R⁷ is cyclopropyl, and -N(R¹⁰)R¹¹ is as defined above in Table 2.

Reference Table 2.7: This table discloses 6 specific compounds of Formula (T-2) wherein n is 1, A¹ is C-R¹, A² is C-R², A³ is C-R³, A⁴ is C-R⁴ and R¹, R², R³, R⁴, R⁵, R⁶ and Rare hydrogen, and -N(R¹⁰)R¹¹ is as defined above in Table 2.

### EXAMPLES

The Examples which follow serve to illustrate the invention. The compounds of the invention can be distinguished from known compounds by virtue of greater efficacy at low application rates, which can be verified by the person skilled in the art using the experimental procedures outlined in the Examples, using lower application rates if necessary, for example 50 ppm, 12.5 ppm, 6 ppm, 3 ppm, 1.5 ppm, 0.8 ppm or 0.2 ppm.

Compounds of Formula (I) may possess any number of benefits including, *inter alia*, advantageous levels of biological activity for protecting plants against diseases that are caused by fungi or superior properties for use as agrochemical active ingredients (for example, greater biological activity, an advantageous spectrum of activity, an increased safety profile (including improved crop tolerance), improved physico-chemical properties, or increased biodegradability).

Throughout this description, temperatures are given in degrees Celsius (°C) and "mp." means melting point. LC/MS means Liquid Chromatography Mass Spectrometry and the description of the apparatus and the method (Methods A, B and C) is as follows:

### The description of the LC/MS apparatus and the method A is:

SQ Detector 2 from Waters
lonisation method: Electrospray
Polarity: positive and negative ions
Capillary (kV) 3.0, Cone (V) 30.00, Extractor (V) 2.00, Source Temperature (°C) 150, Desolvation Temperature (°C) 350, Cone Gas Flow (L/Hr) 0, Desolvation Gas Flow (L/Hr) 650
Mass range: 100 to 900 Da
DAD Wavelength range (nm): 210 to 500

### Method Waters ACQUITY UPLC with the following HPLC gradient conditions:

(Solvent A: Water/Methanol 20:1 + 0.05% formic acid and Solvent B: Acetonitrile+ 0.05% formic acid)

| Time (minutes) | A (%) | B (%) | Flow rate (ml/min) |
|---|---|---|---|
| 0 | 100 | 0 | 0.85 |
| 1.2 | 0 | 100 | 0.85 |
| 1.5 | 0 | 100 | 0.85 |

Type of column: Waters ACQUITY UPLC HSS T3; Column length: 30 mm; Internal diameter of column: 2.1 mm; Particle Size: 1.8 micron; Temperature: 60°C.

### The description of the LC/MS apparatus and the method B is:

SQ Detector 2 from Waters
lonisation method: Electrospray
Polarity: positive ions
Capillary (kV) 3.5, Cone (V) 30.00, Extractor (V) 3.00, Source Temperature (°C) 150, Desolvation Temperature (°C) 400, Cone Gas Flow (L/Hr) 60, Desolvation Gas Flow (L/Hr) 700
Mass range: 140 to 800 Da
DAD Wavelength range (nm): 210 to 400

### Method Waters ACQUITY UPLC with the following HPLC gradient conditions

(Solvent A: Water/Methanol 9:1 + 0.1% formic acid and Solvent B: Acetonitrile + 0.1% formic acid)

| Time (minutes) | A (%) | B (%) | Flow rate (ml/min) |
|---|---|---|---|
| 0 | 100 | 0 | 0.75 |
| 2.5 | 0 | 100 | 0.75 |
| 2.8 | 0 | 100 | 0.75 |
| 3.0 | 100 | 0 | 0.75 |

Type of column: Waters ACQUITY UPLC HSS T3; Column length: 30 mm; Internal diameter of column: 2.1 mm; Particle Size: 1.8 micron; Temperature: 60°C.

### The description of the LC/MS apparatus and the method C is:

SQ Detector 2 from Waters
lonisation method: Electrospray
ACQUITY H Class UPLC, Mass Spectrometer from Waters
Polarity: positive and Negative Polarity Switch
Scan Type MS1 Scan
Capillary (kV) 3.00, Cone (V) 40.00, Desolvation Temperature (°C) 500, Cone Gas Flow (L/Hr) 50, Desolvation Gas Flow (L/Hr) 1000
Mass range: 0 to 2000 Da
DAD Wavelength range (nm): 200 to 350

### Method Waters ACQUITY UPLC with the following HPLC gradient conditions

(Solvent A: Water +,0.1% formic acid and Solvent B: Acetonitrile)

| Time (minutes) | A (%) | B (%) | Flow rate (ml/min) |
|---|---|---|---|
| 0 | 70 | 30 | 0.5 |
| 0.05 | 70 | 30 | 0.5 |
| 0.8 | 5 | 95 | 0.5 |

| | | | |
|---|---|---|---|
| 1.8 | 5 | 95 | 0.5 |
| 2.45 | 70 | 30 | 0.5 |
| 2.50 | 70 | 30 | 0.5 |

Type of column: Waters ACQUITY UPLC BEH C18; Column length: 50 mm; Internal diameter of column: 2.1 mm; Particle Size: 1.7 micron; Temperature: 35°C.

### The description of the LC/MS apparatus and the method D is:

Mass Spectrometer: MSQ Single Quad From Thermo
HPLC : Surveyor Series HPLC From Thermo
lonisation method: Atmospheric Pressure Chemical Ionisation (APCI)
Polarity : Positive and Negative Polarity Switch
Scan Type: MS Full Scan
Cone Voltage (V): 50.00; Probe Temperature (°C): 400; Corona sid: 50
Detector (V): 1400
Mass range: 50 to 700 Da; Scan Time (Sec): 0.3

Gradient conditions: Solvent A: Water with 0.1% formic acid Solvent B: Methanol

| Time (minutes) | A (%) | B (%) | Flow rate (ml/min) |
|---|---|---|---|
| 0 | 90 | 10 | 1.7 |
| 0.5 | 90 | 10 | 1.7 |
| 1.5 | 0 | 100 | 1.7 |
| 4.0 | 0 | 100 | 1.7 |
| 5.0 | 90 | 10 | 1.7 |
| 5.5 | 90 | 10 | 1.7 |

Type of column : WATERS XTERRA C18; Column length: 30 mm; Internal diameter of column: 4.6 mm; Particle Size: 3.5 µ; UV Range (nm): 210-350

Where necessary, enantiomerically pure final compounds may be obtained from racemic materials as appropriate via standard physical separation techniques, such as reverse phase chiral chromatography, or through stereoselective synthetic techniques, e.g., by using chiral starting materials.

### Formulation Examples

| Wettable powders | a) | b) | c) |
|---|---|---|---|
| active ingredient [compound of formula (I)] | 25 % | 50 % | 75 % |
| sodium lignosulfonate | 5 % | 5 % | - |
| sodium lauryl sulfate | 3 % | - | 5 % |
| sodium diisobutylnaphthalenesulfonate | - | 6 % | 10 % |
| phenol polyethylene glycol ether (7-8 mol of ethylene oxide) | - | 2 % | - |
| highly dispersed silicic acid | 5 % | 10 % | 10 % |
| Kaolin | 62 % | 27 % | - |

The active ingredient is thoroughly mixed with the adjuvants and the mixture is thoroughly ground in a suitable mill, affording wettable powders that can be diluted with water to give suspensions of the desired concentration.

| Powders for dry seed treatment | a) | b) | c) |
|---|---|---|---|
| active ingredient [compound of formula (I)] | 25 % | 50 % | 75 % |
| light mineral oil | 5 % | 5 % | 5 % |
| highly dispersed silicic acid | 5 % | 5 % | - |
| Kaolin | 65 % | 40 % | - |
| Talcum | - | | 20 % |

The active ingredient is thoroughly mixed with the adjuvants and the mixture is thoroughly ground in a suitable mill, affording powders that can be used directly for seed treatment.

### Emulsifiable concentrate

| | |
|---|---|
| active ingredient [compound of formula (I)] | 10 % |
| octylphenol polyethylene glycol ether (4-5 mol of ethylene oxide) | 3 % |
| calcium dodecylbenzenesulfonate | 3 % |
| castor oil polyglycol ether (35 mol of ethylene oxide) | 4 % |
| Cyclohexanone | 30 % |
| xylene mixture | 50 % |

Emulsions of any required dilution, which can be used in plant protection, can be obtained from this concentrate by dilution with water.

| Dusts | a) | b) | c) |
|---|---|---|---|
| Active ingredient [compound of formula (I)] | 5 % | 6 % | 4 % |
| Talcum | 95 % | - | - |
| Kaolin | - | 94 % | - |
| mineral filler | - | - | 96 % |

Ready-for-use dusts are obtained by mixing the active ingredient with the carrier and grinding the mixture in a suitable mill. Such powders can also be used for dry dressings for seed.

### Extruder granules

| | |
|---|---|
| Active ingredient [compound of formula (I)] | 15 % |
| sodium lignosulfonate | 2 % |
| Carboxymethylcellulose | 1 % |
| Kaolin | 82 % |

The active ingredient is mixed and ground with the adjuvants, and the mixture is moistened with water. The mixture is extruded and then dried in a stream of air.

### Coated granules

| | |
|---|---|
| Active ingredient [compound of formula (I)] | 8 % |
| polyethylene glycol (mol. wt. 200) | 3 % |
| Kaolin | 89 % |

The finely ground active ingredient is uniformly applied, in a mixer, to the kaolin moistened with polyethylene glycol. Non-dusty coated granules are obtained in this manner.

### Suspension concentrate

| | |
|---|---|
| active ingredient [compound of formula (I)] | 40 % |
| propylene glycol | 10 % |
| nonylphenol polyethylene glycol ether (15 mol of ethylene oxide) | 6 % |
| Sodium lignosulfonate | 10 % |
| Carboxymethylcellulose | 1 % |
| silicone oil (in the form of a 75 % emulsion in water) | 1 % |
| Water | 32 % |

The finely ground active ingredient is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Flowable concentrate for seed treatment

| | |
|---|---|
| active ingredient [compound of formula (I)] | 40 % |
| propylene glycol | 5 % |
| copolymer butanol PO/EO | 2 % |
| tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground active ingredient is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow-Release Capsule Suspension

28 parts of a combination of the compound of formula (I) are mixed with 2 parts of an aromatic solvent and 7 parts of toluene diisocyanate/polymethylene-polyphenylisocyanate-mixture (8:1). This mixture is emulsified in a mixture of 1.2 parts of polyvinylalcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed.

The obtained capsule suspension is stabilized by adding 0.25 parts of a thickener and 3 parts of a dispersing agent. The capsule suspension formulation contains 28% of the active ingredients. The medium capsule diameter is 8-15 microns.

The resulting formulation is applied to seeds as an aqueous suspension in an apparatus suitable for that purpose.

### List of Abbreviations:

- AIBN =: azobisisobutyronitrile
- brs =: broad singlet
- DCM =: dichloromethane
- DIBAL-H =: diisobutylaluminium hydride
- DIPEA =: N,N-diisopropylethylamine
- DMF =: dimethylformamide
- EtOAc =: ethyl acetate
- EtOH =: ethyl alcohol
- HCl =: hydrochloric acid
- mp =: melting point
- MeOH =: methyl alcohol
- N =: normal
- NaH =: sodium hydride
- NaOH =: sodium hydroxide
- NBS =: *N-bromosuccinimide*
- *RT* =: *room temperature*
- *TBME* =: *tert-butyl methyl ether*
- TFAA =: trifluoroacetic acid anhydride
- THF =: tetrahydrofuran

### Preparation Examples

Using the synthetic techniques described both above and below, compounds of Formula (I) may be prepared accordingly.

### Comparative Example 1: This example illustrates the preparation of 1-cyclopropyl-N-[[4-[5-(trifluoromethyl)-1,2,4-oxadiazol-3-yl]phenyl]methyl]methanesulfonamide (Compound 3.3 Table T3)

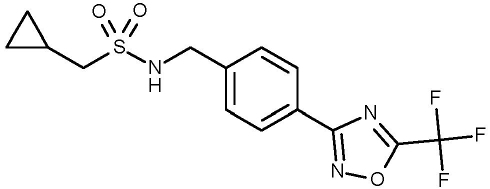

### Step 1: Preparation of 4-[5-(trifluoromethyl)-1,2,4-oxadiazol-3-yl]benzoyl chloride

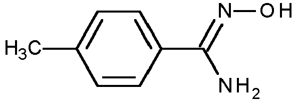

To a stirred suspension of 4-methylbenzonitrile (35 g, 0.29 mol) in ethanol (220 mL) and water (440 mL) was added at room temperature hydroxylamine hydrochloride (41.1 g, 0.58 mol), potassium carbonate (65.4 g, 0.47 mol) and 8-hydroxyquinoline (0.22 g, 1.5 mmol). The reaction mixture was heated at 80°C for 4 hours. The mixture was cooled to room temperature and diluted with 2N HCl until pH 8. Ethanol was evaporated under reduced pressure. The mixture was filtered, washed with water and dried under vacuum to afford 39.1 g of the title compound. LC/MS (Method A) retention time = 0.23 minutes, 151.0 (M+H).

### Step 2: Preparation of 3-(p-tolyl)-5-(trifluoromethyl)-1,2,4-oxadiazole

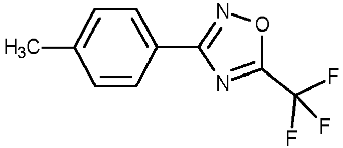

To a stirred solution of N'-hydroxy-4-methyl-benzamidine (38.7 g, 0.25 mol) in 2-methyltetrahydrofuran (750 mL) was added TFAA at 0°C. The reaction mixture was stirred at 15°C for two hours and diluted with water. The organic layer was separated, washed successively with sodium bicarbonate solution, ammonium chloride solution and water, dried over sodium sulfate, filtered and evaporated to dryness. The crude was subject to flash chromatography over silica gel (750g prepacked column) with heptane/EtOAc 99:1 to 90:10 to afford 54.1 g of the title compound as clear oil, which solidified after storage.

LC/MS (Method A) retention time = 1.15 minutes, mass not detected.

¹H NMR (400 MHz, CDCl₃) δ ppm: 8.00 (d, 2H), 7.32 (d, 2H), 2.45 (s,3H).

¹⁹F NMR (400 MHz, CDCl₃) δ ppm: -65.41 (s).

### Step 3a: Preparation of 3-[4-(bromomethyl)phenyl]-5-(trifluoromethyl)-1,2,4-oxadiazole

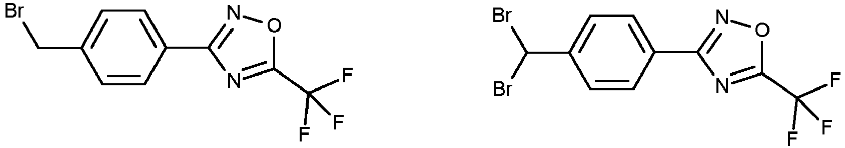

A stirred mixture of 3-(p-tolyl)-5-(trifluoromethyl)-1,2,4-oxadiazole (56.0 g, 0.24 mol) and NBS (45.4 g, 0.25 mol) in tetrachloromethane (480 mL) under argon was heated to 70°C. AIBN (4.03 g, 24 mmol) was added and the reaction mixture was stirred at 65°C for 18 hours. The mixture was cooled to room temperature and diluted with dichloromethane and water. Layers were separated. The organic layer was washed with sodium bicarbonate solution, dried over sodium sulfate, filtered and evaporated to dryness. The crude was subject to flash chromatography over silica gel (750g pre packed column) with cyclohexane/EtOAc 100:0 to 95:5 to afford 44.7 g of the title compound as a white solid mp: 58-63°C.

¹H NMR (400 MHz, CDCl₃) δ ppm: 8.11 (d, 2H), 7.55 (d, 2H), 4.53 (s,2H).

¹⁹F NMR (400 MHz, CDCl₃) δ ppm: -65.32 (s).

3-[4-(dibromomethyl)phenyl]-5-(trifluoromethyl)-1,2,4-oxadiazole was isolated as by-product as white solid mp: 61-66°C.

¹H NMR (400 MHz, CDCl₃) δ ppm: 8.15 (d, 2H), 7.73 (d, 2H), 6.68 (s, 1H).

¹⁹F NMR (400 MHz, CDCl₃) δ ppm: -65.34 (s).

### Step 3b: Preparation of 3-[4-(bromomethyl)phenyl]-5-(trifluoromethyl)-1,2,4-oxadiazole from 3-[4-(dibromomethyl)phenyl]-5-(trifluoromethyl)-1,2,4-oxadiazole

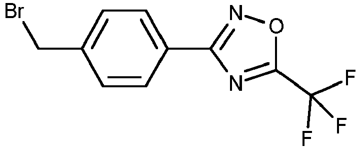

To a 1:9 ratio mixture of 3-[4-(bromomethyl)phenyl]-5-(trifluoromethyl)-1,2,4-oxadiazole and 3-[4-(dibromomethyl)phenyl]-5-(trifluoromethyl)-1,2,4-oxadiazole (10.2 g) in acetonitrile (95 mL), water (1.9 mL) and DIPEA (6.20 ml, 35.7 mmol) was added diethylphosphite (4.7 ml, 35.7 mmol) at 5°C. The mixture was stirred at 5-10°C for two hours, water and 1M HCl was added and acetonitrile was evaporated under reduced pressure. The white slurry was extracted thrice with dichloromethane. The combined organic layers were dried over sodium sulfate, and filtered. The solvent was removed under reduced pressure and the resultant crude was subject to flash chromatography over silica gel (40g prepacked column) with cyclohexane/EtOAc 99:1 to 9:1 to afford 7.10 g of 3-[4-(bromomethyl)phenyl]-5-(trifluoromethyl)-1,2,4-oxadiazole.

¹H NMR (400 MHz, CDCl₃) δ ppm: 8.11 (d, 2H), 7.55 (d, 2H), 4.53 (s,2H).

¹⁹F NMR (400 MHz, CDCl₃) δ ppm: -65.32 (s).

### Step 4: Preparation of [4-[5-(trifluoromethyl)-1,2,4-oxadiazol-3-yl]phenyl]methanamine hydrochloride

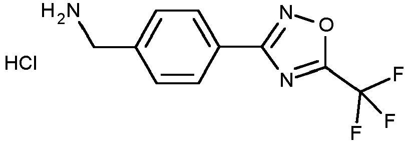

A dry flask equipped with stirrer under argon was charged with sodium hydride (2 equiv., 3.13 mmol, 60 mass% NaH) and tetrahydrofuran (25 mL). To this white suspension was added tert-butyl N-tert-butoxycarbonylcarbamate (1.1 equiv., 1.72 mmol) and while stirring for 5 min gas evolution was observed. 3-[4-(bromomethyl)phenyl]-5-(trifluoromethyl)-1,2,4-oxadiazole (0.500 g, 1.56 mmol) was then introduced and the contents were stirred for 12 hours. Upon reaction completion, the solution was poured into water and extracted with ethyl acetate (2×30mL). The organic layers were combined and dried over sodium sulfate, filtered, and concentrated at reduced pressure to produce a pale yellow oil which partially crystalize upon sitting. The yellow material was dissolved in dioxane (5 mL) and 4M hydrogen chloride in dioxane (15 equiv., 24.7 mmol,) was introduced dropwise. After stirring overnight at 22°C the reaction solution was diluted with ether and provided a white precipitate (70% yield) whose analytics matched the reported values and which was used without further purification. mp: >200°C.

LC/MS (Method A) retention time = 0.61 minutes, 244 (M+H).

¹H NMR (400 MHz, DMSO-*d₆*) δ ppm: 8.56 (brs, 2H), 8.13 (d, 2H), 7.75 (d, 2H), 4.15 (s, 2H).

¹⁹F NMR (400 MHz, DMSO-*d₆*) δ ppm: -64.69 (s).

Alternatively, the titled compound can be prepared using an analogous procedure as described in WO 2013/066839.

To a stirred solution of tert-butyl N-[[4-[5-(trifluoromethyl)-1,2,4-oxadiazol-3-yl]phenyl]methyl]-carbamate, (23.1 g, 65.4 mmol) in 1,4-dioxane (196 mL), was added drop wise at 70°C a solution of 4 M HCl in 1,4-dioxane (41 mL, 163 mmol). Precipitation of a white solid and gas liberation started 5 minutes after addition. The mixture was stirred for 6 hours at 70°C. The white suspension was cooled down to 23°C, filtered over sinter no 4 funnel, washed with 1,4-dioxane and dried under reduced pressure at 40°C to yield 17.3 g of [4-[5-(trifluoromethyl)-1,2,4-oxadiazol-3-yl]phenyl]methanamine hydrochloride as a yellow solid.

### Step 5: Preparation of 1-cyclopropyl-N-[[4-[5-(trifluoromethyl)-1,2,4-oxadiazo1-3-yl]phenyl]methyl] methane sulfonamide

A clear solution of [4-[5-(trifluoromethyl)-1,2,4-oxadiazol-3-yl]phenyl]methanamine hydrochloride (50 mg, 0.18 mmol) and DCM (1.8 mL) was treated with triethylamine (0.08 mL, 0.53 mmol) followed by cyclopropylmethanesulfonyl chloride (35 mg, 0.51 mmol). After 2 hours, Isolute^{®} sorbent was added to the reaction mixture and volatiles were removed under reduced pressure. The crude product was subjected to flash chromatography over silica gel with cyclohexane/EtOAc 99:1 to 1:1 to afford the title compound (47 mg, 73% yield) as a colourless oil. LC/MS (Method A) retention time = 1.05 minutes, 360 (M-H).

1H NMR (400 MHz, CDCl₃) δ ppm: 8.21 (d, 2H), 7.52 (d, 2H), 4.60 (m, 1H), 4.40 (d, 2H), 2.94 (d, 2H), 1.53 (s, 3H), 1.25 (m, 1H), 0.70 (q, 2H), 0.38 (q, 2H).

¹⁹F NMR (400 MHz, CDCl₃) δ ppm: -65.35 (s).

### Comparative Example 2: This example illustrates the preparation of 3-[4-[[methyl(propylsulfamoyl)amino]methyl] phenyl]-5-(trifluoromethyl)-1,2,4-oxadiazole (Compound 1.23 Table T1)

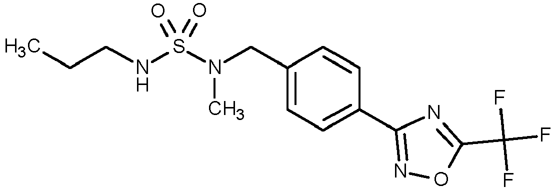

### Step 1: Preparation of N-[[4-[5-(trifluoromethyl)-1,2,4-oxadiazol-3-yl]phenyl]methyl]methanamine

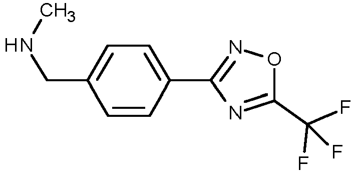

A solution of 3-[4-(bromomethyl)phenyl]-5-(trifluoromethyl)-1,2,4-oxadiazole (15 g, 46.9 mmol) in THF (20 mL) was added drop-wise at room temperature to a solution of methylamine 2M in THF (120 mL, 234.5 mmol). The mixture was stirred at room temperature for 24 hours. The solvent was removed under reduced pressure and the resultant crude material was purified by flash chromatography over silica gel (cyclohexane/EtOAc eluent gradient 1:0 to 1:1) to give 10.3 g of the title compound as a clear oil. LC/MS (Method A) retention time = 0.58 minutes, 258 (M+H).

¹H NMR (400 MHz, CDCl₃) δ ppm: 8.08 (d, 2H), 7.47 (d, 2H), 3.84 (s, 2H), 2.48 (s, 3H).

¹⁹F NMR (400 MHz, CDCl₃) δ ppm: -65.39 (s).

### Step 2: Preparation of 3-[4-[[methyl(propylsulfamoyl)amino]methyl]phenyl]-5-(trifluoromethyl)-1,2,4-oxadiazole

A clear solution of [N-[[4-[5-(trifluoromethyl)-1,2,4-oxadiazol-3-yl]phenyl]methyl]methanamine (0.5 mg, 1.94 mmol) and dichloromethane (5.8 mL) was treated with triethylamine (0.82 mL, 5.8 mmol) followed by N-propylsulfamoyl chloride (1.5 g, 9.7 mmol). After stirring overnight, water was added and the organics were extracted using dichloromethane. The crude product was subjected to flash chromatography over silica gel with cyclohexane/EtOAc 99:1 to 1:1 to afford the title compound (0.02 g, 2% yield) as a colorless oil.

¹H NMR (400 MHz, DMSO-*d₆*) δ ppm: 8.04 (d, 2H), 7.59 (d, 2H), 4.43 (s, 2H), 4.40 (d, 2H), 2.82 (q, 2H), 2.37 (m, 2H), 1.45 (q, 2H), 0.87 (t, 3H).

¹⁹F NMR (400 MHz, DMSO-*d₆*)) δ ppm: -64.71 (s).

### Comparative Example 3: This example illustrates the preparation of N-cyclopropyl-N-[[4-[5-(trifluoromethyl)-1,2,4-oxadiazol-3-yl]phenyl]methyl]ethanesulfonamide (Compound 1.1 Table T1)

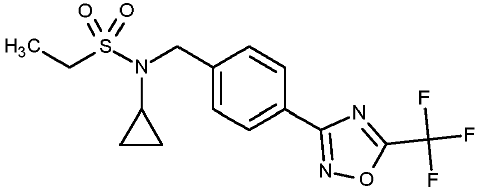

### Step 1: preparation of N-[[4-[5-(trifluoromethyl)-1,2,4-oxadiazol-3-yl]phenyl]methyl]cyclopropanamine

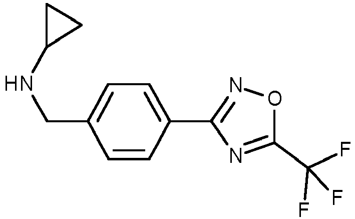

A solution of 3-[4-(bromomethyl)phenyl]-5-(trifluoromethyl)-1,2,4-oxadiazole (2.50 g, 8.1 mmol) in dichloromethane (5 mL) was added over 2 hours at room temperature to a solution of cyclopropanamine (3.7 g, 65 mmol) and N-ethyl-N-isopropyl-propan-2-amine (1.4 mL, 8.1 mmol) in dichloromethane (10 mL). The mixture was stirred at room temperature for 30 minutes, poured into water and then extracted with dichloromethane. The combined organic layers were washed with brine, dried over sodium sulfate, and filtered. The solvent was removed under reduced pressure and the resultant crude residue was purified by flash chromatography over silica gel (cyclohexane/EtOAc eluent gradient 1:0 to 1:1) to give 1.18 g of the title compound as a white solid, LC/MS (Method A) retention time = 0.71 minutes, 300 (M+H).

¹H NMR (400 MHz, CDCl₃) δ ppm: 8.06 (d, 2H), 7.49 (d, 2H), 3.92 (s, 2H), 2.18 (m, 1H), 1.85 (brs, 1H), 0.42 (m, 2H), 0.39 (m, 2H).

¹⁹F NMR (400 MHz, CDCl₃) δ ppm: -65.36 (s).

### Step 2: Preparation of N-cyclopropyl-N-[[4-[5-(trifluoromethyl)-1,2,4-oxadiazo1-3-yl]phenyl]methyl] ethanesulfonamide

A solution of N-[[4-[5-(trifluoromethyl)-1,2,4-oxadiazol-3-yl]phenyl]methyl]cyclopropanamine (0.20 g, 0.7 mmol) and DCM (2 mL) at 0°C was introduced triethylamine (0.2 g, 2.0 mmol) and after 10 minutes ethanesulfonyl chloride (0.1 g, 0.8 mmol). After 4 hours, water was added and the organics were extracted using dichloromethane. The total combined organic fraction was washed with water and brine then concentrated under reduced pressure. The crude product was subjected to flash chromatography over silica gel with cyclohexane/EtOAc 80:20 to 1:1 to afford 3-[4-[[cyclopropyl(ethylsulfamoyl)amino]methyl]phenyl]-5-(trifluoromethyl)-1,2,4-oxadiazole (0.16 g, 60% yield) as a white solid. mp. 108 - 110°C.

1H NMR (400 MHz, CDCl₃) δ ppm: 8.11 (d, 2H), 7.58 (d, 2H), 4.53 (s, 2H), 3.05 (q, 2H), 2.45 (m, 1H), 1.58 (m, 1H), 1.37 (m, 3H), 0.80 (m, 4H).

### Reference Example 4: This example illustrates the preparation of intermediate N-[[4-[5-(trifluoromethyl)-1,2,4-oxadiazol-3-yl]phenyl]methyl]ethanamine

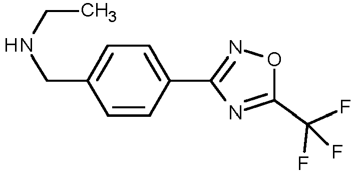

A solution of 3-[4-(bromomethyl)phenyl]-5-(trifluoromethyl)-1,2,4-oxadiazole (1.50 g, 4.69 mmol) in dichloromethane (9.4 mL) was added drop-wise at room temperature to a stirred solution of ethylamine 2 M in MeOH (12 mL, 24.0 mmol). The mixture was stirred at room temperature for 24 hours. The reaction mixture was poured into water and the layers were separated. The aqueous layer was extracted trice with dichloromethane. The combined organic layers were washed with brine, dried over sodium sulfate and filtered. The solvent was removed under reduced pressure and the resultant crude was purified by flash chromatography over silica gel (cyclohexane: EtOAc eluent gradient 1:0 to 1:1) to give 0.92 g of the title compound as a white solid mp: 102-112 °C, LC/MS (Method A) retention time = 0.66 minutes, 272 (M+H).

¹H NMR (400 MHz, CDCl₃) δ ppm: 8.01 (d, 2H), 7.57 (d, 2H), 3.86 (q, 2H), 3.29 (brs, 1H), 2.53 (q, 2H), 1.05 (t, 3H).

¹⁹F NMR (400 MHz, CDCl₃) δ ppm: -64.77 (s).

### Reference Example 5: This example illustrates the preparation of the intermediate 1-[3-fluoro-4-[5-(trifluoromethyl)-1,2,4-oxadiazol-3-yl]phenyl]-N-methoxy-methanamine

### Step 1: Preparation of 2-fluoro-N'-hydroxy-4-methyl-benzamidine

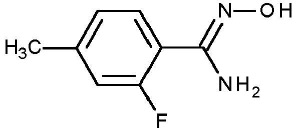

To a suspension of 2-fluoro-4-methylbenzonitrile (5 g, 37.0 mmol) in ethanol (125 mL) at 25°C was added hydroxylamine hydrochloride (7.7 g, 111 mmol). The reaction mixture was heated at 80°C for 2 h. After cooling to room temperature the volatiles were removed under reduced pressure thus affording a white solid that was used in the next step without any purification. LC/MS (Method A) retention time = 1.14 minutes, 169.2 (M+H).

¹H NMR (400 MHz, CDCl₃) δ ppm: 7.96 (t, 1H), 7.11 (m, 2H), 2.45 (s, 3H).

### Step 2: Preparation of 3-(2-fluoro-4-methyl-phenyl)-5-(trifluoromethyl)-1,2,4-oxadiazole

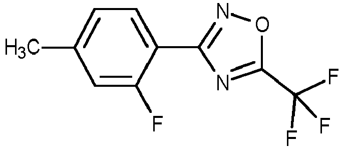

To a solution of 2-fluoro-N'-hydroxy-4-methyl-benzamidine (37 mmol) in tetrahydrofuran (122 mL) cooled *via* an ice bath was added TFAA (7.71 mL, 55.5 mmol). The reaction mixture was stirred at 25°C overnight and then diluted with water. The organic layer was separated, washed successively with sodium bicarbonate solution, ammonium chloride solution, and water then dried over sodium sulfate, filtered and evaporated to dryness. The crude product was subjected to flash chromatography over silica gel with cyclohexane/EtOAc 99:1 to 1:1 to afford the title compound (6.6 g, 72% yield) as an amorphous white solid. LC/MS (Method A) retention time = 1.14 minutes, 247 (M+H).

¹H NMR (400 MHz, CDCl₃) δ ppm: 8.00 (d, 1H), 7.32 (d, 2H), 2.45 (s, 3H).

¹⁹F NMR (400 MHz, CDCl₃) δ ppm: -65.3 (s), 108.1 (s).

### Step 3a: Preparation of 3-[4-(bromomethyl)phenyl]-5-(trifluoromethyl)-1,2,4-oxadiazole

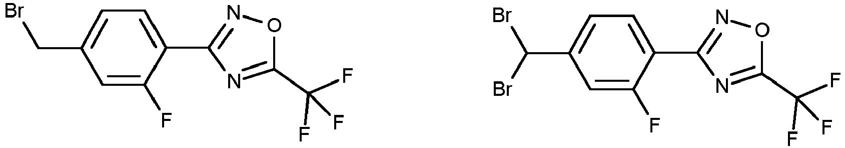

A stirred mixture of 3-(2-fluoro-4-methyl-phenyl)-5-(trifluoromethyl)-1,2,4-oxadiazole (4.2 g, 17.1 mmol) and NBS (3.11 g, 17.1 mmol) in tetrachloromethane (34.3 mL) under argon was heated to 70°C. AIBN (0.29 g, 1.71 mmol) was added and the reaction mixture stirred at 65°C for 18 hours. The mixture was cooled to 25°C then diluted with dichloromethane and water after which the layers were separated. The succinimide by-product was filtrated off, and the solvent was removed under reduced pressure to afford a brown gum. This crude residue was subjected to flash chromatography over silica gel (with cyclohexane/EtOAc 100:0 to 4:1 to afford the title compound as a white solid (1.7 g, 31% yield. LC/MS (Method A) retention time = 1.13 minutes, (M+H) not detected.

¹H NMR (400 MHz, CDCl₃) δ ppm: 8.09 (t, 1H), 7.34 (m, 2H), 4.49 (s, 2H).

¹⁹F NMR (400 MHz, CDCl₃) δ ppm: -65.18 (s), -106.2 (s).

3-[4-(dibromomethyl)phenyl]-5-(trifluoromethyl)-1,2,4-oxadiazole was isolated as by-product in the form of a beige solid (4.0 g, 58% yield) LC/MS (Method A) retention time = 1.20 minutes, (M+H) not detected.

¹H NMR (400 MHz, CDCl₃) δ ppm: 8.14 (d, 1H), 7.52 (dd, 2H), 6.63 (s, 1H).

### Step 3b: Preparation of 3-[4-(bromomethyl)-2-fluoro-phenyl]-5-(trifluoromethyl)-1,2,4-oxadiazole from 33-[4-(dibromomethyl)-2-fluoro-phenyl]-5-(trifluoromethyl)-1,2,4-oxadiazole

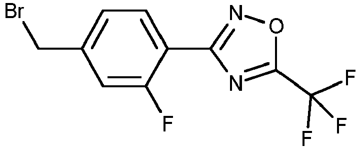

To a 1:20 ratio mixture of 3-[4-(bromomethyl)-2-fluoro-phenyl]-5-(trifluoromethyl)-1,2,4-oxadiazole and 3-[4-(dibromomethyl)-2-fluoro-phenyl]-5-(trifluoromethyl)-1,2,4-oxadiazole (4.0 g, 9.9 mmol) in acetonitrile (37 mL), water (0.8 mL) and DIPEA (2.59 mL, 14.8 mmol) at 5°C was added diethylphosphite (2.0 mL, 14.8 mmol). The mixture was stirred at 5-10°C for 2 hours, water and 1M HCl were added, and volatiles were removed under reduced pressure. The white slurry was extracted three times with dichloromethane and the combined organic layers were dried over sodium sulfate and filtered. The solvent was removed under reduced pressure and the resultant light orange colored crude residue was subjected to flash chromatography over silica gel with cyclohexane/EtOAc 99:1 to 1:1 to afford 3-[4-(bromomethyl)-2-fluoro-phenyl]-5-(trifluoromethyl)-1,2,4-oxadiazole (2.2 g, 68% yield). LC/MS (Method A) retention time = 1.13 minutes, (M+H) not detected.

¹H NMR (400 MHz, CDCl₃) δ ppm: 8.09 (t, 1H), 7.34 (m, 2H), 4.49 (s, 2H).

¹⁹F NMR (400 MHz, CDCl₃) δ ppm: -65.18 (s), -106.2 (s).

### Step 4: Preparation of 1-[3-fluoro-4-[5-(trifluoromethyl)-1,2,4-oxadiazol-3-yl]phenyl]-N-methoxy-methanamine

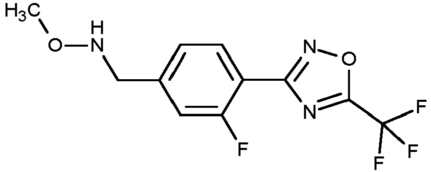

A solution of O-methylhydroxylamine hydrochloride (4.9 g, 59mmol) in dichloromethane (15mL) was treated drop-wise with DIPEA (12 mL, 66 mmol) followed by a solution of 3-[4-(bromomethyl)-2-flurorphenyl]-5-(trifluoromethyl)-1,2,4-oxadiazole (1.2 g, 3.7 mmol) in dichloromethane (5 mL). After 18h, water was introduced (10mL) and the reaction contents were extracted twice with dichloromethane and the combined organic layers were dried over sodium sulfate and filtered. The resultant residue was purified by flash chromatography over silica gel (cyclohexane/EtOAc eluent gradient 1:0 to 1:1) to afford 1-[3-fluoro-4-[5-(trifluoromethyl)-1,2,4-oxadiazol-3-yl]phenyl]-N-methoxy-methanamine as a colourless oil (410 mg, 38% yield). LC/MS (Method A) retention time = 1.01 minutes, (M+H) not detected.

¹H NMR (400 MHz, CDCl₃) δ ppm: 8.05 (t, 1H), 7.45 (m, 2H), 5.85 (brs, 1H), 4.12 (s, 2H), 3.50 (s, 3H).

¹⁹F NMR (400 MHz, CDCl₃) δ ppm: -65.21 (s), -107.33 (s).

### Reference Example 6: This example illustrates the preparation of the intermediate 1-[2,3-difluoro-4-[5-(trifluoromethyl)-1,2,4-oxadiazol-3-yl]phenyl]-N-methoxy-methanamine

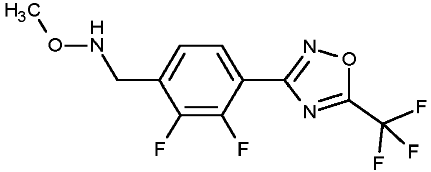

### Step 1: Preparation of 2,3-difluoro-N'-hydroxy-4-methyl-benzamidine

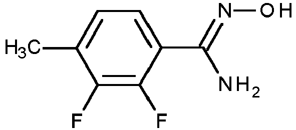

To a suspension of 2,3-difluoro-4-methylbenzonitrile (5.0 g, 32.6 mmol) in ethanol (111 mL) at 25°C was added hydroxylamine hydrochloride (4.5 g, 65.3 mmol). The reaction mixture was heated at 80°C for 2 hours. After cooling to room temperature the volatiles were removed under reduced pressure thus affording a white solid that was used in the next step without purification.

¹H NMR (400 MHz, CDCl₃) δ ppm: 7.30 (m, 1H), 6.95 (m, 1H), 6.50 (brs, 1H), 5.05 (brs, 2H), 2.30 (s, 3H).

### Step 2: Preparation of 3-(2,3-difluoro-4-methyl-phenyl)-5-(trifluoromethyl)-1,2,4-oxadiazole

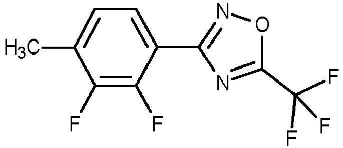

To a solution of 2,3-difluoro-N'-hydroxy-4-methyl-benzamidine (2.6 mmol) in tetrahydrofuran (108 mL) cooled using an ice bath was added TFAA (6.9 mL, 49 mmol). The reaction mixture was stirred at 25°C overnight and then diluted with water. The organic layer was separated, washed successively with sodium bicarbonate solution, ammonium chloride solution, and water then dried over sodium sulfate, filtered and evaporated to dryness. The crude title compound (6.6 g, 72% yield) was isolated as a light brown solid that was used in the next transformation without further purification. LC/MS (Method A) retention time = 1.16 minutes, 265 (M+H).

¹H NMR (400 MHz, CDCl₃) δ ppm: 7.76 (d, 1H), 7.12 (d, 1H), 2.41 (s, 3H).

¹⁹F NMR (400 MHz, CDCl₃) δ ppm: -65.41 (s), -133.3 (s), -140.1 (s).

### Step 3: Preparation of 3-[4-(bromomethyl)-2,3-difluoro-phenyl]-5-(trifluoromethyl)-1,2,4-oxadiazole

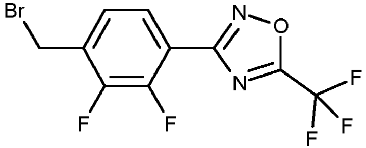

A mixture of 3-(2,3-difluoro-4-methyl-phenyl)-5-(trifluoromethyl)-1,2,4-oxadiazole (6.0 g, 22.6 mmol) and NBS (7.17 g, 10.0 mmol) in tetrachloromethane (79 mL) under argon was heated to 70°C. AIBN (0.68 g, 3.95 mmol) was added and the reaction mixture stirred at 65°C for 36 hours. The mixture was cooled to 25°C, diluted with dichloromethane and water, and the layers were separated. The succinimide by-product was filtered off, and the solvent was removed under vacuum, to afford a brown gum. This crude residue was subjected to flash chromatography over silica gel (cyclohexane/EtOAc eluent gradient 100:0 to 4:1) to afford the title compound as a white solid (4.8 g, 72% yield). LC/MS (Method A) retention time = 1.16 minutes, 344 (M+H).

¹H NMR (400 MHz, CDCl₃) δ ppm: 7.80 (m, 1H), 7.37 (m, 1H), 4.55 (s, 2H).

¹⁹F NMR (400 MHz, CDCl₃) δ ppm: -65.1 (s), -131.2 (s), -139.1 (s).

### Step 4: Preparation of 1-[2,3-difluoro-4-[5-(trifluoromethyl)-1,2,4-oxadiazol-3-yl]phenyl]-N-methoxy-methanamine

A solution of O-methylhydroxylamine hydrochloride (3.5g, 42 mmol) in dichloromethane (8 mL) was treated dropwise with DIPEA (8.3 mL, 47 mmol) followed by a solution of 3-[4-(bromomethyl)-2,3-difluoro-phenyl]-5-(trifluoromethyl)-1,2,4-oxadiazole (2 g, 5.2 mmol) in dichloromethane (5 mL). After 18 hours water was introduced (10 mL) and the reaction contents were extracted twice with dichloromethane and the combined organic layers were dried over sodium sulfate and filtered. The resultant residue was purified by flash chromatography over silica gel (cyclohexane/EtOAc eluent gradient 1:0 to 1:1) to afford 1-[2,3-difluoro-4-[5-(trifluoromethyl)-1,2,4-oxadiazol-3-yl]phenyl]-N-methoxy-methanamine as a pale yellow oil (1.1g, 68% yield). LC/MS (Method A) retention time = 1.03 minutes, 310 (M+H).

¹H NMR (400 MHz, CDCl₃) δ ppm: 7.84 (t, 1H), 7.38 (t, 1H), 5.87 (brs, 1H), 4.20 (s, 2H), 3.52 (s, 3H).

¹⁹F NMR (400 MHz, CDCl₃) δ ppm: -65.21 (s), -132.53 (s), -147.50 (s).

### Reference Example 7: This example illustrates the preparation of intermediate N-methoxy-1-[4-[5-(trifluoromethyl)-1,2,4-oxadiazol-3-yl]phenyl]methanamine

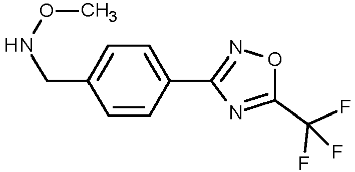

### Step 1: Preparation of 4-[5-(trifluoromethyl)-1,2,4-oxadiazol-3-yl]benzoyl chloride

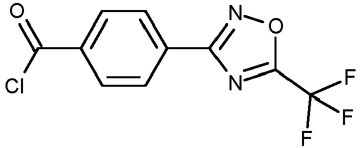

4-[5-(trifluoromethyl)-1,2,4-oxadiazol-3-yl]benzoic acid (4.00 g, 15.0 mmol) was suspended in dichloromethane (90 mL), DMF (0.01 mL, 0.150 mmol) was added followed by oxalyl chloride (1.46 mL, 16.5 mmol). The mixture was heated at reflux for 2 hours. The mixture was evaporated under reduced pressure to afford 4.15 g of 4-[5-(trifluoromethyl)-1,2,4-oxadiazol-3-yl]benzoyl chloride as a yellow solid.

### Step 2: Preparation of N-methoxy-N-methyl-4-[5-(trifluoromethyl)-1,2,4-oxadiazol-3-yl]benzamide

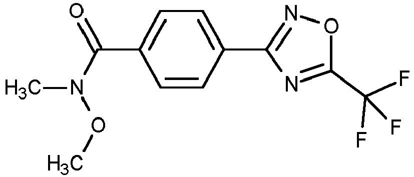

A solution of 4-[5-(trifluoromethyl)-1,2,4-oxadiazol-3-yl]benzoyl chloride from Step 1 (4.15 g, 14.6 mmol) in dichloromethane (20 mL) was added drop-wise at room temperature to a stirred solution of N-methoxymethanamine (1.10 g, 17.5 mmol) and triethylamine (3.10 ml, 21.8 mmol) in dichloromethane (80 mL). The mixture was stirred at room temperature for 18 hours. The reaction mixture was poured into water extracted twice with dichloromethane. The combined organic layers were washed with brine, dried over sodium sulfate, and filtered. The solvent was removed under reduced pressure and the resultant crude residue was subjected to flash chromatography over silica gel (heptane: EtOAc eluent gradient 9:1 to 65:35) to afford 4.12 g of N-methoxy-N-methyl-4-[5-(trifluoromethyl)-1,2,4-oxadiazol-3-yl]benzamide as a solid. LC/MS (Method A) retention time = 0.97 minutes, 302 (M+H).

¹H NMR (400 MHz, CDCl₃) δ ppm: 8.18 (d, 2H), 7.84 (d, 2H), 3.56 (s, 3H), 3.40 (s, 3H).

### Step 3: Preparation of 4-[5-(trifluoromethyl)-1,2,4-oxadiazol-3-yl]benzaldehyde

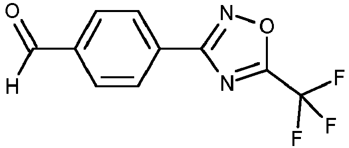

In a 75-mL multi neck flask equipped with stirrer, thermometer at -78°C under argon, DIBAL-H, 1.0M in toluene (16 mL, 16.0 mmol) was added drop-wise to a solution of N-methoxy-N-methyl-4-[5-(trifluoromethyl)-1,2,4-oxadiazol-3-yl]benzamide (4.10 g, 13.3 mmol) in 2-methyltetrahydrofuran (90 mL). The mixture was stirred two hours at -78°C and for one hour temperature was allowed to increase to 0°C. Complete conversion observed by LC/MS. The mixture was quenched by drop wise addition of sat. ammonium chloride solution. Precipitation of a white solid occurred. 4M HCI was added until full solubilisation. The mixture was extracted thrice with ethyl acetate. Combined organics were dried over magnesium sulfate and evaporated to afford the crude as beige solid. The crude was subject to flash chromatography over silica gel (heptane: EtOAc eluent gradient 99:1 to 90:10) to afford 2.93 g of 4-[5-(trifluoromethyl)-1,2,4-oxadiazol-3-yl]benzaldehyde as a white solid mp: 40-50°C.

¹H NMR (400 MHz, CDCl₃) δ ppm: 10.12 (s, 1H), 8.31 (d, 2H), 8.05 (d, 2H).

¹⁹F NMR (400 MHz, CDCl₃) δ ppm: -65.29 (s).

### Step 4: Preparation of N-methoxy-1-[4-[5-(trifluoromethyl)-1,2,4-oxadiazol-3-yl]phenyl]methanimine

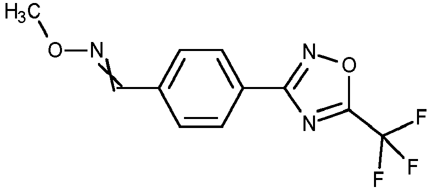

A solution of 4-[5-(trifluoromethyl)-1,2,4-oxadiazol-3-yl]benzaldehyde (0.30 g, 1.1 mmol) in methanol (11 mL) was treated at room temperature with pyridine (0.15 mL, 1.9 mmol) followed by addition of O-methylhydroxylamine hydrochloride (0.15 g, 1.8 mmol). The mixture was stirred at room temperature over the weekend, poured on water, acidified with 1M HCI and extracted trice with ethyl acetate. Combined organics were washed with water, dried over magnesium sulfate and evaporated to afford a resin. The crude was subject to flash chromatography over silica gel (heptane: EtOAc eluent gradient 100:0 to 95:5) to afford the title compound as a white solid mp: 55-65°C.

¹H NMR (400 MHz, CDCl₃) δ ppm: 8.14 (s, 1H), 8.11 (d, 2H), 7.73 (d, 2H).

¹⁹F NMR (400 MHz, CDCl₃) δ ppm: -65.40 (s).

### Step 5: Preparation of N-methoxv-1-[4-[5-(trifluoromethyl)-1,2,4-oxadiazol-3-yl]phenyl]methanamine

A solution of N-methoxy-1-[4-[5-(trifluoromethyl)-1,2,4-oxadiazol-3-yl]phenyl]methanimine (0.81 g, 2.89 mmol) in acetic acid (11 mL) was treated at 15°C with sodium cyano borohydride (0.4 g, 6.1 mmol). The mixture was stirred at 22°C for 18 hours until complete consumption of starting material was observed. The reaction mixture was carefully poured in 0.1M NaOH solution. By addition of 1M NaOH the pH was adjusted to 12-13. The mixture was extracted four times with TBME. Combined organics were washed twice with water, once with brine, then dried over magnesium sulfate and concentrated under reduced. The resultant green oil was purified by flash chromatography over silica gel (heptane: EtOAc eluent gradient 99:1 to 70:30) to give 0.60 g of the title compound as an oil.

¹H NMR (400 MHz, CDCl₃) δ ppm: 8.09 (d, 2H), 7.53 (d, 2H), 5.33 (brs, 1H), 4.12 (s, 2H), 3.50 (s, 3H).

¹⁹F NMR (400 MHz, CDCl₃) δ ppm: -65.32 (s).

### Comparative Example 8: This example illustrates the preparation of N-(dimethylsulfamoyl)-N-methyl-1-[4-[5-(trifluoromethyl)-1,2,4-oxadiazol-3-yl]phenyl]ethanamine (Compound 1.110 Table T1)

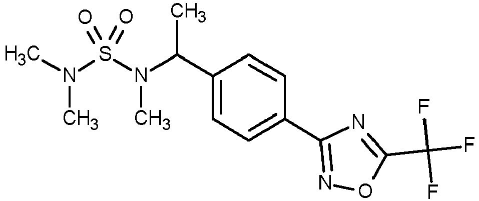

### Step 1: Preparation of 1-[4-[5-(trifluoromethyl)-1,2,4-oxadiazol-3-yl]phenyl]ethanol

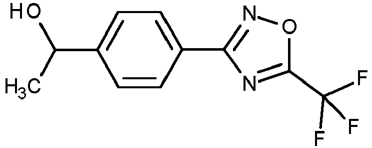

In a 50 mL flask dried and under argon, 4-[5-(trifluoromethyl)-1,2,4-oxadiazol-3-yl]benzaldehyde (1.36 mol) was dissolved in THF (10 mL) and cooled to -78°C *via* a dry ice acetone bath. To this solution was introduced dropwise methyl magnesium bromide (0.70 mL, 2.03M in diethyl ether). The mixture was stirred for 1h at -78°C and then was quenched with a saturated aqueous ammonium chloride solution. The dry ice bath was removed, and the reaction was stirred at room temperature for 5 min. it was then extracted with ethyl acetate. The combined organics were dried over sodium sulfate and concentrated under reduced pressure to afford the crude as a colourless oil. The crude was subject to combiflash chromatography over silica gel (heptane: EtOAc eluent gradient 99:1 to 1:1) to afford 1-[4-[5-(trifluoromethyl)-1,2,4-oxadiazol-3-yl]phenyl]ethanol as a white solid.

¹H NMR (400 MHz, CDCl₃) δ ppm: 8.12 (d, 2H), 7.54 (d, 2H), 5.00 (s, 1H), 1.54 (d, 3H).

¹⁹F NMR (400 MHz, CDCl₃) δ ppm: -65.31 (s).

### Step 2: Preparation of 3-[4-(1-bromoethyl)phenyl]-5-(trifluoromethyl)-1,2,4-oxadiazole

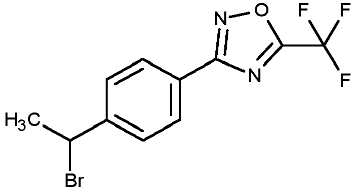

To a solution of 1-[4-[5-(trifluoromethyl)-1,2,4-oxadiazol-3-yl]phenyl]ethanol (1.15 g, 4.45 mmol) in dichloromethane (15 mL) cooled to 0°C using an ice bath was added tribromophosphane (0.465 ml, 4.90 mmol) over 30 minutes and the reaction mixture was stirred for 1.5 hrs. Then, an ice bath was used to cool the contents and a 10% sodium metabisulphite (50 ml) was introduced. After 15 minutes, the aqueous layer was extracted with dichloromethane and the total combined organic layer was washed with water and dried over Na₂SO₄, filtered and concentrated under reduced pressure. The resultant crude residue was purified by combiflash chromatography over silica gel using cyclohexane as eluent to afford pure 3-[4-(1-bromoethyl)phenyl]-5-(trifluoromethyl)-1,2,4-oxadiazole (1 g, 71% yield) as white solid.

¹H NMR (400 MHz, CDCl₃) δ ppm: 8.12 (m, 2H), 7.60 (m, 2H), 5.25 (q, 1H), 2.10 (d, 3H).

¹⁹F NMR (400 MHz, CDCl₃) δ ppm: -65.31 (s).

### Step 3: Preparation of N-methyl-N-[1-[4-[5-(trifluoromethyl)-1,2,4-oxadiazol-3-yl]phenyl]ethyl lmethanesulfonamide

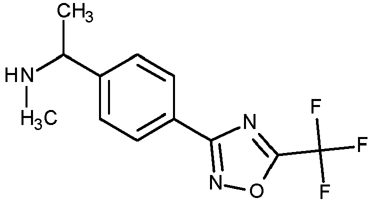

To a solution of 3-[4-(1-bromoethyl)phenyl]-5-(trifluoromethyl)-1,2,4-oxadiazole (1.0 g, 3.14 mmol) in THF (3 mL) at 0°C was added dropwise a solution of methylamine 2M in THF (7.8 mL,15.6 mmol). The mixture was stirred at 60°C for 3 hours. The solvent was removed under reduced pressure and the resultant crude material was purified by flash chromatography over silica gel (dichloromethane/methanol eluent gradient 90:10) to give 0.8 g of the title compound as a clear oil.

¹H NMR (400 MHz, CDCl₃) δ ppm: 8.05 (d, 2H), 7.62 (d, 2H), 3.88 (m, 1H), 3.40 (brs, 1H), 2.23 (m, 3H), 1.34 (m, 3H).

¹⁹F NMR (400 MHz, CDCl₃) δ ppm: -64.74 (s).

### Step 4: Preparation of N-(dimethylsulfamoyl)-N-methyl-1-[4-[5-(trifluoromethyl)-1,2,4-oxadiazol-3-yl]phenyl]ethanamine

A solution of N-methyl-N-[1-[4-[5-(trifluoromethyl)-1,2,4-oxadiazol-3-yl]phenyl]ethyl] methanesulfonamide (0.21 g, 0.37 mmol) and dichloromethane (3.7 mL) at 0°C was added triethylamine (0.16 mL, 1.1 mmol) and after 30 minutes N,N-dimethylsulfamoyl chloride (0.06 mL, 0.55 mmol) was introduced drop-wise over 15 minutes. The contents were stirred overnight and the volatiles were removed under reduced pressure. Ethyl acetate and water was added and the organics were extracted using ethyl acetate. The total combined organic fraction was washed with water, saturated aqueous sodium bicarbonate solution, brine, dried over sodium sulphate, and concentrated under reduced pressure. The crude product was subjected to flash chromatography over silica gel with cyclohexane/EtOAc 80:20 to 1:1 to afford the title compound (0.70 mg, 51% yield) as a white solid.

¹H NMR (400 MHz, CDCl₃) δ ppm: 8.11 (d, 2H), 7.57 (d, 2H), 5.23 (q, 1H), 2.83 (m, 6H), 2.58 (s, 3H), 1.63 (m, 3H).

¹⁹F NMR (400 MHz, CDCl₃) δ ppm: -65.34 (s).

The following procedure was used in a combinatorial fashion using appropriate building blocks (compounds (III) and (II)) to provide the compounds of Formula (I). The compounds prepared via the following combinatorial protocol were analyzed using LC/MS Method B.

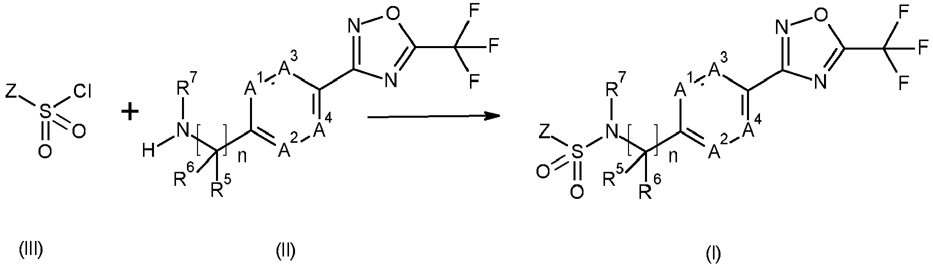

By way of exemplification, solutions of sulfonyl chloride derivatives of formula (III) (0.08 mmol) in ethyl acetate (1 mL) were transferred to a 96 slot deep well plate (DWP96) containing [[5-(trifluoromethyl)-1,2,4-oxadiazol-3-yl]aryl]methanamine derivatives of formula (II) (0.04 mmol) and DIPEA (0.16 mmol) in ethyl acetate (1 mL). The reaction mixtures were stirred at room temperature overnight then the ethyl acetate was removed using the Barkey station. The crude residues were solubilized in a mixture of MeOH (200 µL) and DMA (600 µL) and directly submitted for preparative LC/MS purification which provided the compounds of formula (I) in 10-85% yields.

**Table T1: Melting point (mp) data and/or retention times (Rₜ) for compounds according to Formula (I). The compounds 1.3, 1.5, 1.9, 1.18, 1.22, 1.40, 1.57, 1.58, 1.88to 1.95, 1.101, 1.111 and 1.112 of Table T1 are according to the invention. The compounds 1.1, 1.2, 1.4, 1.6 to 1.8, 1.10 to 1.17, 1.19 to 1.21, 1.23 to 1.39, 1.41 to 1.56, 1.59 to 1.87, 1.96 to 1.100 and 1.102 to 1.110 of Table T1 are not according to the invention.**

| Entry | IUPAC name | **Structure** | Rₜ (min) | [M+H] (measured) | Method | mp (°C) |
|---|---|---|---|---|---|---|
| 1.1 | N-cyclopropyl-N-[[4-[5-(trifluoromethyl)-1,2,4-oxadiazol-3-yl]phenyl]methyl]eth anesulfonamide | 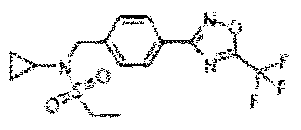 | | | | 86 - 88 |
| 1.2 | N-cyclopropyl-N-[[4-[5-(trifluoromethyl)-1,2,4-oxadiazol-3-yl]phenyl]methyl]cyc lopropanesulfonami de | 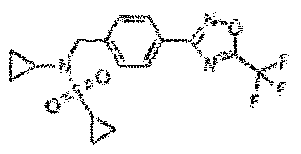 | | | | 90 - 92 |
| 1.3 | N-methoxy-N-[[4-[5-(trifluoromethyl)-1,2,4-oxadiazol-3-yl]phenyl]methyl]eth anesulfonamide | 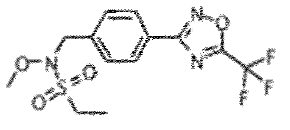 | | | | 44 - 46 |
| 1.4 | N-cyclopropyl-1-phenyl-N-[[4-[5-(trifluoromethyl)-1,2,4-oxadiazol-3-yl]phenyl]methyl]met hanesulfonamide | 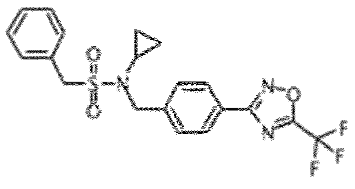 | Not obser ved | | | |
| 1.5 | N-methoxy-N-[[4-[5-(trifluoromethyl)-1,2,4-oxadiazol-3-yl]phenyl]methyl]met hanesulfonamide | 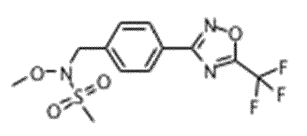 | | | | 94 - 96 |
| 1.6 | N-(dimethylsulfamoyl)-N-[[4-[5-(trifluoromethyl)-1,2,4-oxadiazol-3-yl]phenyl]methyl]cyc lopropanamine | 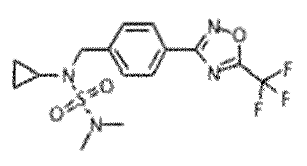 | | | | 82 - 84 |
| 1.7 | N-cyclopropyl-N-[[4-[5-(trifluoromethyl)-1,2,4-oxadiazol-3-yl]phenyl]methyl]mo rpholine-4-sulfonamide | 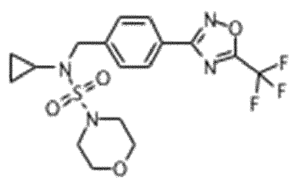 | | | | 99 - 101 |
| 1.8 | N-cyclopropyl-N-[[4-[5-(trifluoromethyl)-1,2,4-oxadiazol-3-yl]phenyl]methyl]met hanesulfonamide | 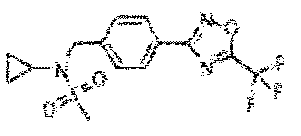 | | | | 84 - 86 |
| 1.9 | N-methoxy-N-[[4-[5-(trifluoromethyl)-1,2,4-oxadiazol-3-yl]phenyl]methyl]cyc lopropanesulfonami de | 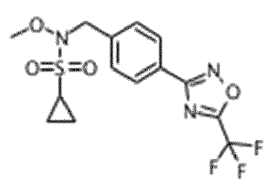 | 1.77 | 378.07 | B | 73-75 |
| 1.10 | N-methyl-N-[[4-[5-(trifluoromethyl)-1,2,4-oxadiazol-3-yl]phenyl]methyl]cyc lopropanesulfonami de | 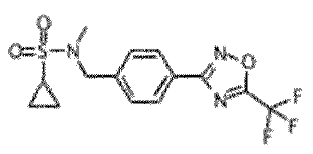 | 1.67 | 362.08 | B | 102-105 |
| 1.11 | N-methyl-N-[[4-[5-(trifluoromethyl)-1,2,4-oxadiazol-3-yl]phenyl]methyl]met hanesulfonamide | 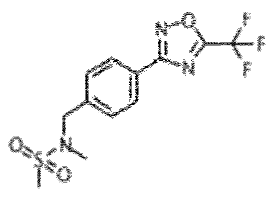 | 1.52 | 336.08 | B | 103-105 |
| 1.12 | N-(dimethylsulfamoyl)-N-methyl-1-[4-[5-(trifluoromethyl)-1,2,4-oxadiazol-3-yl]phenyl]methanam ine | 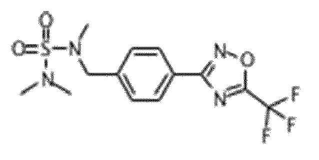 | 1.70 | 365.10 | B | 77-79 |
| 1.13 | N-methyl-N-[[4-[5-(trifluoromethyl)-1,2,4-oxadiazol-3-yl]phenyl]methyl]eth anesulfonamide | 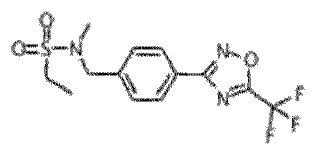 | 1.62 | 350.09 | B | 85-88 |
| 1.14 | N-methyl-1-phenyl-N-[[4-[5-(trifluoromethyl)-1,2,4-oxadiazol-3-yl]phenyl]methyl]met hanesulfonamide | 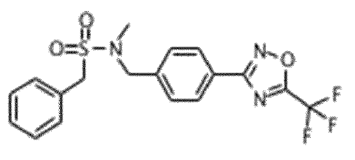 | 1.85 | 412.09 | B | 109-111 |
| 1.15 | N-methyl-N-[[4-[5-(trifluoromethyl)-1,2,4-oxadiazol-3-yl]phenyl]methyl]mo rpholine-4-sulfonamide | 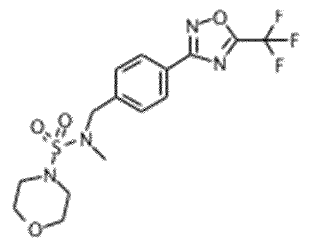 | | | | 118-120 |
| 1.16 | 3-[4-[[ethylsulfamoyl(met hyl)amino]methyl]ph enyl]-5-(trifluoromethyl)-1,2,4-oxadiazole | 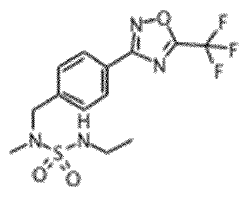 | | | | 63-66 |
| 1.17 | 3-[4-[[cyclopropyl(ethylsu Ifamoyl)amino]meth yl]phenyl]-5-(trifluoromethyl)-1,2,4-oxadiazole | 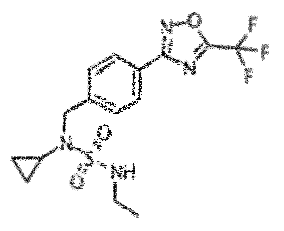 | | | | 108-110 |
| 1.18 | 3-[4-[[ethylsulfamoyl(met hoxy)amino]methyl] phenyl]-5-(trifluoromethyl)-1,2,4-oxadiazole | 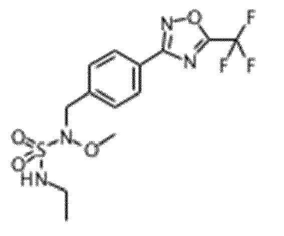 | 1.73 | (M+H) not observed | C | |
| 1.19 | 3-[4-[[methyl(prop-2-ynylsulfamoyl)amino ]methyl]phenyl]-5-(trifluoromethyl)-1,2,4-oxadiazole | 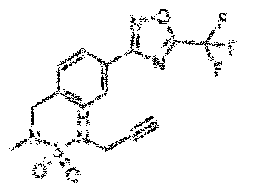 | 1.51 | 374.9 | C | |
| 1.20 | 3-[4-[[cyclopropyl(prop-2-ynylsulfamoyl)amino ]methyl]phenyl]-5-(trifluoromethyl)-1,2,4-oxadiazole | 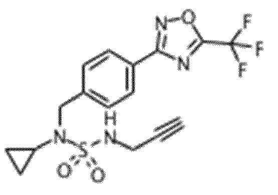 | 1.59 | 401.1 | C | |
| 1.21 | 3-[4-[[cyclopropyl (propyls ulfamoyl)amino]met hyl]phenyl]-5-(trifluoromethyl)-1,2,4-oxadiazole | 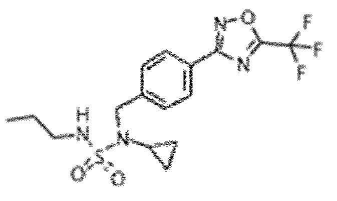 | | | | 70 - 73 |
| 1.22 | 3-[4-[[methoxy(propylsulf amoyl)amino]methyl ]phenyl]-5-(trifluoromethyl)-1,2,4-oxadiazole | 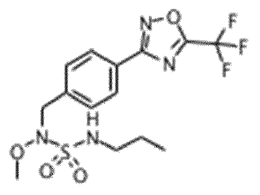 | 1.15 | 395.29 | C | |
| 1.23 | 3-[4-[[methyl(propylsulfa moyl)amino]methyl] phenyl]-5-(trifluoromethyl)-1,2,4-oxadiazole | 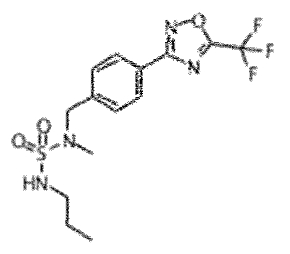 | 1.58 | 379 | C | |
| 1.24 | N-ethyl-N-[[4-[5-(trifluoromethyl)-1,2,4-oxadiazol-3-yl]phenyl]methyl]be nzenesulfonamide | 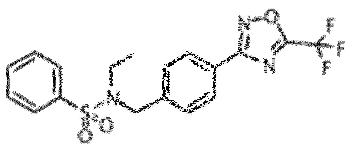 | 1.93 | 412.08 | B | |
| 1.25 | N-methyl-N-[[4-[5-(trifluoromethyl)-1,2,4-oxadiazol-3-yl]phenyl]methyl]pro pane-1-sulfonamide | 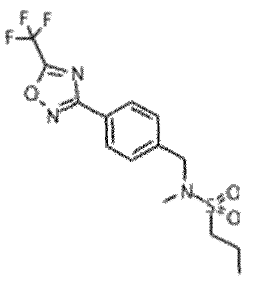 | 1.74 | 364.11 | B | |
| 1.26 | N-propyl-N-[[4-[5-(trifluoromethyl)-1,2,4-oxadiazol-3-yl]phenyl]methyl]pro pane-1-sulfonamide | 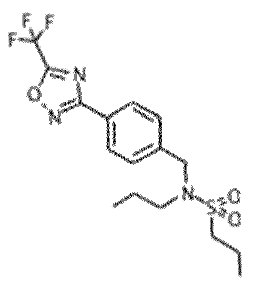 | 1.92 | 392.12 | B | |
| 1.27 | N-propyl-N-[[4-[5-(trifluoromethyl)-1,2,4-oxadiazol-3-yl]phenyl]methyl]be nzenesulfonamide | 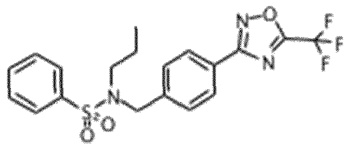 | 2.02 | 426.1 | B | |
| 1.28 | N-isopropyl-N-[[4-[5-(trifluoromethyl)-1,2,4-oxadiazol-3-yl]phenyl]methyl]be nzenesulfonamide | 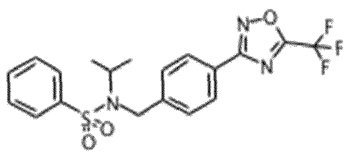 | 1.99 | 426.1 | B | |
| 1.29 | N-ethyl-1-methyl-N [[4-[5-(trifluoromethyl)-1,2,4-oxadiazol-3-yl]phenyl]methyl]pyr azole-3-sulfonamide | 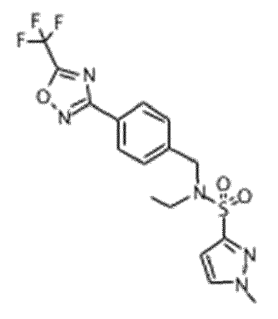 | 1.71 | 416.1 | B | |
| 1.30 | N-ethyl-5-methyl-N-[[4-[5-(trifluoromethyl)-1,2,4-oxadiazol-3-yl]phenyl]methyl]thio phene-2-sulfonamide | 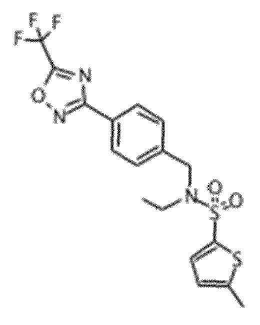 | 2.01 | 432.06 | B | |
| 1.31 | 1-(2,2-dichlorocyclopropyl) -N-ethyl-N-[[4-[5-(trifluoromethyl)-1,2,4-oxadiazol-3-yl]phenyl]methyl]met hanesulfonamide | 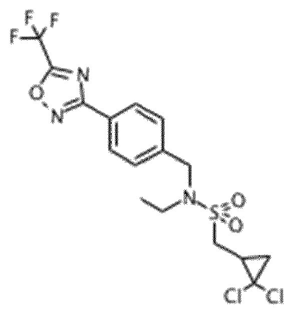 | 1.97 | 458.03 | B | |
| 1.32 | N-ethyl-N-[[4-[5-(trifluoromethyl)-1,2,4-oxadiazol-3-yl]phenyl]methyl]pyr rolidine-1-sulfonamide | 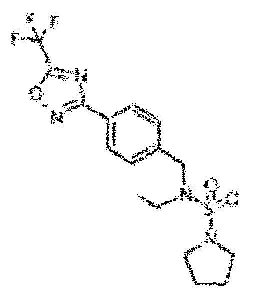 | 1.88 | 405.13 | B | |
| 1.33 | N-ethyl-N-[[4-[5-(trifluoromethyl)-1,2,4-oxadiazol-3-yl]phenyl]methyl]cyc lopropanesulfonami de | 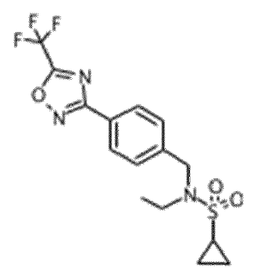 | 1.75 | 376.1 | B | |
| 1.34 | N-ethyl-N-[[4-[5-(trifluoromethyl)-1,2,4-oxadiazol-3-yl]phenyl]methyl]pro p-2-ene-1-sulfonamide | 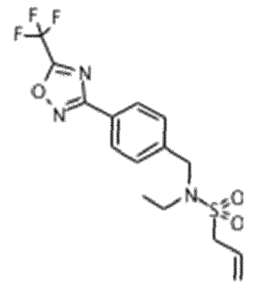 | 1.77 | 376.1 | B | |
| 1.35 | N-ethyl-1-tetra hyd rofu ran-3-yl-N-[[4-[5-(trifluoromethyl)-1,2,4-oxadiazol-3-yl]phenyl]methyl]met hanesulfonamide | 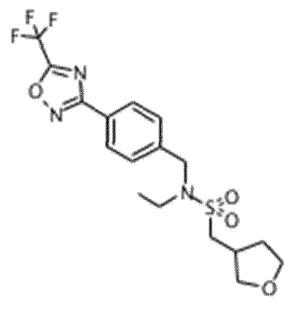 | 1.71 | 420.13 | B | |
| 1.36 | methyl 3-[ethyl-[[4-[5-(trifluoromethyl)-1,2,4-oxadiazol-3-yl]phenyl]methyl]sulf amoyl]propanoate | 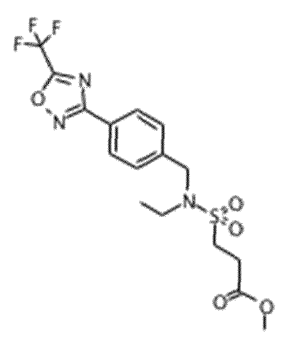 | 1.71 | 422.11 | B | |
| 1.37 | N-ethyl-2-methoxy-N-[[4-[5-(trifluoromethyl)-1,2,4-oxadiazol-3-yl]phenyl]methyl]eth anesulfonamide | 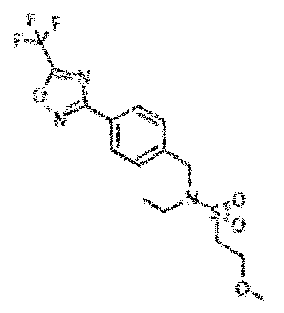 | 1.70 | 394.12 | B | |
| 1.38 | N-ethyl-3,3,3-trifluoro-N-[[4-[5-(trifluoromethyl)-1,2,4-oxadiazol-3-yl]phenyl]methyl]pro pane-1-sulfonamide | 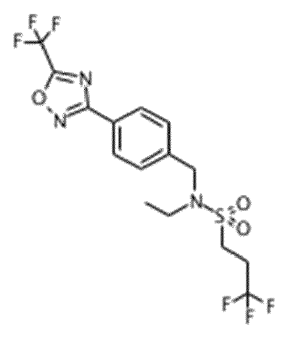 | 1.92 | 432.06 | B | |
| 1.39 | (E)- N-ethyl- N- [[4- [5-(trifluoromethyl)-1,2,4-oxadiazol-3-yl]phenyl]methyl]pro p-1-ene-1-sulfonamide | 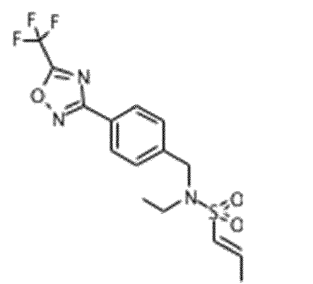 | 1.81 | 376.09 | B | |
| 1.40 | N,2-dimethoxy-N-[[4-[5-(trifluoromethyl)-1,2,4-oxadiazol-3-yl]phenyl]methyl]eth anesulfonamide | 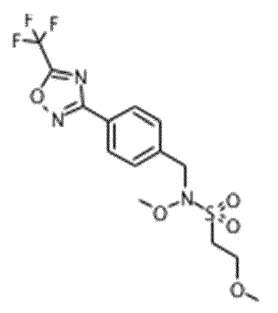 | 1.71 | 396.09 | B | |
| 1.41 | N,1-dimethyl-N-[[4-[5-(trifluoromethyl)-1,2,4-oxadiazol-3-yl]phenyl]methyl]pyr azole-3-sulfonamide | 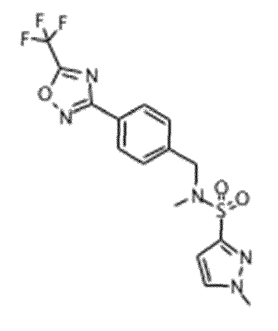 | 1.63 | 402.10 | B | |
| 1.42 | N,5-dimethyl-N-[[4-[5-(trifluoromethyl)-1,2,4-oxadiazol-3-yl]phenyl]methyl]thio phene-2-sulfonamide | 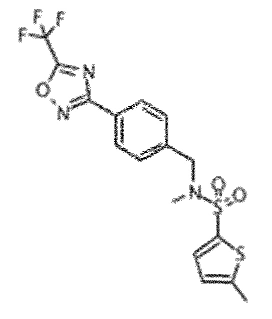 | 1.95 | 418.05 | B | |
| 1.43 | 1-(2,2-dichlorocyclopropyl) -N-methyl-N-[[4-[5-(trifluoromethyl)-1,2,4-oxadiazol-3-yl]phenyl]methyl]met hanesulfonamide | 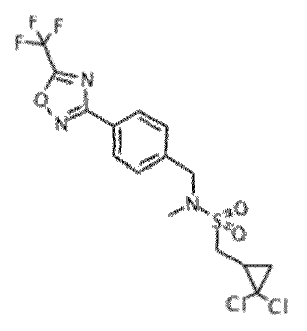 | 1.90 | 444.01 | B | |
| 1.44 | N-methyl-N-[[4-[5-(trifluoromethyl)-1,2,4-oxadiazol-3-yl]phenyl]methyl]pyr rolidine-1-sulfonamide | 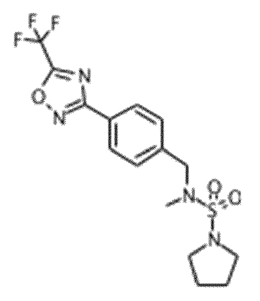 | 1.80 | 391.12 | B | |
| 1.45 | 1-cyclopentyl-N-methyl-N-[[4-[5-(trifluoromethyl)-1,2,4-oxadiazol-3-yl]phenyl]methyl]met hanesulfonamide | 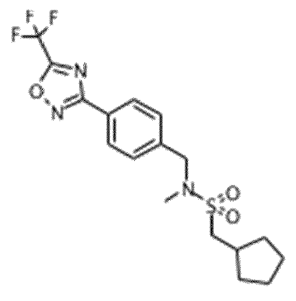 | 1.96 | 404.12 | B | |
| 1.46 | N-methyl-N-[[4-[5-(trifluoromethyl)-1,2,4-oxadiazol-3-yl]phenyl]methyl]pro p-2-ene-1-sulfonamide | 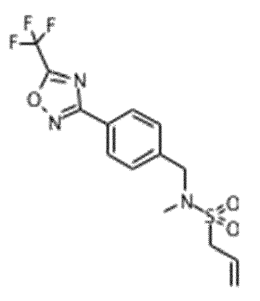 | 1.69 | 362.09 | B | |
| 1.47 | N-methyl-1-tetra hyd rofu ran-3-yl-N-[[4-[5-(trifluoromethyl)-1,2,4-oxadiazol-3-yl]phenyl]methyl]met hanesulfonamide | 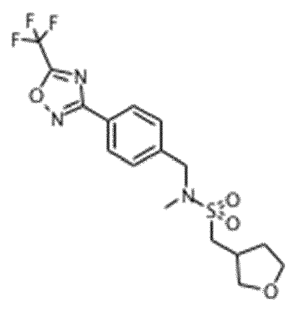 | 1.60 | 406.11 | B | |
| 1.48 | methyl 3-[methyl-[[4-[5-(trifluoromethyl)-1,2,4-oxadiazol-3-yl]phenyl]methyl]sulf amoyl]propanoate | 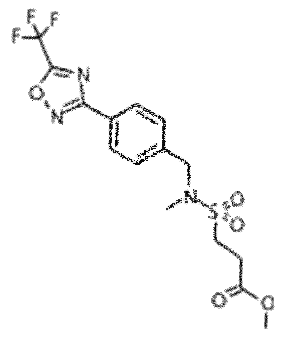 | 1.63 | 408.08 | B | |
| 1.49 | 2-methoxy-N-methyl-N-[[4-[5-(trifluoromethyl)-1,2,4-oxadiazol-3-yl]phenyl]methyl]eth anesulfonamide | 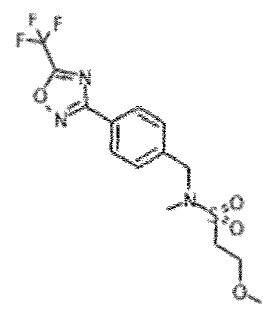 | 1.62 | 380.09 | B | |
| 1.50 | 3,3,3-trifluoro-N-methyl-N-[[4-[5-(trifluoromethyl)-1,2,4-oxadiazol-3-yl]phenyl]methyl]pro pane-1-sulfonamide | 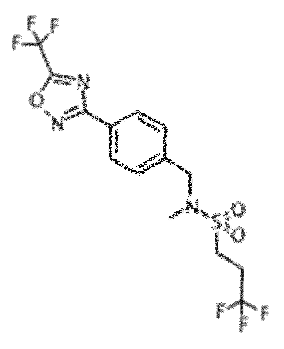 | 1.81 | 418.08 | B | |
| 1.51 | (E)-N-methyl-N-[[4-[5-(trifluoromethyl)-1,2,4-oxadiazol-3-yl]phenyl]methyl]pro p-1-ene-1-sulfonamide | 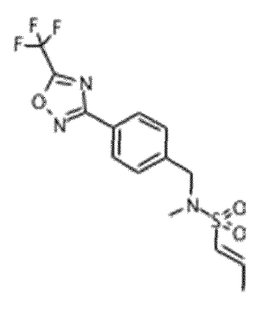 | 1.72 | 362.08 | B | |
| 1.52 | 1-cyclopropyl-N-methyl-N-[[4-[5-(trifluoromethyl)-1,2,4-oxadiazol-3-yl]phenyl]methyl]met hanesulfonamide | 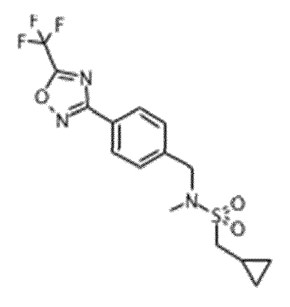 | 1.78 | 376.11 | B | |
| 1.53 | methyl 2-[methyl-[[4-[5-(trifluoromethyl)-1,2,4-oxadiazol-3-yl]phenyl]methyl]sulf amoyl]acetate | 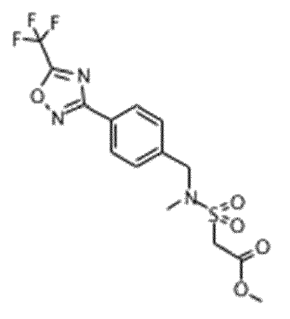 | 1.62 | 394.08 | B | |
| 1.54 | N-methyl-N-[[4-[5-(trifluoromethyl)-1,2,4-oxadiazol-3-yl]phenyl]methyl]pro pane-2-sulfonamide | 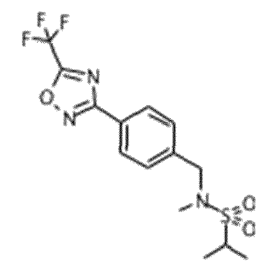 | 1.72 | 364.11 | B | |
| 1.55 | N-methyl-N-[[4-[5-(trifluoromethyl)-1,2,4-oxadiazol-3-yl]phenyl]methyl]but ane-1-sulfonamide | 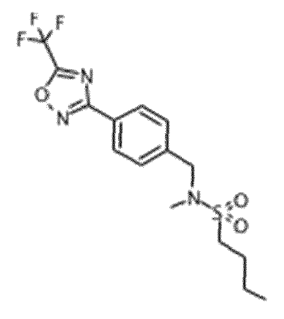 | 1.84 | 378.11 | B | I |
| 1.56 | 3-[4-[[methyl(methylsulfa moyl)amino]methyl] phenyl]-5-(trifluoromethyl)-1,2,4-oxadiazole | 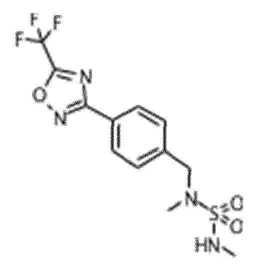 | 1.54 | 351.08 | B | |
| 1.57 | methyl 2-[methoxy-[[4-[5-(trifluoromethyl)-1,2,4-oxadiazol-3-yl]phenyl]methyl]sulf If amoyl]acetate | 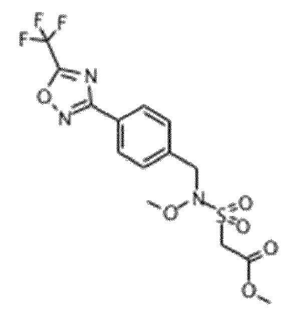 | 1.71 | 410.07 | B | |
| 1.58 | 3-[4-[[methoxy(methylsul famoyl)amino]methy I]phenyl]-5-(trifluoromethyl)-1,2,4-oxadiazole | 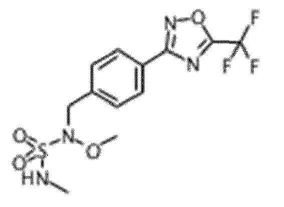 | 1.61 | 367.07 | B | |
| 1.59 | methyl 2-[ethyl-[[4-[5-(trifluoromethyl)-1,2,4-oxadiazol-3-yl]phenyl]methyl]sulf amoyl]acetate | 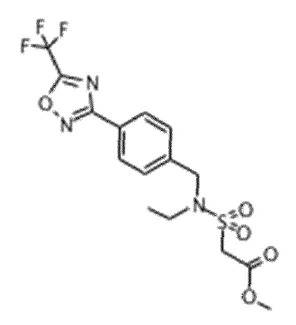 | 1.68 | 408.08 | B | |
| 1.60 | N-ethyl-1-phenyl-N-[[4-[5-(trifluoromethyl)-1,2,4-oxadiazol-3-yl]phenyl]methyl]met hanesulfonamide | 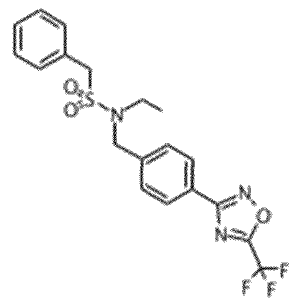 | 1.93 | 426.10 | B | |
| 1.61 | N-ethyl-N-[[4-[5-(trifluoromethyl)-1,2,4-oxadiazol-3-yl]phenyl]methyl]eth anesulfonamide | 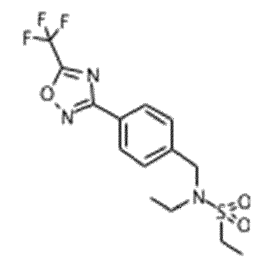 | 1.71 | 364.11 | B | |
| 1.62 | N-ethyl-N-[[4-[5-(trifluoromethyl)-1,2,4-oxadiazol-3-yl]phenyl]methyl]met hanesulfonamide | 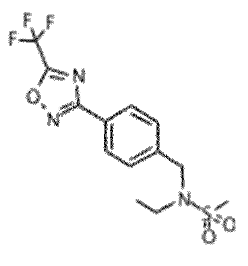 | 1.61 | 350.10 | B | |
| 1.63 | 3-[4-[[ethyl(methylsulfam oyl)amino]methyl]ph enyl]-5-(trifluoromethyl)-1,2,4-oxadiazole | 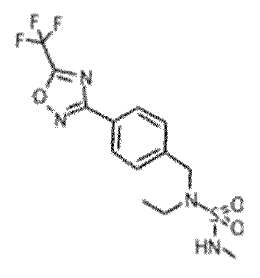 | 1.62 | 365.09 | B | |
| 1.64 | N-(dimethylsulfamoyl)-N-[[4-[5-(trifluoromethyl)-1,2,4-oxadiazol-3-yl]phenyl]methyl]eth anamine | 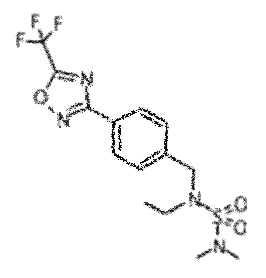 | 1.79 | 379.10 | B | |
| 1.65 | 1-methyl-N-propyl-N-[[4-[5-(trifluoromethyl)-1,2,4-oxadiazol-3-yl]phenyl]methyl]pyr azole-3-sulfonamide | 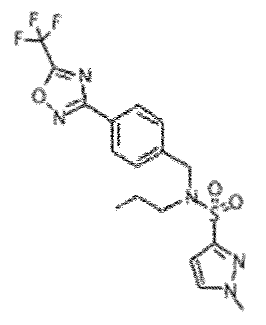 | 1.79 | 430.12 | B | |
| 1.66 | 5-methyl-N-propyl-N-[[4-[5-(trifluoromethyl)-1,2,4-oxadiazol-3-yl]phenyl]methyl]thio phene-2-sulfonamide | 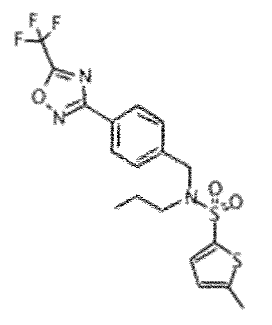 | 2.09 | 446.08 | B | |
| 1.67 | 1-(2,2-dichlorocyclopropyl) -N-propyl-N-[[4-[5-(trifluoromethyl)-1,2,4-oxadiazol-3-yl]phenyl]methyl]met hanesulfonamide | 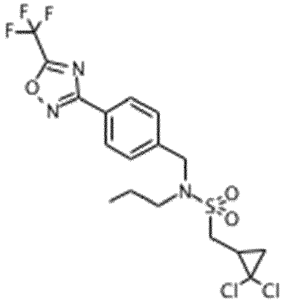 | 2.06 | 472.05 | B | |
| 1.68 | N-propyl-N-[[4-[5-(trifluoromethyl)-1,2,4-oxadiazol-3-yl]phenyl]methyl]pyr rolidine-1-sulfonamide | 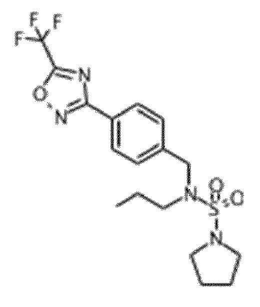 | 1.98 | 419.14 | B | |
| 1.69 | N-propyl-N-[[4-[5-(trifluoromethyl)-1,2,4-oxadiazol-3-yl]phenyl]methyl]cyc lopropanesulfonami de | 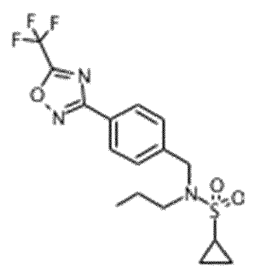 | 1.85 | 390.12 | B | |
| 1.70 | N-propyl-N-[[4-[5-(trifluoromethyl)-1,2,4-oxadiazol-3-yl]phenyl]methyl]pro p-2-ene-1-sulfonamide | 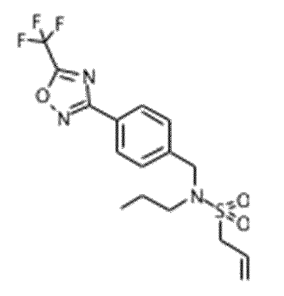 | 1.87 | 390.12 | B | |
| 1.71 | N-propyl-1-tetra hydrofuran-3-yl-N-[[4-[5-(trifluoromethyl)-1,2,4-oxadiazol-3-yl]phenyl]methyl]met hanesulfonamide | 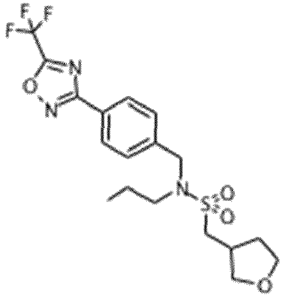 | 1.79 | 434.12 | | |
| 1.72 | methyl 3-[propyl-[[4-[5-(trifluoromethyl)-1,2,4-oxadiazol-3-yl]phenyl]methyl]sulf amoyl]propanoate | 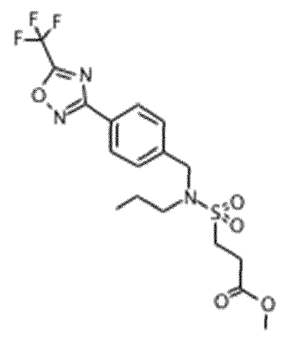 | 1.80 | 436.12 | B | |
| 1.73 | 2-methoxy-N-propyl-N-[[4-[5-(trifluoromethyl)-1,2,4-oxadiazol-3-yl]phenyl]methyl]eth anesulfonamide | 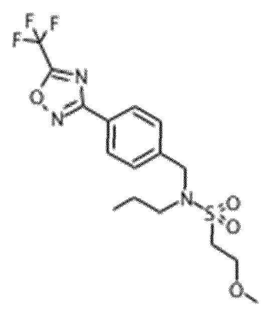 | 1.80 | 408.12 | B | |
| 1.74 | 3,3,3-trifluoro-N-propyl-N-[[4-[5-(trifluoromethyl)-1,2,4-oxadiazol-3-yl]phenyl]methyl]pro pane-1-sulfonamide | 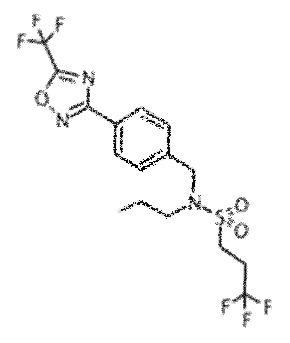 | 2.81 | 446.08 | B | |
| 1.75 | (E)-N-propyl-N-[[4-[5-(trifluoromethyl)-1,2,4-oxadiazol-3-yl]phenyl]methyl]pro p-1-ene-1-sulfonamide | 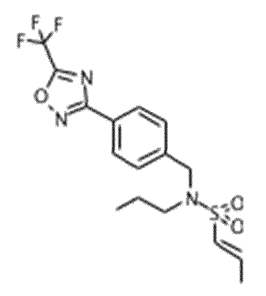 | 1.90 | 390.11 | B | |
| 1.76 | methyl 2-[propyl-[[4-[5-(trifluoromethyl)-1,2,4-oxadiazol-3-yl]phenyl]methyl]sulf amoyl]acetate | 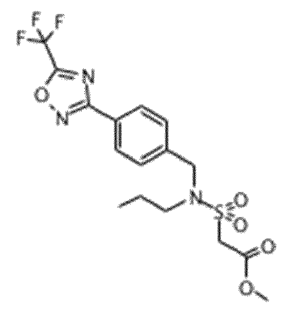 | 1.78 | 422.10 | B | |
| 1.77 | 1-phenyl-N-propyl-N-[[4-[5-(trifluoromethyl)-1,2,4-oxadiazol-3-yl]phenyl]methyl]met hanesulfonamide | 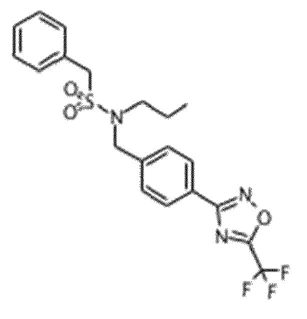 | 2.01 | 440.12 | B | |
| 1.78 | N-propyl-N-[[4-[5-(trifluoromethyl)-1,2,4-oxadiazol-3-yl]phenyl]methyl]eth anesulfonamide | 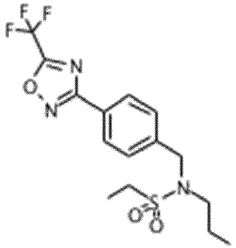 | 2.81 | 378.12 | B | |
| 1.79 | N-propyl-N-[[4-[5-(trifluoromethyl)-1,2,4-oxadiazol-3-yl]phenyl]methyl]met hanesulfonamide | 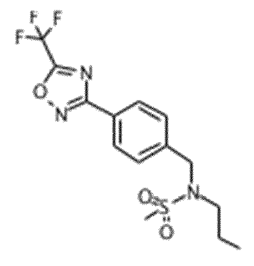 | 1.71 | 364.10 | B | |
| 1.80 | 3-[4-[[methylsulfamoyl(pr opyl)amino]methyl]p henyl]-5-(trifluoromethyl)-1,2,4-oxadiazole | 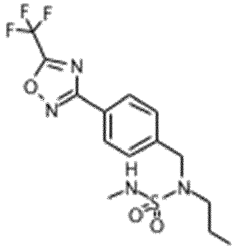 | 1.73 | 379.10 | B | |
| 1.81 | N-(dimethylsulfamoyl)-N-[[4-[5-(trifluoromethyl)-1,2,4-oxadiazol-3-yl]phenyl]methyl]pro pan-1-amine | 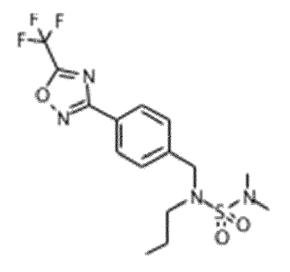 | 1.90 | 393.12 | B | |
| 1.82 | N-isopropyl-1-methyl-N-[[4-[5-(trifluoromethyl)-1,2,4-oxadiazol-3-yl]phenyl]methyl]pyr azole-3-sulfonamide | 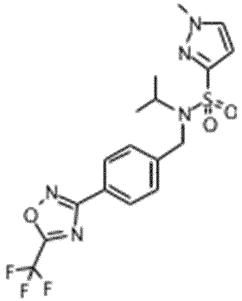 | 1.77 | 430.11 | B | |
| 1.83 | N-isopropyl-2-methoxy-N-[[4-[5-(trifluoromethyl)-1,2,4-oxadiazol-3-yl]phenyl]methyl]eth anesulfonamide | 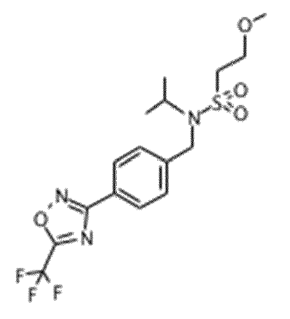 | 1.79 | 408.12 | B | |
| 1.84 | (E)-N-isopropyl-N-[[4-[5-(trifluoromethyl)-1,2,4-oxadiazol-3-yl]phenyl]methyl]pro p-1-ene-1-sulfonamide | 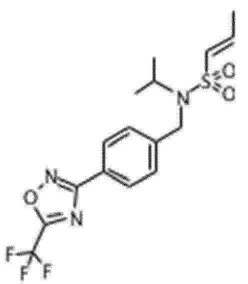 | 1.87 | 390.12 | B | |
| 1.85 | methyl 2-[isopropyl-[[4-[5-(trifluoromethyl)-1,2,4-oxadiazol-3-yl]phenyl]methyl]sulf amoyl]acetate | 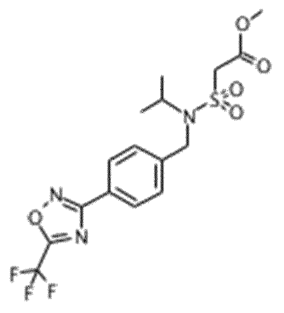 | 1.76 | 422.10 | B | |
| 1.86 | N-isopropyl-N-[[4-[5-(trifluoromethyl)-1,2,4-oxadiazol-3-yl]phenyl]methyl]met hanesulfonamide | 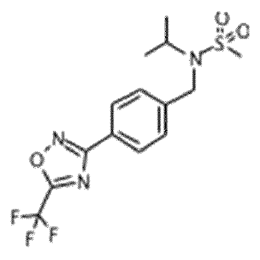 | 1.69 | 364.08 | B | |
| 1.87 | 3-[4-[[isopropyl(methylsul famoyl)amino]methy l]phenyl]-5-(trifluoromethyl)-1,2,4-oxadiazole | 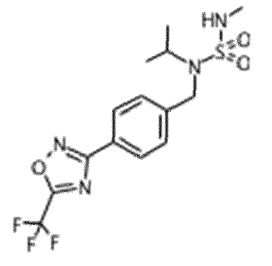 | 1.70 | 379.08 | B | |
| 1.88 | N-methoxy-1-methyl-N-[[4-[5-(trifluoromethyl)-1,2,4-oxadiazol-3-yl]phenyl]methyl]pyr azole-3-sulfonamide | 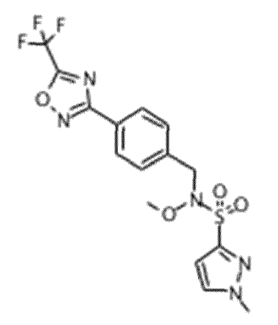 | 1.69 | 418.08 | B | |
| 1.89 | N-methoxy-5-methyl-N-[[4-[5-(trifluoromethyl)-1,2,4-oxadiazol-3-yl]phenyl]methyl]thio phene-2-sulfonamide | 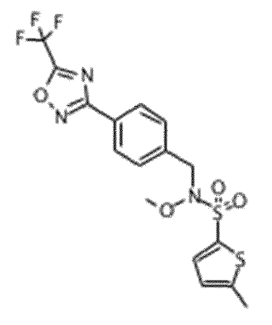 | 2.02 | 434.05 | B | |
| 1.90 | 1-(2,2-dichlorocyclopropyl) -N-methoxy-N-[[4-[5-(trifluoromethyl)-1,2,4-oxadiazol-3-yl]phenyl]methyl]met hanesulfonamide | 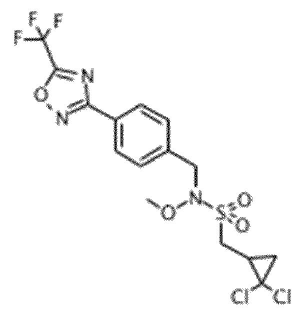 | 2.00 | 460.25 | B | |
| 1.91 | N-methoxy-N-[[4-[5-(trifluoromethyl)-1,2,4-oxadiazol-3-yl]phenyl]methyl]pyr rolidine-1-sulfonamide | 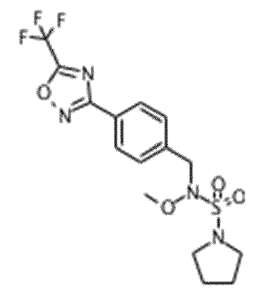 | 1.88 | 407.11 | B | |
| 1.92 | N-methoxy-N-[[4-[5-(trifluoromethyl)-1,2,4-oxadiazol-3-yl]phenyl]methyl]pro p-2-ene-1-sulfonamide | 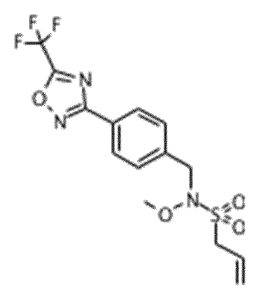 | 1.79 | 378.36 | B | |
| 1.93 | methyl 3-[methoxy-[[4-[5-(trifluoromethyl)-1,2,4-oxadiazol-3-yl]phenyl]methyl]sulf amoyl]propanoate | 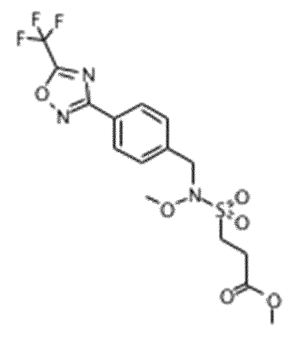 | 1.73 | 424.07 | B | |
| 1.94 | (E)-N-methoxy-N-[[4-[5-(trifluoromethyl)-1,2,4-oxadiazol-3-yl]phenyl]methyl]pro p-1-ene-1-sulfonamide | 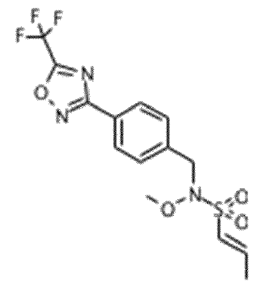 | 1.81 | 378.09 | B | |
| 1.95 | N-(dimethylsulfamoyl)-N-methoxy-1-[4-[5-(trifluoromethyl)-1,2,4-oxadiazol-3-yl]phenyl]methanam ine | 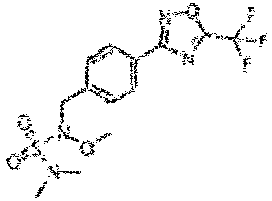 | 1.78 | 381.08 | B | |
| 1.96 | N-isopropyl-5-methyl-N-[[4-[5-(trifluoromethyl)-1,2,4-oxadiazol-3-yl]phenyl]methyl]thio phene-2-sulfonamide | 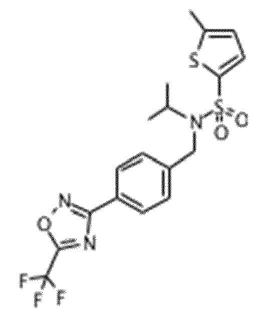 | 2.07 | 446.10 | B | |
| 1.97 | N-isopropyl-N-[[4-[5-(trifluoromethyl)-1,2,4-oxadiazol-3-yl]phenyl]methyl]pyr rolidine-1-sulfonamide | 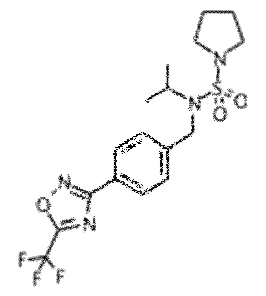 | 1.96 | 419.14 | B | |
| 1.98 | N-isopropyl-N-[[4-[5-(trifluoromethyl)-1,2,4-oxadiazol-3-yl]phenyl]methyl]cyc lopropanesulfonami de | 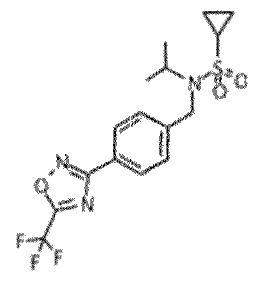 | 1.82 | 390.13 | B | |
| 1.99 | N-isopropyl-1-phenyl-N-[[4-[5-(trifluoromethyl)-1,2,4-oxadiazol-3-yl]phenyl]methyl]met hanesulfonamide | 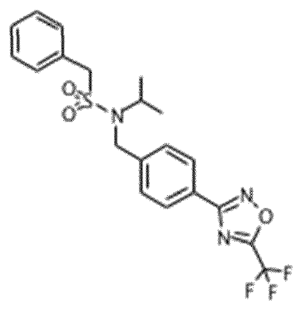 | 1.99 | 440.13 | B | |
| 1.100 | N-(dimethylsulfamoyl)-N-[[4-[5-(trifluoromethyl)-1,2,4-oxadiazol-3-yl]phenyl]methyl]pro pan-2-amine | 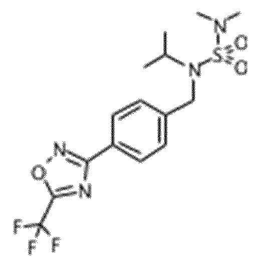 | 1.87 | 393.14 | B | |
| 1.101 | N-[[3-fluoro-4-[5-(trifluoromethyl)-1,2,4-oxadiazol-3-yl]phenyl]methyl]-N-methoxy-methanesulfonamid e | 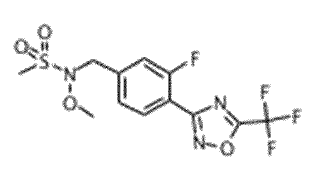 | 1.04 | (M+H) not observed | A | |
| 1.102 | N-ethyl-N-[[3-fluoro-4-[5-(trifluoromethyl)-1,2,4-oxadiazol-3-yl]phenyl]methyl]met hanesulfonamide | 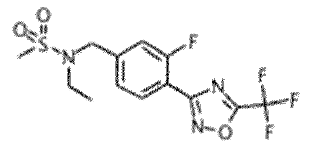 | 1.02 | 368 | A | |
| 1.103 | N-ethyl-N-[[2- fluoro-4-[5-(trifluoromethyl)-1,2,4-oxadiazol-3-yl]phenyl]methyl]met hanesulfonamide | 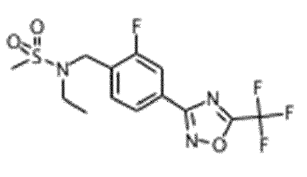 | | | | 90-93 |
| 1.104 | N-[[2,3-difluoro-4-[5-(trifluoromethyl)-1,2,4-oxadiazol-3-yl]phenyl]methyl]-N-ethyl-methanesulfonamid e | 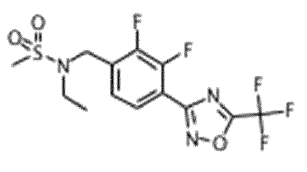 | | | | 81 - 83 |
| 1.105 | N-(dimethylsulfamoyl)-N-[[2-fluoro-4-[5-(trifluoromethyl)-1,2,4-oxadiazol-3-yl]phenyl]methyl]eth anamine | 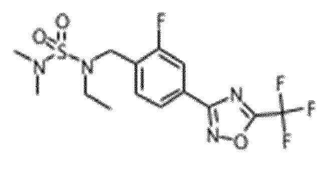 | 1.15 | 397.4 | A | |
| 1.106 | N-(dimethylsulfamoyl)-N-[[3-fluoro-4-[5-6 (trifluoromethyl)-1,2,4-oxadiazol-3-yl]phenyl]methyl]eth anamine | 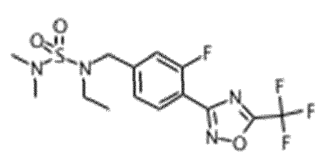 | 1.11 | 397.2 | A | |
| 1.107 | N-[[2,3-difluoro-4-[5-(trifluoromethyl)-1,2,4-oxadiazol-3-yl]phenyl]methyl]-N-(dimethylsulfamoyl) ethanamine | 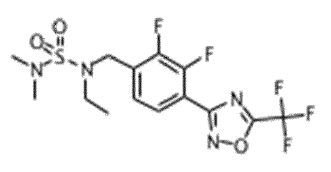 | 1.13 | 415.2 | A | |
| 1.108 | N-methyl-N-[1-[4-[5-(trifluoromethyl)-1,2,4-oxadiazol-3-yl]phenyl]ethyl]meth anesulfonamide | 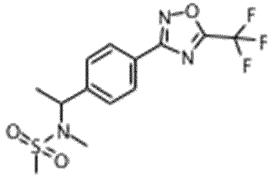 | 1.44 | (M+H) not observed | C | |
| 1.109 | N-cyclopropyl-N-[1-[4-[5-(trifluoromethyl)-1,2,4-oxadiazol-3-yl]phenyl]ethyl]meth anesulfonamide | 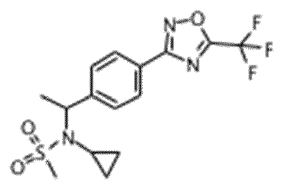 | 1.57 | (M+H) not observed | C | |
| 1.110 | N-(dimethylsulfamoyl)-N-methyl-1-[4-[5-(trifluoromethyl)-1,2,4-oxadiazol-3-yl]phenyl]ethanamin e | 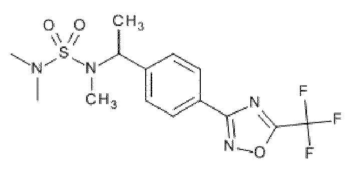 | Not obser ved | | | |
| 1.111 | N-methoxy-N-[[5-[5-(trifluoromethyl)-1,2,4-oxadiazol-3-yl]-2-pyridyl]methyl]benz enesulfonamide | 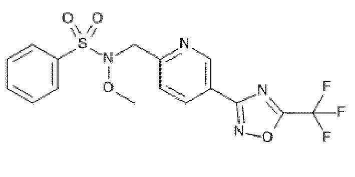 | 1.10 | 415.3 | A | |
| 1.112 | N-(dimethylsulfamoyl)-N-methoxy-1-[5-[5-(trifluoromethyl)-1,2,4-oxadiazol-3-yl]-2-pyridyl]methanamin e | 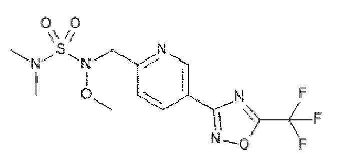 | Not obser ved | | | |

**Table T2: Melting point (mp) data and/or retention times (Rₜ) for compounds according to Formula (I). The compounds 2.1 to 2.39 of Table T2 are not according to the invention.**

| Entry | IUPAC name | **Structure** | Rₜ (min) | [M+H] (measured) | Method | mp (°C) |
|---|---|---|---|---|---|---|
| 2.1 | 2-methoxy-N-[[4-[5-(trifluoromethyl) -1,2,4-oxadiazol-3-yl]phenyl]methy l]ethanesulfona mide | 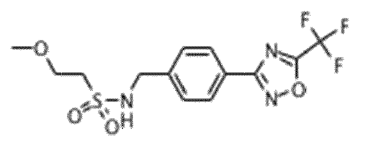 | 1.44 | 366.08 | B | 97 - 100 |
| 2.2 | N-[[4-[5-(trifluoromethyl) -1,2,4-oxadiazol-3-yl]phenyl]methy l]ethanesulfona mide | 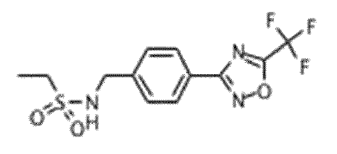 | 1.43 | 336.08 | B | 127 - 129 |
| 2.3 | 3-[4-[(dimethylsulfa moylamino)met hyl]phenyl]-5-(trifluoromethyl) -1,2,4-oxadiazole | 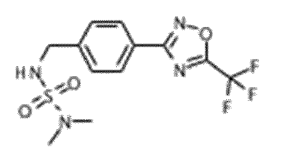 | 1.52 | 351.08 | B | 105 - 107 |
| 2.4 | N-[[4-[5-(trifluoromethyl) -1,2,4-oxadiazol-3-yl]phenyl]methy l]propane-1-sulfonamide | 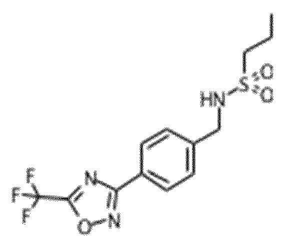 | 2.65 | 350.1 | B | |
| 2.5 | N-[[3-fluoro-4-[5-(trifluoromethyl) -1,2,4-oxadiazol-3-yl]phenyl]methy l]propane-1-sulfonamide | 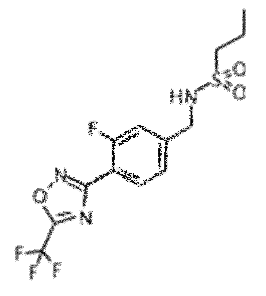 | 1.53 | 368.07 | B | |
| 2.6 | N-[[2-fluoro-4-[5-(trifluoromethyl) -1,2,4-oxadiazol-3-yl]phenyl]methy l]propane-1-sulfonamide | 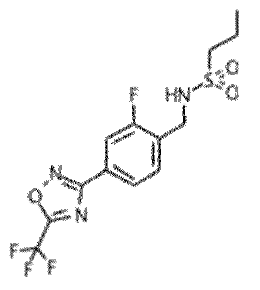 | 1.6 | 368.07 | B | |
| 2.7 | N-[[4-[5-(trifluoromethyl) -1,2,4-oxadiazol-3-yl]phenyl]methy l]prop-2-ene-1-sulfonamide | 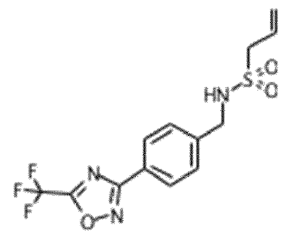 | 1.5 | 348.06 | B | |
| 2.8 | methyl 3-[[4-[5-(trifluoromethyl) -1,2,4-oxadiazol-3-yl]phenyl]methy lsulfamoyl]prop anoate | 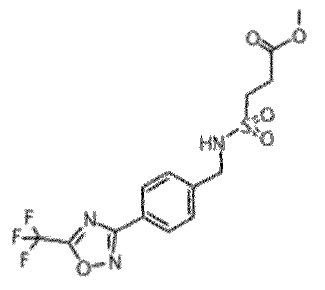 | 1.46 | 394.07 | B | |
| 2.9 | 3,3,3-trifluoro-N-[[4-[5-(trifluoromethyl) -1,2,4-oxadiazol-3-yl]phenyl]methy l]propane-1-sulfonamide | 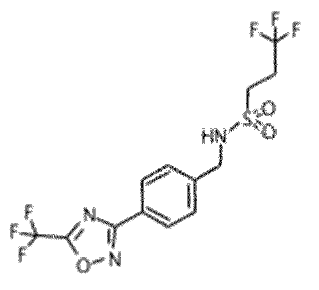 | 2.82 | 404.03 | B | |
| 2.10 | (E)-N-[[4-[5-(trifluoromethyl) -1,2,4-oxadiazol-3-yl]phenyl]methy l]prop-1-ene-1-sulfonamide | 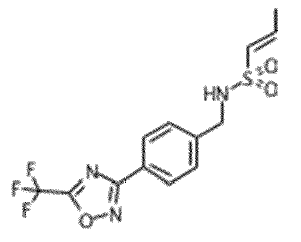 | 1.54 | 348.08 | B | |
| 2.11 | methyl 2-[[4-[5-(trifluoromethyl) -1,2,4-oxadiazol-3-yl]phenyl]methy lsulfamoyl]acet ate | 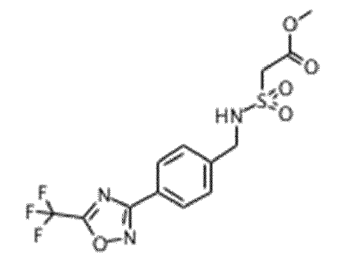 | 2.82 | 380.03 | B | |
| 2.12 | N-[[4-[5-(trifluoromethyl) -1,2,4-oxadiazol-3-yl]phenyl]methy l]butane-1-sulfonamide | 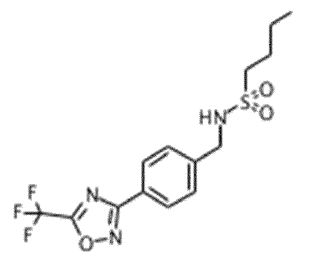 | 1.67 | 364.1 | B | |
| 2.13 | N-[[4-[5-(trifluoromethyl) -1,2,4-oxadiazol-3-yl]phenyl]methyl]methanesulfo namide | 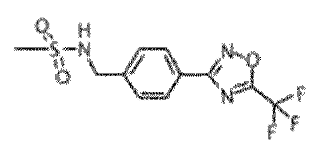 | 1.35 | 322.06 | B | |
| 2.14 | N-(methylsulfamo yl)-1-[4-[5-(trifluoromethyl) -1,2,4-oxadiazol-3-yl]phenyl]meth anamine | 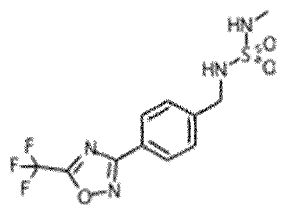 | 1.35 | 337.07 | B | |
| 2.15 | N-[[3-fluoro-4-[5-(trifluoromethyl) -1,2,4-oxadiazol-3-yl]phenyl]methy l]prop-2-ene-1-sulfonamide | 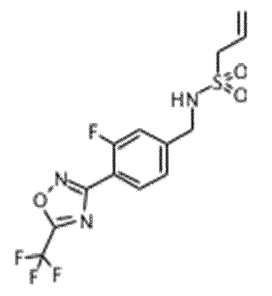 | 1.49 | 366.26 | B | |
| 2.16 | methyl 3-[[3-fluoro-4-[5-(trifluoromethyl) -1,2,4-oxadiazol-3-yl]phenyl]methy lsulfamoyl]prop anoate | 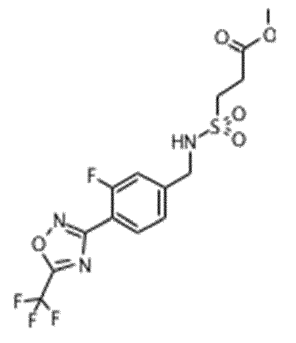 | 1.45 | 412.04 | B | |
| 2.17 | N-[[3-fluoro-4-[5-(trifluoromethyl) -1,2,4-oxadiazol-3-yl]phenyl]methy l]-2-methoxyethanesulfona mide | 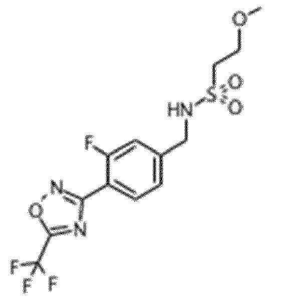 | 1.43 | 384.06 | B | |
| 2.18 | 3,3,3-trifluoro-N-[[3-fluoro-4-[5-(trifluoromethyl) -1,2,4-oxadiazol-3-yl]phenyl]methy l]propane-1-sulfonamide | 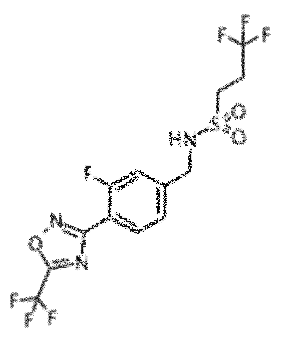 | 1.63 | 422.03 | B | |
| 2.19 | (E)-N-[[3-fluoro-4-[5-(trifluoromethyl) -1,2,4-oxadiazol-3-yl]phenyl]methy l]prop-1-ene-1-sulfonamide | 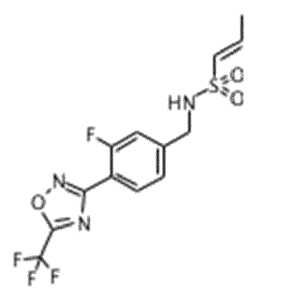 | 1.51 | 366.35 | B | |
| 2.20 | methyl 2-[[3-fluoro-4-[5-(trifluoromethyl) -1,2,4-oxadiazol-3-yl]phenyl]methy lsulfamoyl]acet ate | 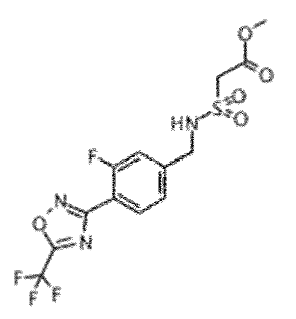 | 1.43 | 398.05 | B | |
| 2.21 | N-[[2-fluoro-4-[5-(trifluoromethyl) -1,2,4-oxadiazol-3-yl]phenyl]methy l]propane-2-sulfonamide | 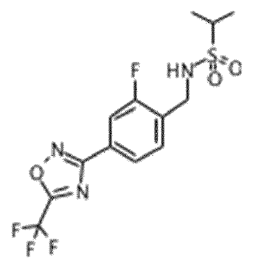 | 1.68 | 368.04 | B | |
| 2.22 | N-[[3-fluoro-4-[5-(trifluoromethyl) -1,2,4-oxadiazol-3-yl]phenyl]methy l]butane-1-sulfonamide | 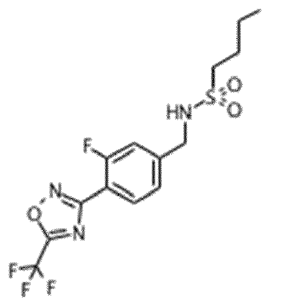 | 1.64 | 382.10 | B | |
| 2.23 | N-[[2-fluoro-4-[5-(trifluoromethyl) -1,2,4-oxadiazol-3-yl]phenyl]methy l]butane-1-sulfonamide | 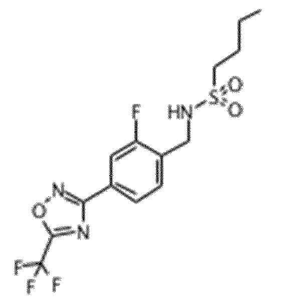 | 1.70 | 382.08 | B | |
| 2.24 | N-[[3-fluoro-4-[5-(trifluoromethyl) -1,2,4-oxadiazol-3-yl]phenyl]methy l]ethanesulfona mide | 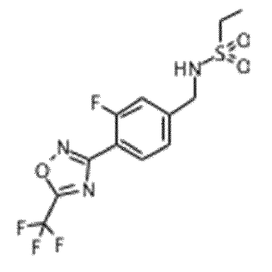 | 1.42 | 354.08 | B | |
| 2.25 | N-[[2-fluoro-4-[5-(trifluoromethyl) -1,2,4-oxadiazol-3-yl]phenyl]methy l]ethanesulfona mide | 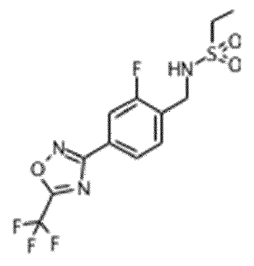 | 1.49 | 354.16 | B | |
| 2.26 | N-[[3-fluoro-4-[5-(trifluoromethyl) -1,2,4-oxadiazol-3-yl]phenyl]methy ]methanesulfo namide | 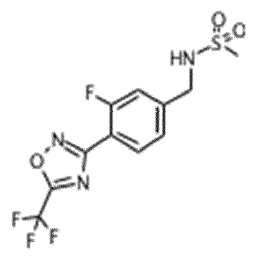 | 1.33 | 340.33 | B | |
| 2.27 | N-[[2-fluoro-4-[5-(trifluoromethyl) -1,2,4-oxadiazol-3-yl]phenyl]methy ]methanesulfo namide | 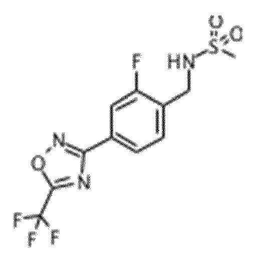 | 1.39 | 340.43 | B | |
| 2.28 | 1-[3-fluoro-4-[5-(trifluoromethyl) -1,2,4-oxadiazol-3-yl]phenyl]-N-(methylsulfamo yl)methanamin e | 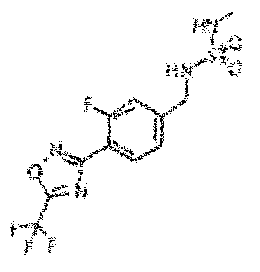 | 1.39 | 355.05 | B | |
| 2.29 | 1-[2-fluoro-4-[5-(trifluoromethyl) -1,2,4-oxadiazol-3-yl]phenyl]-N-(methylsulfamo yl)methanamin e | 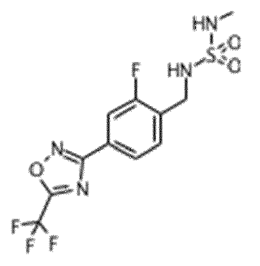 | 1.39 | 355.29 | B | |
| 2.30 | 3-[4-[(dimethylsulfa moylamino)met hyl]-2-fluorophenyl]-5-(trifluoromethyl) -1,2,4-oxadiazole | 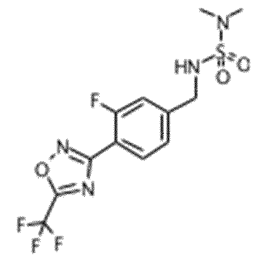 | 1.50 | 369.07 | B | |
| 2.31 | 3-[4-[(dimethylsulfa moylamino)met hyl]-3-fluorophenyl]-5-(trifluoromethyl) -1,2,4-oxadiazole | 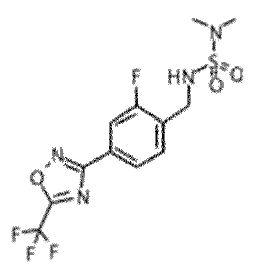 | 1.57 | 369.06 | B | |
| 2.32 | N-[[2-fluoro-4-[5-(trifluoromethyl) -1,2,4-oxadiazol-3-yl]phenyl]methy l]prop-2-ene-1-sulfonamide | 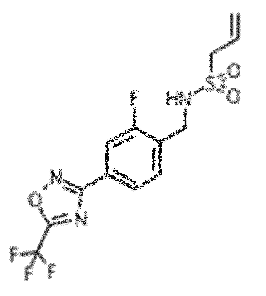 | 1.55 | 366.08 | B | |
| 2.33 | methyl 3-[[2-fluoro-4-[5-(trifluoromethyl) -1,2,4-oxadiazol-3-yl]phenyl]methy lsulfamoyl]prop anoate | 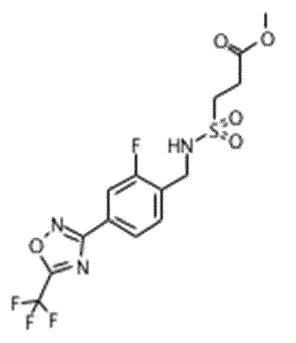 | 1.50 | 412.06 | B | |
| 2.34 | N-[[2-fluoro-4-[5-(trifluoromethyl) -1,2,4-oxadiazol-3-yl]phenyl]methy l]-2-methoxyethanesulfona mide | 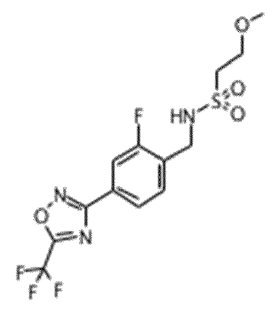 | 1.49 | 384.07 | B | |
| 2.35 | 3,3,3-trifluoro-N-[[2-fluoro-4-[5-(trifluoromethyl) -1,2,4-oxadiazol-3-yl]phenyl]methy l]propane-1-sulfonamide | 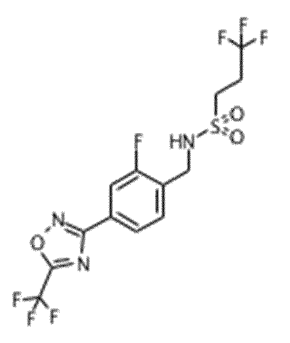 | 1.69 | 422.12 | B | |
| 2.36 | (E)-N-[[2-fluoro-4-[5-(trifluoromethyl) -1,2,4-oxadiazol-3-yl]phenyl]methy l]prop-1-ene-1-sulfonamide | 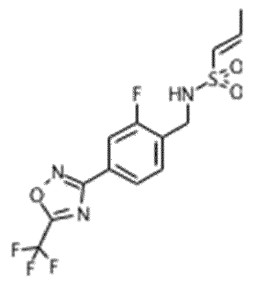 | 1.58 | 366.08 | B | |
| 2.37 | methyl 2-[[2-fluoro-4-[5-(trifluoromethyl) -1,2,4-oxadiazol-3-yl]phenyl]methy lsulfamoyl]acet ate | 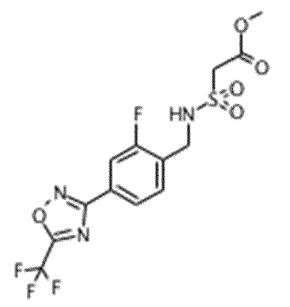 | 1.49 | 398.26 | B | |
| 2.38 | N-[4-[5-(trifluoromethyl) -1,2,4-oxadiazol-3-yl]phenyl]propa ne-1-sulfonamide | 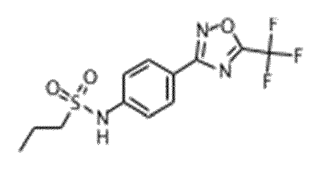 | | | | 65 - 75 |
| 2.39 | 3-[6-[(dimethylsulfa moylamino)met hyl]-3-pyridyl]-5-(trifluoromethyl) -1,2,4-oxadiazole | 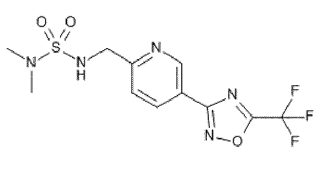 | 0.90 | 352.3 | A | |

**Table T3: Melting point (mp) data and/or retention times (Rₜ) for compounds according to Formula (I). The compounds 3.1 to 3.36 of Table T3 are not according to the invention.**

| Entry | IUPAC name | **Structure** | Rₜ (min) | (measured) | Method | mp |
|---|---|---|---|---|---|---|
| 3.1 | N-[[4-[5-(trifluoromethyl)-1,2,4-oxadiazol-3-yl]phenyl]methyl]b enzenesulfonamid e | 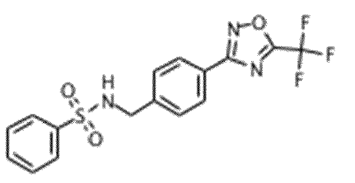 | 1.67 | 384.06 | B | 116 - 124 |
| 3.2 | N-[[4-[5-(trifluoromethyl)-1,2,4-oxadiazol-3-yl]phenyl]methyl]c yclopropanesulfon amide | 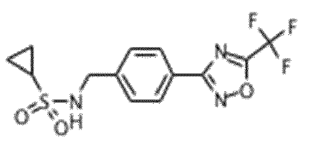 | 2.81 | 348.08 | B | 130 - 134 |
| 3.3 | 1-cyclopropyl-N-[[4-[5-(trifluoromethyl)-1,2,4-oxadiazol-3-yl]phenyl]methyl]m ethanesulfonamid e | 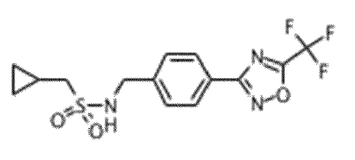 | 1.57 | 362.09 | B | 140 - 144 |
| 3.4 | 1-tetrahydrofuran-3-yl-N-[[4-[5-(trifluoromethyl)-1,2,4-oxadiazol-3-yl]phenyl]methyl]m ethanesulfonamid e | 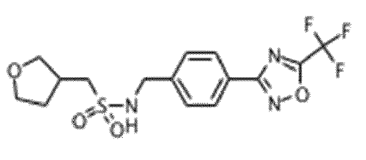 | 1.43 | 392.1 | B | 117 - 122 |
| 3.5 | 2,4-difluoro-N-[[4-[5-(trifluoromethyl)-1,2,4-oxadiazol-3-yl]phenyl]methyl]b enzenesulfonamid e | 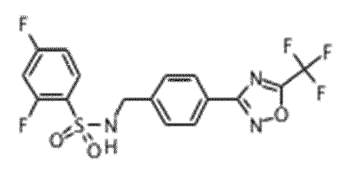 | | | | 112 - 115 |
| 3.6 | 1-(4-chloro-2-fluoro-phenyl)-N-[[4-[5-(trifluoromethyl)-1,2,4-oxadiazol-3-yl]phenyl]methyl]m ethanesulfonamid e | 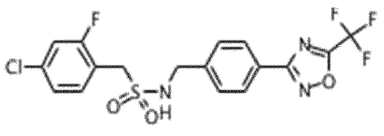 | | | | 140 - 144 |
| 3.7 | 2-fluoro-N-[[4-[5-(trifluoromethyl)-1,2,4-oxadiazol-3-yl]phenyl]methyl]b enzenesulfonamid e | 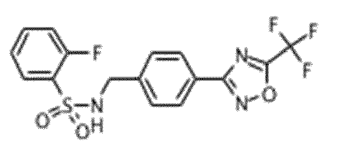 | | | | 120 - 123 |
| 3.8 | N-[[4-[5-(trifluoromethyl)-1,2,4-oxadiazol-3-yl]phenyl]methyl]m orpholine-4-sulfonamide | 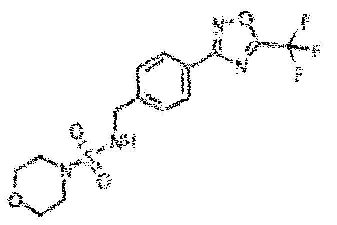 | | | | 108 - 110 |
| 3.9 | N-[[3-fluoro-4-[5-(trifluoromethyl)-1,2,4-oxadiazol-3-yl]phenyl]methyl]b enzenesulfonamid e | 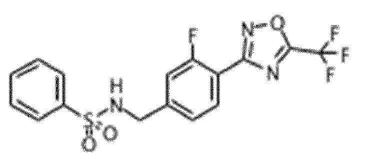 | 2.75 | 402.13 | B | |
| 3.10 | 1-methyl-N-[[4-[5-(trifluoromethyl)-1,2,4-oxadiazol-3-yl]phenyl]methyl]p yrazole-3-sulfonamide | 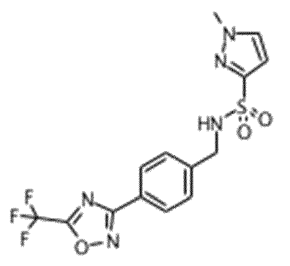 | 1.42 | 388.08 | B | |
| 3.11 | 5-methyl-N-[[4-[5-(trifluoromethyl)-1,2,4-oxadiazol-3-yl]phenyl]methy l]t h iophene-2-sulfonamide | 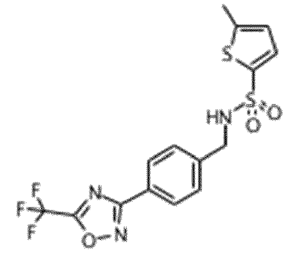 | 1.74 | 404.02 | B | |
| 3.12 | 1-(2,2-dichlorocyclopropy l)-N-[[4-[5-(trifluoromethyl)-1,2,4-oxadiazol-3 - yl]phenyl]methyl]m ethanesulfonamid e | 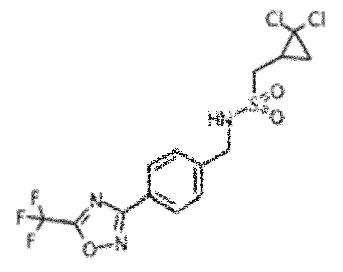 | 1.73 | 429.99 | B | |
| 3.13 | N-[[4-[5-(trifluoromethyl)-1,2,4-oxadiazol-3-yl]phenyl]methyl]p yrrolidine-1-sulfonamide | 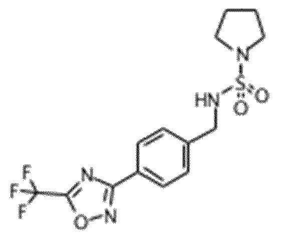 | 1.61 | 377.09 | B | |
| 3.14 | N-[[3-fluoro-4-[5-(trifluoromethyl)-1,2,4-oxadiazol-3-yl]phenyl]methyl]-1-methyl-pyrazole-3-sulfonamide | 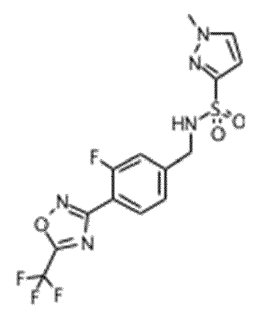 | 1.41 | 406.04 | B | |
| 3.15 | N-[[3-fluoro-4-[5-(trifluoromethyl)-1,2,4-oxadiazol-3-yl]phenyl]methyl]-5-methylthiophene-2-sulfonamide | 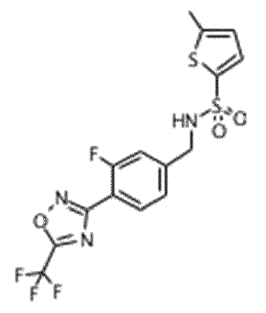 | 1.71 | 422.00 | B | |
| 3.16 | 1-(2,2-dichlorocyclopropy l)-N-[[3-fluoro-4-[5-(trifluoromethyl)-1,2,4-oxadiazol-3-yl]phenyl]methyl]m ethanesulfonamid e | 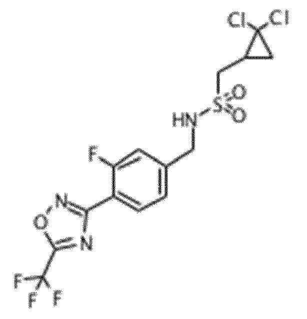 | 1.71 | 448.19 | B | |
| 3.17 | N-[[3-fluoro-4-[5-(trifluoromethyl)-1,2,4-oxadiazol-3-yl]phenyl]methyl]p yrrolidine-1-sulfonamide | 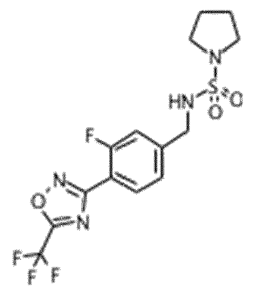 | 1.59 | 395.08 | B | |
| 3.18 | 1-cyclopentyl-N-[[3-fluoro-4-[5-(trifluoromethyl)-1,2,4-oxadiazol-3-yl]phenyl]methyl]m ethanesulfonamid e | 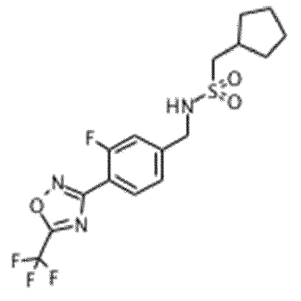 | 1.75 | 408.08 | B | |
| 3.19 | N-[[3-fluoro-4-[5-(trifluoromethyl)-1,2,4-oxadiazol-3-yl]phenyl]methyl]c yclopropanesulfon amide | 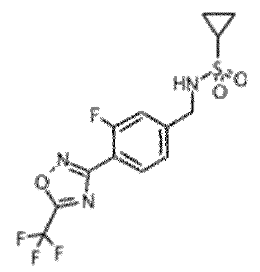 | 1.47 | 366.36 | B | |
| 3.20 | N-[[3-fluoro-4-[5-(trifluoromethyl)-1,2,4-oxadiazol-3-yl]phenyl]methyl]-1-tetrahydrofuran-3-yl-methanesulfonami de | 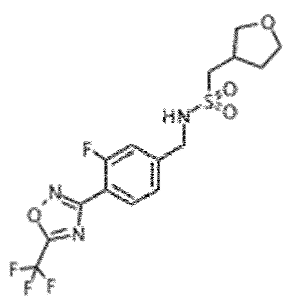 | 1.41 | 410.18 | B | |
| 3.21 | 1-cyclopropyl-N-[[3-fluoro-4-[5-(trifluoromethyl)-1,2,4-oxadiazol-3-yl]phenyl]methyl]m ethanesulfonamid e | 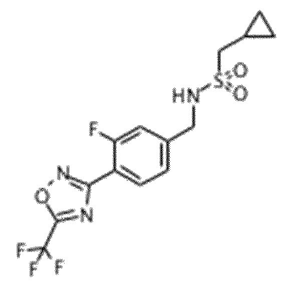 | 1.54 | 379.89 | B | |
| 3.22 | N-[[3-fluoro-4-[5-(trifluoromethyl)-1,2,4-oxadiazol-3-yl]phenyl]methyl]-1-phenyl-methanesulfonami de | 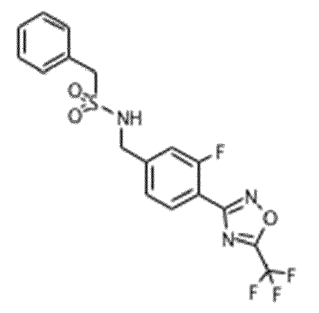 | 1.72 | 416.06 | B | |
| 3.23 | N-[[2-fluoro-4-[5-(trifluoromethyl)-1,2,4-oxadiazol-3-yl]phenyl]methyl]-1-phenyl-methanesulfonami de | 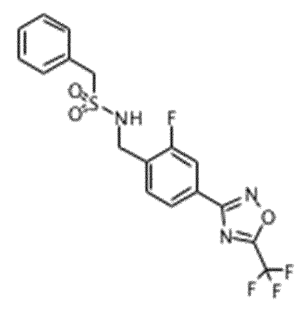 | 1.72 | 416.09 | B | |
| 3.24 | N-[[2-fluoro-4-[5-(trifluoromethyl)-1,2,4-oxadiazol-3-yl]phenyl]methyl]-1-methyl-pyrazole-3-sulfonamide | 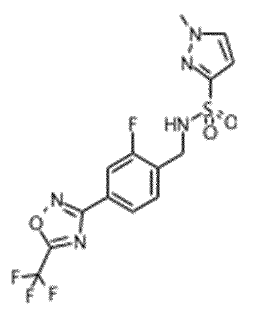 | 1.41 | 406.04 | B | |
| 3.25 | N-[[2-fluoro-4-[5-(trifluoromethyl)-1,2,4-oxadiazol-3-yl]phenyl]methyl]-5-methylthiophene-2-sulfonamide | 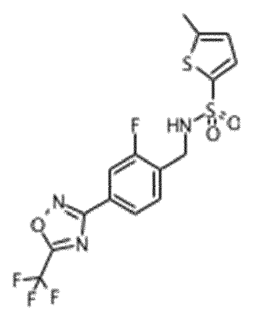 | 1.71 | 422.00 | B | |
| 3.26 | 1-(2,2-dichlorocyclopropy l)-N-[[2-fluoro-4-[5-(trifluoromethyl)-1,2,4-oxadiazol-3-yl]phenyl]methyl]m ethanesulfonamid e | 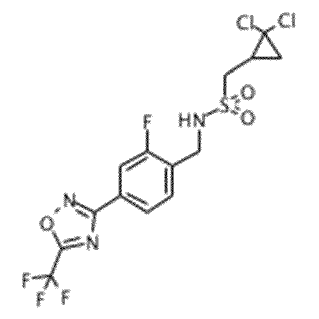 | 1.78 | 448.27 | B | |
| 3.27 | N-[[2-fluoro-4-[5-(trifluoromethyl)-1,2,4-oxadiazol-3-yl]phenyl]methyl]p yrrolidine-1-sulfonamide | 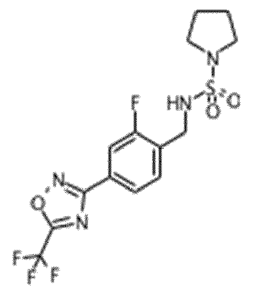 | 1.66 | 395.40 | B | |
| 3.28 | 1-cyclopentyl-N-[[2-fluoro-4-[5-(trifluoromethyl)-1,2,4-oxadiazol-3-yl]phenyl]methyl]m ethanesulfonamid e | 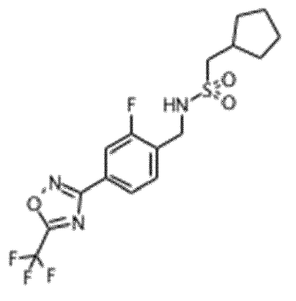 | 1.82 | 408.10 | B | |
| 3.29 | N-[[2-fluoro-4-[5-(trifluoromethyl)-1,2,4-oxadiazol-3-yl]phenyl]methyl]c yclopropanesulfon amide | 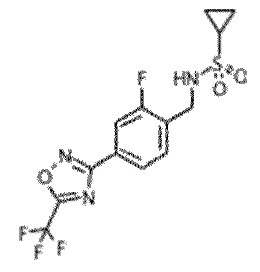 | 1.53 | 366.44 | B | |
| 3.30 | N-[[2-fluoro-4-[5-(trifluoromethyl)-1,2,4-oxadiazol-3-yl]phenyl]methyl]-1-tetrahydrofuran-3-yl-methanesulfonami de | 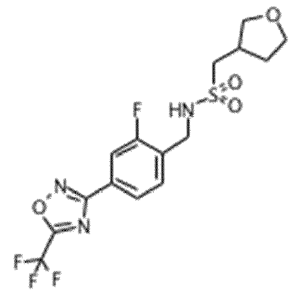 | 1.47 | 410.47 | B | |
| 3.31 | 1-cyclopropyl-N-[[2-fluoro-4-[5-(trifluoromethyl)-1,2,4-oxadiazol-3-yl]phenyl]methyl]m ethanesulfonamid e | 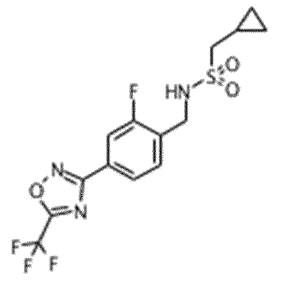 | 1.61 | 380.05 | B | |
| 3.32 | N-[[2-fluoro-4-[5-(trifluoromethyl)-1,2,4-oxadiazol-3-yl]phenyl]methyl]b enzenesulfonamid e | 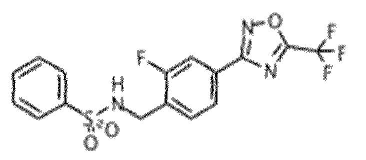 | 1.70 | 402.42 | B | |
| 3.33 | N-[4-[5-(trifluoromethyl)-1,2,4-oxadiazol-3-yl]phenyl]cyclopro panesulfonamide | 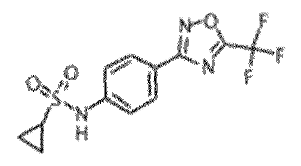 | | | | 130 - 140 |
| 3.34 | 1-phenyl-N-[4-[5-(trifluoromethyl)-1,2,4-oxadiazol-3-yl]phenyl]methane sulfonamide | 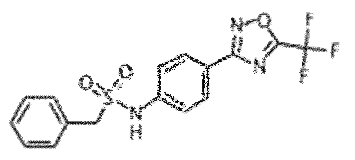 | | | | 125-135 |
| 3.35 | N-[4-[5-(trifluoromethyl)-1,2,4-oxadiazol-3-yl]phenyl]benzene sulfonamide | 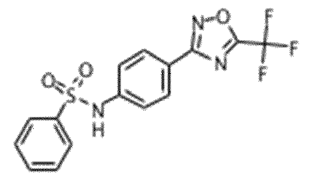 | | | | 115-130 |
| 3.36 | N-[[5-[5-(trifluoromethyl)-1,2,4-oxadiazol-3-yl]-2-pyridyl]methyl]ben zenesulfonamide | 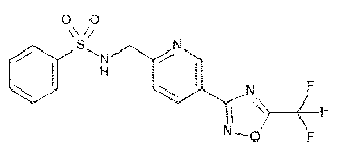 | 0.98 | 385 | A | |

**Table T4: Melting point (mp) data and/or retention times (Rₜ) for compounds according to Formula (I). The compound 4.2 of Table T4 is according to the invention. The compound 4.1 of Table T4 is not according to the invention.**

| Entry | IUPAC name | **STRUCTURE** | RT (min) | [M+H] (measured) | Method | MP °C |
|---|---|---|---|---|---|---|
| 4.1 | *N*-ethyl-*N*-[1-[4-[5-(trifluoromethyl)-1,2,4-oxadiazol-3-yl]phenyl]ethyl]met hanesulfonamide | 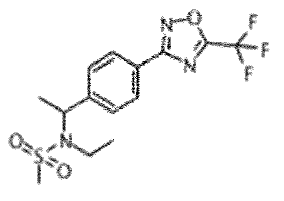 | 2.85 | 363.87 | D | |
| 4.2 | *N*-methoxy-*N*-[1-methyl-2-[4-[5-(trifluoromethyl)-1,2,4-oxadiazol-3-yl]phenyl]ethyl]met hanesulfonamide | 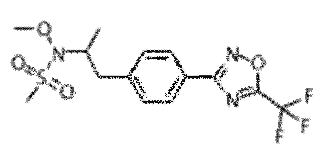 | 1.12 | 380.4 | A | |

### BIOLOGICAL EXAMPLES

### General examples of leaf disk tests in well plates:

Leaf disks or leaf segments of various plant species are cut from plants grown in a greenhouse. The cut leaf disks or segments are placed in multiwell plates (24-well format) onto water agar. The leaf disks are sprayed with a test solution before (preventative) or after (curative) inoculation. Compounds to be tested are prepared as DMSO solutions (max. 10 mg/ml) which are diluted to the appropriate concentration with 0.025% Tween20 just before spraying. The inoculated leaf disks or segments are incubated under defined conditions (temperature, relative humidity, light, etc.) according to the respective test system. A single evaluation of disease level is carried out 3 to 14 days after inoculation, depending on the pathosystem. Percent disease control relative to the untreated check leaf disks or segments is then calculated.

### General examples of liquid culture tests in well plates:

*Mycelia* fragments or conidia suspensions of a fungus prepared either freshly from liquid cultures of the fungus or from cryogenic storage, are directly mixed into nutrient broth. DMSO solutions of the test compound (max. 10 mg/ml) are diluted with 0.025% Tween20 by a factor of 50 and 10 µl of this solution is pipetted into a microtiter plate (96-well format). The nutrient broth containing the fungal spores/mycelia fragments is then added to give an end concentration of the tested compound. The test plates are incubated in the dark at 24°C and 96% relative humidity. The inhibition of fungal growth is determined photometrically after 2 to 7 days, depending on the pathosystem, and percent antifungal activity relative to the untreated check is calculated.

### Example 1: Fungicidal activity against Puccinia recondita f. sp. tritici / wheat / leaf disc preventative (Brown rust)

Wheat leaf segments cv. Kanzler were placed on agar in multiwell plates (24-well format) and sprayed with the formulated test compound diluted in water. The leaf disks were inoculated with a spore suspension of the fungus 1 day after application. The inoculated leaf segments were incubated at 19°C and 75% relative humidity (rh) under a light regime of 12 hours light / 12 hours darkness in a climate cabinet and the activity of a compound was assessed as percent disease control compared to untreated when an appropriate level of disease damage appears in untreated check leaf segments (7 to 9 days after application).

The following compounds at 200 ppm in the applied formulation give at least 80% disease control in this test when compared to untreated control leaf disks under the same conditions, which show extensive disease development.

Compounds (from Table T1) 1.1, 1.2, 1.3, 1.6, 1.7, 1.8, 1.10, 1.11, 1.12, 1.13, 1.14, 1.15, 1.16, 1.17, 1.18, 1.19, 1.22, 1.23, 1.25, 1.29, 1.32, 1.33, 1.34, 1.35, 1.36, 1.37, 1.39, 1.41, 1.44, 1.46, 1.47, 1.48, 1.49, 1.51, 1.52, 1.53, 1.54, 1.56, 1.57, 1.59, 1.60, 1.61, 1.62, 1.63, 1.64, 1.65, 1.68, 1.69, 1.71, 1.73, 1.76, 1.79, 1.80, 1.82, 1.83, 1.84, 1.85, 1.86, 1.87, 1.88, 1.93, 1.97, 1.98, 1.100, 1.102, 1.103, 1.104, 1.105, 1.106, 1.107, and 1.111.

Compounds (from Table T2) 2.1, 2.2, 2.3, 2.4, 2.5, 2.6, 2.7, 2.8, 2.10, 2.12, 2.13, 2.14, 2.15, 2.16, 2.17, 2.18, 2.19, 2.22, 2.24, 2.25, 2.26, 2.27, 2.28, 2.29, 2.30, 2.31, 2.32, 2.33, 2.34, 2.36, and 2.37.

Compounds (from Table T3) 3.1, 3.2, 3.3, 3.4, 3.5, 3.6, 3.7, 3.8, 3.9, 3.10, 3.11, 3.12, 3.13, 3.14, 3.16, 3.17, 3.19, 3.20, 3.21, 3.22, 3.23, 3.24, 3.27, 3.29, 3.30, 3.31, 3.32, 3.33, and 3.35.

Compounds (from Table T4) 4.1 and 4.2.

### Example 2: Fungicidal activity against Puccinia recondita f. sp. tritici / wheat / leaf disc curative (Brown rust)

Wheat leaf segments cv. Kanzler are placed on agar in multiwell plates (24-well format). The leaf segments are then inoculated with a spore suspension of the fungus. Plates were stored in darkness at 19°C and 75% relative humidity. The formulated test compound diluted in water was applied 1 day after inoculation. The leaf segments were incubated at 19°C and 75% relative humidity under a light regime of 12 hours light / 12 hours darkness in a climate cabinet and the activity of a compound was assessed as percent disease control compared to untreated when an appropriate level of disease damage appears in untreated check leaf segments (6 to 8 days after application).

The following compounds at 200 ppm in the applied formulation give at least 80% disease control in this test when compared to untreated control leaf disks under the same conditions, which show extensive disease development.

Compounds (from Table T1) 1.1, 1.3, 1.5, 1.8, 1.10, 1.11, 1.12, 1.13, 1.15, 1.16, 1.17, 1.19, 1.20, 1.23, 1.25, 1.29, 1.33, 1.34, 1.35, 1.37, 1.39, 1.41, 1.46, 1.47, 1.49, 1.51, 1.52, 1.56, 1.59, 1.61, 1.62, 1.63, 1.64, 1.69, 1.70, 1.73, 1.76, 1.79, 1.81, 1.82, 1.83, 1.84, 1.85, 1.86, 1.87, 1.98, 1.100, 1.101, 1.102, 1.103, 1.104, 1.105, 1.106, and 1.107.

Compounds (from Table T2) 2.1, 2.2, 2.3, 2.4, 2.5, 2.6, 2.7, 2.8, 2.13, 2.14, 2.15, 2.16, 2.17, 2.19, 2.21, 2.24, 2.25, 2.26, 2.27, 2.28, 2.29, 2.30, 2.31, 2.32, 2.34, and 2.36.

Compounds (from Table T3) 3.2, 3.3, 3.4, 3.8, 3.10, 3.14, 3.17, 3.19, 3.20, 3.21, 3.24, 3.27, 3.29, 3.30, and 3.31.

Compounds (from Table T4) 4.1.

### Example 3: Fungicidal activity against Phakopsora pachyrhizi / soybean / leaf disc preventative (Asian soybean rust)

Soybean leaf disks are placed on water agar in multiwell plates (24-well format) and sprayed with the formulated test compound diluted in water. One day after application leaf discs are inoculated by spraying a spore suspension on the lower leaf surface. After an incubation period in a climate cabinet of 24-36 hours in darkness at 20°C and 75% rh leaf disc are kept at 20°C with 12 h light/day and 75% rh. The activity of a compound is assessed as percent disease control compared to untreated when an appropriate level of disease damage appears in untreated check leaf disks (12 to 14 days after application).

The following compounds at 200 ppm in the applied formulation give at least 80% disease control in this test when compared to untreated control leaf disks under the same conditions, which show extensive disease development.

Compounds (from Table T1) 1.1, 1.2, 1.3, 1.8, 1.10, 1.11, 1.12, 1.13, 1.15, 1.16, 1.17, 1.23, 1.101, 1.102, 1.103, and 1.104.

Compounds (from Table T2) 2.1, 2.2, 2.3, and 2.38.

Compounds (from Table T3) 3.2, 3.3, 3.4, 3.7, 3.33, and 3.35.

Compounds (from Table T4) 4.1 and 4.2.

### Example 4: Fungicidal activity against Glomerella lagenarium (Colletotrichum lagenarium) liquid culture / cucumber / preventative (Anthracnose)

Conidia of the fungus from cryogenic storage are directly mixed into nutrient broth (PDB - potato dextrose broth). After placing a (DMSO) solution of test compound into a microtiter plate (96-well format), the nutrient broth containing the fungal spores is added. The test plates are incubated at 24°C and the inhibition of growth is measured photometrically 3 to 4 days after application.

The following compounds at 20 ppm in the applied formulation give at least 80% disease control in this test when compared to untreated control under the same conditions, which show extensive disease development.

Compounds (from Table T1) 1.1, 1.2, 1.3, 1.4, 1.5, 1.6, 1.7, 1.8, 1.9, 1.10, 1.11, 1.12, 1.13, 1.14, 1.15, 1.16, 1.17, 1.18, 1.19, 1.20, 1.21, 1.22, 1.23, 1.24, 1.25, 1.26, 1.29, 1.31, 1.32, 1.33, 1.34, 1.35, 1.36, 1.37, 1.39, 1.40, 1.41, 1.44, 1.46, 1.47, 1.48, 1.49, 1.51, 1.52, 1.53, 1.54, 1.55, 1.56, 1.58, 1.59, 1.60, 1.61, 1.62, 1.63, 1.64, 1.65, 1.68, 1.69, 1.70, 1.71, 1.72, 1.73, 1.75, 1.76, 1.77, 1.78, 1.79, 1.80, 1.81, 1.82, 1.83, 1.84, 1.85, 1.86, 1.87, 1.92, 1.93, 1.94, 1.97, 1.98, 1.99, 1.100, 1.101, 1.102, 1.103, 1.104, 1.105, 1.106, 1.107, and 1.111.

Compounds (from Table T2) 2.1, 2.2, 2.3, 2.4, 2.5, 2.6, 2.7, 2.8, 2.10, 2.11, 2.12, 2.13, 2.14, 2.15, 2.16, 2.17, 2.18, 2.19, 2.20, 2.22, 2.23, 2.24, 2.25, 2.26, 2.27, 2.28, 2.29, 2.30, 2.31, 2.32, 2.33, 2.34, 2.36, 2.37, and 2.38

Compounds (from Table T3) 3.1, 3.2, 3.3, 3.4, 3.7, 3.8, 3.9, 3.10, 3.11, 3.12, 3.13, 3.14, 3.15, 3.16, 3.17, 3.19, 3.20, 3.21, 3.24, 3.25, 3.27, 3.29, 3.30, 3.31, 3.33, 3.34, and 3.35.

Compounds (from Table T4) 4.1 and 4.2.

## Claims

1. A compound of Formula (I): 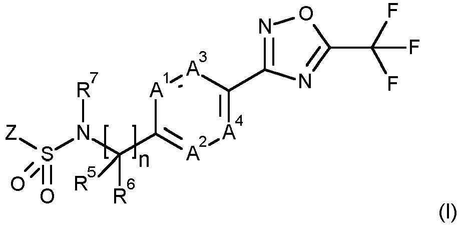 wherein
n is 0, 1, or 2;
A¹ represents N or CR¹, wherein R¹ represents hydrogen, halogen, or methyl;
A² represents N or CR², wherein R² represents hydrogen, halogen, or methyl;
A³ represents N or CR³, wherein R³ represents hydrogen or fluoro;
A⁴ represents N or CR⁴, wherein R⁴ represents hydrogen or fluoro; and
wherein at least 2 of A¹, A², A³ and A⁴ are C-H;
R⁵ and R⁶ independently represent hydrogen, methyl, cyano, difluoromethyl, or trifluoromethyl;
R⁷ represents hydroxy, C₁₋₄haloalkyl, C₁₋₄alkoxy, hydroxyC₂₋₄alkyl, C₁₋₂alkoxyC₂₋₄alkyl, C₁-₂haloalkoxy, C₁₋₂haloalkoxyC₂₋₄alkyl, C₃₋₄alkenyloxy, C₃₋₄alkynyloxy or C₁₋₄alkylcarbonyloxy;
Z represents R⁸, wherein
R⁸ is C₁₋₆alkyl, C₂₋₆alkenyl, C₂₋₆alkynyl, cyanoC₁₋₄alkyl, C₁₋₄haloalkyl, C₁₋₄alkoxyC₂₋₄alkyl, C₁-₂haloalkoxyC₂₋₄alkyl, C₁₋₄alkylcarbonylC₁₋₄alkyl, C₁₋₄alkoxycarbonylC₁₋₄alkyl, C₁₋₄alkylcarbonyloxyC₂₋₄alkyl, N-C₁₋₄alkylaminocarbonylC₁₋₄alkyl, N,N-diC₁₋₄alkylaminocarbonylC₁₋₄alkyl, N-C₁₋₄alkylaminoC₂₋salkyl, N,N-diC₁₋₄alkylaminoC₂₋₅alkyl, C₁₋₃alkylcarbonylaminoC₂₋₅alkyl, or C₁₋₄alkoxycarbonylaminoC₂₋salkyl; or
R⁸ is C₃₋₆cycloalkyl, C₃₋₆cycloalkylC₁₋₂alkyl, phenyl, phenylC₁₋₂alkyl, heteroaryl or heteroarylC₁₋₂alkyl, wherein the heteroaryl moiety is a 5- or 6-membered monocyclic aromatic ring which comprises 1, 2, 3 or 4 heteroatoms individually selected from N, O and S, heterocyclyl or heterocyclylC₁₋₂alkyl, wherein the heterocyclyl moiety is a 4- to 6-membered non-aromatic ring which comprises 1 or 2 heteroatoms individually selected from N, O and S;
wherein when R⁸ is C₃₋₆cycloalkyl, C₃₋₆cycloalkylC₁₋₂alkyl, phenyl, phenylC₁₋₂alkyl, heteroaryl, heteroarylC₁₋₂alkyl, heterocyclyl, or heterocyclylC₁₋₂alkyl, the C₃₋₆cycloalkyl, phenyl, heteroaryl, or heterocyclyl motif is optionally substituted by 1 or 2 substituents, which may be the same or different, selected from R⁹;
R⁹ represents cyano, fluoro, chloro, bromo, methyl, difluoromethyl, trifluoromethyl, methoxy, ethoxy, or difluoromethoxy; and wherein when R⁸ is substituted C₃₋₆cycloalkyl or heterocyclyl, these cycles may contain a carbonyl (C=O) or sulfonyl (S(O)₂) group; or
Z represents -N(R¹⁰)R¹¹, wherein
R¹⁰ represents hydrogen, C₁₋₆alkyl, C₁₋₆haloalkyl, cyanoC₁₋₄alkyl, hydroxyC₂₋₄alkyl, C₁-₂alkoxyC₂₋₄alkyl, C₁₋₂haloalkoxyC₁₋₄alkyl, C₃₋₆alkenyl, C₃₋₆alkynyl, aminoC₂₋₄alkyl, N-C₁₋₄alkylaminoC₂₋₄alkyl, N,N-diC₁₋₄alkylaminoC₂₋₄alkyl, formyl, C₁₋₄alkylcarbonyl, C₁₋₄alkylcarbonylC₁₋₄alkyl, C₁₋₄alkoxycarbonylC₁₋₄alkyl, C₁₋₄alkylcarbonyloxyC₁₋₄alkyl, N-C₁₋₄alkylaminocarbonylC₁₋₄alkyl, N,N-diC₁₋₄alkylaminocarbonylC₁₋₄alkyl, C₁₋₄alkoxycarbonylaminoC₂₋₄alkyl, C₁₋₄alkylcarbonylaminoC₂₋₄alkyl, or C₁₋₄haloalkylcarbonylaminoC₂₋₄alkyl; or
R¹⁰ is C₃₋₆cycloalkyl, C₃₋₆cycloalkylC₁₋₂alkyl, phenyl, phenylC₁₋₂alkyl, heteroaryl or heteroarylC₁₋₂alkyl, wherein the heteroaryl moiety is a 5- or 6-membered monocyclic aromatic ring which comprises 1, 2, 3 or 4 heteroatoms individually selected from N, O and S, heterocyclyl or heterocyclylC₁₋₂alkyl, wherein the heterocyclyl moiety is a 4- to 6-membered non-aromatic ring which comprises 1 or 2 heteroatoms individually selected from N, O and S;
wherein when R¹⁰ is C₃₋₆cycloalkyl, C₃₋₆cycloalkylC₁₋₂alkyl, phenylC₁₋₂alkyl, phenyl, heteroaryl, heteroarylC₁₋₂alkyl, heterocyclyl, or heterocyclylC₁₋₂alkyl, the C₃₋₆cycloalkyl, phenyl, heteroaryl, or heterocyclyl motif is optionally substituted by 1 or 2 substituents, which may be the same or different, selected from R¹²;
R¹² represents cyano, fluoro, chloro, bromo, methyl, difluoromethyl, trifluoromethyl, methoxy, ethoxy, or difluoromethoxy; and wherein when R¹⁰ is substituted C₃₋₆cycloalkyl or heterocyclyl, these cycles may contain a carbonyl (C=O) or sulfonyl (S(O)₂) group;
R¹¹ represents hydrogen, hydroxyl, C₁₋₄alkyl, C₁₋₂haloalkyl, C₃₋₄alkenyl, C₃₋₄alkynyl, C₃₋₄cycloalkyl, C₃-₄cycloalkylC₁₋₂alkyl, C₁₋₄alkoxy, C₃₋₄alkenyloxy, or C₃₋₄alkynyloxy; or
a salt or an N-oxide thereof.

2. A compound according to claim 1, wherein A¹ and A² each independently represent N, C-H or C-F, and A³ and A⁴ each independently represent C-H or C-F, wherein at least 2 of A¹, A², A³ and A⁴ are C-H.

3. A compound according to claim 1 or claim 2, wherein R⁷ is methoxy.

4. A compound according to any one of claims 1 to 3, wherein R⁵ and R⁶ independently represent hydrogen or methyl.

5. A compound according to any one of claims 1 to 4, wherein Z represents R⁸, wherein R⁸ is C₁-₆alkyl, C₂₋₆alkenyl, C₂₋₆alkynyl, cyanoC₁₋₄alkyl, C₁₋₄haloalkyl, C₁₋₄alkoxyC₂₋₄alkyl, C₁₋₂haloalkoxyC₂₋₄alkyl, C₁₋₄alkylcarbonylC₁₋₄alkyl, C₁₋₄alkoxycarbonylC₁₋₄alkyl, or C₁₋₄alkylcarbonyloxyC₂₋₄alkyl; or
R⁸ is C₃₋₆cycloalkyl, C₃₋₆cycloalkylC₁₋₂alkyl, phenyl, phenylC₁₋₂alkyl, heteroaryl or heteroarylC₁₋₂alkyl, wherein the heteroaryl moiety is a 5- or 6-membered monocyclic aromatic ring which comprises 1, 2, or 3 heteroatoms individually selected from N, O and S, heterocyclyl or heterocyclylC₁₋₂alkyl, wherein the heterocyclyl moiety is a 4- to 6-membered non-aromatic ring which comprises 1 or 2 heteroatoms individually selected from N, O and S;
wherein when R⁸ is C₃₋₆cycloalkyl, C₃₋₆cycloalkylC₁₋₂alkyl, phenyl, phenylC₁₋₂alkyl, heteroaryl, heteroarylC₁₋₂alkyl, heterocyclyl, or heterocyclylC₁₋₂alkyl, the C₃₋₆cycloalkyl, phenyl, heteroaryl, or heterocyclyl motif is optionally substituted by 1 or 2 substituents, which may be the same or different, selected from R⁹;
R⁹ represents cyano, fluoro, chloro, bromo, methyl, difluoromethyl, trifluoromethyl, methoxy, ethoxy, or difluoromethoxy; and wherein when R⁸ is substituted C₃₋₆cycloalkyl or heterocyclyl, these cycles may contain a carbonyl (C=O) or sulfonyl (S(O)₂) group.

6. A compound according to any one of claims 1 to 5, wherein Z represents R⁸, wherein R⁸ is C₁₋₄alkyl, C₂₋₄alkenyl, C₂₋₄alkynyl, cyanoC₁₋₂alkyl, C₁₋₃haloalkyl, C₁₋₄alkoxyC₂₋₄alkyl, C₁₋₂haloalkoxyC₂₋₄alkyl, Cₗ-₃alkylcarbonylCi-₃alkyl, C₁₋₃alkoxycarbonylC₁₋₃alkyl, or C₁₋₃alkylcarbonyloxyC₂₋₄alkyl; or
R⁸ is C₃₋₅cycloalkyl, C₃₋₅cycloalkylC₁₋₂alkyl, phenyl, phenylC₁₋₂alkyl, heteroaryl or heteroarylC₁₋₂alkyl, wherein the heteroaryl moiety is a 5- or 6-membered monocyclic aromatic ring which comprises 1, 2, or 3 heteroatoms individually selected from N, O, and S, heterocyclyl or heterocyclylC₁₋₂alkyl, wherein the heterocyclyl moiety is a 4- to 6-membered non-aromatic ring which comprises 1 or 2 heteroatoms individually selected from N, O, and S;
wherein when R⁸ is C₃₋₅cycloalkyl, C₃₋₅cycloalkylC₁₋₂alkyl, phenyl, phenylC₁₋₂alkyl, heteroaryl, heteroarylC₁₋₂alkyl, heterocyclyl, or heterocyclylC₁₋₂alkyl, the C₃₋₅cycloalkyl, phenyl, heteroaryl, or heterocyclyl motif is optionally substituted by 1 or 2 substituents, which may be the same or different, selected from R⁹;
R⁹ represents cyano, fluoro, chloro, bromo, methyl, difluoromethyl, trifluoromethyl or methoxy; and wherein when R⁸ is substituted C₃₋₅cycloalkyl or heterocyclyl, these cycles may contain a carbonyl (C=O) group.

7. A compound according to any one of claims 1 to 6, wherein Z represents R⁸, wherein R⁸ is C₁₋₄alkyl, C₃₋₄alkenyl, C₁₋₃haloalkyl, C₁₋₂alkoxyC₂₋₃alkyl or C₁₋₂alkoxycarbonylC₁₋₂alkyl; or
R⁸ is C₃₋₅cycloalkyl, (C₃₋₅cycloalkyl)methyl, phenyl, benzyl, heteroaryl, (heteroaryl)methyl, wherein the heteroaryl moiety is a 5-membered monocyclic aromatic ring which comprises 1 or 2 heteroatoms individually selected from N, O, and S, heterocyclyl or (heterocyclyl)methyl, wherein the heterocyclyl moiety is a 5- or 6-membered non-aromatic ring which comprises 1 or 2 heteroatoms individually selected from N and O;
wherein when R⁸ is C₃₋₅cycloalkyl, (C₃₋₅cycloalkyl)methyl, phenyl, benzyl, heteroaryl, (heteroaryl)methyl, heterocyclyl, or (heterocyclyl)methyl, the C₃₋₅cycloalkyl, phenyl, heteroaryl, or heterocyclyl motif is optionally substituted by 1 or 2 substituents, which may be the same or different, selected from R⁹;
R⁹ represents cyano, fluoro, chloro, bromo, methyl or methoxy.

8. A compound according to any one of claims 1 to 7, wherein Z represents R⁸, wherein R⁸ is methyl, ethyl, *n*-propyl, isopropyl, *n*-butyl, *t*-butyl, allyl, prop-1-enyl, isopropenyl, 1,1-difluoromethyl, 1,1,1-trifluoromethyl, 2,2-difluoroethyl, 2,2,2-trifluoroethyl, 3,3,3-trifluoropropyl, methoxymethyl, methoxyethyl, methoxycarbonylmethyl, methoxycarbonylethyl, ethoxycarbonylmethyl, ethoxycarbonylethyl, cyclopropyl, cyclobutyl, cyclopentyl, cyclopropylmethyl, cyclobutylmethyl, cyclopentylmethyl, 2,2-difluorocyclopropylmethyl, 2,2-dichlorocyclopropylmethyl, phenyl, benzyl, 2-fluorophenyl, 2,4-difluorophenyl, 2,4-dichlorophenyl, 4-chloro-2-fluorophenyl, 4-fluoro-2-chlorophenyl, pyrazol-3-yl, pyrazol-4-yl, 1-methylpyrazol-3-yl, 1-methylpyrazol-4-yl, thiophene-2-yl, thiophene-3-yl, 2-methylthiophene-5-yl, 2-methylthiophene-4-yl, tetrahydrofuran-2-yl, tetrahydrofuran-3-yl, tetrahydrofuran-2-yl methyl, tetrahydrofuran-3-yl methyl, morpholinyl, pyrrolidin-1-yl, pyrrolidin-2-yl, pyrrolidin-3-yl, piperidin-1-yl, piperidin-2-yl, piperidin-3-yl, or piperidin-4-yl.

9. A compound according to any one of claims 1 to 4, wherein Z represents -N(R¹⁰)R¹¹, wherein R¹⁰ represents hydrogen, C₁₋₆alkyl, C₁₋₆haloalkyl, cyanoC₁₋₄alkyl, hydroxyC₂₋₄alkyl, C₁₋₂alkoxyC₂₋₄alkyl, C₁₋₂haloalkoxyC₁₋₄alkyl, C₃₋₆alkenyl, C₃₋₆alkynyl, formyl, C₁₋₄alkylcarbonyl, C₁₋₄alkylcarbonylC₁₋₄alkyl, C₁₋₄alkoxycarbonylC₁₋₄alkyl, or C₁₋₄alkylcarbonyloxyC₁₋₄alkyl; or
R¹⁰ is C₃₋₆cycloalkyl, C₃₋₆cycloalkylC₁₋₂alkyl, phenyl, or phenylC₁₋₂alkyl;
wherein when R¹⁰ is C₃₋₆cycloalkyl, C₃₋₆cycloalkylC₁₋₂alkyl, phenyl, or phenylC₁₋₂alkyl, the C₃₋₆cycloalkyl or phenyl motif is optionally substituted by 1 or 2 substituents, which may be the same or different, selected from R¹²;
R¹² represents cyano, fluoro, chloro, bromo, methyl, difluoromethyl, trifluoromethyl, methoxy, ethoxy, or difluoromethoxy; and wherein when R¹⁰ is substituted C₃₋₆cycloalkyl or heterocyclyl, these cycles may contain a carbonyl (C=O) or sulfonyl (S(O)₂) group; and
R¹¹ represents hydrogen, hydroxy, C₁₋₄alkyl, C₁₋₂haloalkyl, C₃₋₄alkenyl, C₃₋₄alkynyl, C₃₋₄cycloalkyl, C₃₋₄cycloalkylC₁₋₂alkyl, C₁₋₄alkoxy, C₃₋₄alkenyloxy, or C₃₋₄alkynyloxy.

10. A compound according to any one of claims 1 to 4, or claim 9, wherein Z represents -N(R¹⁰)R¹¹, wherein R¹⁰ is hydrogen, C₁₋₄alkyl, C₁₋₄haloalkyl, cyanoC₁₋₂alkyl, hydroxyC₂₋₄alkyl, C₁₋₂alkoxyC₂₋₄alkyl, C₁₋₂haloalkoxyC₁₋₂alkyl, C₃₋₄alkenyl, C₃₋₄alkynyl, formyl, C₁₋₂alkylcarbonyl, C₁₋₂alkylcarbonylC₁₋₂alkyl, C₁₋₂alkoxycarbonylC₁₋₂alkyl, or C₁₋₂alkylcarbonyloxyC₁₋₂alkyl; or
R¹⁰ is C₃₋₆cycloalkyl or C₃-₆cycloalkylC₁₋₂alkyl;
wherein when R¹⁰ is C₃₋₆cycloalkyl or C₃₋₆cycloalkylC₁₋₂alkyl, the C₃₋₆cycloalkyl motif is optionally substituted by 1 or 2 substituents, which may be the same or different, selected from R¹²;
R¹² represents cyano, fluoro, chloro, bromo, methyl, or methoxy; and wherein when R¹⁰ is substituted C₃₋₆cycloalkyl or heterocyclyl, these cycles may contain a carbonyl (C=O) group; and
R¹¹ represents hydrogen, hydroxyl, C₁₋₄alkyl, C₁₋₂haloalkyl, C₃₋₄alkenyl, C₃₋₄alkynyl, C₃₋₄cycloalkyl, C₃₋₄cycloalkylC₁₋₂alkyl or C₁₋₄alkoxy.

11. A compound according to any one of claims 1 to 10, wherein n is 0 or 1.

12. An agrochemical composition comprising a fungicidally effective amount of a compound of Formula (I) according to any one of claims 1 to 11.

13. The composition according to claim 12, further comprising at least one additional active ingredient and/or an agrochemically-acceptable diluent or carrier.

14. A method of controlling or preventing infestation of useful plants by phytopathogenic microorganisms, wherein a fungicidally effective amount of a compound of Formula (I) according to any of claims 1 to 11, or a composition comprising this compound as active ingredient, is applied to the plants, to parts thereof or the locus thereof.

15. Use of a compound of Formula (I) according to any one of claims 1 to 11 as a fungicide, with the proviso that the use excludes methods for the treatment of the human or animal body by surgery or therapy.

## Patentansprüche

1. Verbindung der Formel (I): 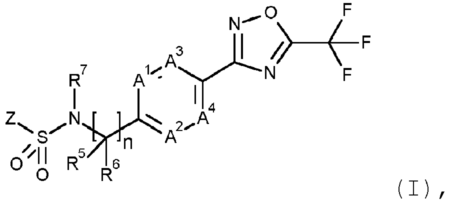 wobei
n für 0, 1 oder 2 steht,
A¹ für N oder CR¹ steht, wobei R¹ für Wasserstoff, Halogen oder Methyl steht;
A² für N oder CR² steht, wobei R² für Wasserstoff, Halogen oder Methyl steht;
A³ für N oder CR³ steht, wobei R³ für Wasserstoff oder Fluor steht;
A⁴ für N oder CR⁴ steht, wobei R⁴ für Wasserstoff oder Fluor steht;
wobei mindestens 2 der Reste A¹, A², A³ und A⁴ für C-H stehen;
R⁵ und R⁶ unabhängig für Wasserstoff, Methyl, Cyano, Difluormethyl oder Trifluormethyl stehen;
R⁷ für Hydroxy, C₁₋₄-Halogenalkyl, C₁₋₄-Alkoxy, Hydroxy-C₂₋₄-alkyl, C₁₋₂-Alkoxy-C₂₋₄-alkyl, C₁₋₂-Halogen-alkoxy, C₁₋₂-Halogenalkoxy-C₂₋₄-alkyl, C₃₋₄-Alkenyloxy, C₃₋₄-Alkinyloxy oder C₁₋₄-Alkylcarbonyloxy steht;
Z für R⁸ steht, wobei
R⁸ für C₁₋₆-Alkyl, C₂₋₆-Alkenyl, C₂₋₆-Alkinyl, Cyano-C₁₋₄-alkyl, C₁₋₄-Halogenalkyl, C₁₋₄-Alkoxy-C₂₋₄-alkyl, C₁₋₂-Halogenalkoxy-C₂₋₄-alkyl, C₁₋₄-Alkylcarbonyl-C₁₋₄-alkyl, C₁₋₄-Alkoxycarbonyl-C₁₋₄-alkyl, C₁₋₄-Alkylcarbonyloxy-C₂₋₄-alkyl, N-C₁₋₄-Alkylaminocarbonyl-C₁₋₄-alkyl, N,N-Di-C₁₋₄-alkylaminocarbonyl-C₁₋₄-alkyl, N-C₁₋₄-Alkylamino-C₂₋₅-alkyl, N,N-Di-C₁₋₄-alkylamino-C₂₋₅-alkyl, C₁₋₃-Alkyl-carbonylamino-C₂₋₅-alkyl oder C₁₋₄-Alkoxycarbonylamino-C₂₋₅-alkyl steht oder
R⁸ für C₃₋₆-Cycloalkyl, C₃₋₆-Cycloalkyl-C₁₋₂-alkyl, Phenyl, Phenyl-C₁₋₂-alkyl, Heteroaryl oder Heteroaryl-C₁₋₂-alkyl, wobei es sich bei der Heteroarylgruppierung um einen 5- oder 6-gliedrigen monocyclischen aromatischen Ring mit 1, 2, 3 oder 4 Heteroatomen, die individuell aus N, 0 und S ausgewählt sind, handelt, Heterocyclyl oder Heterocyclyl-C₁₋₂-alkyl, wobei es sich bei der Heterocyclylgruppierung um einen 4- bis 6-gliedrigen nichtaromatischen Ring mit 1 oder 2 Heteroatomen, die individuell aus N, 0 und S ausgewählt sind, handelt, steht;
wobei dann, wenn R⁸ für C₃₋₆-Cycloalkyl, C₃₋₆-Cycloalkyl-C₁₋₂-alkyl, Phenyl, Phenyl-C₁₋₂-alkyl, Heteroaryl, Heteroaryl-C₁₋₂-alkyl, Heterocyclyl oder Heterocyclyl-C₁₋₂-alkyl steht, die C₃₋₆-Cycloalkyl-, Phenyl-, Heteroaryl- oder Heterocyclyleinheit gegebenenfalls durch 1 oder 2 aus R⁹ ausgewählte Substituenten, die gleich oder verschieden sein können, substituiert ist;
R⁹ für Cyano, Fluor, Chlor, Brom, Methyl, Difluormethyl, Trifluormethyl, Methoxy, Ethoxy oder Difluormethoxy steht; und wobei dann, wenn R⁸ für substituiertes C₃₋₆-Cycloalkyl oder Heterocyclyl steht, diese Ringe eine Carbonylgruppe (C=0) oder Sulfonylgruppe (S(O)₂) enthalten können; oder
Z für -N(R¹⁰)R¹¹ steht, wobei
R¹⁰ für Wasserstoff, C₁₋₆-Alkyl, C₁₋₆-Halogenalkyl, Cyano-C₁₋₄-alkyl, Hydroxy-C₂₋₄-alkyl, C₁₋₂-Alkoxy-C₂₋₄-alkyl, C₁₋₂-Halogenalkoxy-C₁₋₄-alkyl, C₃₋₆-Alkenyl, C₃₋₆-Alkinyl, Amino-C₂₋₄-alkyl, N-C₁₋₄-Alkylamino-C₂₋₄-alkyl, N,N-Di-C₁₋₄-alkylamino-C₂₋₄-alkyl, Formyl, C₁₋₄-Alkylcarbonyl, C₁₋₄-Alkylcarbonyl-C₁₋₄-alkyl, C₁₋₄-Alkoxycarbonyl-C₁₋₄-alkyl, C₁₋₄-Alkylcarbonyloxy-C₁₋₄-alkyl, N-C₁₋₄-Alkylaminocarbonyl-C₁₋₄-alkyl, N,N-Di-C₁₋₄-alkyl-aminocarbonyl-C₁₋₄-alkyl, C₁₋₄-Alkoxycarbonylamino-C₂₋₄-alkyl, C₁₋₄-Alkylcarbonylamino-C₂₋₄-alkyl oder C₁₋₄-Halogenalkylcarbonylamino-C₂₋₄-alkyl steht oder
R¹⁰ für C₃₋₆-Cycloalkyl, C₃₋₆-Cycloalkyl-C₁₋₂-alkyl, Phenyl, Phenyl-C₁₋₂-alkyl, Heteroaryl oder Heteroaryl-C₁₋₂-alkyl, wobei es sich bei der Heteroarylgruppierung um einen 5- oder 6-gliedrigen monocyclischen aromatischen Ring mit 1, 2, 3 oder 4 Heteroatomen, die individuell aus N, 0 und S ausgewählt sind, handelt, Heterocyclyl oder Heterocyclyl-C₁₋₂-alkyl, wobei es sich bei der Heterocyclylgruppierung um einen 4- bis 6-gliedrigen nichtaromatischen Ring mit 1 oder 2 Heteroatomen, die individuell aus N, 0 und S ausgewählt sind, handelt, steht;
wobei dann, wenn R¹⁰ für C₃₋₆-Cycloalkyl, C₃₋₆-Cycloalkyl-C₁₋₂-alkyl, Phenyl-C₁₋₂-alkyl, Phenyl, Heteroaryl, Heteroaryl-C₁₋₂-alkyl, Heterocyclyl oder Heterocyclyl-C₁₋₂-alkyl steht, die C₃₋₆-Cycloalkyl-, Phenyl-, Heteroaryl- oder Heterocyclyleinheit gegebenenfalls durch 1 oder 2 aus R¹² ausgewählte Substituenten, die gleich oder verschieden sein können, substituiert ist;
R¹² für Cyano, Fluor, Chlor, Brom, Methyl, Difluormethyl, Trifluormethyl, Methoxy, Ethoxy oder Difluormethoxy steht; und wobei dann, wenn R¹⁰ für substituiertes C₃₋₆-Cycloalkyl oder Heterocyclyl steht, diese Ringe eine Carbonylgruppe (C=0) oder Sulfonylgruppe (S(O)₂) enthalten können;
R¹¹ für Wasserstoff, Hydroxyl, C₁₋₄-Alkyl, C₁₋₂-Halogenalkyl, C₃₋₄-Alkenyl, C₃₋₄-Alkinyl, C₃₋₄-Cycloalkyl, C₃₋₄-Cycloalkyl-C₁₋₂-alkyl, C₁₋₄-Alkoxy, C₃₋₄-Alkenyloxy oder C₃₋₄-Alkinyloxy steht; oder
ein Salz oder ein N-Oxid davon.

2. Verbindung nach Anspruch 1, wobei A¹ und A² jeweils unabhängig für N, C-H oder C-F stehen und A³ und A⁴ jeweils unabhängig für C-H oder C-F stehen, wobei mindestens 2 der Reste A¹, A², A³ und A⁴ für C-H stehen.

3. Verbindung nach Anspruch 1 oder Anspruch 2, wobei R⁷ für Methoxy steht.

4. Verbindung nach einem der Ansprüche 1 bis 3, wobei R⁵ und R⁶ unabhängig für Wasserstoff oder Methyl stehen.

5. Verbindung nach einem der Ansprüche 1 bis 4, wobei Z für R⁸ steht, wobei R⁸ für C₁₋₆-Alkyl, C₂₋₆-Alkenyl, C₂₋₆-Alkinyl, Cyano-C₁₋₄-alkyl, C₁₋₄-Halogenalkyl, C₁₋₄-Alkoxy-C₂₋₄-alkyl, C₁₋₂-Halogenalkoxy-C₂₋₄-alkyl, C₁₋₄-Alkylcarbonyl-C₁₋₄-alkyl, C₁₋₄-Alkoxycarbonyl-C₁₋₄-alkyl oder C₁₋₄-Alkylcarbonyloxy-C₂₋₄-alkyl steht oder
R⁸ für C₃₋₆-Cycloalkyl, C₃₋₆-Cycloalkyl-C₁₋₂-alkyl, Phenyl, Phenyl-C₁₋₂-alkyl, Heteroaryl oder Heteroaryl-C₁₋₂-alkyl, wobei es sich bei der Heteroarylgruppierung um einen 5- oder 6-gliedrigen monocyclischen aromatischen Ring mit 1, 2 oder 3 Heteroatomen, die individuell aus N, 0 und S ausgewählt sind, handelt, Heterocyclyl oder Heterocyclyl-C₁₋₂-alkyl, wobei es sich bei der Heterocyclylgruppierung um einen 4- bis 6-gliedrigen nichtaromatischen Ring mit 1 oder 2 Heteroatomen, die individuell aus N, 0 und S ausgewählt sind, handelt, steht;
wobei dann, wenn R⁸ für C₃₋₆-Cycloalkyl, C₃₋₆-Cycloalkyl-C₁₋₂-alkyl, Phenyl, Phenyl-C₁₋₂-alkyl, Heteroaryl, Heteroaryl-C₁₋₂-alkyl, Heterocyclyl oder Heterocyclyl-C₁₋₂-alkyl steht, die C₃₋₆-Cycloalkyl-, Phenyl-, Heteroaryl- oder Heterocyclyleinheit gegebenenfalls durch 1 oder 2 aus R⁹ ausgewählte Substituenten, die gleich oder verschieden sein können, substituiert ist;
R⁹ für Cyano, Fluor, Chlor, Brom, Methyl, Difluormethyl, Trifluormethyl, Methoxy, Ethoxy oder Difluormethoxy steht; und wobei dann, wenn R⁸ für substituiertes C₃₋₆-Cycloalkyl oder Heterocyclyl steht, diese Ringe eine Carbonylgruppe (C=0) oder Sulfonylgruppe (S(O)₂) enthalten können.

6. Verbindung nach einem der Ansprüche 1 bis 5, wobei Z für R⁸ steht, wobei R⁸ für C₁₋₄-Alkyl, C₂₋₄-Alkenyl, C₂₋₄-Alkinyl, Cyano-C₁₋₂-alkyl, C₁₋₃-Halogenalkyl, C₁₋₄-Alkoxy-C₂₋₄-alkyl, C₁₋₂-Halogenalkoxy-C₂₋₄-alkyl, C₁₋₃-Alkylcarbonyl-C₁₋₃-alkyl, C₁₋₃-Alkoxycarbonyl-C₁₋₃-alkyl oder C₁₋₃-Alkylcarbonyloxy-C₂₋₄-alkyl steht oder
R⁸ für C₃₋₅-Cycloalkyl, C₃₋₅-Cycloalkyl-C₁₋₂-alkyl, Phenyl, Phenyl-C₁₋₂-alkyl, Heteroaryl oder Heteroaryl-C₁₋₂-alkyl, wobei es sich bei der Heteroarylgruppierung um einen 5- oder 6-gliedrigen monocyclischen aromatischen Ring mit 1, 2 oder 3 Heteroatomen, die individuell aus N, 0 und S ausgewählt sind, handelt, Heterocyclyl oder Heterocyclyl-C₁₋₂-alkyl, wobei es sich bei der Heterocyclylgruppierung um einen 4- bis 6-gliedrigen nichtaromatischen Ring mit 1 oder 2 Heteroatomen, die individuell aus N, 0 und S ausgewählt sind, handelt, steht;
wobei dann, wenn R⁸ für C₃₋₅-Cycloalkyl, C₃₋₅-Cycloalkyl-C₁₋₂-alkyl, Phenyl, Phenyl-C₁₋₂-alkyl, Heteroaryl, Heteroaryl-C₁₋₂-alkyl, Heterocyclyl oder Heterocyclyl-C₁₋₂-alkyl steht, die C₃₋₅-Cycloalkyl-, Phenyl-, Heteroaryl- oder Heterocyclyleinheit gegebenenfalls durch 1 oder 2 aus R⁹ ausgewählte Substituenten, die gleich oder verschieden sein können, substituiert ist;
R⁹ für Cyano, Fluor, Chlor, Brom, Methyl, Difluormethyl, Trifluormethyl oder Methoxy steht; und wobei dann, wenn R⁸ für substituiertes C₃₋₅-Cycloalkyl oder Heterocyclyl steht, diese Ringe eine Carbonylgruppe (C=0) enthalten können.

7. Verbindung nach einem der Ansprüche 1 bis 6, wobei Z für R⁸ steht, wobei R⁸ für C₁₋₄-Alkyl, C₃₋₄-Alkenyl, C₁₋₃-Halogenalkyl, C₁₋₂-Alkoxy-C₂₋₃-alkyl oder C₁₋₂-Alkylcarbonyl-C₁₋₂-alkyl steht oder
R⁸ für C₃₋₅-Cycloalkyl, (C₃₋₅-Cycloalkyl) methyl, Phenyl, Benzyl, Heteroaryl, (Heteroaryl)methyl, wobei es sich bei der Heteroarylgruppierung um einen 5-gliedrigen monocyclischen aromatischen Ring mit 1 oder 2 Heteroatomen, die individuell aus N, 0 und S ausgewählt sind, handelt, Heterocyclyl oder (Heterocyclyl)methyl, wobei es sich bei der Heterocyclylgruppierung um einen 5- oder 6-gliedrigen nichtaromatischen Ring mit 1 oder 2 Heteroatomen, die individuell aus N und 0 ausgewählt sind, handelt, steht;
wobei dann, wenn R⁸ für C₃₋₅-Cycloalkyl, (C₃₋₅-Cycloalkyl)methyl, Phenyl, Benzyl, Heteroaryl, (Heteroaryl)methyl, Heterocyclyl oder (Heterocyclyl)methyl steht, die C₃₋₅-Cycloalkyl-, Phenyl-, Heteroaryl- oder Heterocyclyleinheit gegebenenfalls durch 1 oder 2 aus R⁹ ausgewählte Substituenten, die gleich oder verschieden sein können, substituiert ist;
R⁹ für Cyano, Fluor, Chlor, Brom, Methyl oder Methoxy steht.

8. Verbindung nach einem der Ansprüche 1 bis 7, wobei Z für R⁸ steht, wobei R⁸ für Methyl, Ethyl, n-Propyl, Isopropyl, n-Butyl, t-Butyl, Allyl, Prop-1-enyl, Isopropenyl, 1,1-Difluormethyl, 1,1,1-Trifluormethyl, 2,2-Difluorethyl, 2,2,2-Trifluorethyl, 3,3,3-Trifluorpropyl, Methoxymethyl, Methoxyethyl, Methoxycarbonylmethyl, Methoxycarbonylethyl, Ethoxycarbonylmethyl, Ethoxycarbonylethyl, Cyclopropyl, Cyclobutyl, Cyclopentyl, Cyclopropylmethyl, Cyclobutylmethyl, Cyclopentylmethyl, 2,2-Difluorcyclopropylmethyl, 2,2-Dichlorcyclopropylmethyl, Phenyl, Benzyl, 2-Fluorphenyl, 2,4-Difluorphenyl, 2,4-Dichlorphenyl, 4-Chlor-2-fluorphenyl, 4-Fluor-2-chlorphenyl, Pyrazol-3-yl, Pyrazol-4-yl, 1-Methylpyrazol-3-yl, 1-Methyl-pyrazol-4-yl, Thiophen-2-yl, Thiophen-3-yl, 2-Methylthiophen-5-yl, 2-Methylthiophen-4-yl, Tetrahydrofuran-2-yl, Tetrahydrofuran-3-yl, Tetrahydrofuran-2-yl-methyl, Tetrahydrofuran-3-ylmethyl, Morpholinyl, Pyrrolidin-1-yl, Pyrrolidin-2-yl, Pyrrolidin-3-yl, Piperidin-1-yl, Piperidin-2-yl, Piperidin-3-yl oder Piperidin-4-yl steht.

9. Verbindung nach einem der Ansprüche 1 bis 4, wobei Z für -N(R¹⁰)R¹¹ steht, wobei R¹⁰ für Wasserstoff, C₁₋₆-Alkyl, C₁₋₆-Halogenalkyl, Cyano-C₁₋₄-alkyl, Hydroxy-C₂₋₄-alkyl, C₁₋₂-Alkoxy-C₂₋₄-alkyl, C₁₋₂-Halogenalkoxy-C₁₋₄-alkyl, C₃₋₆-Alkenyl, C₃₋₆-Alkinyl, Formyl, C₁₋₄-Alkylcarbonyl, C₁₋₄-Alkylcarbonyl-C₁₋₄-alkyl, C₁₋₄-Alkoxycarbonyl-C₁₋₄-alkyl oder C₁₋₄-Alkylcarbonyloxy-C₁₋₄-alkyl steht oder
R¹⁰ für C₃₋₆-Cycloalkyl, C₃₋₆-Cycloalkyl-C₁₋₂-alkyl, Phenyl oder Phenyl-C₁₋₂-alkyl steht;
wobei dann, wenn R¹⁰ für C₃₋₆-Cycloalkyl, C₃₋₆-Cycloalkyl-C₁₋₂-alkyl, Phenyl oder Phenyl-C₁₋₂-alkyl steht, die C₃₋₆-Cycloalkyl- oder Phenyleinheit gegebenenfalls durch 1 oder 2 aus R¹² ausgewählte Substituenten, die gleich oder verschieden sein können, substituiert ist;
R¹² für Cyano, Fluor, Chlor, Brom, Methyl, Difluormethyl, Trifluormethyl, Methoxy, Ethoxy oder Difluormethoxy steht; und wobei dann, wenn R¹⁰ für substituiertes C₃₋₆-Cycloalkyl oder Heterocyclyl steht, diese Ringe eine Carbonylgruppe (C=0) oder Sulfonylgruppe (S(O)₂) enthalten können; und
R¹¹ für Wasserstoff, Hydroxy, C₁₋₄-Alkyl, C₁₋₂-Halogenalkyl, C₃₋₄-Alkenyl, C₃₋₄-Alkinyl, C₃₋₄-Cycloalkyl, C₃₋₄-Cycloalkyl-C₁₋₂-alkyl, C₁₋₄-Alkoxy, C₃₋₄-Alkenyloxy oder C₃₋₄-Alkinyloxy steht.

10. Verbindung nach einem der Ansprüche 1 bis 4 oder Anspruch 9, wobei Z für -N(R¹⁰)R¹¹ steht, wobei R¹⁰ für Wasserstoff, C₁₋₄-Alkyl, C₁₋₄-Halogenalkyl, Cyano-C₁₋₂-alkyl, Hydroxy-C₂₋₄-alkyl, C₁₋₂-Alkoxy-C₂₋₄-alkyl, C₁₋₂-Halogenalkoxy-C₂₋₄-alkyl, C₃₋₄-Alkenyl, C₃₋₄-Alkinyl, Formyl, C₁₋₂-Alkylcarbonyl, C₁₋₂-Alkylcarbonyl-C₁₋₂-alkyl, C₁₋₂-Alkoxycarbonyl-C₁₋₂-alkyl oder C₁₋₂-Alkylcarbonyloxy-C₁₋₂-alkyl steht oder
R¹⁰ für C₃₋₆-Cycloalkyl oder C₃₋₆-Cycloalkyl-C₁₋₂-alkyl steht;
wobei dann, wenn R¹⁰ für C₃₋₆-Cycloalkyl oder C₃₋₆-Cycloalkyl-C₁₋₂-alkyl steht, die C₃₋₆-Cycloalkyleinheit gegebenenfalls durch 1 oder 2 aus R¹² ausgewählte Substituenten, die gleich oder verschieden sein können, substituiert ist;
R¹² für Cyano, Fluor, Chlor, Brom, Methyl oder Methoxy steht; und wobei dann, wenn R¹⁰ für substituiertes C₃₋₆-Cycloalkyl oder Heterocyclyl steht, diese Ringe eine Carbonylgruppe (C=0) enthalten können; und
R¹¹ für Wasserstoff, Hydroxyl, C₁₋₄-Alkyl, C₁₋₂-Halogenalkyl, C₃₋₄-Alkenyl, C₃₋₄-Alkinyl, C₃₋₄-Cycloalkyl, C₃₋₄-Cycloalkyl-C₁₋₂-alkyl oder C₁₋₄-Alkoxy steht.

11. Verbindung nach einem der Ansprüche 1 bis 10, wobei n für 0 oder 1 steht.

12. Agrochemische Zusammensetzung, umfassend eine fungizid wirksame Menge einer Verbindung der Formel (I) nach einem der Ansprüche 1 bis 11.

13. Zusammensetzung nach Anspruch 12, weiterhin umfassend mindestens einen zusätzlichen Wirkstoff und/oder ein agrochemisch unbedenkliches Verdünnungsmittel oder einen agrochemisch unbedenklichen Träger.

14. Verfahren zur Bekämpfung oder Prävention des Befalls von Nutzpflanzen durch phytopathogene Mikroorganismen, bei dem man eine fungizid wirksame Menge einer Verbindung der Formel (I) nach einem der Ansprüche 1 bis 11 oder eine Zusammensetzung, die diese Verbindung als Wirkstoff umfasst, auf Pflanzen, Teile davon oder deren Standort ausbringt.

15. Verwendung einer Verbindung der Formel (I) nach einem der Ansprüche 1 bis 11 als Fungizid, mit der Maßgabe, dass diese Verwendung Verfahren zur chirurgischen oder therapeutischen Behandlung des menschlichen oder tierischen Körpers ausschließt.

## Revendications

1. Composé de formule (I) : 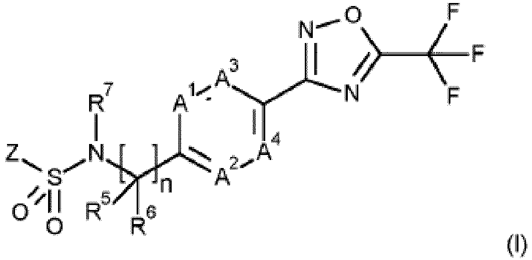
n étant 0, 1 ou 2 ;
A¹ représentant N ou CR¹, R¹ représentant hydrogène, halogène, ou méthyle ;
A² représentant N ou CR², R² représentant hydrogène, halogène, ou méthyle ;
A³ représentant N ou CR³, R³ représentant hydrogène ou fluoro ;
A⁴ représentant N ou CR⁴, R⁴ représentant hydrogène ou fluoro ; et
au moins 2 parmi A¹, A², A³ et A⁴ étant C-H ;
R⁵ et R⁶ représentant indépendamment hydrogène, méthyle, cyano, difluorométhyle, ou trifluorométhyle ;
R⁷ représentant hydroxy, C₁₋₄halogénoalkyle, C₁₋₄alcoxy, hydroxyC₂₋₄alkyle, C₁₋₂alcoxyC₂₋₄alkyle, C₁₋₂halogénoalcoxy, C₁₋₂halogénoalcoxyC₂₋₄alkyle, C₃₋₄alcényloxy, C₃₋₄alcynyloxy ou C₁₋₄alkylcarbonyloxy ; Z représentant R⁸,
R⁸ étant C₁₋₆alkyle, C₂₋₆alcényle, C₂₋₆alcynyle, cyanoC₁₋₄alkyle, C₁₋₄halogénoalkyle, C₁₋₄alcoxyC₂₋₄alkyle, C₁₋₂halogénoalcoxyC₂₋₄alkyle, C₁₋₄alkylcarbonylC₁₋₄alkyle, C₁₋₄alcoxycarbonylC₁₋₄alkyle, C₁₋₄alkylcarbonyloxyC₂₋₄alkyle, N-C₁₋₄alkylaminocarbonylC₁₋₄alkyle, N,N-diC₁₋₄alkylaminocarbonylC₁₋₄alkyle, N-C₁₋₄alkylaminoC₂₋₅alkyle, N,N-diC₁₋₄alkylaminoC₂₋₅alkyle, C₁₋₃alkylcarbonylaminoC₂₋₅alkyle, ou C₁₋₄alcoxycarbonylaminoC₂₋₅alkyle ; ou
R⁸ étant C₃₋₆cycloalkyle, C₃₋₆cycloalkylC₁₋₂alkyle, phényle, phénylC₁₋₂alkyle, hétéroaryle ou hétéroarylC₁₋₂alkyle, le groupement hétéroaryle étant un cycle aromatique monocyclique à 5 ou 6 chaînons qui comprend 1, 2, 3 ou 4 hétéroatomes individuellement choisis parmi N, O et S, hétérocyclyle ou hétérocyclylC₁₋₂alkyle, le groupement hétérocyclyle étant un cycle non aromatique à 4 à 6 chaînons qui comprend 1 ou 2 hétéroatomes individuellement choisis parmi N, O et S ;
lorsque R⁸ est C₃₋₆cycloalkyle, C₃₋₆cycloalkylC₁₋₂alkyle, phényle, phénylC₁₋₂alkyle, hétéroaryle, hétéroarylC₁₋₂alkyle, hétérocyclyle, ou hétérocyclylC₁₋₂alkyle, le motif C₃₋₆cycloalkyle, phényle, hétéroaryle, ou hétérocyclyle est éventuellement substitué par 1 ou 2 substituants, qui peuvent être identiques ou différents, choisis parmi R⁹ ;
R⁹ représentant cyano, fluoro, chloro, bromo, méthyle, difluorométhyle, trifluorométhyle, méthoxy, éthoxy, ou difluorométhoxy ; et lorsque R⁸ est C₃₋₆cycloalkyle ou hétérocyclyle substitué, ces cycles peuvent contenir un groupe carbonyle (C=O) ou sulfonyle (S(O)₂) ; ou
Z représentant -N(R¹⁰)R¹¹,
R¹⁰ représentant hydrogène, C₁₋₆alkyle, C₁₋₆halogénoalkyle, cyanoC₁₋₄alkyle, hydroxyC₂₋₄alkyle, C₁₋₂alcoxyC₂₋₄alkyle, C₁₋₂halogénoalcoxyC₁₋₄alkyle, C₃₋₆alcényle, C₃₋₆alcynyle, aminoC₂₋₄alkyle, N-C₁₋₄alkylaminoC₂₋₄alkyle, N,N-diC₁₋₄alkylaminoC₂₋₄alkyle, formyle, C₁₋₄alkylcarbonyle, C₁₋₄alkylcarbonylC₁₋₄alkyle, C₁₋₄alcoxycarbonylC₁₋₄alkyle, C₁₋₄alkylcarbonyloxyC₁₋₄alkyle, N-C₁₋₄alkylaminocarbonylC₁₋₄alkyle, N,N-diC₁₋₄alkylaminocarbonylC₁₋₄alkyle, C₁₋₄alcoxycarbonylaminoC₂₋₄alkyle, C₁₋₄alkylcarbonylaminoC₂₋₄alkyle, ou C₁₋₄halogénoalkylcarbonylaminoC₂₋₄alkyle ; ou
R¹⁰ étant C₃₋₆cycloalkyle, C₃₋₆cycloalkylC₁₋₂alkyle, phényle, phénylC₁₋₂alkyle, hétéroaryle ou hétéroarylC₁₋₂alkyle, le groupement hétéroaryle étant un cycle aromatique monocyclique à 5 ou 6 chaînons qui comprend 1, 2, 3 ou 4 hétéroatomes individuellement choisis parmi N, O et S, hétérocyclyle ou hétérocyclylC₁₋₂alkyle, le groupement hétérocyclyle étant un cycle non aromatique à 4 à 6 chaînons qui comprend 1 ou 2 hétéroatomes individuellement choisis parmi N, O et S ;
lorsque R¹⁰ est C₃₋₆cycloalkyle, C₃₋₆cycloalkylC₁₋₂alkyle, phénylC₁₋₂alkyle, phényle, hétéroaryle, hétéroarylC₁₋₂alkyle, hétérocyclyle, ou hétérocyclylC₁₋₂alkyle, le motif C₃₋₆cycloalkyle, phényle, hétéroaryle, ou hétérocyclyle est éventuellement substitué par 1 ou 2 substituants, qui peuvent être identiques ou différents, choisis parmi R¹² ;
R¹² représentant cyano, fluoro, chloro, bromo, méthyle, difluorométhyle, trifluorométhyle, méthoxy, éthoxy, ou difluorométhoxy ; et lorsque R¹⁰ est C₃₋₆cycloalkyle ou hétérocyclyle substitué, ces cycles peuvent contenir un groupe carbonyle (C=O) ou sulfonyle (S(O)₂) ;
R¹¹ représentant hydrogène, hydroxyle, C₁₋₄alkyle, C₁₋₂halogénoalkyle, C₃₋₄alcényle, C₃₋₄alcynyle, C₃₋₄cycloalkyle, C₃₋₄cycloalkylC₁₋₂alkyle, C₁₋₄alcoxy, C₃₋₄alcényloxy, ou C₃₋₄alcynyloxy ; ou
sel ou N-oxyde correspondant.

2. Composé selon la revendication 1, A¹ et A² représentant chacun indépendamment N, C-H ou C-F, et A³ et A⁴ représentant chacun indépendamment C-H ou C-F, au moins 2 parmi A¹, A², A³ et A⁴ étant C-H.

3. Composé selon la revendication 1 ou la revendication 2, R⁷ étant méthoxy.

4. Composé selon l'une quelconque des revendications 1 à 3, R⁵ et R⁶ représentant indépendamment hydrogène ou méthyle.

5. Composé selon l'une quelconque des revendications 1 à 4, Z représentant R⁸, R⁸ étant C₁₋₆alkyle, C₂₋₆alcényle, C₂₋₆alcynyle, cyanoC₁₋₄alkyle, C₁₋₄halogénoalkyle, C₁₋₄alcoxyC₂₋₄alkyle, C₁₋₂halogénoalcoxyC₂₋₄alkyle, C₁₋₄alkylcarbonylC₁₋₄alkyle, C₁₋₄alcoxycarbonylC₁₋₄alkyle, ou C₁₋₄alkylcarbonyloxyC₂₋₄alkyle ; ou
R⁸ étant C₃₋₆cycloalkyle, C₃₋₆cycloalkylC₁₋₂alkyle, phényle, phénylC₁₋₂alkyle, hétéroaryle ou hétéroarylC₁₋₂alkyle, le groupement hétéroaryle étant un cycle aromatique monocyclique à 5 ou 6 chaînons qui comprend 1, 2, ou 3 hétéroatomes individuellement choisis parmi N, O et S, hétérocyclyle ou hétérocyclylC₁₋₂alkyle, le groupement hétérocyclyle étant un cycle non aromatique à 4 à 6 chaînons qui comprend 1 ou 2 hétéroatomes individuellement choisis parmi N, O et S ;
lorsque R⁸ est C₃₋₆cycloalkyle, C₃₋₆cycloalkylC₁₋₂alkyle, phényle, phénylC₁₋₂alkyle, hétéroaryle, hétéroarylC₁₋₂alkyle, hétérocyclyle, ou hétérocyclylC₁₋₂alkyle, le motif C₃₋₆cycloalkyle, phényle, hétéroaryle, ou hétérocyclyle est éventuellement substitué par 1 ou 2 substituants, qui peuvent être identiques ou différents, choisis parmi R⁹ ;
R⁹ représentant cyano, fluoro, chloro, bromo, méthyle, difluorométhyle, trifluorométhyle, méthoxy, éthoxy, ou difluorométhoxy ; et lorsque R⁸ est C₃₋₆cycloalkyle ou hétérocyclyle substitué, ces cycles peuvent contenir un groupe carbonyle (C=O) ou sulfonyle (S(O)₂).

6. Composé selon l'une quelconque des revendications 1 à 5, Z représentant R⁸, R⁸ étant C₁₋₄alkyle, C₂₋₄alcényle, C₂₋₄alcynyle, cyanoC₁₋₂alkyle, C₁₋₃halogénoalkyle, C₁₋₄alcoxyC₂₋₄alkyle, C₁₋₂halogénoalcoxyC₂₋₄alkyle, C₁₋₃alkylcarbonylC₁₋₃alkyle, C₁₋₃alcoxycarbonylC₁₋₃alkyle, ou C₁₋₃alkylcarbonyloxyC₂₋₄alkyle ; ou
R⁸ étant C₃₋₅cycloalkyle, C₃₋₅cycloalkylC₁₋₂alkyle, phényle, phénylC₁₋₂alkyle, hétéroaryle ou hétéroarylC₁₋₂alkyle, le groupement hétéroaryle étant un cycle aromatique monocyclique à 5 ou 6 chaînons qui comprend 1, 2, ou 3 hétéroatomes individuellement choisis parmi N, O, et S, hétérocyclyle ou hétérocyclylC₁₋₂alkyle, le groupement hétérocyclyle étant un cycle non aromatique à 4 à 6 chaînons qui comprend 1 ou 2 hétéroatomes individuellement choisis parmi N, O, et S ;
lorsque R⁸ est C₃₋₅cycloalkyle, C₃₋₅cycloalkylC₁₋₂alkyle, phényle, phénylC₁₋₂alkyle, hétéroaryle, hétéroarylC₁₋₂alkyle, hétérocyclyle, ou hétérocyclylC₁₋₂alkyle, le motif C₃₋₅cycloalkyle, phényle, hétéroaryle, ou hétérocyclyle est éventuellement substitué par 1 ou 2 substituants, qui peuvent être identiques ou différents, choisis parmi R⁹ ;
R⁹ représentant cyano, fluoro, chloro, bromo, méthyle, difluorométhyle, trifluorométhyle ou méthoxy ; et lorsque R⁸ est C₃₋₅cycloalkyle ou hétérocyclyle substitué, ces cycles peuvent contenir un groupe carbonyle (C=O).

7. Composé selon l'une quelconque des revendications 1 à 6, Z représentant R⁸, R⁸ étant C₁₋₄alkyle, C₃₋₄alcényle, C₁₋₃halogénoalkyle, C₁₋₂alcoxyC₂₋₃alkyle ou C₁₋₂alcoxycarbonylC₁₋₂alkyle ; ou
R⁸ étant C₃₋₅cycloalkyle, (C₃₋₅cycloalkyl)méthyle, phényle, benzyle, hétéroaryle, (hétéroaryl)méthyle, le groupement hétéroaryle étant un cycle aromatique monocyclique à 5 chaînons qui comprend 1 ou 2 hétéroatomes individuellement choisis parmi N, O, et S, hétérocyclyle ou (hétérocyclyl)méthyle, le groupement hétérocyclyle étant un cycle non aromatique à 5 ou 6 chaînons qui comprend 1 ou 2 hétéroatomes individuellement choisis parmi N et O ;
lorsque R⁸ est C₃₋₅cycloalkyle, (C₃₋₅cycloalkyl)méthyle, phényle, benzyle, hétéroaryle, (hétéroaryl)méthyle, hétérocyclyle, ou (hétérocyclyl)méthyle, le motif C₃₋₅cycloalkyle, phényle, hétéroaryle, ou hétérocyclyle est éventuellement substitué par 1 ou 2 substituants, qui peuvent être identiques ou différents, choisis parmi R⁹ ;
R⁹ représentant cyano, fluoro, chloro, bromo, méthyle ou méthoxy.

8. Composé selon l'une quelconque des revendications 1 à 7, Z représentant R⁸, R⁸ étant méthyle, éthyle, n-propyle, isopropyle, n-butyle, t-butyle, allyle, prop-1-ényle, isopropényle, 1,1-difluorométhyle, 1,1,1-trifluorométhyle, 2,2-difluoroéthyle, 2,2,2-trifluoroéthyle, 3,3,3-trifluoropropyle, méthoxyméthyle, méthoxyéthyle, méthoxycarbonylméthyle, méthoxycarbonyléthyle, éthoxycarbonylméthyle, éthoxycarbonyléthyle, cyclopropyle, cyclobutyle, cyclopentyle, cyclopropylméthyle, cyclobutylméthyle, cyclopentylméthyle, 2,2-difluorocyclopropylméthyle, 2,2-dichlorocyclopropylméthyle, phényle, benzyle, 2-fluorophényle, 2,4-difluorophényle, 2,4-dichlorophényle, 4-chloro-2-fluorophényle, 4-fluoro-2-chlorophényle, pyrazol-3-yle, pyrazol-4-yle, 1-méthylpyrazol-3-yle, 1-méthylpyrazol-4-yle, thiophén-2-yle, thiophén-3-yle, 2-méthylthiophén-5-yle, 2-méthylthiophén-4-yle, tétrahydrofuran-2-yle, tétrahydrofuran-3-yle, tétrahydrofuran-2-yl-méthyle, tétrahydrofuran-3-yl-méthyle, morpholinyle, pyrrolidin-1-yle, pyrrolidin-2-yle, pyrrolidin-3-yle, pipéridin-1-yle, pipéridin-2-yle, pipéridin-3-yle, ou pipéridin-4-yle.

9. Composé selon l'une quelconque des revendications 1 à 4, Z représentant -N(R¹⁰)R¹¹, R¹⁰ représentant hydrogène, C₁₋₆alkyle, C₁₋₆halogénoalkyle, cyanoC₁₋₄alkyle, hydroxyC₂₋₄alkyle, C₁₋₂alcoxyC₂₋₄alkyle, C₁₋₂halogénoalcoxyC₁₋₄alkyle, C₃₋₆alcényle, C₃₋₆alcynyle, formyle, C₁₋₄alkylcarbonyle, C₁₋₄alkylcarbonylC₁₋₄alkyle, C₁₋₄alcoxycarbonylC₁₋₄alkyle, ou C₁₋₄alkylcarbonyloxyC₁₋₄alkyle ; ou
R¹⁰ étant C₃₋₆cycloalkyle, C₃₋₆cycloalkylC₁₋₂alkyle, phényle, ou phénylC₁₋₂alkyle ;
lorsque R¹⁰ est C₃₋₆cycloalkyle, C₃₋₆cycloalkylC₁₋₂alkyle, phényle, ou phénylC₁₋₂alkyle, le motif C₃₋₆cycloalkyle ou phényle est éventuellement substitué par 1 ou 2 substituants, qui peuvent être identiques ou différents, choisis parmi R¹² ; R¹² représentant cyano, fluoro, chloro, bromo, méthyle, difluorométhyle, trifluorométhyle, méthoxy, éthoxy, ou difluorométhoxy ; et lorsque R¹⁰ est C₃₋₆cycloalkyle ou hétérocyclyle substitué, ces cycles peuvent contenir un groupe carbonyle (C=O) ou sulfonyle (S(O)₂) ; et
R¹¹ représentant hydrogène, hydroxy, C₁₋₄alkyle, C₁₋₂halogénoalkyle, C₃₋₄alcényle, C₃₋₄alcynyle, C₃₋₄cycloalkyle, C₃₋₄cycloalkylC₁₋₂alkyle, C₁₋₄alcoxy, C₃₋₄alcényloxy, ou C₃₋₄alcynyloxy.

10. Composé selon l'une quelconque des revendications 1 à 4, ou la revendication 9, Z représentant - N(R¹⁰)R¹¹, R¹⁰ étant hydrogène, C₁₋₄alkyle, C₁₋₄halogénoalkyle, cyanoC₁₋₂alkyle, hydroxyC₂₋₄alkyle, C₁₋₂alcoxyC₂₋₄alkyle, C₁₋₂halogénoalcoxyC₁₋₂alkyle, C₃₋₄alcényle, C₃₋₄alcynyle, formyle, C₁₋₂alkylcarbonyle, C₁₋₂alkylcarbonylC₁₋₂alkyle, C₁₋₂alcoxycarbonylC₁₋₂alkyle, ou C₁₋₂alkylcarbonyloxyC₁₋₂alkyle ; ou
R¹⁰ étant C₃₋₆cycloalkyle ou C₃₋₆cycloalkylC₁₋₂alkyle ;
lorsque R¹⁰ est C₃₋₆cycloalkyle ou C₃₋₆cycloalkylC₁₋₂alkyle, le motif C₃₋₆cycloalkyle est éventuellement substitué par 1 ou 2 substituants, qui peuvent être identiques ou différents, choisis parmi R¹² ; R¹² représentant cyano, fluoro, chloro, bromo, méthyle, ou méthoxy ; et lorsque R¹⁰ est C₃₋₆cycloalkyle ou hétérocyclyle substitué, ces cycles peuvent contenir un groupe carbonyle (C=O) ; et
R¹¹ représentant hydrogène, hydroxyle, C₁₋₄alkyle, C₁₋₂halogénoalkyle, C₃₋₄alcényle, C₃₋₄alcynyle, C₃₋₄cycloalkyle, C₃₋₄cycloalkylC₁₋₂alkyle ou C₁₋₄alcoxy.

11. Composé selon l'une quelconque des revendications 1 à 10, n étant 0 ou 1.

12. Composition agrochimique comprenant une quantité efficace sur le plan fongicide d'un composé de Formule (I) selon l'une quelconque des revendications 1 à 11.

13. Composition selon la revendication 12, comprenant en outre au moins un ingrédient actif supplémentaire et/ou un diluant ou support acceptable sur le plan agrochimique.

14. Procédé de régulation ou de prévention d'une infestation de végétaux utiles par des micro-organismes phytopathogènes, une quantité efficace sur le plan fongicide d'un composé de Formule (I) selon l'une quelconque des revendications 1 à 11, ou d'une composition comprenant ce composé en tant qu'ingrédient actif, étant appliquée aux végétaux, à des parties de ceux-ci ou au site de ceux-ci.

15. Utilisation d'un composé de Formule (I) selon l'une quelconque des revendications 1 à 11 en tant que fongicide, à la condition que l'utilisation exclut des procédés pour le traitement du corps humain ou animal par chirurgie ou thérapie.
